# EUROPEAN PATENT APPLICATION

(11) **EP 3 029 031 A1**
(43) Date of publication of application: **08.06.2016**
(21) Application number: 14832018.7
(22) Date of filing: 29.07.2014
(51) Int. Cl.: C07D 401/04, A61K 31/4439, A61K 31/4545, A61K 31/4709, A61K 31/4725, A61K 31/496, A61K 31/497, A61K 31/501, A61K 31/506, A61K 31/5377, A61K 31/541, A61P 1/04, A61P 17/06, A61P 19/02, A61P 25/00, A61P 27/02, A61P 29/00, A61P 37/02, A61P 43/00, C07D 401/14, C07D 405/14

(54) **HETEROCYCLIC COMPOUND**

(30) Priority: 30.07.2013 JP 2013158306; 27.09.2013 JP 2013202738
(71) Applicant: Takeda Pharmaceutical Company Limited, Osaka-shi, Osaka 541-0045 (JP)
(72) Inventor: SAITOH, Morihisa, Fujisawa-shi Kanagawa 251-0012 (JP); YOGO, Takatoshi, Fujisawa-shi Kanagawa 251-0012 (JP); KAMEI, Taku, Fujisawa-shi Kanagawa 251-0012 (JP); TOKUNAGA, Norihito, Fujisawa-shi Kanagawa 251-0012 (JP); OHBA, Yusuke, Takatsuki-shi Osaka 569-1115 (JP); YUKAWA, Takafumi, Fujisawa-shi Kanagawa 251-0012 (JP)
(74) Representative: Held, Stephan
(86) International application number: PCT/JP2014/069907
(87) International publication number: WO 2015/016206

(57) **Abstract**

The present invention provides an agent for the prophylaxis or treatment of autoimmune diseases (e.g., psoriasis, rheumatoid arthritis, inflammatory bowel disease, Sjogren's syndrome, Behcet's disease, multiple sclerosis, systemic lupus erythematosus etc.) and the like, which has a superior Tyk2 inhibitory action.

The present invention relates to a compound represented by the formula 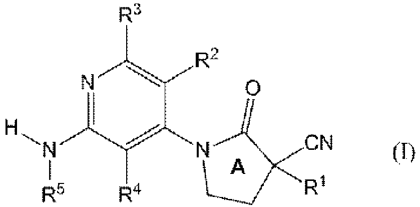 wherein each symbol is as defined in the specification, or a salt thereof.

## Description

### Technical Field

The present invention relates to a heterocyclic compound having a tyrosine kinase 2 (In the present specification, sometimes to be abbreviated as "Tyk2") inhibitory action, which is useful as an agent for the prophylaxis or treatment of autoimmune diseases (e.g., psoriasis, rheumatoid arthritis, inflammatory bowel disease, Sjogren's syndrome, Behcet's disease, multiple sclerosis, systemic lupus erythematosus etc.) and the like, a pharmaceutical composition containing thereof, and the like.

### (Background of the Invention)

Cytokines are proteins secreted by a cell of the immune system and transduce a signal to a specific cell. Cytokines have various kinds, and many of them are especially associated with immunity and inflammation and also associated with cell growth, differentiation, cell death, wound healing and the like (Curr Opin Cell Biol. 1991 Apr;3(2):171-5.).

The janus kinase (JAK) family plays a role in cytokine-dependent regulation of the function of cells associated with growth and immune response. Tyk2 is one of the four kinds of janus kinases (JAK1 (janus kinase 1), JAK2 (janus kinase 2), JAK3 (janus kinase 3) and Tyk2 (tyrosine kinase 2)), and it is known to be involved in signal transduction of cytokines such as IFN(interferon)-α, IFN-β, IL(interleukin)-6, IL-10 family (IL-10, IL-19, IL-20, IL-22, IL-28, IL-29), IL-12, IL-23 and the like (Nature Immunology 10, 356 - 360 (2009), New York Academy of Science 1246, 34-40 (2011)). These cytokines play an important role in immune response when exist in an appropriate amount. However, excessive production of them is involved in many autoimmune diseases such as psoriasis, rheumatoid arthritis, inflammatory bowel disease, Sjogren's syndrome, Behcet's disease, multiple sclerosis, systemic lupus erythematosus and the like (Journal of Allergy and Clinical Immunology 127, 3,701-721.e70 (2011), Cytokine & Growth Factor Reviews 19, 41-52 (2008), Invest Ophthalmol Vis Sci. 2008 Jul; 49(7):3058-3064, Ann Rheum Dis. 2010 Jul;69(7):1325-1328). For example, Ustekinumab, which is an anti-IL-12/23 monoclonal antibody, has been approved as a therapeutic drug for moderate to severe psoriasis patient in Europe, and furthermore, clinical trials for various diseases in which the IL-12/23 signaling pathway is suggested to be involved are performed. From the foregoing, a Tyk2 inhibitor is a therapeutic drug for various autoimmune diseases such as psoriasis, rheumatoid arthritis, inflammatory bowel disease, Sjogren's syndrome, Behcet's disease, multiple sclerosis, systemic lupus erythematosus and the like (Front Biosci. 2011 Jun 1;17:3214-32).

Examples of the compound having a structure similar to the compound described in the present specification include the following compounds.
(1) A compound represented by the following formula: 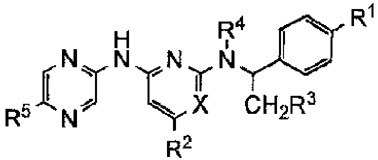 wherein
   (I)
      X is CH or N;
      R¹ is halogen;
      R² is H, halogen, cyano, an optionally substituted nitrogen-containing non-aromatic heterocyclic group or the like;
      R³ is H or alkyl;
      R⁴ is H or alkyl; and
      R⁵ is H or alkyl; or
   (II)
      X is -CR^{A};
      R^{A} is a group represented by the following formula: 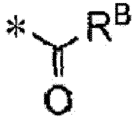 wherein
         * means a binding position; and
         R^{B} is an optionally substituted amino or the like;
      R¹ is halogen;
      R² is H;
      R³ is H or hydroxy;
      R⁴ is H or alkyl; and
      R⁵ is H or alkyl,
      which is a JAK2 inhibitor, and is useful for the treatment of inflammatory disease and the like (Patent Document 1).
(2) A compound represented by the following formula: 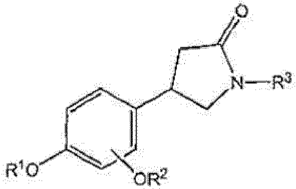 wherein
   R¹ is optionally substituted C₁₋₄ alkyl or the like;
   R² is optionally substituted phenyl or the like; and
   R³ is an optionally substituted aromatic heterocyclic group or the like, which is a HIV transcription inhibitor, and is useful for the treatment of HIV and the like (Patent Document 2).
(3) A compound represented by the following formula: 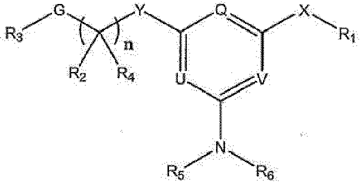 wherein
   R¹ is

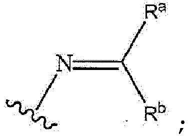

   R², R³, R⁴, R^{a} and R^{b} are independently a hydrogen atom, an optionally substituted alkyl group or the like;
   R⁵ and R⁶ are independently a nitrogen-containing heterocycle or the like;
   X is O, S, N(R^{k}) or the like;
   Y is O, S, (CHR^{g})ₘ, N(R^{k}) or the like;
   Q, U and V are independently N or CR^{g} or the like;
   R^{k} is a hydrogen atom, an optionally substituted alkyl group or the like;
   R^{g} is a hydrogen atom, an optionally substituted alkyl group or the like; and
   M is 0, 1, 2, 3 or 4,
   which is a c-Rel inhibitor, and is useful as an agent for the prophylaxis or treatment of inflammatory disease (rheumatoid arthritis, psoriatic arthropathy, systemic lupus erythematosus and the like) and the like (Patent Documents 3 and 4).

### Document List

### Patent Document

[Patent Document 1] WO 2010/090290
[Patent Document 2] WO 2004/037784
[Patent Document 3] WO 2006/128172
[Patent Document 4] WO 2006/128129

### Summary of the Invention

### Problems to be Solved by the Invention

An object of the present invention is to provide an agent for the prophylaxis or treatment of autoimmune diseases (e.g., psoriasis, rheumatoid arthritis, inflammatory bowel disease, Sjogren's syndrome, Behcet's disease, multiple sclerosis, systemic lupus erythematosus etc.) and the like, which has a superior Tyk2 inhibitory action.

### Means of Solving the Problems

The present inventors have conducted intensive studies in an attempt to solve the above-mentioned problem and found that a compound represented by the following formula (I) has a superior Tyk2 inhibitory action, which resulted in the completion of the present invention.

Accordingly, the present invention provides the following.
[1] A compound represented by the formula (I): 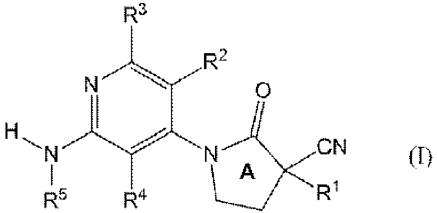 wherein
   Ring A is an optionally further substituted pyrrolidine ring;
   R¹, R² and R⁴ are independently a hydrogen atom or a substituent;
   R³ is a hydrogen atom, a halogen atom, an optionally halogenated C₁₋₆ alkyl group, a mono- or di-(optionally halogenated C₁₋₆ alkyl)amino group or an amino group; and
   R⁵ is a hydrogen atom, an optionally substituted C₁₋₆ alkyl group, an optionally substituted C₆₋₁₄ aryl group, an optionally substituted aromatic heterocyclic group or an acyl group, or a salt thereof (hereinafter sometime to be referred to as "compound (I)").
[2] The compound or salt of the above-mentioned [1], wherein R³ is a hydrogen atom or an amino group.
[3] The compound or salt of the above-mentioned [1], wherein
   R¹ is an optionally substituted C₁₋₆ alkyl group or an optionally substituted C₃₋₁₀ cycloalkyl group;
   R² and R⁴ are independently a hydrogen atom, a halogen atom or an optionally substituted C₁₋₆ alkyl group;
   R³ is a hydrogen atom or an amino group; and
   R⁵ is
   (1) a hydrogen atom,
   (2) an optionally substituted C₃₋₁₀ cycloalkyl-carbonyl group,
   (3) an optionally substituted C₆₋₁₄ aryl group,
   (4) an optionally substituted 5- to 14-membered aromatic heterocyclic group,
   (5) an optionally substituted C₁₋₆ alkyl-carbonyl group,
   (6) an optionally substituted C₆₋₁₄ aryl-carbonyl group,
   (7) an optionally substituted 5- or 6-membered aromatic heterocyclylcarbonyl group,
   (8) an optionally substituted C₁₋₆ alkyl-carbamoyl group,
   (9) an optionally substituted C₆₋₁₄ aryl-carbamoyl group,
   (10) an optionally substituted C₃₋₁₀ cycloalkyl-carbamoyl group, or
   (11) an optionally substituted 3- to 8-membered monocyclic non-aromatic heterocyclylcarbamoyl group.
[4] (3S)-3-Cyclopropyl-1-(2-((5-(morpholin-4-yl)pyridin-2-yl)amino)pyridin-4-yl)-2-oxopyrrolidine-3-carbonitrile or a salt thereof.
[5] (3S)-3-Cyclopropyl-1-(2-((5-(2-hydroxypropan-2-yl)pyridin-2-yl)amino)pyridin-4-yl)-2-oxopyrrolidine-3-carbonitrile or a salt thereof.
[6] 3-((4-((3S)-3-Cyano-3-cyclopropyl-2-oxopyrrolidin-1-yl)pyridin-2-yl)amino)-N,1-dimethyl-1H-pyrazole-5-carboxamide or a salt thereof.
[7] A medicament comprising the compound or salt of the above-mentioned [1].
[8] The medicament of the above-mentioned [7], which is a tyrosine kinase 2 inhibitor.
[9] The medicament of the above-mentioned [7], which is an agent for the prophylaxis or treatment of autoimmune diseases.
[10] The medicament of the above-mentioned [9], wherein the autoimmune disease is psoriasis, rheumatoid arthritis, inflammatory bowel disease, Sjogren's syndrome, Behcet's disease, multiple sclerosis or systemic lupus erythematosus.
[11] The compound or salt of the above-mentioned [1] for use in the prophylaxis or treatment of autoimmune diseases.
[12] The compound or salt of the above-mentioned [11], wherein the autoimmune disease is psoriasis, rheumatoid arthritis, inflammatory bowel disease, Sjogren's syndrome, Behcet's disease, multiple sclerosis or systemic lupus erythematosus.
[13] A method of inhibiting tyrosine kinase 2 in a mammal, which comprises administering an effective amount of the compound or salt of the above-mentioned [1] to the mammal.
[14] A method for the prophylaxis or treatment of autoimmune diseases in a mammal, which comprises administering an effective amount of the compound or salt of the above-mentioned [1] to the mammal.
[15] The method of the above-mentioned [14], wherein the autoimmune disease is psoriasis, rheumatoid arthritis, inflammatory bowel disease, Sjogren's syndrome, Behcet's disease, multiple sclerosis or systemic lupus erythematosus.
[16] Use of the compound or salt of the above-mentioned [1] for the production of an agent for the prophylaxis or treatment of autoimmune diseases.
[17] The use of the above-mentioned [16], wherein the autoimmune disease is psoriasis, rheumatoid arthritis, inflammatory bowel disease, Sjogren's syndrome, Behcet's disease, multiple sclerosis or systemic lupus erythematosus.

### Effect of the Invention

Compound (I) has a superior Tyk2 inhibitory action, which is useful as an agent for the prophylaxis or treatment of autoimmune diseases (e.g., psoriasis, rheumatoid arthritis, inflammatory bowel disease (e.g., Crohn's disease, ulcerative colitis etc.), Sjogren's syndrome, Behcet's disease, multiple sclerosis, systemic lupus erythematosus etc.) and the like.

### (Detailed Description of the Invention)

The definition of each substituent used in the present specification is described in detail in the following. Unless otherwise specified, each substituent has the following definition.

In the present specification, examples of the "halogen atom" include fluorine, chlorine, bromine and iodine.

In the present specification, examples of the "C₁₋₆ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl.

In the present specification, examples of the "optionally halogenated C₁₋₆ alkyl group" include a C₁₋₆ alkyl group optionally having 1 to 7, preferably 1 to 5, halogen atoms. Specific examples thereof include methyl, chloromethyl, difluoromethyl, trichloromethyl, trifluoromethyl, ethyl, 2-bromoethyl, 2,2,2-trifluoroethyl, tetrafluoroethyl, pentafluoroethyl, propyl, 2,2-difluoropropyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl and 6,6,6-trifluorohexyl.

In the present specification, examples of the "C₂₋₆ alkenyl group" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl and 5-hexenyl.

In the present specification, examples of the "C₂₋₆ alkynyl group" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and 4-methyl-2-pentynyl.

In the present specification, examples of the "C₃₋₁₀ cycloalkyl group" include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl and adamantyl.

In the present specification, examples of the "optionally halogenated C₃₋₁₀ cycloalkyl group" include a C₃₋₁₀ cycloalkyl group optionally having 1 to 7, preferably 1 to 5, halogen atoms. Specific examples thereof include cyclopropyl, 2,2-difluorocyclopropyl, 2,3-difluorocyclopropyl, cyclobutyl, difluorocyclobutyl, cyclopentyl, cyclohexyl, cycloheptyl and cyclooctyl.

In the present specification, examples of the "C₃₋₁₀ cycloalkenyl group" include cyclopropenyl, cyclobutenyl, cyclopentenyl, cyclohexenyl, cycloheptenyl and cyclooctenyl.

In the present specification, examples of the "C₆₋₁₄ aryl group" include phenyl, 1-naphthyl, 2-naphthyl, 1-anthryl, 2-anthryl and 9-anthryl.

In the present specification, examples of the "C₇₋₁₆ aralkyl group" include benzyl, phenethyl, naphthylmethyl and phenylpropyl.

In the present specification, examples of the "C₁₋₆ alkoxy group" include methoxy, ethoxy, propoxy, isopropoxy, butoxy, isobutoxy, sec-butoxy, tert-butoxy, pentyloxy and hexyloxy.

In the present specification, examples of the "optionally halogenated C₁₋₆ alkoxy group" include a C₁₋₆ alkoxy group optionally having 1 to 7, preferably 1 to 5, halogen atoms. Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy and hexyloxy.

In the present specification, examples of the "C₃₋₁₀ cycloalkyloxy group" include cyclopropyloxy, cyclobutyloxy, cyclopentyloxy, cyclohexyloxy, cycloheptyloxy and cyclooctyloxy.

In the present specification, examples of the "C₁₋₆ alkylthio group" include methylthio, ethylthio, propylthio, isopropylthio, butylthio, sec-butylthio, tert-butylthio, pentylthio and hexylthio.

In the present specification, examples of the "optionally halogenated C₁₋₆ alkylthio group" include a C₁₋₆ alkylthio group optionally having 1 to 7, preferably 1 to 5, halogen atoms. Specific examples thereof include methylthio, difluoromethylthio, trifluoromethylthio, ethylthio, propylthio, isopropylthio, butylthio, 4,4,4-trifluorobutylthio, pentylthio and hexylthio.

In the present specification, examples of the "C₁₋₆ alkyl-carbonyl group" include acetyl, propanoyl, butanoyl, 2-methylpropanoyl, pentanoyl, 3-methylbutanoyl, 2-methylbutanoyl, 2,2-dimethylpropanoyl, hexanoyl and heptanoyl.

In the present specification, examples of the "optionally halogenated C₁₋₆ alkyl-carbonyl group" include a C₁₋₆ alkyl-carbonyl group optionally having 1 to 7, preferably 1 to 5, halogen atoms. Specific examples thereof include acetyl, chloroacetyl, trifluoroacetyl, trichloroacetyl, propanoyl, butanoyl, pentanoyl and hexanoyl.

In the present specification, examples of the "C₁₋₆ alkoxy-carbonyl group" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl and hexyloxycarbonyl.

In the present specification, examples of the "C₆₋₁₄ aryl-carbonyl group" include benzoyl, 1-naphthoyl and 2-naphthoyl.

In the present specification, examples of the "C₇₋₁₆ aralkyl-carbonyl group" include phenylacetyl and phenylpropionyl.

In the present specification, examples of the "5- to 14-membered aromatic heterocyclylcarbonyl group" include nicotinoyl, isonicotinoyl, thenoyl and furoyl.

In the present specification, examples of the "3- to 14-membered non-aromatic heterocyclylcarbonyl group" include morpholinylcarbonyl, piperidinylcarbonyl and pyrrolidinylcarbonyl.

In the present specification, examples of the "mono- or di-C₁₋₆ alkyl-carbamoyl group" include methylcarbamoyl, ethylcarbamoyl, dimethylcarbamoyl, diethylcarbamoyl and N-ethyl-N-methylcarbamoyl.

In the present specification, examples of the "mono- or di-C₇₋₁₆ aralkyl-carbamoyl group" include benzylcarbamoyl and phenethylcarbamoyl.

In the present specification, examples of the "C₁₋₆ alkylsulfonyl group" include methylsulfonyl, ethylsulfonyl, propylsulfonyl, isopropylsulfonyl, butylsulfonyl, sec-butylsulfonyl and tert-butylsulfonyl.

In the present specification, examples of the "optionally halogenated C₁₋₆ alkylsulfonyl group" include a C₁₋₆ alkylsulfonyl group optionally having 1 to 7, preferably 1 to 5, halogen atoms. Specific examples thereof include methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl and hexylsulfonyl.

In the present specification, examples of the "C₆₋₁₄ arylsulfonyl group" include phenylsulfonyl, 1-naphthylsulfonyl and 2-naphthylsulfonyl.

In the present specification, examples of the "substituent" include a halogen atom, a cyano group, a nitro group, an optionally substituted hydrocarbon group, an optionally substituted heterocyclic group, an acyl group, an optionally substituted amino group, an optionally substituted carbamoyl group, an optionally substituted thiocarbamoyl group, an optionally substituted sulfamoyl group, an optionally substituted hydroxy group, an optionally substituted sulfanyl (SH) group and an optionally substituted silyl group.

In the present specification, examples of the "hydrocarbon group" (including "hydrocarbon group" of "optionally substituted hydrocarbon group") include a C₁₋₆ alkyl group, a C₂₋₆ alkenyl group, a C₂₋₆ alkynyl group, a C₃₋₁₀ cycloalkyl group, a C₃₋₁₀ cycloalkenyl group, a C₆₋₁₄ aryl group and a C₇₋₁₆ aralkyl group.

In the present specification, examples of the "optionally substituted hydrocarbon group" include a hydrocarbon group optionally having substituent(s) selected from the following Substituent Group A.

### [Substituent Group A]

(1) a halogen atom,
(2) a nitro group,
(3) a cyano group,
(4) an oxo group,
(5) a hydroxy group,
(6) an optionally halogenated C₁₋₆ alkoxy group,
(7) a C₆₋₁₄ aryloxy group (e.g., phenoxy, naphthoxy),
(8) a C₇₋₁₆ aralkyloxy group (e.g., benzyloxy),
(9) a 5- to 14-membered aromatic heterocyclyloxy group (e.g., pyridyloxy),
(10) a 3- to 14-membered non-aromatic heterocyclyloxy group (e.g., morpholinyloxy, piperidinyloxy),
(11) a C₁₋₆ alkyl-carbonyloxy group (e.g., acetoxy, propanoyloxy),
(12) a C₆₋₁₄ aryl-carbonyloxy group (e.g., benzoyloxy, 1-naphthoyloxy, 2-naphthoyloxy),
(13) a C₁₋₆ alkoxy-carbonyloxy group (e.g., methoxycarbonyloxy, ethoxycarbonyloxy, propoxycarbonyloxy, butoxycarbonyloxy),
(14) a mono- or di-C₁₋₆ alkyl-carbamoyloxy group (e.g., methylcarbamoyloxy, ethylcarbamoyloxy, dimethylcarbamoyloxy, diethylcarbamoyloxy),
(15) a C₆₋₁₄ aryl-carbamoyloxy group (e.g., phenylcarbamoyloxy, naphthylcarbamoyloxy),
(16) a 5- to 14-membered aromatic heterocyclylcarbonyloxy group (e.g., nicotinoyloxy),
(17) a 3- to 14-membered non-aromatic heterocyclylcarbonyloxy group (e.g., morpholinylcarbonyloxy, piperidinylcarbonyloxy),
(18) an optionally halogenated C₁₋₆ alkylsulfonyloxy group (e.g., methylsulfonyloxy, trifluoromethylsulfonyloxy),
(19) a C₆₋₁₄ arylsulfonyloxy group optionally substituted by a C₁₋₆ alkyl group (e.g., phenylsulfonyloxy, toluenesulfonyloxy),
(20) an optionally halogenated C₁₋₆ alkylthio group,
(21) a 5- to 14-membered aromatic heterocyclic group,
(22) a 3- to 14-membered non-aromatic heterocyclic group,
(23) a formyl group,
(24) a carboxy group,
(25) an optionally halogenated C₁₋₆ alkyl-carbonyl group,
(26) a C₆₋₁₄ aryl-carbonyl group,
(27) a 5- to 14-membered aromatic heterocyclylcarbonyl group,
(28) a 3- to 14-membered non-aromatic heterocyclylcarbonyl group,
(29) a C₁₋₆ alkoxy-carbonyl group,
(30) a C₆₋₁₄ aryloxy-carbonyl group (e.g., phenyloxycarbonyl, 1-naphthyloxycarbonyl, 2-naphthyloxycarbonyl),
(31) a C₇₋₁₆ aralkyloxy-carbonyl group (e.g., benzyloxycarbonyl, phenethyloxycarbonyl),
(32) a carbamoyl group,
(33) a thiocarbamoyl group,
(34) a mono- or di-C₁₋₆ alkyl-carbamoyl group,
(35) a C₆₋₁₄ aryl-carbamoyl group (e.g., phenylcarbamoyl),
(36) a 5- to 14-membered aromatic heterocyclylcarbamoyl group (e.g., pyridylcarbamoyl, thienylcarbamoyl),
(37) a 3- to 14-membered non-aromatic heterocyclylcarbamoyl group (e.g., morpholinylcarbamoyl, piperidinylcarbamoyl),
(38) an optionally halogenated C₁₋₆ alkylsulfonyl group,
(39) a C₆₋₁₄ arylsulfonyl group,
(40) a 5- to 14-membered aromatic heterocyclylsulfonyl group (e.g., pyridylsulfonyl, thienylsulfonyl),
(41) an optionally halogenated C₁₋₆ alkylsulfinyl group,
(42) a C₆₋₁₄ arylsulfinyl group (e.g., phenylsulfinyl, 1-naphthylsulfinyl, 2-naphthylsulfinyl),
(43) a 5- to 14-membered aromatic heterocyclylsulfinyl group (e.g., pyridylsulfinyl, thienylsulfinyl),
(44) an amino group,
(45) a mono- or di-C₁₋₆ alkylamino group (e.g., methylamino, ethylamino, propylamino, isopropylamino, butylamino, dimethylamino, diethylamino, dipropylamino, dibutylamino, N-ethyl-N-methylamino),
(46) a mono- or di-C₆₋₁₄ arylamino group (e.g., phenylamino),
(47) a 5- to 14-membered aromatic heterocyclylamino group (e.g., pyridylamino),
(48) a C₇₋₁₆ aralkylamino group (e.g., benzylamino),
(49) a formylamino group,
(50) a C₁₋₆ alkyl-carbonylamino group (e.g., acetylamino, propanoylamino, butanoylamino),
(51) a (C₁₋₆ alkyl) (C₁₋₆ alkyl-carbonyl) amino group (e.g., N-acetyl-N-methylamino),
(52) a C₆₋₁₄ aryl-carbonylamino group (e.g., phenylcarbonylamino, naphthylcarbonylamino),
(53) a C₁₋₆ alkoxy-carbonylamino group (e.g., methoxycarbonylamino, ethoxycarbonylamino, propoxycarbonylamino, butoxycarbonylamino, tert-butoxycarbonylamino),
(54) a C₇₋₁₆ aralkyloxy-carbonylamino group (e.g., benzyloxycarbonylamino),
(55) a C₁₋₆ alkylsulfonylamino group (e.g., methylsulfonylamino, ethylsulfonylamino),
(56) a C₆₋₁₄ arylsulfonylamino group optionally substituted by a C₁₋₆ alkyl group (e.g., phenylsulfonylamino, toluenesulfonylamino),
(57) an optionally halogenated C₁₋₆ alkyl group,
(58) a C₂₋₆ alkenyl group,
(59) a C₂₋₆ alkynyl group,
(60) a C₃₋₁₀ cycloalkyl group,
(61) a C₃₋₁₀ cycloalkenyl group and
(62) a C₆₋₁₄ aryl group.

The number of the above-mentioned substituents in the "optionally substituted hydrocarbon group" is, for example, 1 to 5, preferably 1 to 3. When the number of the substituents is two or more, the respective substituents may be the same or different.

In the present specification, examples of the "heterocyclic group" (including "heterocyclic group" of "optionally substituted heterocyclic group") include (i) an aromatic heterocyclic group, (ii) a non-aromatic heterocyclic group and (iii) a 7- to 10-membered bridged heterocyclic group, each containing, as a ring-constituting atom besides carbon atom, 1 to 4 hetero atoms selected from a nitrogen atom, a sulfur atom and an oxygen atom.

In the present specification, examples of the "aromatic heterocyclic group" (including "5- to 14-membered aromatic heterocyclic group") include a 5- to 14-membered (preferably 5- to 10-membered) aromatic heterocyclic group containing, as a ring-constituting atom besides carbon atom, 1 to 4 hetero atoms selected from a nitrogen atom, a sulfur atom and an oxygen atom.

Preferable examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and 8- to 14-membered fused polycyclic (preferably bi or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl and the like.

In the present specification, examples of the "non-aromatic heterocyclic group" (including "3- to 14-membered non-aromatic heterocyclic group") include a 3- to 14-membered (preferably 4- to 10-membered) non-aromatic heterocyclic group containing, as a ring-constituting atom besides carbon atom, 1 to 4 hetero atoms selected from a nitrogen atom, a sulfur atom and an oxygen atom.

Preferable examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisooxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl and the like; and 9- to 14-membered fused polycyclic (preferably bi or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl and the like.

In the present specification, preferable examples of the "7- to 10-membered bridged heterocyclic group" include quinuclidinyl and 7-azabicyclo[2.2.1]heptanyl.

In the present specification, examples of the "nitrogen-containing heterocyclic group" include a "heterocyclic group" containing at least one nitrogen atom as a ring-constituting atom.

In the present specification, examples of the "optionally substituted heterocyclic group" include a heterocyclic group optionally having substituent(s) selected from the aforementioned Substituent Group A.

The number of the substituents in the "optionally substituted heterocyclic group" is, for example, 1 to 3. When the number of the substituents is two or more, the respective substituents may be the same or different.

In the present specification, examples of the "acyl group" include a formyl group, a carboxy group, a carbamoyl group, a thiocarbamoyl group, a sulfino group, a sulfo group, a sulfamoyl group and a phosphono group, each optionally having "1 or 2 substituents selected from a C₁₋₆ alkyl group, a C₂₋₆ alkenyl group, a C₃₋₁₀ cycloalkyl group, a C₃₋₁₀ cycloalkenyl group, a C₆₋₁₄ aryl group, a C₇₋₁₆ aralkyl group, a 5- to 14-membered aromatic heterocyclic group and a 3- to 14-membered non-aromatic heterocyclic group, each of which optionally has 1 to 3 substituents selected from a halogen atom, an optionally halogenated C₁₋₆ alkoxy group, a hydroxy group, a nitro group, a cyano group, an amino group and a carbamoyl group".

Examples of the "acyl group" also include a hydrocarbon-sulfonyl group, a heterocyclylsulfonyl group, a hydrocarbon-sulfinyl group and a heterocyclylsulfinyl group.

Here, the hydrocarbon-sulfonyl group means a hydrocarbon group-bonded sulfonyl group, the heterocyclylsulfonyl group means a heterocyclic group-bonded sulfonyl group, the hydrocarbon-sulfinyl group means a hydrocarbon group-bonded sulfinyl group and the heterocyclylsulfinyl group means a heterocyclic group-bonded sulfinyl group.

Preferable examples of the "acyl group" include a formyl group, a carboxy group, a C₁₋₆ alkyl-carbonyl group, a C₂₋₆ alkenyl-carbonyl group (e.g., crotonoyl), a C₃₋₁₀ cycloalkyl-carbonyl group (e.g., cyclobutanecarbonyl, cyclopentanecarbonyl, cyclohexanecarbonyl, cycloheptanecarbonyl), a C₃₋₁₀ cycloalkenyl-carbonyl group (e.g., 2-cyclohexenecarbonyl) , a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a C₆₋₁₄ aryloxy-carbonyl group (e.g., phenyloxycarbonyl, naphthyloxycarbonyl), a C₇₋₁₆ aralkyloxy-carbonyl group (e.g., benzyloxycarbonyl, phenethyloxycarbonyl), a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl), a mono- or di-C₆₋₁₄ aryl-carbamoyl group (e.g., phenylcarbamoyl), a mono- or di-C₇₋₁₆ aralkyl-carbamoyl group, a 5- to 14-membered aromatic heterocyclylcarbamoyl group (e.g., pyridylcarbamoyl), a thiocarbamoyl group, a mono- or di-C₁₋₆ alkyl-thiocarbamoyl group (e.g., methylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a 5- to 14-membered aromatic heterocyclylthiocarbamoyl group (e.g., pyridylthiocarbamoyl), a sulfino group, a C₁₋₆ alkylsulfinyl group (e.g., methylsulfinyl, ethylsulfinyl), a sulfo group, a C₁₋₆ alkylsulfonyl group, a C₆₋₁₄ arylsulfonyl group, a phosphono group and a mono- or di-C₁₋₆ alkylphosphono group (e.g., dimethylphosphono, diethylphosphono, diisopropylphosphono, dibutylphosphono).

In the present specification, examples of the "optionally substituted amino group" include an amino group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from Substituent Group A".

Preferable examples of the optionally substituted amino group include an amino group, a mono- or di-(optionally halogenated C₁₋₆ alkyl) amino group (e.g., methylamino, trifluoromethylamino, dimethylamino, ethylamino, diethylamino, propylamino, dibutylamino), a mono- or di-C₂₋₆ alkenylamino group (e.g., diallylamino), a mono- or di-C₃₋₁₀ cycloalkylamino group (e.g., cyclopropylamino, cyclohexylamino), a mono- or di-C₆₋₁₄ arylamino group (e.g., phenylamino), a mono- or di-C₇₋₁₆ aralkylamino group (e.g., benzylamino, dibenzylamino), a mono-or di-(optionally halogenated C₁₋₆ alkyl)-carbonylamino group (e.g., acetylamino, propionylamino), a mono- or di-C₆₋₁₄ aryl-carbonylamino group (e.g., benzoylamino), a mono- or di-C₇₋₁₆ aralkyl-carbonylamino group (e.g., benzylcarbonylamino), a mono- or di-5- to 14-membered aromatic heterocyclylcarbonylamino group (e.g., nicotinoylamino, isonicotinoylamino), a mono- or di-3- to 14-membered non-aromatic heterocyclylcarbonylamino group (e.g., piperidinylcarbonylamino), a mono- or di-C₁₋₆ alkoxy-carbonylamino group (e.g., tert-butoxycarbonylamino), a 5- to 14-membered aromatic heterocyclylamino group (e.g., pyridylamino), a carbamoylamino group, a (mono- or di-C₁₋₆ alkyl-carbamoyl)amino group (e.g., methylcarbamoylamino), a (mono- or di-C₇₋₁₆ aralkyl-carbamoyl) amino group (e.g., benzylcarbamoylamino), a C₁₋₆ alkylsulfonylamino group (e.g., methylsulfonylamino, ethylsulfonylamino), a C₆₋₁₄ arylsulfonylamino group (e.g., phenylsulfonylamino), a (C₁₋₆ alkyi) (C₁₋₆ alkyl-carbonyl)amino group (e.g., N-acetyl-N-methylamino) and a (C₁₋₆ alkyl) (C₆₋₁₄ aryl-carbonyl) amino group (e.g., N-benzoyl-N-methylamino).

In the present specification, examples of the "optionally substituted carbamoyl group" include a carbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from Substituent Group A".

Preferable examples of the optionally substituted carbamoyl group include a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl, cyclohexylcarbamoyl), a mono- or di-C₆₋₁₄ aryl-carbamoyl group (e.g., phenylcarbamoyl), a mono- or di-C₇₋₁₆ aralkyl-carbamoyl group, a mono- or di-C₁₋₆ alkyl-carbonyl-carbamoyl group (e.g., acetylcarbamoyl, propionylcarbamoyl), a mono- or di-C₆₋₁₄ aryl-carbonyl-carbamoyl group (e.g., benzoylcarbamoyl) and a 5- to 14-membered aromatic heterocyclylcarbamoyl group (e.g., pyridylcarbamoyl).

In the present specification, examples of the "optionally substituted thiocarbamoyl group" include a thiocarbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from Substituent Group A".

Preferable examples of the optionally substituted thiocarbamoyl group include a thiocarbamoyl group, a mono- or di-C₁₋₆ alkyl-thiocarbamoyl group (e.g., methylthiocarbamoyl, ethylthiocarbamoyl, dimethylthiocarbamoyl, diethylthiocarbamoyl, N-ethyl-N-methylthiocarbamoyl), a mono-or di-C₂₋₆ alkenyl-thiocarbamoyl group (e.g., diallylthiocarbamoyl), a mono- or di-C₃₋₁₀ cycloalkylthiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkylthiocarbamoyl group (e.g., benzylthiocarbamoyl, phenethylthiocarbamoyl), a mono- or di-C₁₋₆ alkyl-carbonylthiocarbamoyl group (e.g., acetylthiocarbamoyl, propionylthiocarbamoyl), a mono- or di-C₆₋₁₄ aryl-carbonylthiocarbamoyl group (e.g., benzoylthiocarbamoyl) and a 5- to 14-membered aromatic heterocyclylthiocarbamoyl group (e.g., pyridylthiocarbamoyl).

In the present specification, examples of the "optionally substituted sulfamoyl group" include a sulfamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from Substituent Group A".

Preferable examples of the optionally substituted sulfamoyl group include a sulfamoyl group, a mono- or di-C₁₋₆ alkyl-sulfamoyl group (e.g., methylsulfamoyl, ethylsulfamoyl, dimethylsulfamoyl, diethylsulfamoyl, N-ethyl-N-methylsulfamoyl), a mono- or di-C₂₋₆ alkenyl-sulfamoyl group (e.g., diallylsulfamoyl), a mono- or di-C₃₋₁₀ cycloalkylsulfamoyl group (e.g., cyclopropylsulfamoyl, cyclohexylsulfamoyl), a mono- or di-C₆₋₁₄ aryl-sulfamoyl group (e.g., phenylsulfamoyl), a mono- or di-C₇₋₁₆ aralkyl-sulfamoyl group (e.g., benzylsulfamoyl, phenethylsulfamoyl), a mono- or di-C₁₋₆ alkyl-carbonyl-sulfamoyl group (e.g., acetylsulfamoyl, propionylsulfamoyl), a mono- or di-C₆₋₁₄ aryl-carbonyl-sulfamoyl group (e.g., benzoylsulfamoyl) and a 5- to 14-membered aromatic heterocyclylsulfamoyl group (e.g., pyridylsulfamoyl).

In the present specification, examples of the "optionally substituted hydroxy group" include a hydroxyl group optionally having "a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from Substituent Group A".

Preferable examples of the optionally substituted hydroxy group include a hydroxy group, a C₁₋₆ alkoxy group, a C₂₋₆ alkenyloxy group (e.g., allyloxy, 2-butenyloxy, 2-pentenyloxy, 3-hexenyloxy), a C₃₋₁₀ cycloalkyloxy group (e.g., cyclohexyloxy), a C₆₋₁₄ aryloxy group (e.g., phenoxy, naphthyloxy), a C₇₋₁₆ aralkyloxy group (e.g., benzyloxy, phenethyloxy), a C₁₋₆ alkyl-carbonyloxy group (e.g., acetyloxy, propionyloxy, butyryloxy, isobutyryloxy, pivaloyloxy), a C₆₋₁₄ aryl-carbonyloxy group (e.g., benzoyloxy), a C₇₋₁₆ aralkyl-carbonyloxy group (e.g., benzylcarbonyloxy), a 5- to 14-membered aromatic heterocyclylcarbonyloxy group (e.g., nicotinoyloxy), a 3- to 14-membered non-aromatic heterocyclylcarbonyloxy group (e.g., piperidinylcarbonyloxy), a C₁₋₆ alkoxy-carbonyloxy group (e.g., tert-butoxycarbonyloxy), a 5- to 14-membered aromatic heterocyclyloxy group (e.g., pyridyloxy), a carbamoyloxy group, a C₁₋₆ alkyl-carbamoyloxy group (e.g., methylcarbamoyloxy), a C₇₋₁₆ aralkyl-carbamoyloxy group (e.g., benzylcarbamoyloxy), a C₁₋₆ alkylsulfonyloxy group (e.g., methylsulfonyloxy, ethylsulfonyloxy) and a C₆₋₁₄ arylsulfonyloxy group (e.g., phenylsulfonyloxy).

In the present specification, examples of the "optionally substituted sulfanyl group" include a sulfanyl group optionally having "a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group and a 5- to 14-membered aromatic heterocyclic group, each of which optionally has 1 to 3 substituents selected from Substituent Group A" and a halogenated sulfanyl group.

Preferable examples of the optionally substituted sulfanyl group include a sulfanyl (-SH) group, a C₁₋₆ alkylthio group, a C₂₋₆ alkenylthio group (e.g., allylthio, 2-butenylthio, 2-pentenylthio, 3-hexenylthio), a C₃₋₁₀ cycloalkylthio group (e.g., cyclohexylthio), a C₆₋₁₄ arylthio group (e.g., phenylthio, naphthylthio), a C₇₋₁₆ aralkylthio group (e.g., benzylthio, phenethylthio), a C₁₋₆ alkyl-carbonylthio group (e.g., acetylthio, propionylthio, butyrylthio, isobutyrylthio, pivaloylthio), a C₆₋₁₄ aryl-carbonylthio group (e.g., benzoylthio), a 5- to 14-membered aromatic heterocyclylthio group (e.g., pyridylthio) and a halogenated thio group (e.g., pentafluorothio).

In the present specification, examples of the "optionally substituted silyl group" include a silyl group optionally having "1 to 3 substituents selected from a C₁₋₆ alkyl group, a C₂₋₆ alkenyl group, a C₃₋₁₀ cycloalkyl group, a C₆₋₁₄ aryl group and a C₇₋₁₆ aralkyl group, each of which optionally has 1 to 3 substituents selected from Substituent Group A".

Preferable examples of the optionally substituted silyl group include a tri-C₁₋₆ alkylsilyl group (e.g., trimethylsilyl, tert-butyl(dimethyl)silyl).

Each symbol of the formula (I) is explained below.

In the formula (I), Ring A is an optionally further substituted pyrrolidine ring.

The "pyrrolidine ring" of the "optionally further substituted pyrrolidine ring" represented by Ring A optionally has 1 to 3 substituents at substitutable position(s). Examples of the substituent include substituents selected from the above-mentioned Substituent Group A. Among them, a C₁₋₆ alkyl group (e.g., methyl) is preferable. When the number of the substituents is plural, the respective substituents may be the same or different.

In one embodiment, Ring A is a pyrrolidine ring wherein the 4-position is not substituted, and the 5-position is optionally substituted.

In this embodiment, Ring A is preferably a pyrrolidine ring wherein the 4-position is not substituted, and the 5-position is optionally further substituted by 1 or 2 (preferably 1) C₁₋₆ alkyl group (s) (e.g., methyl).

In another embodiment, Ring A is a pyrrolidine ring wherein the 4-position is not substituted.

In this embodiment, Ring A is preferably a pyrrolidine ring wherein the 4-position and the 5-position are not substituted.

Moreover, in another embodiment, Ring A is a pyrrolidine ring wherein the 4-position is not substituted, and the 5-position is substituted.

In this embodiment, Ring A is preferably a pyrrolidine ring wherein the 4-position is not substituted, and the 5-position is further substituted by 1 or 2 (preferably 1) C₁₋₆ alkyl group(s) (e.g., methyl).

Moreover, in another embodiment, Ring A is a pyrrolidine ring wherein the 4-position is substituted, and the 5-position position is not substituted.

Moreover, in another embodiment, Ring A is a pyrrolidine ring wherein the 4-position and the 5-position are substituted.

In the formula (I) , R¹, R² and R⁴ are independently a hydrogen atom or a substituent.

R¹ is preferably an optionally substituted C₁₋₆ alkyl group or an optionally substituted C₃₋₁₀ cycloalkyl group.
Specifically, R¹ is preferably
(1) a C₁₋₆ alkyl group (e.g., ethyl, isopropyl), or
(2) a C₃₋₁₀ cycloalkyl group (e.g., cyclopropyl).

R¹ is more preferably an optionally substituted C₃₋₁₀ cycloalkyl group.

Specifically, R¹ is more preferably a C₃₋₁₀ group (e.g., cyclopropyl).

R² is preferably a hydrogen atom.

R⁴ is preferably a hydrogen atom.

In another embodiment, R² and R⁴ are preferably independently a hydrogen atom, a halogen atom or an optionally substituted C₁₋₆ alkyl group.

Specifically, R² and R⁴ are preferably independently
(1) a hydrogen atom,
(2) a halogen atom (e.g., a fluorine atom, a chlorine atom), or
(3) a C₁₋₆ alkyl group (e.g., methyl).

R² and R⁴ are more preferably both hydrogen atoms.

In the formula (I), R³ is a hydrogen atom, a halogen atom, an optionally halogenated C₁₋₆ alkyl group, a mono- or di-(optionally halogenated C₁₋₆ alkyl)amino group or an amino group.

R³ is preferably a hydrogen atom.

In another embodiment, R³ is preferably a hydrogen atom or an amino group, more preferably a hydrogen atom.

In the formula (I), R⁵ is a hydrogen atom, an optionally substituted C₁₋₆ alkyl group, an optionally substituted C₆₋₁₄ aryl group, an optionally substituted aromatic heterocyclic group or an acyl group.

The "C₁₋₆ alkyl group" of the "optionally substituted C₁₋₆ alkyl group" represented by R⁵ optionally has 1 to 3 substituents at substitutable position(s). Examples of the substituent include substituents selected from the above-mentioned Substituent Group A. When the number of the substituents is plural, the respective substituents may be the same or different.

The "C₆₋₁₄ aryl group" of the "optionally substituted C₆₋₁₄ aryl group" represented by R⁵ optionally has 1 to 3 substituents at substitutable position(s). Examples of the substituent include substituents selected from the above-mentioned Substituent Group A. When the number of the substituents is plural, the respective substituents may be the same or different.

The "aromatic heterocyclic group" of the "optionally substituted aromatic heterocyclic group" represented by R⁵ optionally has 1 to 3 substituents at substitutable position(s). Examples of the substituent include substituents selected from the above-mentioned Substituent Group A. When the number of the substituents is plural, the respective substituents may be the same or different.

R⁵ is preferably
(1) a hydrogen atom,
(2) an optionally substituted C₃₋₁₀ cycloalkyl-carbonyl group,
(3) an optionally substituted C₆₋₁₄ aryl group, or
(4) an optionally substituted 5- to 14-membered aromatic heterocyclic group (preferably a 5- or 6-membered monocyclic aromatic heterocyclic group).

Specifically, R⁵ is preferably
(1) a hydrogen atom,
(2) a C₃₋₁₀ cycloalkyl-carbonyl group (e.g., cyclopropylcarbonyl),
(3) a C₆₋₁₄ aryl group (e.g., phenyl), or
(4) a 5- to 14-membered aromatic heterocyclic group (e.g., pyridyl, pyrimidinyl, pyrazinyl, pyridazinyl, pyrazolyl, imidazolyl, isoxazolyl, quinolyl, isoquinolyl) (preferably a 5- or 6-membered monocyclic aromatic heterocyclic group) optionally substituted by 1 to 3 substituents selected from
   (a) a halogen atom (e.g., a fluorine atom),
   (b) a C₁₋₆ alkyl group (e.g., methyl, ethyl, isopropyl, tert-butyl) optionally substituted by 1 to 3 substituents selected from
      (i) a hydroxy group,
      (ii) a halogen atom (e.g., a fluorine atom),
      (iii) a cyano group,
      (iv) a carboxy group,
      (v) a C₁₋₆ alkoxy group (e.g., methoxy),
      (vi) a C₁₋₆ alkoxy-carbonyl group (e.g., tert-butoxycarbonyl),
      (vii) an amino group optionally mono- or di-substituted by substituent(s) selected from a C₁₋₆ alkyl group (e.g., methyl), a C₁₋₆ alkyl-carbonyl group (e.g., acetyl) and a C₁₋₆ alkylsulfonyl group (e.g., methylsulfonyl),
      (viii) a carbamoyl group optionally mono- or di-substituted by C₁₋₆ alkyl group(s) (e.g., methyl), and
      (ix) a 3- to 8-membered monocyclic non-aromatic heterocyclic group (e.g., morpholinyl),
   (c) a C₃₋₁₀ cycloalkyl group (e.g., cyclopropyl, cyclobutyl, cyclohexyl) optionally substituted by 1 to 3 substituents selected from
      (i) a hydroxy group,
      (ii) a halogen atom (e.g., a fluorine atom), and
      (iii) a cyano group,
   (d) a C₁₋₆ alkoxy group (e.g., methoxy, ethoxy, isopentyloxy) optionally substituted by 1 to 3 substituents selected from
      (i) a hydroxy group,
      (ii) a C₁₋₆ alkoxy group (e.g., methoxy), and
      (iii) a carbamoyl group optionally mono- or di-substituted by C₁₋₆ alkyl group (s) (e.g., methyl),
   (e) a C₆₋₁₄ aryl group (e.g., phenyl),
   (f) a C₁₋₆ alkoxy-carbonyl group (e.g., methoxycarbonyl, tert-butoxycarbonyl),
   (g) a carbamoyl group optionally mono- or di-substituted by substituent(s) selected from
      (i) a C₁₋₆ alkyl group (e.g., methyl) optionally substituted by 1 to 3 hydroxy groups, and
      (ii) a 3- to 8-membered monocyclic non-aromatic heterocyclic group (e.g., tetrahydropyranyl),
   (h) a sulfamoyl group optionally mono- or di-substituted by C₃₋₁₀ cycloalkyl group (s) (e.g., cyclopropyl),
   (i) an amino group optionally mono- or di-substituted by C₁₋₆ alkyl-carbonyl group(s) (e.g., acetyl),
   (j) a cyano group,
   (k) a 3- to 8-membered monocyclic non-aromatic heterocyclic group (e.g., morpholinyl, 1,1-dioxidothiomorpholinyl, dihydropyranyl, tetrahydropyranyl, tetrahydropyridyl, piperidyl, piperazinyl, pyrrolidinyl, azetidinyl) optionally substituted by 1 to 3 substituents selected from
      (i) an oxo group,
      (ii) a cyano group,
      (iii) a hydroxy group,
      (iv) a halogen atom (e.g., a fluorine atom),
      (v) a C₁₋₆ alkyl group (e.g., methyl) optionally substituted by 1 to 3 substituents selected from a hydroxy group and a halogen atom (e.g., a fluorine atom),
      (vi) a C₁₋₆ alkoxy group (e.g., methoxy),
      (vii) a C₃₋₁₀ cycloalkyl group (e.g., cyclopropyl),
      (viii) a C₁₋₆ alkyl-carbonyl group (e.g., acetyl),
      (ix) a C₁₋₆ alkoxy-carbonyl group (e.g., tert-butoxycarbonyl),
      (x) a carbamoyl group, and
      (xi) a C₁₋₆ alkylsulfonyl group (e.g., isopropylsulfonyl),
   (l) a 3- to 8-membered monocyclic non-aromatic heterocyclyloxy group (e.g., tetrahydropyranyloxy),
   (m) a 3- to 8-membered monocyclic non-aromatic heterocyclylcarbonyl group (e.g., morpholinylcarbonyl, piperidylcarbonyl) optionally substituted by 1 to 3 substituents selected from
      (i) a halogen atom (e.g., a fluorine atom), and
      (ii) a C₁₋₆ alkyl group (e.g., methyl), and
   (n) a carboxy group.

In another embodiment, specifically, R⁵ is preferably
(1) a hydrogen atom,
(2) a C₃₋₁₀ cycloalkyl-carbonyl group (e.g., cyclopropylcarbonyl),
(3) a C₆₋₁₄ aryl group (e.g., phenyl), or
(4) a 5- to 14-membered aromatic heterocyclic group (e.g., pyridyl, pyrimidinyl, pyrazinyl, pyridazinyl, pyrazolyl, imidazolyl, isoxazolyl, quinolyl, isoquinolyl, pyrrolo[3,2-c]pyridyl, [1,2,4]triazolo[4,3-c]pyrimidinyl, pyrrolo[3,2-c]pyridyl) (preferably a 5- or 6-membered monocyclic aromatic heterocyclic group) optionally substituted by 1 to 3 substituents selected from
   (a) a halogen atom (e.g., a fluorine atom, a chlorine atom),
   (b) a C₁₋₆ alkyl group (e.g., methyl, ethyl, isopropyl, tert-butyl) optionally substituted by 1 to 3 substituents selected from
      (i) a hydroxy group,
      (ii) a halogen atom (e.g., a fluorine atom),
      (iii) a cyano group,
      (iv) a carboxy group,
      (v) a C₁₋₆ alkoxy group (e.g., methoxy),
      (vi) a C₁₋₆ alkoxy-carbonyl group (e.g., tert-butoxycarbonyl),
      (vii) an amino group optionally mono- or di-substituted by substituent(s) selected from a C₁₋₆ alkyl group (e.g., methyl), a C₁₋₆ alkyl-carbonyl group (e.g., acetyl) and a C₁₋₆ alkylsulfonyl group (e.g., methylsulfonyl),
      (viii) a carbamoyl group optionally mono- or di-substituted by substituent(s) selected from
         (I) a C₁₋₆ alkyl group (e.g., methyl, ethyl) optionally substituted by 1 to 3 substituents selected from a hydroxy group and a halogen atom (e.g., a fluorine atom), and
         (II) a 3- to 8-membered monocyclic non-aromatic heterocyclic group (e.g., oxetanyl),
      (ix) a 3- to 8-membered monocyclic non-aromatic heterocyclic group (e.g., morpholinyl, piperidyl) optionally substituted by 1 to 3 substituents selected from a hydroxy group and a C₁₋₆ alkyl group (e.g., methyl), and
      (x) a 3- to 8-membered monocyclic non-aromatic heterocyclylcarbonyl group (e.g., morpholinylcarbonyl),
   (c) a C₃₋₁₀ cycloalkyl group (e.g., cyclopropyl, cyclobutyl, cyclopentyl, cyclohexyl) optionally substituted by 1 to 3 substituents selected from
      (i) a hydroxy group,
      (ii) a halogen atom (e.g., a fluorine atom),
      (iii) a cyano group,
      (iv) a 3- to 8-membered monocyclic non-aromatic heterocyclylcarbonyl group (e.g., azetidinylcarbonyl, pyrrolidinylcarbonyl, 3-oxa-8-azabicyclo[3.2.1]octyl) optionally substituted by 1 to 3 substituents selected from a halogen atom (e.g., a fluorine atom) and a hydroxy group, and
      (v) a carbamoyl group optionally mono- or di-substituted by substituent(s) selected from
         (I) a C₁₋₆ alkyl group (e.g., methyl, ethyl) optionally substituted by 1 to 3 substituents selected from a halogen atom (e.g., a fluorine atom) and a C₁₋₆ alkoxy group (e.g., methoxy),
         (II) a C₃₋₁₀ cycloalkyl group (e.g., cyclopropyl), and
         (III) a 3- to 8-membered monocyclic non-aromatic heterocyclic group (e.g., oxetanyl),
   (d) a C₁₋₆ alkoxy group (e.g., methoxy, ethoxy, isopentyloxy) optionally substituted by 1 to 3 substituents selected from
      (i) a hydroxy group,
      (ii) a C₁₋₆ alkoxy group (e.g., methoxy), and
      (iii) a carbamoyl group optionally mono- or di-substituted by C₁₋₆ alkyl group(s) (e.g., methyl),
   (e) a C₆₋₁₄ aryl group (e.g., phenyl),
   (f) a C₁₋₆ alkoxy-carbonyl group (e.g., methoxycarbonyl, tert-butoxycarbonyl),
   (g) a carbamoyl group optionally mono- or di-substituted by substituent(s) selected from
      (i) a C₁₋₆ alkyl group (e.g., methyl, ethyl, isobutyl, tert-butyl) optionally substituted by 1 to 3 substituents selected from
         (I) a hydroxy group,
         (II) a cyano group,
         (III) a halogen atom (e.g., a fluorine atom),
         (IV) a C₁₋₆ alkoxy group (e.g., methoxy),
         (V) a C₃₋₁₀ cycloalkyl group (e.g., cyclopropyl), and
         (VI) a 3- to 8-membered monocyclic non-aromatic heterocyclic group (e.g., oxetanyl, tetrahydrofuryl),
      (ii) a 3- to 8-membered monocyclic non-aromatic heterocyclic group (e.g., tetrahydropyranyl, tetrahydrofuryl, oxetanyl, 1,1-dioxidotetrahydrothiopyranyl) optionally substituted by 1 to 3 C₁₋₆ alkyl groups (e.g., methyl), and
      (iii) a C₃₋₁₀ cycloalkyl group (e.g., cyclopropyl, cyclobutyl) optionally substituted by 1 to 3 substituents selected from a halogen atom (e.g., a fluorine atom) and a cyano group,
   (h) a sulfamoyl group optionally mono- or di-substituted by C₃₋₁₀ cycloalkyl group (s) (e.g., cyclopropyl),
   (i) an amino group optionally mono- or di-substituted by substituent(s) selected from
      (i) a C₁₋₆ alkyl-carbonyl group (e.g., acetyl, propionyl) optionally substituted by 1 to 3 substituents selected from a halogen atom (e.g., a fluorine atom), a hydroxy group, a cyano group and a C₁₋₆ alkoxy group (e.g., methoxy),
      (ii) a C₁₋₆ alkoxy-carbonyl group (e.g., methoxycarbonyl, tert-butoxycarbonyl), and
      (iii) a C₃₋₁₀ cycloalkyl-carbonyl group (e.g., cyclopropylcarbonyl),
   (j) a cyano group,
   (k) a 3- to 8-membered monocyclic non-aromatic heterocyclic group (e.g., morpholinyl, 1,1-dioxidothiomorpholinyl, dihydropyranyl, tetrahydropyranyl, tetrahydropyridyl, piperidyl, piperazinyl, pyrrolidinyl, azetidinyl, oxadiazolinyl, oxazepanyl, 8-oxa-3-azabicyclo[3.2.1]octyl, 6-oxa-3-azabicyclo[3.1.1]heptyl, 3-oxa-8-azabicyclo[3.2.1]octyl) optionally substituted by 1 to 3 substituents selected from
      (i) an oxo group,
      (ii) a cyano group,
      (iii) a hydroxy group,
      (iv) a halogen atom (e.g., a fluorine atom),
      (v) a C₁₋₆ alkyl group (e.g., methyl) optionally substituted by 1 to 3 substituents selected from a hydroxy group and a halogen atom (e.g., a fluorine atom),
      (vi) a C₁₋₆ alkoxy group (e.g., methoxy),
      (vii) a C₃₋₁₀ cycloalkyl group (e.g., cyclopropyl),
      (viii) a C₁₋₆ alkyl-carbonyl group (e.g., acetyl, propionyl) optionally substituted by 1 to 3 halogen atoms (e.g., a fluorine atom),
      (ix) a C₁₋₆ alkoxy-carbonyl group (e.g., tert-butoxycarbonyl),
      (x) a carbamoyl group optionally mono- or di-substituted by C₁₋₆ alkyl group (s) (e.g., methyl),
      (xi) a C₁₋₆ alkylsulfonyl group (e.g., methylsulfonyl, isopropylsulfonyl), and
      (xii) a C₃₋₁₀ cycloalkyl-carbonyl group (e.g., cyclobutylcarbonyl) optionally substituted by 1 to 3 halogen atoms (e.g., a fluorine atom),
   (l) a 3- to 8-membered monocyclic non-aromatic heterocyclyloxy group (e.g., tetrahydropyranyloxy),
   (m) a 3- to 8-membered monocyclic non-aromatic heterocyclylcarbonyl group (e.g., morpholinylcarbonyl, piperidylcarbonyl, pyrrolidinylcarbonyl, oxazepanylcarbonyl, azetidinylcarbonyl, 6-oxa-3-azabicyclo[3.1.1]heptylcarbonyl, 3-oxa-8-azabicyclo[3.2.1]octylcarbonyl, 8-oxa-3-azabicyclo[3.2.1]octylcarbonyl, 2-oxa-6-azaspiro[3.4]octylcarbonyl, 3-azabicyclo[3.1.0]hexylcarbonyl, 1,3-dihydro-2H-pyrrolo[3,4-c]pyridylcarbonyl, 5,7-dihydro-6H-pyrrolo[3,4-b]pyridylcarbonyl, 5,7-dihydro-6H-pyrrolo[3,4-d]pyrimidinylcarbonyl) optionally substituted by 1 to 3 substituents selected from
      (i) a halogen atom (e.g., a fluorine atom),
      (ii) a C₁₋₆ alkyl group (e.g., methyl) optionally substituted by 1 to 3 halogen atoms (e.g., a fluorine atom),
      (iii) a hydroxy group, and
      (iv) a C₁₋₆ alkoxy group (e.g., methoxy), and
   (n) a carboxy group.

R⁵ is more preferably an optionally substituted 5- or 6-membered monocyclic aromatic heterocyclic group.

Specifically, R⁵ is more preferably a 5- or 6-membered monocyclic aromatic heterocyclic group (e.g., pyridyl, pyrazolyl) optionally substituted by 1 to 3 substituents selected from
(i) a C₁₋₆ alkyl group (e.g., methyl, isopropyl) optionally substituted by 1 to 3 hydroxy groups,
(ii) a carbamoyl group optionally mono- or di-substituted by C₁₋₆ alkyl group (s) (e.g., methyl),
(iii) a 3- to 8-membered monocyclic non-aromatic heterocyclic group (e.g., morpholinyl),
(iv) a C₁₋₆ alkoxy-carbonyl group (e.g., tert-butoxycarbonyl), and
(v) a carboxy group.

R⁵ is particularly preferably a 5- or 6-membered monocyclic aromatic heterocyclic group (e.g., pyridyl, pyrazolyl) optionally substituted by 1 to 3 substituents selected from
(i) a C₁₋₆ alkyl group (e.g., methyl, isopropyl) optionally substituted by 1 to 3 hydroxy groups,
(ii) a carbamoyl group optionally mono- or di-substituted by C₁₋₆ alkyl group (s) (e.g., methyl), and
(iii) a 3- to 8-membered monocyclic non-aromatic heterocyclic group (e.g., morpholinyl).

In another embodiment, R⁵ is particularly preferably a 5-or 6-membered monocyclic aromatic heterocyclic group (e.g., pyrazolyl) optionally substituted by 1 to 3 substituents selected from
(i) a C₁₋₆ alkyl group (e.g., methyl),
(ii) a carbamoyl group optionally mono- or di-substituted by C₁₋₆ alkyl group (s) (e.g., methyl),
(iii) a C₁₋₆ alkoxy-carbonyl group (e.g., tert-butoxycarbonyl), and
(iv) a carboxy group.

Moreover, in another embodiment, R⁵ is preferably
(1) a hydrogen atom,
(2) an optionally substituted C₃₋₁₀ cycloalkyl-carbonyl group,
(3) an optionally substituted C₆₋₁₄ aryl group,
(4) an optionally substituted 5- to 14-membered aromatic heterocyclic group (preferably a 5- or 6-membered monocyclic aromatic heterocyclic group),
(5) an optionally substituted C₁₋₆ alkyl-carbonyl group,
(6) an optionally substituted C₆₋₁₄ aryl-carbonyl group,
(7) an optionally substituted 5- or 6-membered aromatic heterocyclylcarbonyl group,
(8) an optionally substituted C₁₋₆ alkyl-carbamoyl group,
(9) an optionally substituted C₆₋₁₄ aryl-carbamoyl group,
(10) an optionally substituted C₃₋₁₀ cycloalkyl-carbamoyl group, or
(11) an optionally substituted 3- to 8-membered monocyclic non-aromatic heterocyclylcarbamoyl group.

Specifically, R⁵ is preferably
(1) a hydrogen atom,
(2) a C₃₋₁₀ cycloalkyl-carbonyl group (e.g., cyclopropylcarbonyl),
(3) a C₆₋₁₄ aryl group (e.g., phenyl),
(4) a 5- to 14-membered aromatic heterocyclic group (e.g., pyridyl, pyrimidinyl, pyrazinyl, pyridazinyl, pyrazolyl, imidazolyl, isoxazolyl, quinolyl, isoquinolyl, pyrrolo[3,2-c]pyridyl, [1,2,4]triazolo[4,3-c]pyrimidinyl, pyrrolo[3,2-c]pyridyl) (preferably a 5- or 6-membered monocyclic aromatic heterocyclic group) optionally substituted by 1 to 3 substituents selected from
   (a) a halogen atom (e.g., a fluorine atom, a chlorine atom),
   (b) a C₁₋₆ alkyl group (e.g., methyl, ethyl, isopropyl, tert-butyl) optionally substituted by 1 to 3 substituents selected from
      (i) a hydroxy group,
      (ii) a halogen atom (e.g., a fluorine atom),
      (iii) a cyano group,
      (iv) a carboxy group,
      (v) a C₁₋₆ alkoxy group (e.g., methoxy),
      (vi) a C₁₋₆ alkoxy-carbonyl group (e.g., tert-butoxycarbonyl),
      (vii) an amino group optionally mono- or di-substituted by substituent(s) selected from a C₁₋₆ alkyl group (e.g., methyl), a C₁₋₆ alkyl-carbonyl group (e.g., acetyl) and a C₁₋₆ alkylsulfonyl group (e.g., methylsulfonyl),
      (viii) a carbamoyl group optionally mono- or di-substituted by substituent(s) selected from
         (I) a C₁₋₆ alkyl group (e.g., methyl, ethyl) optionally substituted by 1 to 3 substituents selected from a hydroxy group and a halogen atom (e.g., a fluorine atom), and
         (II) a 3- to 8-membered monocyclic non-aromatic heterocyclic group (e.g., oxetanyl),
      (ix) a 3- to 8-membered monocyclic non-aromatic heterocyclic group (e.g., morpholinyl, piperidyl) optionally substituted by 1 to 3 substituents selected from a hydroxy group and a C₁₋₆ alkyl group (e.g., methyl), and
      (x) a 3- to 8-membered monocyclic non-aromatic heterocyclylcarbonyl group (e.g., morpholinylcarbonyl),
   (c) a C₃₋₁₀ cycloalkyl group (e.g., cyclopropyl, cyclobutyl, cyclopentyl, cyclohexyl) optionally substituted by 1 to 3 substituents selected from
      (i) a hydroxy group,
      (ii) a halogen atom (e.g., a fluorine atom),
      (iii) a cyano group,
      (iv) a 3- to 8-membered monocyclic non-aromatic heterocyclylcarbonyl group (e.g., azetidinylcarbonyl, pyrrolidinylcarbonyl, 3-oxa-8-azabicyclo[3.2.1]octyl) optionally substituted by 1 to 3 substituents selected from a halogen atom (e.g., a fluorine atom) and a hydroxy group, and
      (v) a carbamoyl group optionally mono- or di-substituted by substituent(s) selected from
         (I) a C₁₋₆ alkyl group (e.g., methyl, ethyl) optionally substituted by 1 to 3 substituents selected from a halogen atom (e.g., a fluorine atom) and a C₁₋₆ alkoxy group (e.g., methoxy),
         (II) a C₃₋₁₀ cycloalkyl group (e.g., cyclopropyl), and
         (III) a 3- to 8-membered monocyclic non-aromatic heterocyclic group (e.g., oxetanyl),
   (d) a C₁₋₆ alkoxy group (e.g., methoxy, ethoxy, isopropoxy, isopentyloxy, tert-butoxy) optionally substituted by 1 to 3 substituents selected from
      (i) a hydroxy group,
      (ii) a C₁₋₆ alkoxy group (e.g., methoxy),
      (iii) a carbamoyl group optionally mono- or di-substituted by C₁₋₆ alkyl group(s) (e.g., methyl), and
      (iv) a C₆₋₁₄ aryl group (e.g., phenyl),
   (e) a C₆₋₁₄ aryl group (e.g., phenyl),
   (f) a C₁₋₆ alkyl-carbonyl group (e.g., acetyl),
   (g) a C₁₋₆ alkoxy-carbonyl group (e.g., methoxycarbonyl, tert-butoxycarbonyl),
   (h) a carbamoyl group optionally mono- or di-substituted by substituent(s) selected from
      (i) a C₁₋₆ alkyl group (e.g., methyl, ethyl, isobutyl, tert-butyl) optionally substituted by 1 to 3 substituents selected from
         (I) a hydroxy group,
         (II) a cyano group,
         (III) a halogen atom (e.g., a fluorine atom),
         (IV) a C₁₋₆ alkoxy group (e.g., methoxy),
         (V) a C₃₋₁₀ cycloalkyl group (e.g., cyclopropyl),
         (VI) a 3- to 8-membered monocyclic non-aromatic heterocyclic group (e.g., oxetanyl, tetrahydrofuryl), and
         (VII) an amino group optionally mono- or di-substituted by C₁₋₆ alkyl group (s) (e.g., methyl),
      (ii) a 3- to 8-membered monocyclic non-aromatic heterocyclic group (e.g., tetrahydropyranyl, tetrahydrofuryl, oxetanyl, 1,1-dioxidotetrahydrothiopyranyl, pyrrolidinyl) optionally substituted by 1 to 3 C₁₋₆ alkyl groups (e.g., methyl),
      (iii) a C₃₋₁₀ cycloalkyl group (e.g., cyclopropyl, cyclobutyl, cyclohexyl) optionally substituted by 1 to 3 substituents selected from
         (I) a halogen atom (e.g., a fluorine atom),
         (II) a cyano group, and
         (III) an amino group optionally mono- or di-substituted by C₁₋₆ alkyl group(s) (e.g., methyl), and
      (iv) a 5- or 6-membered monocyclic aromatic heterocyclic group (e.g., pyrazolyl) optionally substituted by 1 to 3 C₁₋₆ alkyl groups (e.g., methyl),
   (i) a sulfamoyl group optionally mono- or di-substituted by C₃₋₁₀ cycloalkyl group (s) (e.g., cyclopropyl),
   (j) an amino group optionally mono- or di-substituted by substituent(s) selected from
      (i) a C₁₋₆ alkyl-carbonyl group (e.g., acetyl, propionyl) optionally substituted by 1 to 3 substituents selected from a halogen atom (e.g., a fluorine atom), a hydroxy group, a cyano group and a C₁₋₆ alkoxy group (e.g., methoxy),
      (ii) a C₁₋₆ alkoxy-carbonyl group (e.g., methoxycarbonyl, tert-butoxycarbonyl),
      (iii) a C₃₋₁₀ cycloalkyl-carbonyl group (e.g., cyclopropylcarbonyl),
      (iv) a C₁₋₆ alkyl group (e.g., methyl),
      (v) a 3- to 8-membered monocyclic non-aromatic heterocyclic group (e.g., tetrahydropyranyl), and
      (vi) a C₃₋₁₀ cycloalkyl group (e.g., cyclopropyl, cyclohexyl) optionally substituted by amino group(s) optionally mono- or di-substituted by substituent(s) selected from
         (I) a C₁₋₆ alkyl group (e.g., methyl), and
         (II) a C₁₋₆ alkoxy-carbonyl group (e.g., tert-butoxycarbonyl),
   (k) a cyano group,
   (l) a 3- to 8-membered monocyclic non-aromatic heterocyclic group (e.g., morpholinyl, 1,1-dioxidothiomorpholinyl, dihydropyranyl, tetrahydropyranyl, tetrahydropyridyl, piperidyl, piperazinyl, pyrrolidinyl, azetidinyl, oxadiazolinyl, oxazepanyl, 8-oxa-3-azabicyclo[3.2.1]octyl, 6-oxa-3-azabicyclo[3.1.1]heptyl, 3-oxa-8-azabicyclo[3.2.1]octyl, diazapane, 2-oxa-6-azaspiro[3.5]nonyl, 2-oxa-7-azaspiro[4.5]decyl) optionally substituted by 1 to 3 substituents selected from
      (i) an oxo group,
      (ii) a cyano group,
      (iii) a hydroxy group,
      (iv) a halogen atom (e.g., a fluorine atom),
      (v) a C₁₋₆ alkyl group (e.g., methyl, ethyl) optionally substituted by 1 to 3 substituents selected from a hydroxy group and a halogen atom (e.g., a fluorine atom),
      (vi) a C₁₋₆ alkoxy group (e.g., methoxy),
      (vii) a C₃₋₁₀ cycloalkyl group (e.g., cyclopropyl),
      (viii) a C₁₋₆ alkyl-carbonyl group (e.g., acetyl, propionyl) optionally substituted by 1 to 3 halogen atoms (e.g., a fluorine atom),
      (ix) a C₁₋₆ alkoxy-carbonyl group (e.g., tert-butoxycarbonyl),
      (x) a carbamoyl group optionally mono- or di-substituted by C₁₋₆ alkyl group (s) (e.g., methyl),
      (xi) a C₁₋₆ alkylsulfonyl group (e.g., methylsulfonyl, isopropylsulfonyl),
      (xii) a C₃₋₁₀ cycloalkyl-carbonyl group (e.g., optionally substituted by 1 to 3 halogen atoms (e.g., a fluorine atom),
      (xiii) a 5- or 6-membered monocyclic aromatic heterocyclic group (e.g., pyrimidinyl), and
      (xiv) an amino group optionally mono- or di-substituted by C₁₋₆ alkyl group (s) (e.g., methyl),
   (m) a 5- or 6-membered monocyclic aromatic heterocyclic group (e.g., pyrazolyl, isoxazolyl) optionally substituted by 1 to 3 C₁₋₆ alkyl groups (e.g., methyl),
   (n) a 3- to 8-membered monocyclic non-aromatic heterocyclyloxy group (e.g., tetrahydropyranyloxy),
   (o) a 3- to 8-membered monocyclic non-aromatic heterocyclylcarbonyl group (e.g., morpholinylcarbonyl, piperidylcarbonyl, piperazinylcarbonyl, pyrrolidinylcarbonyl, oxazepanylcarbonyl, azetidinylcarbonyl, 6-oxa-3-azabicyclo[3.1.1]heptylcarbonyl, 3-oxa-8-azabicyclo[3.2.1]octylcarbonyl, 8-oxa-3-azabicyclo[3.2.1]octylcarbonyl, 2-oxa-6-azaspiro[3.4]octylcarbonyl, 3-azabicyclo[3.1.0]hexylcarbonyl, 1,3-dihydro-2H-pyrrolo[3,4-c]pyridylcarbonyl, 5,7-dihydro-6H-pyrrolo[3,4-b]pyridylcarbonyl, 5,7-dihydro-6H-pyrrolo[3,4-d]pyrimidinylcarbonyl) optionally substituted by 1 to 3 substituents selected from
      (i) a halogen atom (e.g., a fluorine atom),
      (ii) a C₁₋₆ alkyl group (e.g., methyl) optionally substituted by 1 to 3 halogen atoms (e.g., a fluorine atom),
      (iii) a hydroxy group, and
      (iv) a C₁₋₆ alkoxy group (e.g., methoxy), and
   (p) a carboxy group,
(5) a C₁₋₆ alkyl-carbonyl group (e.g., acetyl, propionyl) optionally substituted by 1 to 3 substituents selected from
   (a) a C₆₋₁₄ aryl group (e.g., phenyl), and
   (b) a 3- to 8-membered monocyclic non-aromatic heterocyclic group (e.g., morpholinyl),
(6) a C₆₋₁₄ aryl-carbonyl group (e.g., benzoyl) optionally substituted by 1 to 3 halogen atoms (e.g., a fluorine atom),
(7) a 5- or 6-membered aromatic heterocyclylcarbonyl group (e.g., pyrazolylcarbonyl) optionally substituted by 1 to 3 C₁₋₆ alkyl groups (e.g., methyl),
(8) a C₁₋₆ alkyl-carbamoyl group (e.g., methylcarbamoyl, ethylcarbamoyl) optionally mono- or di-substituted by substituent(s) selected from
   (a) a C₆₋₁₄ aryl group (e.g., phenyl) optionally substituted by 1 to 3 hydroxy groups,
   (b) a 5- to 14-membered aromatic heterocyclic group (e.g., pyridyl, imidazolyl, benzofuryl, benzoxazolyl, benzimidazolyl), and
   (c) a 3- to 8-membered monocyclic non-aromatic heterocyclic group (e.g., piperazinyl) optionally substituted by 1 to 3 oxo groups,
(9) a C₆₋₁₄ aryl-carbamoyl group (e.g., phenylcarbamoyl),
(10) a C₃₋₁₀ cycloalkyl-carbamoyl group (e.g., cyclohexylcarbamoyl), or
(11) a 3- to 8-membered monocyclic non-aromatic heterocyclylcarbamoyl group (e.g., tetrahydropyranylcarbamoyl).

Preferable examples of compound (I) include the following compounds.

### [Compound A1-1]

Compound (I) wherein
Ring A is an optionally further substituted pyrrolidine ring;
R¹ is an optionally substituted C₁₋₆ alkyl group or an optionally substituted C₃₋₁₀ cycloalkyl group;
R² is a hydrogen atom;
R³ is a hydrogen atom;
R⁴ is a hydrogen atom; and
R⁵ is
(1) a hydrogen atom,
(2) an optionally substituted C₃₋₁₀ cycloalkyl-carbonyl group,
(3) an optionally substituted C₆₋₁₄ aryl group, or
(4) an optionally substituted 5- to 14-membered aromatic heterocyclic group (preferably a 5- or 6-membered monocyclic aromatic heterocyclic group).

### [Compound A1a-1]

Compound (I) wherein
Ring A is a pyrrolidine ring wherein the 4-position is not substituted, and the 5-position is optionally further substituted by 1 or 2 (preferably 1) C₁₋₆ alkyl group(s) (e.g., methyl);
R¹ is
(1) a C₁₋₆ alkyl group (e.g., ethyl, isopropyl), or
(2) a C₃₋₁₀ cycloalkyl group (e.g., cyclopropyl) ;
   R² is a hydrogen atom;
   R³ is a hydrogen atom;
   R⁴ is a hydrogen atom; and
   R⁵ is
   (1) a hydrogen atom,
   (2) a C₃₋₁₀ cycloalkyl-carbonyl group (e.g., cyclopropylcarbonyl),
   (3) a C₆₋₁₄ aryl group (e.g., phenyl), or
   (4) a 5- to 14-membered aromatic heterocyclic group (e.g., pyridyl, pyrimidinyl, pyrazinyl, pyridazinyl, pyrazolyl, imidazolyl, isoxazolyl, quinolyl, isoquinolyl) (preferably a 5- or 6-membered monocyclic aromatic heterocyclic group) optionally substituted by 1 to 3 substituents selected from
      (a) a halogen atom (e.g., a fluorine atom),
      (b) a C₁₋₆ alkyl group (e.g., methyl, ethyl, isopropyl, tert-butyl) optionally substituted by 1 to 3 substituents selected from
         (i) a hydroxy group,
         (ii) a halogen atom (e.g., a fluorine atom),
         (iii) a cyano group,
         (iv) a carboxy group,
         (v) a C₁₋₆ alkoxy group (e.g., methoxy),
         (vi) a C₁₋₆ alkoxy-carbonyl group (e.g., tert-butoxycarbonyl),
         (vii) an amino group optionally mono- or di-substituted by substituent(s) selected from a C₁₋₆ alkyl group (e.g., methyl), a C₁₋₆ alkyl-carbonyl group (e.g., acetyl) and a C₁₋₆ alkylsulfonyl group (e.g., methylsulfonyl),
         (viii) a carbamoyl group optionally mono- or di-substituted by C₁₋₆ alkyl group(s) (e.g., methyl), and
         (ix) a 3- to 8-membered monocyclic non-aromatic heterocyclic group (e.g., morpholinyl),
      (c) a C₃₋₁₀ cycloalkyl group (e.g., cyclopropyl, cyclobutyl, cyclohexyl) optionally substituted by 1 to 3 substituents selected from
         (i) a hydroxy group,
         (ii) a halogen atom (e.g., a fluorine atom), and
         (iii) a cyano group,
      (d) a C₁₋₆ alkoxy group (e.g., methoxy, ethoxy, isopentyloxy) optionally substituted by 1 to 3 substituents selected from
         (i) a hydroxy group,
         (ii) a C₁₋₆ alkoxy group (e.g., methoxy), and
         (iii) a carbamoyl group optionally mono- or di-substituted by C₁₋₆ alkyl group(s) (e.g., methyl),
      (e) a C₆₋₁₄ aryl group (e.g., phenyl),
      (f) a C₁₋₆ alkoxy-carbonyl group (e.g., methoxycarbonyl, tert-butoxycarbonyl),
      (g) a carbamoyl group optionally mono- or di-substituted by substituent(s) selected from
         (i) a C₁₋₆ alkyl group (e.g., methyl) optionally substituted by 1 to 3 hydroxy groups, and
         (ii) a 3- to 8-membered monocyclic non-aromatic heterocyclic group (e.g., tetrahydropyranyl),
      (h) a sulfamoyl group optionally mono- or di-substituted by C₃₋₁₀ cycloalkyl group (s) (e.g., cyclopropyl),
      (i) an amino group optionally mono- or di-substituted by C₁₋₆ alkyl-carbonyl group(s) (e.g., acetyl),
      (j) a cyano group,
      (k) a 3- to 8-membered monocyclic non-aromatic heterocyclic group (e.g., morpholinyl, 1,1-dioxidothiomorpholinyl, dihydropyranyl, tetrahydropyranyl, tetrahydropyridyl, piperidyl, piperazinyl, pyrrolidinyl, azetidinyl) optionally substituted by 1 to 3 substituents selected from
         (i) an oxo group,
         (ii) a cyano group,
         (iii) a hydroxy group,
         (iv) a halogen atom (e.g., a fluorine atom),
         (v) a C₁₋₆ alkyl group (e.g., methyl) optionally substituted by 1 to 3 substituents selected from a hydroxy group and a halogen atom (e.g., a fluorine atom),
         (vi) a C₁₋₆ alkoxy group (e.g., methoxy),
         (vii) a C₃₋₁₀ cycloalkyl group (e.g., cyclopropyl),
         (viii) a C₁₋₆ alkyl-carbonyl group (e.g., acetyl),
         (ix) a C₁₋₆ alkoxy-carbonyl group (e.g., tert-butoxycarbonyl),
         (x) a carbamoyl group, and
         (xi) a C₁₋₆ alkylsulfonyl group (e.g., isopropylsulfonyl),
      (l) a 3- to 8-membered monocyclic non-aromatic heterocyclyloxy group (e.g., tetrahydropyranyloxy),
      (m) a 3- to 8-membered monocyclic non-aromatic heterocyclylcarbonyl group (e.g., morpholinylcarbonyl, piperidylcarbonyl) optionally substituted by 1 to 3 substituents selected from
         (i) a halogen atom (e.g., a fluorine atom), and
         (ii) a C₁₋₆ alkyl group (e.g., methyl), and
      (n) a carboxy group.

### [Compound A1a-2]

Compound (I) wherein
Ring A is a pyrrolidine ring wherein the 4-position is not substituted, and the 5-position is optionally further substituted by 1 or 2 (preferably 1) C₁₋₆ alkyl group(s) (e.g., methyl);
R¹ is
(1) a C₁₋₆ alkyl group (e.g., ethyl, isopropyl), or
(2) a C₃₋₁₀ cycloalkyl group (e.g., cyclopropyl);
R² is a hydrogen atom;
R³ is a hydrogen atom;
R⁴ is a hydrogen atom; and
R⁵ is
(1) a hydrogen atom,
(2) a C₃₋₁₀ cycloalkyl-carbonyl group (e.g., cyclopropylcarbonyl),
(3) a C₆₋₁₄ aryl group (e.g., phenyl), or
(4) a 5- to 14-membered aromatic heterocyclic group (e.g., pyridyl, pyrimidinyl, pyrazinyl, pyridazinyl, pyrazolyl, imidazolyl, isoxazolyl, quinolyl, isoquinolyl, pyrrolo[3,2-c]pyridyl, [1,2,4]triazolo[4,3-c]pyrimidinyl, pyrrolo[3,2-c]pyridyl) (preferably a 5- or 6-membered monocyclic aromatic heterocyclic group) optionally substituted by 1 to 3 substituents selected from
   (a) a halogen atom (e.g., a fluorine atom, a chlorine atom),
   (b) a C₁₋₆ alkyl group (e.g., methyl, ethyl, isopropyl, tert-butyl) optionally substituted by 1 to 3 substituents selected from
      (i) a hydroxy group,
      (ii) a halogen atom (e.g., a fluorine atom),
      (iii) a cyano group,
      (iv) a carboxy group,
      (v) a C₁₋₆ alkoxy group (e.g., methoxy),
      (vi) a C₁₋₆ alkoxy-carbonyl group (e.g., tert-butoxycarbonyl),
      (vii) an amino group optionally mono- or di-substituted by substituent(s) selected from a C₁₋₆ alkyl group (e.g., methyl), a C₁₋₆ alkyl-carbonyl group (e.g., acetyl) and a C₁₋₆ alkylsulfonyl group (e.g., methylsulfonyl),
      (viii) a carbamoyl group optionally mono- or di-substituted by substituent(s) selected from
         (I) a C₁₋₆ alkyl group (e.g., methyl, ethyl) optionally substituted by 1 to 3 substituents selected from a hydroxy group and a halogen atom (e.g., a fluorine atom), and
         (II) a 3- to 8-membered monocyclic non-aromatic heterocyclic group (e.g., oxetanyl),
      (ix) a 3- to 8-membered monocyclic non-aromatic heterocyclic group (e.g., morpholinyl, piperidyl) optionally substituted by 1 to 3 substituents selected from a hydroxy group and a C₁₋₆ alkyl group (e.g., methyl), and
      (x) a 3- to 8-membered monocyclic non-aromatic heterocyclylcarbonyl group (e.g., morpholinylcarbonyl),
   (c) a C₃₋₁₀ cycloalkyl group (e.g., cyclopropyl, cyclobutyl, cyclopentyl, cyclohexyl) optionally substituted by 1 to 3 substituents selected from
      (i) a hydroxy group,
      (ii) a halogen atom (e.g., a fluorine atom),
      (iii) a cyano group,
      (iv) a 3- to 8-membered monocyclic non-aromatic heterocyclylcarbonyl group (e.g., azetidinylcarbonyl, pyrrolidinylcarbonyl, 3-oxa-8-azabicyclo[3.2.1]octyl) optionally substituted by 1 to 3 substituents selected from a halogen atom (e.g., a fluorine atom) and a hydroxy group, and
      (v) a carbamoyl group optionally mono- or di-substituted by substituent(s) selected from
         (I) a C₁₋₆ alkyl group (e.g., methyl, ethyl) optionally substituted by 1 to 3 substituents selected from a halogen atom (e.g., a fluorine atom) and a C₁₋₆ alkoxy group (e.g., methoxy),
         (II) a C₃₋₁₀ cycloalkyl group (e.g., cyclopropyl), and
         (III) a 3- to 8-membered monocyclic non-aromatic heterocyclic group (e.g., oxetanyl),
   (d) a C₁₋₆ alkoxy group (e.g., methoxy, ethoxy, isopentyloxy) optionally substituted by 1 to 3 substituents selected from
      (i) a hydroxy group,
      (ii) a C₁₋₆ alkoxy group (e.g., methoxy), and
      (iii) a carbamoyl group optionally mono- or di-substituted by C₁₋₆ alkyl group (s) (e.g., methyl),
   (e) a C₆₋₁₄ aryl group (e.g., phenyl),
   (f) a C₁₋₆ alkoxy-carbonyl group (e.g., methoxycarbonyl, tert-butoxycarbonyl),
   (g) a carbamoyl group optionally mono- or di-substituted by substituent(s) selected from
      (i) a C₁₋₆ alkyl group (e.g., methyl, ethyl, isobutyl, tert-butyl) optionally substituted by 1 to 3 substituents selected from
         (I) a hydroxy group,
         (II) a cyano group,
         (III) a halogen atom (e.g., a fluorine atom),
         (IV) a C₁₋₆ alkoxy group (e.g., methoxy),
         (V) a C₃₋₁₀ cycloalkyl group (e.g., cyclopropyl), and
         (VI) a 3- to 8-membered monocyclic non-aromatic heterocyclic group (e.g., oxetanyl, tetrahydrofuryl),
      (ii) a 3- to 8-membered monocyclic non-aromatic heterocyclic group (e.g., tetrahydropyranyl, tetrahydrofuryl, oxetanyl, 1,1-dioxidotetrahydrothiopyranyl) optionally substituted by 1 to 3 C₁₋₆ alkyl groups (e.g., methyl), and
      (iii) a C₃₋₁₀ cycloalkyl group (e.g., cyclopropyl, cyclobutyl) optionally substituted by 1 to 3 substituents selected from a halogen atom (e.g., a fluorine atom) and a cyano group,
   (h) a sulfamoyl group optionally mono- or di-substituted by C₃₋₁₀ cycloalkyl group (s) (e.g., cyclopropyl),
   (i) an amino group optionally mono- or di-substituted by substituent(s) selected from
      (i) a C₁₋₆ alkyl-carbonyl group (e.g., acetyl, propionyl) optionally substituted by 1 to 3 substituents selected from a halogen atom (e.g., a fluorine atom), a hydroxy group, a cyano group and a C₁₋₆ alkoxy group (e.g., methoxy),
      (ii) a C₁₋₆ alkoxy-carbonyl group (e.g., methoxycarbonyl, tert-butoxycarbonyl), and
      (iii) a C₃₋₁₀ cycloalkyl-carbonyl group (e.g., cyclopropylcarbonyl),
   (j) a cyano group,
   (k) a 3- to 8-membered monocyclic non-aromatic heterocyclic group (e.g., morpholinyl, 1,1-dioxidothiomorpholinyl, dihydropyranyl, tetrahydropyranyl, tetrahydropyridyl, piperidyl, piperazinyl, pyrrolidinyl, azetidinyl, oxadiazolinyl, oxazepanyl, 8-oxa-3-azabicyclo[3.2.1]octyl, 6-oxa-3-azabicyclo[3.1.1]heptyl, 3-oxa-8-azabicyclo[3.2.1]octyl) optionally substituted by 1 to 3 substituents selected from
      (i) an oxo group,
      (ii) a cyano group,
      (iii) a hydroxy group,
      (iv) a halogen atom (e.g., a fluorine atom),
      (v) a C₁₋₆ alkyl group (e.g., methyl) optionally substituted by 1 to 3 substituents selected from a hydroxy group and a halogen atom (e.g., a fluorine atom),
      (vi) a C₁₋₆ alkoxy group (e.g., methoxy),
      (vii) a C₃₋₁₀ cycloalkyl group (e.g., cyclopropyl),
      (viii) a C₁₋₆ alkyl-carbonyl group (e.g., acetyl, propionyl) optionally substituted by 1 to 3 halogen atoms (e.g., a fluorine atom),
      (ix) a C₁₋₆ alkoxy-carbonyl group (e.g., tert-butoxycarbonyl),
      (x) a carbamoyl group optionally mono- or di-substituted by C₁₋₆ alkyl group (s) (e.g., methyl),
      (xi) a C₁₋₆ alkylsulfonyl group (e.g., methylsulfonyl, isopropylsulfonyl), and
      (xii) a C₃₋₁₀ cycloalkyl-carbonyl group (e.g., optionally substituted by 1 to 3 halogen atoms (e.g., a fluorine atom),
   (l) a 3- to 8-membered monocyclic non-aromatic heterocyclyloxy group (e.g., tetrahydropyranyloxy),
   (m) a 3- to 8-membered monocyclic non-aromatic heterocyclylcarbonyl group (e.g., morpholinylcarbonyl, piperidylcarbonyl, pyrrolidinylcarbonyl, oxazepanylcarbonyl, azetidinylcarbonyl, 6-oxa-3-azabicyclo[3.1.1]heptylcarbonyl, 3-oxa-8-azabicyclo[3.2.1]octylcarbonyl, 8-oxa-3-azabicyclo[3.2.1]octylcarbonyl, 2-oxa-6-azaspiro[3.4]octylcarbonyl, 3-azabicyclo[3.1.0]hexylcarbonyl, 1,3-dihydro-2H-pyrrolo[3,4-c]pyridylcarbonyl, 5,7-dihydro-6H-pyrrolo[3,4-b]pyridylcarbonyl, 5,7-dihydro-6H-pyrrolo[3,4-d]pyrimidinylcarbonyl) optionally substituted by 1 to 3 substituents selected from
      (i) a halogen atom (e.g., a fluorine atom),
      (ii) a C₁₋₆ alkyl group (e.g., methyl) optionally substituted by 1 to 3 halogen atoms (e.g., a fluorine atom),
      (iii) a hydroxy group, and
      (iv) a C₁₋₆ alkoxy group (e.g., methoxy), and
   (n) a carboxy group.

### [Compound A2-1]

Compound (I) wherein
Ring A is an optionally further substituted pyrrolidine ring; R¹ is an optionally substituted C₁₋₆ alkyl group or an optionally substituted C₃₋₁₀ cycloalkyl group;
R² and R⁴ are independently a hydrogen atom, a halogen atom or an optionally substituted C₁₋₆ alkyl group;
R³ is a hydrogen atom or an amino group; and
R⁵ is
(1) a hydrogen atom,
(2) an optionally substituted C₃₋₁₀ cycloalkyl-carbonyl group,
(3) an optionally substituted C₆₋₁₄ aryl group,
(4) an optionally substituted 5- to 14-membered aromatic heterocyclic group (preferably a 5- or 6-membered monocyclic aromatic heterocyclic group),
(5) an optionally substituted C₁₋₆ alkyl-carbonyl group,
(6) an optionally substituted C₆₋₁₄ aryl-carbonyl group,
(7) an optionally substituted 5- or 6-membered aromatic heterocyclylcarbonyl group,
(8) an optionally substituted C₁₋₆ alkyl-carbamoyl group,
(9) an optionally substituted C₆₋₁₄ aryl-carbamoyl group,
(10) an optionally substituted C₃₋₁₀ cycloalkyl-carbamoyl group, or
(11) an optionally substituted 3- to 8-membered monocyclic non-aromatic heterocyclylcarbamoyl group.

### [Compound A2a-1]

Compound (I) wherein
Ring A is a pyrrolidine ring wherein the 4-position is not substituted, and the 5-position is optionally further substituted by 1 or 2 (preferably 1) C₁₋₆ alkyl group(s) (e.g., methyl);
R¹ is
(1) a C₁₋₆ alkyl group (e.g., ethyl, isopropyl), or
(2) a C₃₋₁₀ cycloalkyl group (e.g., cyclopropyl) ;
R² and R⁴ are independently
(1) a hydrogen atom,
(2) a halogen atom (e.g., a fluorine atom, a chlorine atom), or
(3) a C₁₋₆ alkyl group (e.g., methyl);
R³ is a hydrogen atom or an amino group; and
R⁵ is
(1) a hydrogen atom,
(2) a C₃₋₁₀ cycloalkyl-carbonyl group (e.g., cyclopropylcarbonyl),
(3) a C₆₋₁₄ aryl group (e.g., phenyl),
(4) a 5- to 14-membered aromatic heterocyclic group (e.g., pyridyl, pyrimidinyl, pyrazinyl, pyridazinyl, pyrazolyl, imidazolyl, isoxazolyl, quinolyl, isoquinolyl, pyrrolo[3,2-c]pyridyl, [1,2,4]triazolo[4,3-c]pyrimidinyl, pyrrolo[3,2-c]pyridyl) (preferably a 5- or 6-membered monocyclic aromatic heterocyclic group) optionally substituted by 1 to 3 substituents selected from
   (a) a halogen atom (e.g., a fluorine atom, a chlorine atom),
   (b) a C₁₋₆ alkyl group (e.g., methyl, ethyl, isopropyl, tert-butyl) optionally substituted by 1 to 3 substituents selected from
      (i) a hydroxy group,
      (ii) a halogen atom (e.g., a fluorine atom),
      (iii) a cyano group,
      (iv) a carboxy group,
      (v) a C₁₋₆ alkoxy group (e.g., methoxy),
      (vi) a C₁₋₆ alkoxy-carbonyl group (e.g., tert-butoxycarbonyl),
      (vii) an amino group optionally mono- or di-substituted by substituent(s) selected from a C₁₋₆ alkyl group (e.g., methyl), a C₁₋₆ alkyl-carbonyl group (e.g., acetyl) and a C₁₋₆ alkylsulfonyl group (e.g., methylsulfonyl),
      (viii) a carbamoyl group optionally mono- or di-substituted by substituent(s) selected from
         (I) a C₁₋₆ alkyl group (e.g., methyl, ethyl) optionally substituted by 1 to 3 substituents selected from a hydroxy group and a halogen atom (e.g., a fluorine atom), and
         (II) a 3- to 8-membered monocyclic non-aromatic heterocyclic group (e.g., oxetanyl),
      (ix) a 3- to 8-membered monocyclic non-aromatic heterocyclic group (e.g., morpholinyl, piperidyl) optionally substituted by 1 to 3 substituents selected from a hydroxy group and a C₁₋₆ alkyl group (e.g., methyl), and
      (x) a 3- to 8-membered monocyclic non-aromatic heterocyclylcarbonyl group (e.g., morpholinylcarbonyl),
   (c) a C₃₋₁₀ cycloalkyl group (e.g., cyclopropyl, cyclobutyl, cyclopentyl, cyclohexyl) optionally substituted by 1 to 3 substituents selected from
      (i) a hydroxy group,
      (ii) a halogen atom (e.g., a fluorine atom),
      (iii) a cyano group,
      (iv) a 3- to 8-membered monocyclic non-aromatic heterocyclylcarbonyl group (e.g., azetidinylcarbonyl, pyrrolidinylcarbonyl, 3-oxa-8-azabicyclo[3.2.1]octyl) optionally substituted by 1 to 3 substituents selected from a halogen atom (e.g., a fluorine atom) and a hydroxy group, and
      (v) a carbamoyl group optionally mono- or di-substituted by substituent(s) selected from
         (I) a C₁₋₆ alkyl group (e.g., methyl, ethyl) optionally substituted by 1 to 3 substituents selected from a halogen atom (e.g., a fluorine atom) and a C₁₋₆ alkoxy group (e.g., methoxy),
         (II) a C₃₋₁₀ cycloalkyl group (e.g., cyclopropyl), and
         (III) a 3- to 8-membered monocyclic non-aromatic heterocyclic group (e.g., oxetanyl),
   (d) a C₁₋₆ alkoxy group (e.g., methoxy, ethoxy, isopropoxy, isopentyloxy, tert-butoxy) optionally substituted by 1 to 3 substituents selected from
      (i) a hydroxy group,
      (ii) a C₁₋₆ alkoxy group (e.g., methoxy),
      (iii) a carbamoyl group optionally mono- or di-substituted by C₁₋₆ alkyl group(s) (e.g., methyl), and
      (iv) a C₆₋₁₄ aryl group (e.g., phenyl),
   (e) a C₆₋₁₄ aryl group (e.g., phenyl),
   (f) a C₁₋₆ alkyl-carbonyl group (e.g., acetyl),
   (g) a C₁₋₆ alkoxy-carbonyl group (e.g., methoxycarbonyl, tert-butoxycarbonyl),
   (h) a carbamoyl group optionally mono- or di-substituted by substituent(s) selected from
      (i) a C₁₋₆ alkyl group (e.g., methyl, ethyl, isobutyl, tert-butyl) optionally substituted by 1 to 3 substituents selected from
         (I) a hydroxy group,
         (II) a cyano group,
         (III) a halogen atom (e.g., a fluorine atom),
         (IV) a C₁₋₆ alkoxy group (e.g., methoxy),
         (V) a C₃₋₁₀ cycloalkyl group (e.g., cyclopropyl),
         (VI) a 3- to 8-membered monocyclic non-aromatic heterocyclic group (e.g., oxetanyl, tetrahydrofuryl), and
         (VII) an amino group optionally mono- or di-substituted by C₁₋₆ alkyl group(s) (e.g., methyl),
      (ii) a 3- to 8-membered monocyclic non-aromatic heterocyclic group (e.g., tetrahydropyranyl, tetrahydrofuryl, oxetanyl, 1,1-dioxidotetrahydrothiopyranyl, pyrrolidinyl) optionally substituted by 1 to 3 C₁₋₆ alkyl groups (e.g., methyl),
      (iii) a C₃₋₁₀ cycloalkyl group (e.g., cyclopropyl, cyclobutyl, cyclohexyl) optionally substituted by 1 to 3 substituents selected from
         (I) a halogen atom (e.g., a fluorine atom),
         (II) a cyano group, and
         (III) an amino group optionally mono- or di-substituted by C₁₋₆ alkyl group(s) (e.g., methyl), and
      (iv) a 5- or 6-membered monocyclic aromatic heterocyclic group (e.g., pyrazolyl) optionally substituted by 1 to 3 C₁₋₆ alkyl groups (e.g., methyl),
   (i) a sulfamoyl group optionally mono- or di-substituted by C₃₋₁₀ cycloalkyl group (s) (e.g., cyclopropyl),
   (j) an amino group optionally mono- or di-substituted by substituent(s) selected from
      (i) a C₁₋₆ alkyl-carbonyl group (e.g., acetyl, propionyl) optionally substituted by 1 to 3 substituents selected from a halogen atom (e.g., a fluorine atom), a hydroxy group, a cyano group and a C₁₋₆ alkoxy group (e.g., methoxy),
      (ii) a C₁₋₆ alkoxy-carbonyl group (e.g., methoxycarbonyl, tert-butoxycarbonyl),
      (iii) a C₃₋₁₀ cycloalkyl-carbonyl group (e.g., cyclopropylcarbonyl),
      (iv) a C₁₋₆ alkyl group (e.g., methyl),
      (v) a 3- to 8-membered monocyclic non-aromatic heterocyclic group (e.g., tetrahydropyranyl), and
      (vi) a C₃₋₁₀ cycloalkyl group (e.g., cyclopropyl, cyclohexyl) optionally substituted by amino group(s) optionally mono- or di-substituted by substituent(s) selected from
         (I) a C₁₋₆ alkyl group (e.g., methyl), and
         (II) a C₁₋₆ alkoxy-carbonyl group (e.g., tert-butoxycarbonyl),
   (k) a cyano group,
   (l) a 3- to 8-membered monocyclic non-aromatic heterocyclic group (e.g., morpholinyl, 1,1-dioxidothiomorpholinyl, dihydropyranyl, tetrahydropyranyl, tetrahydropyridyl, piperidyl, piperazinyl, pyrrolidinyl, azetidinyl, oxadiazolinyl, oxazepanyl, 8-oxa-3-azabicyclo[3.2.1]octyl, 6-oxa-3-azabicyclo[3.1.1]heptyl, 3-oxa-8-azabicyclo[3.2.1]octyl, diazapane, 2-oxa-6-azaspiro[3.5]nonyl, 2-oxa-7-azaspiro[4.5]decyl) optionally substituted by 1 to 3 substituents selected from
      (i) an oxo group,
      (ii) a cyano group,
      (iii) a hydroxy group,
      (iv) a halogen atom (e.g., a fluorine atom),
      (v) a C₁₋₆ alkyl group (e.g., methyl, ethyl) optionally substituted by 1 to 3 substituents selected from a hydroxy group and a halogen atom (e.g., a fluorine atom),
      (vi) a C₁₋₆ alkoxy group (e.g., methoxy),
      (vii) a C₃₋₁₀ cycloalkyl group (e.g., cyclopropyl),
      (viii) a C₁₋₆ alkyl-carbonyl group (e.g., acetyl, propionyl) optionally substituted by 1 to 3 halogen atoms (e.g., a fluorine atom),
      (ix) a C₁₋₆ alkoxy-carbonyl group (e.g., tert-butoxycarbonyl),
      (x) a carbamoyl group optionally mono- or di-substituted by C₁₋₆ alkyl group (s) (e.g., methyl),
      (xi) a C₁₋₆ alkylsulfonyl group (e.g., methylsulfonyl, isopropylsulfonyl),
      (xii) a C₃₋₁₀ cycloalkyl-carbonyl group (e.g., cyclobutylcarbonyl) optionally substituted by 1 to 3 halogen atoms (e.g., a fluorine atom),
      (xiii) a 5- or 6-membered monocyclic aromatic heterocyclic group (e.g., pyrimidinyl), and (xiv) an amino group optionally mono- or di-substituted by C₁₋₆ alkyl group (s) (e.g., methyl),
   (m) a 5- or 6-membered monocyclic aromatic heterocyclic group (e.g., pyrazolyl, isoxazolyl) optionally substituted by 1 to 3 C₁₋₆ alkyl groups (e.g., methyl),
   (n) a 3- to 8-membered monocyclic non-aromatic heterocyclyloxy group (e.g., tetrahydropyranyloxy),
   (o) a 3- to 8-membered monocyclic non-aromatic heterocyclylcarbonyl group (e.g., morpholinylcarbonyl, piperidylcarbonyl, piperazinylcarbonyl, pyrrolidinylcarbonyl, oxazepanylcarbonyl, azetidinylcarbonyl, 6-oxa-3-azabicyclo[3.1.1]heptylcarbonyl, 3-oxa-8-azabicyclo[3.2.1]octylcarbonyl, 8-oxa-3-azabicyclo[3.2.1]octylcarbonyl, 2-oxa-6-azaspiro[3.4]octylcarbonyl, 3-azabicyclo[3.1.0]hexylcarbonyl, 1,3-dihydro-2H-pyrrolo[3,4-c]pyridylcarbonyl, 5,7-dihydro-6H-pyrrolo[3,4-b]pyridylcarbonyl, 5,7-dihydro-6H-pyrrolo[3,4-d]pyrimidinylcarbonyl) optionally substituted by 1 to 3 substituents selected from
      (i) a halogen atom (e.g., a fluorine atom),
      (ii) a C₁₋₆ alkyl group (e.g., methyl) optionally substituted by 1 to 3 halogen atoms (e.g., a fluorine atom),
      (iii) a hydroxy group, and
      (iv) a C₁₋₆ alkoxy group (e.g., methoxy), and
   (p) a carboxy group,
(5) a C₁₋₆ alkyl-carbonyl group (e.g., acetyl, propionyl) optionally substituted by 1 to 3 substituents selected from
   (a) a C₆₋₁₄ aryl group (e.g., phenyl), and
   (b) a 3- to 8-membered monocyclic non-aromatic heterocyclic group (e.g., morpholinyl),
(6) a C₆₋₁₄ aryl-carbonyl group (e.g., benzoyl) optionally substituted by 1 to 3 halogen atoms (e.g., a fluorine atom),
(7) a 5- or 6-membered aromatic heterocyclylcarbonyl group (e.g., pyrazolylcarbonyl) optionally substituted by 1 to 3 C₁₋₆ alkyl groups (e.g., methyl),
(8) a C₁₋₆ alkyl-carbamoyl group (e.g., methylcarbamoyl, ethylcarbamoyl) optionally mono- or di-substituted by substituent(s) selected from
   (a) a C₆₋₁₄ aryl group (e.g., phenyl) optionally substituted by 1 to 3 hydroxy groups,
   (b) a 5- to 14-membered aromatic heterocyclic group (e.g., pyridyl, imidazolyl, benzofuryl, benzoxazolyl, benzimidazolyl), and
   (c) a 3- to 8-membered monocyclic non-aromatic heterocyclic group (e.g., piperazinyl) optionally substituted by 1 to 3 oxo groups,
(9) a C₆₋₁₄ aryl-carbamoyl group (e.g., phenylcarbamoyl),
(10) a C₃₋₁₀ cycloalkyl-carbamoyl group (e.g., cyclohexylcarbamoyl), or
(11) a 3- to 8-membered monocyclic non-aromatic heterocyclylcarbamoyl group (e.g., tetrahydropyranylcarbamoyl).

### [Compound B-1]

Compound (I) wherein
Ring A is a pyrrolidine ring wherein the 4-position is not substituted, and the 5-position is optionally substituted;
R¹ is an optionally substituted C₃₋₁₀ cycloalkyl group;
R² is a hydrogen atom;
R³ is a hydrogen atom;
R⁴ is a hydrogen atom; and
R⁵ is an optionally substituted 5- or 6-membered monocyclic aromatic heterocyclic group.

### [Compound Ba-1]

Compound (I) wherein
Ring A is a pyrrolidine ring wherein the 4-position is not substituted, and the 5-position is optionally further substituted by 1 or 2 (preferably 1) C₁₋₆ alkyl group(s) (e.g., methyl);
R¹ is a C₃₋₁₀ cycloalkyl group (e.g., cyclopropyl);
R² is a hydrogen atom;
R³ is a hydrogen atom;
R⁴ is a hydrogen atom; and
R⁵ is a 5- or 6-membered monocyclic aromatic heterocyclic group (e.g., pyridyl, pyrazolyl) optionally substituted by 1 to 3 substituents selected from
(i) a C₁₋₆ alkyl group (e.g., methyl, isopropyl) optionally substituted by 1 to 3 hydroxy groups,
(ii) a carbamoyl group optionally mono- or di-substituted by C₁₋₆ alkyl group(s) (e.g., methyl),
(iii) a 3- to 8-membered monocyclic non-aromatic heterocyclic group (e.g., morpholinyl),
(iv) a C₁₋₆ alkoxy-carbonyl group (e.g., tert-butoxycarbonyl), and
(v) a carboxy group.

### [Compound C-1]

Compound (I) wherein
Ring A is a pyrrolidine ring wherein the 4-position and the 5-position are not substituted;
R¹ is an optionally substituted C₃₋₁₀ cycloalkyl group;
R² is a hydrogen atom;
R³ is a hydrogen atom;
R⁴ is a hydrogen atom; and
R⁵ is an optionally substituted 5- or 6-membered monocyclic aromatic heterocyclic group.

### [Compound Ca-1]

Compound (I) wherein
Ring A is a pyrrolidine ring wherein the 4-position and the 5-position are not substituted;
R¹ is a C₃₋₁₀ cycloalkyl group (e.g., cyclopropyl);
R² is a hydrogen atom;
R³ is a hydrogen atom;
R⁴ is a hydrogen atom; and
R⁵ is a 5- or 6-membered monocyclic aromatic heterocyclic group (e.g., pyridyl, pyrazolyl) optionally substituted by 1 to 3 substituents selected from
(i) a C₁₋₆ alkyl group (e.g., methyl, isopropyl) optionally substituted by 1 to 3 hydroxy groups,
(ii) a carbamoyl group optionally mono- or di-substituted by C₁₋₆ alkyl group(s) (e.g., methyl), and
(iii) a 3- to 8-membered monocyclic non-aromatic heterocyclic group (e.g., morpholinyl).

### [Compound D-1]

Compound (I) wherein
Ring A is a pyrrolidine ring wherein the 4-position is not substituted, and the 5-position is substituted;
R¹ is an optionally substituted C₃₋₁₀ cycloalkyl group;
R² is a hydrogen atom;
R³ is a hydrogen atom;
R⁴ is a hydrogen atom; and
R⁵ is an optionally substituted 5- or 6-membered monocyclic aromatic heterocyclic group.

### [Compound Da-1]

Compound (I) wherein
Ring A is a pyrrolidine ring wherein the 4-position is not substituted, and the 5-position is further substituted by 1 or 2 (preferably 1) C₁₋₆ alkyl group (s) (e.g., methyl);
R¹ is a C₃₋₁₀ cycloalkyl group (e.g., cyclopropyl);
R² is a hydrogen atom;
R³ is a hydrogen atom;
R⁴ is a hydrogen atom; and
R⁵ is a 5- or 6-membered monocyclic aromatic heterocyclic group (e.g., pyrazolyl) optionally substituted by 1 to 3 substituents selected from
(i) a C₁₋₆ alkyl group (e.g., methyl),
(ii) a carbamoyl group optionally mono- or di-substituted by C₁₋₆ alkyl group(s) (e.g., methyl),
(iii) a C₁₋₆ alkoxy-carbonyl group (e.g., tert-butoxycarbonyl), and
(iv) a carboxy group.

### [Compound E-1]

(3S)-3-cyclopropyl-1-(2-((5-(morpholin-4-yl)pyridin-2-yl)amino)pyridin-4-yl)-2-oxopyrrolidine-3-carbonitrile or a salt thereof;
(3S)-3-cyclopropyl-1-(2-((5-(2-hydroxypropan-2-yl)pyridin-2-yl)amino)pyridin-4-yl)-2-oxopyrrolidine-3-carbonitrile or a salt thereof; or
3-((4-((3S)-3-cyano-3-cyclopropyl-2-oxopyrrolidin-1-yl)pyridin-2-yl)amino)-N,1-dimethyl-1H-pyrazole-5-carboxamide or a salt thereof.

When compound (I) is in a form of a salt, examples thereof include metal salts, an ammonium salt, salts with organic base, salts with inorganic acid, salts with organic acid, salts with basic or acidic amino acid, and the like. Preferable examples of the metal salt include alkali metal salts such as sodium salt, potassium salt and the like; alkaline earth metal salts such as calcium salt, magnesium salt, barium salt and the like; an aluminum salt, and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferable examples of the salt with inorganic acid include salts with hydrochloric acid, hydrobromic acid, nitric acid, sulfuric acid, phosphoric acid and the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of the salt with basic amino acid include salts with arginine, lysine, ornithine and the like. Preferable examples of the salt with acidic amino acid include salts with aspartic acid, glutamic acid and the like.

Among them, a pharmaceutically acceptable salt is preferable. For example, when a compound has an acidic functional group, examples thereof include inorganic salts such as alkali metal salts (e.g., sodium salt, potassium salt etc.), alkaline earth metal salts (e.g., calcium salt, magnesium salt etc.) and the like, ammonium salt etc., and when a compound has a basic functional group, examples thereof include salts with inorganic acid such as hydrochloric acid, hydrobromic acid, nitric acid, sulfuric acid, phosphoric acid and the like, and salts with organic acid such as acetic acid, phthalic acid, fumaric acid, oxalic acid, tartaric acid, maleic acid, citric acid, succinic acid, methanesulfonic acid, benzenesulfonic acid, p-toluenesulfonic acid and the like.

### [Production Method]

The production method of compound (I) or a salt thereof of the present invention are explained in the followings.

The compound (I) and the raw material compounds can be produced according to a method known per se, for example, method shown in the following scheme and the like. In each step in the following production method, the "room temperature" generally means 10 to 35°C and, unless otherwise specified, each symbol in the chemical formulas described in the schemes is as defined above. In the compounds in the formulas, each compound includes salts, and examples of such salt include those similar to the salts of compound (I) and the like.

In each reaction, when the raw material compound or intermediate has an amino group, a carboxy group or a hydroxy group as a substituent, these groups may be protected by a protecting group generally used in peptide chemistry and the like. By removing the protecting group as necessary after the reaction, the objective compound can be obtained.

The introduction and removal of the protecting group can be performed according to a method known per se, for example, the method described in "Protective Groups in Organic Synthesis, 4th Ed", Wiley-Interscience, Inc. (2006) (Theodora W. Greene, Peter G. M. Wuts).

In the present specification, Examples of the amino-protecting group include a C₁₋₆ alkyl group, a C₇₋₁₂ aralkyl group (e.g., benzyl, p-methoxybenzyl ), a formyl group, a C₁₋₆ alkyl-carbonyl group, a C₁₋₆ alkoxy-carbonyl group (e.g., tert-butyloxycarbonyl), a benzoyl group, a p-fluorobenzoyl group, a C₇₋₁₀ aralkyl-carbonyl group (e.g., benzylcarbonyl), a C₇₋₁₂ aralkyloxy-carbonyl group (e.g., benzyloxycarbonyl, a 9-fluorenylmethoxycarbonyl), a trityl group, a 2-(trimethylsilyl)ethoxymethyl group, a phthaloyl group, an N,N-dimethylaminomethylene group, a substituted silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl), a pyrrolyl group (e.g., 2,5-dimethylpyrrolyl) and the like.

In the present specification, Examples of the carboxyl-protecting group include a C₁₋₆ alkyl group, a C₇₋₁₂ aralkyl group (e.g., benzyl ), a phenyl group, a trityl group, a substituted silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like.

In the present specification, Examples of the hydroxyl-protecting group include a C₁₋₆ alkyl group (e.g., methyl, ethyl), a phenyl group, a trityl group, a C₇₋₁₂ aralkyl group (e.g., benzyl ), a formyl group, a C₁₋₆ alkyl-carbonyl group, a benzoyl group, a C₇₋₁₂ aralkyl-carbonyl group (e.g., benzylcarbonyl), 2-tetrahydropyranyl group, 2-tetrahydrofuranyl group, a substituted silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like.

These groups are optionally substituted by 1 to 3 substituents selected from a halogen atom, a C₁₋₆ alkyl group, a C₁₋₆ alkoxy group and a nitro group.

These protecting groups can be removed according to a method known per se, for example, the method described in "Protective Groups in Organic Synthesis, 4thEd." Wiley-Interscience, Inc. (2006) (Theodora W. Greene, Peter G. M. Wuts) or the like. Specifically, a method using acid, base, ultraviolet rays, hydrazine, phenylhydrazine, sodium N-methyldithiocarbamate, tetrabutylammonium fluoride, palladium acetate, trialkylsilyl halide (e.g., trimethylsilyl iodide, trimethylsilyl bromide) and the like, a reduction method, and the like can be employed.

The compound obtained in each step can be used directly for the next step as the reaction mixture or a crude product, or can be isolated from the reaction mixture according to a conventional means, and can be easily purified according to a separation means such as recrystallization, distillation, chromatography and the like.

Compound (I) can be produced, for example, according to the following Method A or Method B. The raw material compound in each method may be a commercially available product, or can also be produced according to a method known per se or a method analogous thereto. In each step of the following production methods, the raw material compounds may be in the form of a salt, and examples of such salt include those similar to the salts of compound (I).

### [Method A]

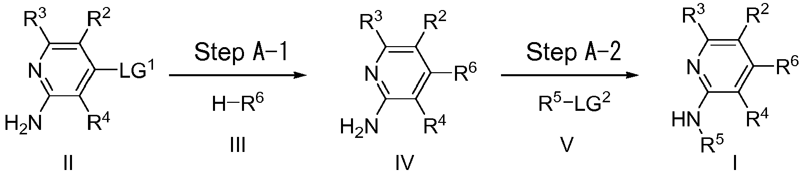

wherein LG¹ and LG² are independently leaving groups, R⁶ is a group represented by 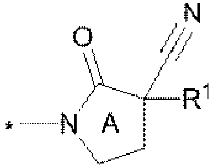 wherein each symbol is as defined above, and the other symbols are as defined above.

Examples of the leaving group represented by LG¹ or LG² include a halogen atom (e.g., a fluorine atom, a chlorine atom, a bromine atom, an iodine atom), a substituted sulfonyloxy group (e.g., a C₁₋₆ alkylsulfonyloxy group optionally substituted by 1 to 3 halogen atoms (e.g., a methanesulfonyloxy group, an ethanesulfonyloxy group, a trifluoromethanesulfonyloxy group etc.); a C₆₋₁₄ arylsulfonyloxy group optionally substituted by 1 to 3 C₁₋₆ alkyl groups (e.g., a benzenesulfonyloxy group, a p-toluenesulfonyloxy group etc.); a C₇₋₁₄ aralkylsulfonyloxy group (e.g., a benzylsulfonyloxy group etc.) etc.) and the like.

The compounds represented by compound (II), compound (III) and compound (V), which are used as raw materials in this method, may be a commercially available product, or can also be produced according to a method known per se or a method analogous thereto.

### (Step A-1)

This step is a step of reacting compound (II) with compound (III) by a coupling reaction using a transition metal catalyst to convert compound (II) into compound (IV).

The coupling reaction using a transition metal catalyst can be carried out according to a method known per se [e.g., Chemical Science, 2011, vol.2, page 27, Coordination of Chemistry Reviews, 2004, vol.48, page 2337, and the like], for example, can be carried out in the presence of a transition metal catalyst and a base, in a solvent that does not adversely influence the reaction.

The amount of compound (III) to be used is about 1 mol to 100 mol equivalent, preferably about 1 mol to 5 mol equivalent, per 1 mol of compound (II).

Examples of the transition metal catalyst include palladium catalysts (e.g., palladium(II) acetate, tris(dibenzylideneacetone)dipalladium(0), palladium(II) chloride, tetrakis(triphenylphosphine)palladium(0), (1,1'-bis(diphenylphosphino)ferrocene)dichloropalladium(II), chloro(2-dicyclohexylphosphino-2',4',6'-triisopropyl-1,1'-biphenyl)[2-(2-aminoethyl)phenyl)]palladium(II), chloro[2-(di-tert-butylphosphino)-2',4',6'-triisopropyl-1,1'-biphenyl][2-(2-aminoethyl)phenyl)]palladium(II), chloro(2-dicyclohexylphosphino-2',6'-dimethoxy-1,1'-biphenyl)[2-(2-aminoethylphenyl)]palladium(II) methyl-tert-butyl ether adduct, chloro-(2-dicyclohexylphosphino-2',6'-diisopropoxy-1,1'-biphenyl)[2-(2-aminoethyl)phenyl]palladium(II), chloro[2-(2-dicyclohexylphosphino)-3,6-dimethoxy-2',4',6'-triisopropyl-1,1'-biphenyl)][2-(2-aminoethyl)phenyl)]palladium(II), chloro(2-dicyclohexylphosphino-2',4',6'-triisopropyl-1,1'-biphenyl)[2-(2'-amino-1,1'-biphenyl)]palladium(II), chloro(2-dicyclohexylphosphino-2',6'-dimethoxy-1,1'-biphenyl)[2-(2'-amino-1,1'-biphenyl)]palladium(II), chloro(2-dicyclohexylphosphino-2',6'-diisopropoxy-1,1'-biphenyl)[2-(2'-amino-1,1'-biphenyl)]palladium(II), etc.), copper catalysts (e.g., copper(I) iodide, copper(II) acetate, copper(I) bromide, copper(I) chloride, etc.), nickel catalysts (e.g., nickel chloride, etc.) and the like. Where necessary, a ligand (e.g., 4,5-bis(diphenylphosphino)-9,9-dimethylxanthene, 2,2'-bis(diphenylphosphino)-1,1'-binaphthyl, 2,2'-bis(di-p-tolylphosphino)-1,1'-binaphthyl, 2-dicyclohexylphosphino-2',4',6'-triisopropyl-biphenyl, 2-dicyclohexylphosphino-2',6'-dimethoxybiphenyl, 2-dicyclohexylphosphino-2',6'-diisopropoxybiphenyl, 2-(dicyclohexylphosphino)-3,6-dimethoxy-2',4',6'-triisopropyl-1,1'-biphenyl, 2-di-tert-butylphosphino-2',4',6'-triisopropylbiphenyl, triphenylphosphine, tri-tert-butylphosphine, N,N'-dimethylethylenediamine, proline, L-proline, D-proline, trans-1,2-cyclohexyldiamine, phenanthroline, etc.) may be added, or a metal oxide (e.g., copper oxide, silver oxide, etc.) may be used as a co-catalyst.

While the amount of the transition metal catalyst to be used varies depending on the kind of the catalyst, it is generally about 0.0001 to about 1 mol equivalent, preferably about 0.01 to about 0.5 mol equivalent, per 1 mol of compound (II). The amount of the ligand to be used is generally about 0.0001 to about 4 mol equivalent, preferably about 0.01 to about 2 mol equivalent, per 1 mol of compound (II).

Examples of the base include organic amines (e.g., trimethylamine, triethylamine, N-ethyldiisopropylamine, N-methylmorpholine, 1,8-diazabicyclo[5.4.0]undec-7-ene, pyridine, N,N-dimethylaniline, etc.), alkali metal salts (e.g., sodium hydrogencarbonate, potassium hydrogencarbonate, sodium carbonate, potassium carbonate, cesium carbonate, sodium acetate, potassium acetate, sodium phosphate, potassium phosphate, sodium hydroxide, potassium hydroxide, etc.), metal hydrides (potassium hydride, sodium hydride, etc.), alkali metal alkoxides (sodium methoxide, sodium ethoxide, sodium tert-butoxide, potassium tert-butoxide, etc.), alkali disilazides (e.g., lithium disilazide, sodium disilazide, potassium disilazide, etc.) and the like. Among them, a alkali metal salt (sodium carbonate, potassium carbonate, cesium carbonate, etc.) is preferable.

The amount of the base to be used is about 0.1 to 100 mol equivalent, preferably about 1 to 10 mol equivalent, per 1 mol of compound (II).

In this reaction, the solvent is not particularly limited as long as the reaction proceeds. Examples of the solvent include hydrocarbons (e.g., benzene, toluene, xylene, hexane, heptane, etc.), halogenated hydrocarbons (e.g., chloroform, dichloromethane, etc.), ethers (e.g., diethyl ether, diisopropyl ether, tert-butyl methyl ether, tetrahydrofuran, dioxane, 1,2-dimethoxyethane, etc.), nitriles (e.g., acetonitrile, etc.), aprotic polar solvents (e.g., N,N-dimethylformamide, dimethyl sulfoxide, hexamethylphosphoroamide, etc.), protic polar solvents (e.g., water, methanol, ethanol, 1-propanol, 2-propanol, 1-butanol, 2-methyl-2-propanol, 2-methyl-2-butanol, etc.) and mixtures thereof.

The reaction temperature in this step is generally about -50 to 200°C, preferably -10°C to 150°C. The reaction time in this step is generally 0.1 hr to 100 hr.

The thus-obtained compound (IV) can be isolated and purified by a known means, for example, concentration, concentration under reduced pressure, solvent extraction, crystallization, recrystallization, phase transfer, chromatography and the like. Alternatively, compound (IV) may be directly used for the next reaction without isolation.

### (Step A-2)

This step is a step of reacting compound (IV) with compound (V) by a coupling reaction using a transition metal catalyst to convert compound (IV) into compound (I). This step can be carried out in the same manner as in the method of the method of Step A-1.

The thus-obtained compound (I) can be isolated and purified by a known means, for example, concentration, concentration under reduced pressure, solvent extraction, crystallization, recrystallization, phase transfer, chromatography and the like.

### [Method B]

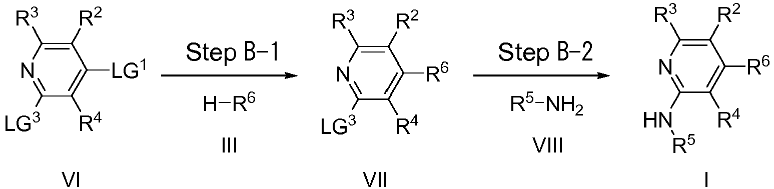

wherein LG³ is a leaving group, and the other symbols are as defined above.

Examples of the leaving group represented by LG³ include those similar to the leaving group represented by LG¹ or LG².

Compound (VI), compound (III) and compound (VIII), which are used as raw materials in this method, may be a commercially available product, or can also be produced according to a method known per se or a method analogous thereto.

### (Step B-1)

This step is a step of reacting compound (VI) with compound (III) to convert compound (VI) into compound (VII).

This step can be carried out in the presence of a base, in a solvent that does not adversely influence the reaction, if necessary.

The amount of compound (III) to be used is preferably about 1 to 100 mol equivalent, particularly preferably about 1 to 10 mol equivalent, per 1 mol of compound (VI).

Examples of the base include organic amines (e.g., trimethylamine, triethylamine, N-ethyldiisopropylamine, N-methylmorpholine, 1,8-diazabicyclo[5.4.0]undec-7-ene, pyridine, N,N-dimethylaniline, etc.), alkali metal salts (e.g., sodium hydrogencarbonate, potassium hydrogencarbonate, sodium carbonate, potassium carbonate, cesium carbonate, sodium acetate, potassium acetate, sodium phosphate, potassium phosphate, sodium hydroxide, potassium hydroxide, etc.), metal hydrides (potassium hydride, sodium hydride, etc.), alkali metal alkoxides (sodium methoxide, sodium ethoxide, sodium tert-butoxide, potassium tert-butoxide, etc.), alkali disilazides (e.g., lithium disilazide, sodium disilazide, potassium disilazide, etc.) and the like.

The amount of the base to be used is preferably about 0.1 to 100 mol equivalent, particularly preferably about 1 to 10 mol equivalent, per 1 mol of compound (VI).

In this step, the solvent is not particularly limited as long as the reaction proceeds. Examples of the solvent include hydrocarbons (e.g., benzene, toluene, xylene, hexane, heptane, etc.), halogenated hydrocarbons (e.g., chloroform, dichloromethane, etc.), ethers (e.g., diethyl ether, diisopropyl ether, tert-butyl methyl ether, tetrahydrofuran, dioxane, dimethoxyethane, etc.), nitriles (e.g., acetonitrile, etc.), aprotic polar solvents (e.g., N,N-dimethylformamide, dimethyl sulfoxide, hexamethylphosphoroamide, etc.) and mixtures thereof.

The reaction temperature in this step is generally about -80 to 200°C, preferably 0°C to 80°C. The reaction time in this step is generally 0.1 hr to 100 hr.

The thus-obtained compound (VII) can be isolated and purified by a known means, for example, concentration, concentration under reduced pressure, solvent extraction, crystallization, recrystallization, phase transfer, chromatography and the like. Alternatively, compound (VII) may be directly used for the next reaction without isolation.

### (Step B-2)

This step is a step of reacting compound (VII) with compound (VIII) by a coupling reaction using a transition metal catalyst to convert compound (VII) into compound (I). This step can be carried out in the same manner as in the method of the method of Step A-1.

The thus-obtained compound (I) can be isolated and purified by a known means, for example, concentration, concentration under reduced pressure, solvent extraction, crystallization, recrystallization, phase transfer, chromatography and the like.

### Among compound (III), a compound represented by the formula (III-A)

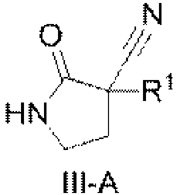

wherein each symbol is as defined above (hereinafter to be referred to as compound (III-A)) can be produced according to the following Method C or a method analogous thereto. In each step of the following production methods, the raw material compounds may be in the form of a salt, and examples of such salt include salts exemplified as the salts of compound (I).

### [Method C]

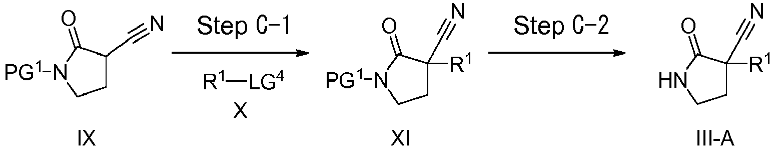

wherein LG⁴ is a leaving group, PG¹ is an amino-protecting group, and the other symbols are as defined above.

Examples of the leaving group represented by LG⁴ include those similar to the leaving group represented by LG¹ or LG².

The compounds represented by compound (IX) and compound (X), which are used as raw materials in this method, may be a commercially available product, or can also be produced according to a method known per [e.g., Journal Heterocyclic Chemistry, 2005, vol.42, page 543, and the like] or a method analogous thereto.

### (Step C-1)

This step is a step of reacting compound (IX) with compound (X) to convert compound (IX) into compound (XI).

This step can be carried out in the presence of a base, in a solvent that does not adversely influence the reaction, if necessary.

The amount of compound (X) to be used is preferably about 1 to 100 mol equivalent, particularly preferably about 1 to 10 mol equivalent, per 1 mol of compound (IX).

Examples of the base include organic amines (e.g., trimethylamine, triethylamine, N-ethyldiisopropylamine, N-methylmorpholine, 1,8-diazabicyclo[5.4.0]undec-7-ene, pyridine, N,N-dimethylaniline, etc.), alkali metal salts (e.g., sodium hydrogencarbonate, potassium hydrogencarbonate, sodium carbonate, potassium carbonate, cesium carbonate, sodium acetate, potassium acetate, sodium phosphate, potassium phosphate, sodium hydroxide, potassium hydroxide, etc.), metal hydrides (potassium hydride, sodium hydride, etc.), alkali metal alkoxides (sodium methoxide, sodium ethoxide, sodium tert-butoxide, potassium tert-butoxide, etc.), alkali disilazides (e.g., lithium disilazide, sodium disilazide, potassium disilazide, etc.) and the like.

The amount of the base to be used is preferably about 0.1 to 100 mol equivalent, particularly preferably about 1 to 10 mol equivalent, per 1 mol of compound (IX).

In this step, the solvent is not particularly limited as long as the reaction proceeds. Examples of the solvent include hydrocarbons (e.g., benzene, toluene, xylene, hexane, heptane, etc.), halogenated hydrocarbons (e.g., chloroform, dichloromethane, etc.), ethers (e.g., diethyl ether, diisopropyl ether, tert-butyl methyl ether, tetrahydrofuran, dioxane, dimethoxyethane, etc.), nitriles (e.g., acetonitrile, etc.), aprotic polar solvents (e.g., N,N-dimethylformamide, dimethyl sulfoxide, hexamethylphosphoroamide, etc.) and mixtures thereof.

The reaction temperature in this step is generally about -80 to 200°C, preferably -80 to 30°C. The reaction time in this step is generally 0.1 hr to 100 hr.

The thus-obtained compound (XI) can be isolated and purified by a known means, for example, concentration, concentration under reduced pressure, solvent extraction, crystallization, recrystallization, phase transfer, chromatography and the like. Alternatively, compound (XI) may be directly used for the next reaction without isolation.

### (Step C-2)

This step is a step of removing the amino-protecting group of compound (XI) to convert compound (XI) into compound (III-A).

This step can be carried out in a solvent that does not adversely influence the reaction, if necessary.

The thus-obtained compound (III-A) can be isolated and purified by a known means, for example, concentration, concentration under reduced pressure, solvent extraction, crystallization, recrystallization, phase transfer, chromatography and the like. Alternatively, compound (III-A) may be directly used for the next reaction without isolation.

### Among compound (III), a compound represented by the formula (III-B)

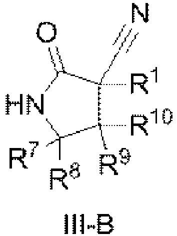

wherein R⁷, R⁸, R⁹ and R¹⁰ are independently a hydrogen atom or an optionally substituted C₁₋₆ alkyl group, and the other symbols are as defined above (hereinafter to be referred to as compound (III-B)) can be carried out according to the following Method D or Method E, or a method analogous thereto. In each step of the following production methods, the raw material compounds may be in the form of a salt, and examples of such salt include salts exemplified as the salts of compound (I).

### [Method D]

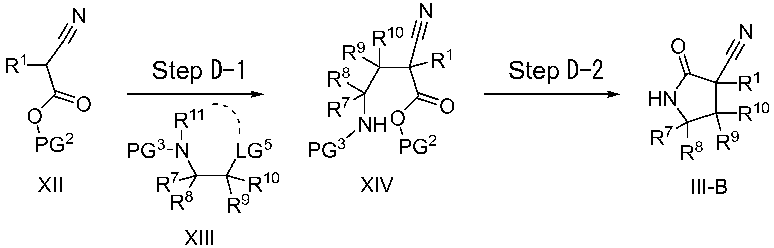

wherein LG⁵ is a leaving group, PG² is a carboxyl-protecting group, PG³ is an amino-protecting group, R¹¹ is a hydrogen atom, or R¹¹ and LG⁵ in combination form a heterocycle (e.g., 2,2-dioxido-1,2,3-oxathiazolidine ring, etc.), and the other symbols are as defined above.

Examples of the leaving group represented by LG⁵ include those similar to the leaving group represented by LG¹ or LG².

Compound (XII) and compound (XIII), which are used as raw materials in this method, may be a commercially available product, or can also be produced according to a method known per [Journal of Organic Chemistry, 2002, vol.67, page 5164, and the like] or a method analogous thereto.

### (Step D-1)

This step is a step of reacting compound (XII) with compound (XIII) to convert compound (XII) into compound (XIV).

This step can be carried out in the presence of a base, in the presence of a phase-transfer catalyst if necessary, in a solvent that does not adversely influence the reaction.

The amount of compound (XIII) to be used is preferably about 1 to 100 mol equivalent, particularly preferably about 1 to 10 mol equivalent, per 1 mol of compound (XII).

Examples of the base include organic amines (e.g., trimethylamine, triethylamine, N-ethyldiisopropylamine, N-methylmorpholine, 1,8-diazabicyclo[5.4.0]undec-7-ene, pyridine, N,N-dimethylaniline, etc.), alkali metal salts (e.g., sodium hydrogencarbonate, potassium hydrogencarbonate, sodium carbonate, potassium carbonate, cesium carbonate, sodium acetate, potassium acetate, sodium phosphate, potassium phosphate, sodium hydroxide, potassium hydroxide, etc.), metal hydrides (potassium hydride, sodium hydride, etc.), alkali metal alkoxides (sodium methoxide, sodium ethoxide, sodium tert-butoxide, potassium tert-butoxide, etc.), alkali metal disilazides (e.g., lithium disilazide, sodium disilazide, potassium disilazide, etc.) and the like.

The amount of the base to be used is preferably about 0.1 to 100 mol equivalent, particularly preferably about 1 to 10 mol equivalent, per 1 mol of compound (XII).

Examples of the phase-transfer catalyst include quaternary ammonium compounds (e.g., tetramethylammonium fluoride, tetraethylammonium fluoride, tetrabutylammonium fluoride, tetrabutylammonium chloride, tetrabutylammonium bromide, tetrabutylammonium iodide, (11bS)-(+)-4,4-dibutyl-4,5-dihydro-2,6-bis(3,4,5-trifluorophenyl)-3H-dinaphtho[2,1-c:1'2'-e]azepinium bromide, (11bR)-(-)-4,4-dibutyl-4,5-dihydro-2,6-bis(3,4,5-trifluorophenyl)-3H-dinaphtho[2,1-c:1'2'-e]azepinium bromide, etc.), phosphonium compounds (e.g., tributyl-n-octylphosphonium bromide, etc.), pyridinium compounds (e.g., 1-butylpyridinium tetrafluoroborate, etc.) and the like.

The amount of the phase-transfer catalyst to be used is preferably about 0.1 to 100 mol equivalent, preferably particularly about 0.1 to 10 mol equivalent, per 1 mol of compound (XII).

In this step, the solvent is not particularly limited as long as the reaction proceeds. Examples of the solvent include hydrocarbons (e.g., benzene, toluene, xylene, hexane, heptane, etc.), halogenated hydrocarbons (e.g., chloroform, dichloromethane, etc.), ethers (e.g., diethyl ether, diisopropyl ether, tert-butyl methyl ether, tetrahydrofuran, 1,4-dioxane, 1,2-dimethoxyethane, etc.), nitriles (e.g., acetonitrile, etc.), aprotic polar solvents (e.g., N,N-dimethylformamide, dimethyl sulfoxide, hexamethylphosphoramide, etc.) and mixtures thereof.

The reaction temperature in this step is generally about -50 to 200°C, preferably -10 to 100°C. The reaction time in this step is generally 0.1 hr to 100 hr.

The thus-obtained compound (XIV) can be isolated and purified by a known means, for example, concentration, concentration under reduced pressure, solvent extraction, crystallization, recrystallization, phase transfer, chromatography and the like. Alternatively, compound (XIV) may be directly used for the next reaction without isolation.

### (Step D-2)

This step is a step of treating compound (XIV) with a base, or removing the amino-protecting group of compound (XIV) if necessary, and treating the resulting compound with a base, to convert compound (XIV) into compound (III-B).

The treatment step with a base can be carried out in the presence of a base, in a solvent that does not adversely influence the reaction.

Examples of the base include organic amines (e.g., trimethylamine, triethylamine, N-ethyldiisopropylamine, N-methylmorpholine, 1,8-diazabicyclo[5.4.0]undec-7-ene, pyridine, N,N-dimethylaniline, etc.), alkali metal salts (e.g., sodium hydrogencarbonate, potassium hydrogencarbonate, sodium carbonate, potassium carbonate, cesium carbonate, sodium acetate, potassium acetate, sodium phosphate, potassium phosphate, sodium hydroxide, potassium hydroxide, etc.), metal hydrides (potassium hydride, sodium hydride, etc.), alkali metal alkoxides (sodium methoxide, sodium ethoxide, sodium tert-butoxide, potassium tert-butoxide, etc.), alkali metal disilazides (e.g., lithium disilazide, sodium disilazide, potassium disilazide, etc.) and the like.

The amount of the base to be used is preferably about 0.1 to 100 mol equivalent, particularly preferably about 1 to 10 mol equivalent, per 1 mol of compound (XIV).

In this step, the solvent is not particularly limited as long as the reaction proceeds. Examples of the solvent include hydrocarbons (e.g., benzene, toluene, xylene, hexane, heptane, etc.), halogenated hydrocarbons (e.g., chloroform, dichloromethane, etc.), ethers (e.g., diethyl ether, diisopropyl ether, tert-butyl methyl ether, tetrahydrofuran, 1,4-dioxane, 1,2-dimethoxyethane, etc.), nitriles (e.g., acetonitrile, etc.), aprotic polar solvents (e.g., N,N-dimethylformamide, dimethyl sulfoxide, hexamethylphosphoramide, etc.) and mixtures thereof.

The reaction temperature in this step is generally about -50 to 200°C, preferably -10 to 100°C. The reaction time in this step is generally 0.1 hr to 100 hr.

The thus-obtained compound (III-B) can be isolated and purified by a known means, for example, concentration, concentration under reduced pressure, solvent extraction, crystallization, recrystallization, phase transfer, chromatography and the like. Alternatively, compound (III-B) may be directly used for the next reaction without isolation.

### [Method E]

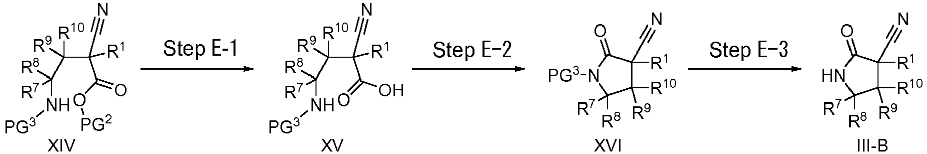

wherein each symbol is as defined above.

### (Step E-1)

This step is a step of removing the carboxyl-protecting group of compound (XIV) to convert compound (XIV) into compound (XV).

This step can be carried out in a solvent that does not adversely influence the reaction, if necessary.

The thus-obtained compound (XV) can be isolated and purified by a known means, for example, concentration, concentration under reduced pressure, solvent extraction, crystallization, recrystallization, phase transfer, chromatography and the like. Alternatively, compound (XV) may be directly used for the next reaction without isolation.

### (Step E-2)

This step is a step of subjecting compound (XV) to an intramolecular amidation reaction to convert compound (XV) into compound (XVI).

This step is can be carried out in the presence of a condensing agent and a base if necessary, in a solvent that does not adversely influence the reaction.

Examples of the condensing agent include N,N'-di-substituted carbodiimides (N,N'-dicyclohexylcarbodiimide, 1-ethyl-3-(3-dimethylaminopropyl)carbodiimide (WSC) hydrochloride, etc.), azolides (N,N'-carbonyldiimidazole, etc.), phosphorylcyanides (diethylphosphorylcyanide, etc.), 2-halogeno pyridinium salts (2-chloromethylpyridinium iodide, 2-fluoro-1-methylpyridinium iodide, etc.), 2-(7-azabenzotriazol-1-yl)-1,1,3,3-hexafluorophosphate (HATU), O-(7-azabenzotriazol-1-yl)-N,N,N',N'-tetramethyluronium tetrafluoroborate (TATU), N-ethoxycarbonyl-2-ethoxy-1,2-dihydroquinoline, 4-(4,6-dimethoxy-1,3,5-triazin-2-yl)-4-methylmorpholinium chloride (DMT-MM), oxalyl chloride, thionyl chloride, phosphorus oxychloride, alkoxyacethylenes, 2,4,6-trichlorobenzoyl chloride, 2-methyl-6-nitrobenzoic anhydride and the like.

The amount of the condensing agent to be used is preferably about 0.1 to 100 mol equivalent, particularly preferably about 1 to 10 mol equivalent, per 1 mol of compound (XV).

Examples of the base include organic amines (e.g., trimethylamine, triethylamine, N-ethyldiisopropylamine, N-methylmorpholine, 1,8-diazabicyclo[5.4.0]undec-7-ene, pyridine, N,N-dimethylaniline, etc.), alkali metal salts (e.g., sodium hydrogencarbonate, potassium hydrogencarbonate, sodium carbonate, potassium carbonate, cesium carbonate, sodium acetate, potassium acetate, sodium phosphate, potassium phosphate, sodium hydroxide, potassium hydroxide, etc.), metal hydrides (potassium hydride, sodium hydride, etc.), alkali metal alkoxides (sodium methoxide, sodium ethoxide, sodium tert-butoxide, potassium tert-butoxide, etc.), alkali metal disilazides (e.g., lithium disilazide, sodium disilazide, potassium disilazide, etc.) and the like.

The amount of the base to be used is preferably about 0.1 to 100 mol equivalent, particularly preferably about 1 to 10 mol equivalent, per 1 mol of compound (XV).

In this step, the solvent is not particularly limited as long as the reaction proceeds. Examples of the solvent include hydrocarbons (e.g., benzene, toluene, xylene, hexane, heptane, etc.), halogenated hydrocarbons (e.g., chloroform, dichloromethane, etc.), ethers (e.g., diethyl ether, diisopropyl ether, tert-butyl methyl ether, tetrahydrofuran, 1,4-dioxane, 1,2-dimethoxyethane, etc.), nitriles (e.g., acetonitrile, etc.), aprotic polar solvents (e.g., N,N-dimethylformamide, dimethyl sulfoxide, hexamethylphosphoramide, etc.) and mixtures thereof.

The reaction temperature in this step is generally about -50 to 200°C, preferably -10 to 100°C. The reaction time in this step is generally 0.1 hr to 100 hr.

The thus-obtained compound (XVI) can be isolated and purified by a known means, for example, concentration, concentration under reduced pressure, solvent extraction, crystallization, recrystallization, phase transfer, chromatography and the like. Alternatively, compound (XVI) may be directly used for the next reaction without isolation.

### (Step E-3)

This step is a step of removing the amino-protecting group of compound (XVI) to convert compound (XVI) into compound (III-B).

This step can be carried out in a solvent that does not adversely influence the reaction, if necessary.

The thus-obtained compound (III-B) can be isolated and purified by a known means, for example, concentration, concentration under reduced pressure, solvent extraction, crystallization, recrystallization, phase transfer, chromatography and the like. Alternatively, compound (III-B) may be directly used for the next reaction without isolation.

Compound (I) obtained according to the above-mentioned Method A or Method B can also be modified by subjecting to a known reaction such as condensation reaction (e.g. various acylation reaction, alkylation reaction etc.), oxidation reaction, reduction reaction, dehydration reaction and the like. Such reaction can be carried out according to a known method per se.

The compound (I) obtained according to the above-mentioned methods can be isolated and purified by a known separation means such as recrystallization, distillation, chromatography and the like.

When compound (I) has an optical isomer, a stereoisomer, a regioisomer or a rotamer, these are also encompassed in compound (I), and can be obtained as a single product according to a synthesis method and separation method known per se (e.g., concentration, solvent extraction, column chromatography, recrystallization etc.). For example, when compound (I) has an optical isomer, the optical isomer resolved from the compound is also encompassed in compound (I).

The optical isomer can be produced according to a method known per se. Specifically, the optical isomer is obtained using an optically active synthetic intermediate or by subjecting the racemic final product to an optical resolution according to a known method.

The optical resolution may be a method known per se, such as a fractional recrystallization method, a chiral column method, a diastereomer method etc.

### 1) Fractional recrystallized method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine etc.) is formed, which is separated by a fractional recrystallized method, and if desired, a neutralization step to give a free optical isomer.

### 2) Chiral column method

A method wherein a racemate or a salt thereof is applied to a column (a chiral column) for separation of an optical isomer to allow separation. In the case of a liquid chromatography, for example, a mixture of the optical isomers is applied to a chiral column such as ENANTIO-OVM (manufactured by Tosoh Corporation), CHIRAL series (manufactured by Daicel Chemical Industries, Ltd.) and the like, and developed with water, various buffers (e.g., phosphate buffer, etc.) and organic solvents (e.g., ethanol, methanol, 2-propanol, acetonitrile, trifluoroacetic acid, diethylamine, etc.) as an eluent, solely or in admixture to separate the optical isomer. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (e.g., a fractional recrystallized method, a chromatography method etc.) and the like, and is subjected to a chemical treatment such as hydrolysis and the like to separate an optically active reagent moiety, whereby an optical isomer is obtained. For example, when compound (I) contains hydroxy or primary or secondary amino in a molecule, the compound and an optically active organic acid (e.g., MTPA [α-methoxy-α-(trifluoromethyl)phenylacetic acid], (-)-menthoxyacetic acid etc.) and the like are subjected to condensation reaction to give diastereomers of the ester compound or the amide compound, respectively. When compound (I) has a carboxy group, the compound and an optically active amine or an optically active alcohol reagent are subjected to condensation reaction to give diastereomers of the amide compound or the ester compound, respectively. The separated diastereomer is converted to an optical isomer of the original compound by acid hydrolysis or base hydrolysis.

Compound (I) may be a crystal.

The crystal of compound (I) can be produced according to a crystallization method known per se.

Examples of the crystallization method include crystallization method from a solution, crystallization method from vapor crystallization method from a melt, and the like.

The "crystallization method from a solution" is typically a method of shifting a non-saturated state to supersaturated state by varying factors involved in solubility of compounds (solvent composition, pH, temperature, ionic strength, redox state, etc.) or the amount of solvent. Specific examples thereof include a concentration method, a slow cooling method, a reaction method (a diffusion method, an electrolysis method), a hydrothermal growth method, a flux method and the like. Examples of the solvent to be used include aromatic hydrocarbons (e.g., benzene, toluene, xylene, etc.), halogenated hydrocarbons (e.g., dichloromethane, chloroform, etc.), saturated hydrocarbons (e.g., hexane, heptane, cyclohexane, etc.), ethers (e.g., diethyl ether, diisopropyl ether, tetrahydrofuran, 1,4-dioxane, etc.), nitriles (e.g., acetonitrile, etc.), ketones (e.g., acetone, etc.), sulfoxides (e.g., dimethyl sulfoxide, etc.), acid amides (e.g., N,N-dimethylformamide, etc.), esters (e.g., ethyl acetate, etc.), alcohols (e.g., methanol, ethanol, 2-propanol, etc.), water and the like. These solvents are used alone or in a combination of two or more at a suitable ratio (e.g., 1:1 to 1:100 (a volume ratio)). Where necessary, a seed crystal can be used.

The "crystallization method from vapor" is, for example, a vaporization method (a sealed tube method, a gas stream method), a gas phase reaction method, a chemical transportation method and the like.

The "crystallization method from a melt" is, for example, a normal freezing method (a pulling method, a temperature gradient method, a Bridgman method), a zone melting method (a zone leveling method, a floating zone method), a special growth method (a VLS method, a liquid phase epitaxy method) and the like.

Preferable examples of the crystallization method include a method comprising dissolving compound (I) in a suitable solvent (e.g., alcohols such as methanol, ethanol etc.) at 20°C to 120°C, and cooling the obtained solution to a temperature (e.g., 0 to 50°C, preferably 0 to 20°C) not higher than the dissolution temperature, and the like.

The thus-obtained crystals of the present invention can be isolated, for example, by filtration and the like.

An analysis method of the obtained crystal is generally a method of crystal analysis by powder X-ray diffraction. As a method of determining crystal orientation, a mechanical method or an optical method and the like can also be used.

The crystal of compound (I) obtained by the above-mentioned production method (hereinafter to be abbreviated as "the crystal of the present invention") has high purity, high quality, and low hygroscopicity, is not denatured even after a long-term preservation under general conditions, and is extremely superior in the stability. In addition, it is also superior in the biological properties (e.g., pharmacokinetics (absorption, distribution, metabolism, excretion), efficacy expression etc.) and is extremely useful as a medicament.

In the present specification, specific optical rotation ([α]D) means a specific optical rotation measured using, for example, polarimeter (JASCO, P-1030 Polarimeter (No.AP-2)) and the like.

In the present specification, the melting point means a melting point measured using, for example, a micro melting point determination apparatus (YANACO, MP-500D), a DSC (differential scanning calorimetry) apparatus (SEIKO, EXSTAR6000) or the like.

The prodrug of compound (I) means a compound which is converted to compound (I) with a reaction due to an enzyme, gastric acid and the like under the physiological condition in the living body, that is, a compound which is converted to compound (I) by enzymatic oxidation, reduction, hydrolysis and the like; a compound which is converted to compound (I) by hydrolysis and the like due to gastric acid, and the like. Examples of the prodrug for compound (I) include a compound obtained by subjecting an amino group in compound (I) to acylation, alkylation or phosphorylation (e.g., a compound obtained by subjecting an amino group in compound (I) to eicosanoylation, alanylation, pentylaminocarbonylation, (5-methyl-2-oxo-1,3-dioxolen-4-yl)methoxycarbonylation, tetrahydrofurylation, pyrrolidylmethylation, pivaloyloxymethylation, tert-butylation, and the like); a compound obtained by subjecting a hydroxy group in compound (I) to acylation, alkylation, phosphorylation or boration (e.g., a compound obtained by subjecting a hydroxy group in compound (I) to acetylation, palmitoylation, propanoylation, pivaloylation, succinylation, fumarylation, alanylation or dimethylaminomethylcarbonylation, and the like); a compound obtained by subjecting a carboxyl group in compound (I) to esterification or amidation (e.g., a compound obtained by subjecting a carboxyl group in compound (I) to ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation, and the like) and the like. These compounds can be produced from compound (I) according to a method known *per se.*

The prodrug of compound (I) may also be one which is converted to compound (I) under physiological conditions as described in "IYAKUHIN no KAIHATSU (Development of Pharmaceuticals)", Vol. 7, Design of Molecules, p. 163-198, Published by HIROKAWA SHOTEN (1990).

In the present specification, compound (I) and the prodrug of compound (I) are sometimes collectively abbreviated as "the compound of the present invention".

Compound (I) may be a hydrate, a non-hydrate, a solvate or a non-solvate.

Compound (I) also encompasses a compound labeled with an isotope (e.g., ³H, ¹⁴C, ³⁵S, ¹²⁵I etc.) and the like.

Compound (I) also encompasses a deuterium conversion form wherein ¹H is converted to ²H(D).

Compound (I) also encompasses a tautomer thereof.

Compound (I) may be a pharmaceutically acceptable cocrystal or a salt thereof. The cocrystal or a salt thereof means a crystalline substance constituted with two or more special solids at room temperature, each having different physical properties (e.g., structure, melting point, melting heat, hygroscopicity, solubility and stability etc.). The cocrystal or a salt thereof can be produced according to a cocrystallization a method known *per se.*

Compound (I) may also be used as a PET tracer.

Since the compound of the present invention has a superior Tyk2 inhibitory action, it is also useful as safe medicaments based on such action.

Since the compound of the present invention has also an IFN-α inhibitory action, IFN-β inhibitory action, IL-6 inhibitory action, IL-10 inhibitory action, IL-19 inhibitory action, IL-20 inhibitory action, IL-22 inhibitory action, IL-28 inhibitory action, IL-29 inhibitory action, IL-12 inhibitory action and/or IL-23 inhibitory action, it is also useful as safe medicaments based on such action.

For example, the medicament of the present invention containing the compound of the present invention can be used for a mammal (e.g., mouse, rat, hamster, rabbit, cat, dog, bovine, sheep, monkey, human etc.) as a prophylactic or therapeutic agent for Tyk2 associated diseases, more specifically, the diseases described in (1) - (4) below.
(1) inflammatory diseases (e.g., acute pancreatitis, chronic pancreatitis, asthma, adult respiratory distress syndrome, chronic obstructive pulmonary disease (COPD), inflammatory bone disease, inflammatory pulmonary disease, inflammatory bowel disease, celiac disease, hepatitis, systemic inflammatory response syndrome (SIRS), postoperative or posttraumatic inflammation, pneumonia, nephritis, meningitis, cystitis, pharyngolaryngitis, gastric mucosal injury, spondylitis, arthritis, dermatitis, chronic pneumonia, bronchitis, pulmonary infarction, silicosis, pulmonary sarcoidosis etc.),
(2) autoimmune diseases (e.g., psoriasis, rheumatoid arthritis, inflammatory bowel disease (e.g., Crohn's disease, ulcerative colitis etc.), Sjogren's syndrome, Behcet's disease, multiple sclerosis, systemic lupus erythematosus, ankylopoietic spondylarthritis, polymyositis, dermatomyositis (DM), polyarteritis nodosa (PN), mixed connective tissue disease (MCTD), scleroderma, profundus lupus erythematosus, chronic thyroiditis, Graves' disease, autoimmune gastritis, type I and type II diabetes, autoimmune hemolytic anemia, autoimmune neutropenia, thrombocytopenia, atopic dermatitis, chronic active hepatitis, myasthenia gravis, graft versus host disease, Addison's disease, abnormal immunoresponse, arthritis, dermatitis, radiodermatitis etc.),
(3) osteoarticular degenerative disease (e.g., rheumatoid arthritis, osteoporosis, osteoarthritis etc.),
(4) neoplastic diseases [e.g., malignant tumor, angiogenesis glaucoma, infantile hemangioma, multiple myeloma, acute myeloblastic leukemia, chronic sarcoma, chronic myelogenous leukemia, metastasis melanoma, Kaposi's sacroma, vascular proliferation, cachexia, metastasis of the breast cancer, cancer (e.g., colorectal cancer (e.g., familial colorectal cancer, hereditary nonpolyposis colorectal cancer, gastrointestinal stromal tumor etc.), lung cancer (e.g., non-small cell lung cancer, small cell lung cancer, malignant mesothelioma etc.), mesothelioma, pancreatic cancer (e.g., pancreatic duct cancer etc.), gastric cancer (e.g., papillary adenocarcinoma, mucinous adenocarcinoma, adenosquamous carcinoma, etc.), breast cancer (e.g., invasive ductal carcinoma, ductal carcinoma in situ, inflammatory breast cancer etc.), ovarian cancer (e.g., ovarian epithelial carcinoma, extragonadal germ cell tumor, ovarian germ cell tumor, ovarian low malignant potential tumor etc.), prostate cancer (e.g., hormone-dependent prostate cancer, non-hormone dependent prostate cancer etc.), liver cancer (e.g., primary liver cancer, extrahepatic bile duct cancer etc.), thyroid cancer (e.g., medullary thyroid carcinoma etc.), kidney cancer (e.g., renal cell carcinoma, transitional cell carcinoma in kidney and urinary duct etc.), uterine cancer, brain tumor (e.g., pineal astrocytoma, pilocytic astrocytoma, diffuse astrocytoma, anaplastic astrocytoma etc.), melanoma, sarcoma, urinary bladder cancer, hematologic cancer and the like including multiple myeloma, hypophyseal adenoma, glioma, acoustic neurinoma, retinoblastoma, pharyngeal cancer, laryngeal cancer, cancer of the tongue, thymoma, esophagus cancer, duodenal cancer, colorectal cancer, rectal cancer, hepatoma, pancreatic endocrine tumor, bile duct cancer, gallbladder cancer, penile cancer, urinary duct cancer, testis tumor, vulvar cancer, cervix cancer, endometrial cancer, uterus sarcoma, cholionic disease, vaginal cancer, skin cancer, fungoid mycosis, basal cell tumor, soft tissue sarcoma, malignant lymphoma, Hodgkin's disease, myelodysplastic syndrome, acute lymphocytic leukemia, chronic lymphocytic leukemia, adult T cell leukemia, chronic bone marrow proliferative disease, pancreatic endocrine tumor fibrous histiocytoma, leiomyosarcoma, rhabdomyosarcoma, cancer of unknown primary).

The medicament of the present invention can be preferably used as an agent for the prophylaxis or treatment of autoimmune diseases, inflammatory disease, osteoarticular degenerative disease or neoplastic disease, particularly preferably psoriasis, rheumatoid arthritis, inflammatory bowel disease (preferably Crohn's disease or ulcerative colitis), Sjogren's syndrome, Behcet's disease, multiple sclerosis, or systemic lupus erythematosus.

Here, the above-mentioned "prophylaxis" of a disease means, for example, administration of a medicament containing the compound of the present invention to patients who are expected to have a high risk of the onset due to some factor relating to the disease but have not developed the disease or patients who have developed the disease but do not have a subjective symptom, or administration of a medicament containing the compound of the present invention to patients who are feared to show recurrence of the disease after treatment of the disease.

The medicament of the present invention shows superior pharmacokinetics (e.g., a half-life of the drug in plasma), low toxicity (e.g., HERG inhibition, CYP inhibition, CYP induction), and decreased drug interaction. The compound of the present invention can be directly used as a medicament, or as the medicament of the present invention by producing a pharmaceutical composition by mixing with a pharmaceutically acceptable carrier by a means known per se and generally used in a production method of pharmaceutical preparations. The medicament of the present invention can be orally or parenterally administered safely to mammals (e.g., humans, monkeys, cows, horses, pigs, mice, rats, hamsters, rabbits, cats, dogs, sheep and goats).

A medicament containing the compound of the present invention can be safely administered solely or by mixing with a pharmacologically acceptable carrier according to a method known per se (e.g., the method described in the Japanese Pharmacopoeia etc.) as the production method of a pharmaceutical preparation, and in the form of, for example, tablet (including sugar-coated tablet, film-coated tablet, sublingual tablet, orally disintegrating tablet, buccal etc.), pill, powder, granule, capsule (including soft capsule, microcapsule), troche, syrup, liquid, emulsion, suspension, release control preparation (e.g., immediate-release preparation, sustained-release preparation, sustained-release microcapsule), aerosol, film (e.g., orally disintegrating film, oral mucosa-adhesive film), injection (e.g., subcutaneous injection, intravenous injection, intramuscular injection, intraperitoneal injection), drip infusion, transdermal absorption type preparation, cream, ointment, lotion, adhesive preparation, suppository (e.g., rectal suppository, vaginal suppository), pellet, nasal preparation, pulmonary preparation (inhalant), eye drop and the like, orally or parenterally (e.g., intravenous, intramuscular, subcutaneous, intraorgan, intranasal, intradermal, instillation, intracerebral, intrarectal, intravaginal, intraperitoneal and intratumor administrations, administration to the vicinity of tumor and direct administration to the lesion).

The content of the compound of the present invention in the medicament of the present invention is about 0.01 to 100% by weight of the entire medicament. The dose varies depending on administration subject, administration route, disease and the like. For example, for oral administration to patients (body weight about 60 kg) with psoriasis, rheumatoid arthritis, inflammatory bowel disease, Sjogren's syndrome, Behcet's disease, multiple sclerosis or systemic lupus erythematosus, about 0.01 mg/kg body weight - about 500 mg/kg body weight, preferably about 0.1 mg/kg body weight - about 50 mg/kg body weight, more preferably about 1 mg/kg body weight - about 30 mg/kg body weight of an active ingredient (compound (I)) can be administered once to several portions per day.

The pharmaceutically acceptable carrier, which may be used for the production of the medicament of the present invention, may be exemplified by various organic or inorganic carrier materials that are conventionally used as preparation materials, for example, excipient, lubricant, bin ding agent and disintegrant for solid preparations; or solvent, solubilizing agent, suspending agent, isotonic agent, buffering agent, soothing agent and the like for liquid preparations. Furthermore, when necessary, ordinary additives such as preservative, antioxidant, colorant, sweetening agent, adsorbing agent, wetting agent and the like can be also used as appropriate in an appropriate amount.

When the compound of the present invention is used as an ointment, the ointment is prepared by mixing the compound of the present invention with a general ointment base so that the concentration is adjusted to about 0.001 to 3% (W/W), preferably about 0.01 to 1% (W/W). The preparation of ointment preferably comprises a powderization step of the compound of the present invention, and a sterilization step of the formulation. The ointment is administered once to four times a day depending on condition of the patient.

Examples of the ointment base include purified lanolin, white vaseline, macrogol, plastibase, liquid paraffin and the like.

Examples of the excipient include lactose, white sugar, D-mannitol, starch, corn starch, crystalline cellulose, light anhydrous silicic acid and the like.

Examples of the lubricant include magnesium stearate, calcium stearate, talc, colloidal silica and the like.

Examples of the binding agent include crystalline cellulose, white sugar, D-mannitol, dextrin, hydroxypropylcellulose, hydroxypropylmethylcellulose, polyvinylpyrrolidone, starch, sucrose, gelatin, methylcellulose, carboxymethylcellulose sodium and the like.

Examples of the disintegrant include starch, carboxymethylcellulose, carboxymethylcellulose calcium, carboxymethylstarch sodium, L-hydroxypropylcellulose and the like.

Examples of the solvent include water for injection, alcohol, propylene glycol, Macrogol, sesame oil, corn oil, olive oil and the like.

Examples of the solubilizing agent include polyethylene glycol, propylene glycol, D-mannitol, benzyl benzoate, ethanol, trisaminomethane, cholesterol, triethanolamine, sodium carbonate, sodium citrate and the like.

Examples of the suspending agent include surfactants such as stearyl triethanolamine, sodium lauryl sulfate, laurylaminopropionic acid, lecithin, benzalkonium chloride, benzetonium chloride, glycerin monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like; and the like.

Examples of the isotonic agent include glucose, D-sorbitol, sodium chloride, glycerin, D-mannitol and the like.

Examples of the buffering agent include buffer solutions such as phosphates, acetates, carbonates, citrates and the like.

Examples of the soothing agent include benzyl alcohol and the like.

Examples of the preservative include parahydroxybenzoates, chlorobutanol, benzyl alcohol, phenylethyl alcohol, dehydroacetic acid, sorbic acid and the like.

Examples of the antioxidant include sulfites, ascorbic acid, α-tocopherol and the like.

For the prophylaxis or treatment of various diseases, the compound of the present invention can also be used together with other medicaments. In the following, a medicament to be used when the compound of the present invention is used together with other drug is referred to as "the combination agent of the present invention".

For example, when the compound of the present invention is used as a Tyk2 inhibitor, IFN-α inhibitor, IFN-β inhibitor, IL-6 inhibitor, IL-10 inhibitor, IL-19 inhibitor, IL-20 inhibitor, IL-22 inhibitor, IL-28 inhibitor, IL-29 inhibitor, IL-12 inhibitor and/or IL-23 inhibitor, it can be used together with the following drugs.
(1) non-steroidal anti-inflammatory drug (NSAIDs)
   (i) Classical NSAIDs
      alcofenac, aceclofenac, sulindac, tolmetin, etodolac, fenoprofen, thiaprofenic acid, meclofenamic acid, meloxicam, tenoxicam, lornoxicam, nabumeton, acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, piroxicam, epirizole, tiaramide hydrochloride, zaltoprofen, gabexate mesylate, camostat mesylate, ulinastatin, colchicine, probenecid, sulfinpyrazone, benzbromarone, allopurinol, sodium aurothiomalate, hyaluronate sodium, sodium salicylate, morphine hydrochloride, salicylic acid, atropine, scopolamine, morphine, pethidine, levorphanol, oxymorphone or a salt thereof and the like.
   (ii) cyclooxygenase inhibitor (COX-1 selective inhibitor COX-2 selective inhibitor etc.)
      salicylic acid derivatives (e.g., celecoxib, aspirin), etoricoxib, valdecoxib, diclofenac, indomethacin, loxoprofen and the like.
   (iii) nitric oxide-releasing NSAIDs.
   (iv) JAK inhibitor
      tofacitinib, ruxolitinib and the like.
(2) disease-modifying anti-rheumatic drugs (DMARDs)
   (i) Gold preparation
      auranofin and the like.
   (ii) penicillamine
      D-penicillamine and the like.
   (iii) aminosalicylic acid preparation
      sulfasalazine, mesalamine, olsalazine, balsalazide and the like.
   (iv) antimalarial drug
      chloroquine and the like.
   (v) pyrimidine synthesis inhibitor
      leflunomide and the like.
   (vi) prograf
(3) anti-cytokine drug
   (I) protein drug
      (i) TNF inhibitor
         etanercept, infliximab, adalimumab, certolizumab pegol, golimumab, PASSTNF-α, soluble TNF-α receptor TNF-α binding protein, anti-TNF-α antibody and the like.
      (ii) interleukin-1 inhibitor
         anakinra (interleukin-1 receptor antagonist), soluble interleukin-1 receptor and the like.
      (iii) interleukin-6 inhibitor
         tocilizumab (anti-interleukin-6 receptor antibody), anti-interleukin-6 antibody and the like.
      (iv) interleukin-10 drug
         interleukin-10 and the like.
      (v) interleukin-12/23 inhibitor
         ustekinumab, briakinumab (anti-interleukin-12/23 antibody) and the like.
   (II) non-protein drug
      (i) MAPK inhibitor
         BMS-582949 and the like.
      (ii) gene modulator
         inhibitor of molecule involved in signal transduction, such as NF-κ, NF-κB, IKK-1, IKK-2, AP-1 and the like, and the like.
      (iii) cytokine production inhibitor
         iguratimod, tetomilast and the like.
      (iv) TNF-α converting enzyme inhibitor
      (v) interleukin-1β converting enzyme inhibitor
         VX-765 and the like.
      (vi) interleukin-6 antagonist
         HMPL-004 and the like.
      (vii) interleukin-8 inhibitor
         IL-8 antagonist, CXCR1 & CXCR2 antagonist, reparixin and the like.
      (viii) chemokine antagonist
         CCR9 antagonist (CCX-282, CCX-025), MCP-1 antagonist and the like.
      (ix) interleukin-2 receptor antagonist
         denileukin, diftitox and the like.
      (x) therapeutic vaccines
         TNF-α vaccine and the like.
      (xi) gene therapy drug
         gene therapy drugs aiming at promoting the expression of gene having an anti-inflammatory action such as interleukin-4, interleukin-10, soluble interleukin-1 receptor soluble TNF-α receptor and the like.
      (xii) antisense compound
         ISIS 104838 and the like.
(4) integrin inhibitor
   natalizumab, vedolizumab, AJM300, TRK-170, E-6007 and the like.
(5) immunomodulator (immunosuppressant)
   methotrexate, cyclophosphamide, MX-68, atiprimod dihydrochloride, BMS-188667, CKD-461, rimexolone, cyclosporine, tacrolimus, gusperimus, azathiopurine, antilymphocyte serum, freeze-dried sulfonated normal immunoglobulin, erythropoietin, colony stimulating factor interleukin, interferon and the like.
(6) steroid
   dexamethasone, hexestrol, methimazole, betamethasone, triamcinolone, triamcinolone acetonide, fluocinonide, fluocinolone acetonide, predonisolone, methylpredonisolone, cortisone acetate, hydrocortisone, fluorometholone, beclomethasone dipropionate, estriol and the like.
(7) angiotensin converting enzyme inhibitor
   enalapril, captopril, ramipril, lisinopril, cilazapril, perindopril and the like.
(8) angiotensin II receptor antagonist
   candesartan, candesartan cilexetil, azilsartan, azilsartan medoxomil, valsartan, irbesartan, olmesartan, eprosartan and the like.
(9) diuretic drug
   hydrochlorothiazide, spironolactone, furosemide, indapamide, bendrofluazide, cyclopenthiazide and the like.
(10) cardiotonic drug
   digoxin, dobutamine and the like.
(11) β receptor antagonist
   carvedilol, metoprolol, atenolol and the like.
(12) Ca sensitizer
   MCC-135 and the like.
(13) Ca channel antagonist
   nifedipine, diltiazem, verapamil and the like.
(14) anti-platelet drug, anticoagulator
   heparin, aspirin, warfarin and the like.
(15) HMG-CoA reductase inhibitor
   atorvastatin, simvastatin and the like.
(16) contraceptive
   (i) sex hormone or derivatives thereof
      gestagen or a derivative thereof (progesterone, 17α-hydroxy progesterone, medroxyprogesterone, medroxyprogesterone acetate, norethisterone, norethisterone enanthate, norethindrone, norethindrone acetate, norethynodrel, levonorgestrel, norgestrel, ethynodiol diacetate, desogestrel, norgestimate, gestodene, progestin, etonogestrel, drospirenone, dienogest, trimegestone, nestorone, chlormadinone acetate, mifepristone, nomegestrol acetate, Org-30659, TX-525, EMM-310525) or a combination agent of a gestagen or a derivative thereof and an estrogen or a derivative thereof (estradiol, estradiol benzoate, estradiol cypionate, estradiol dipropionate, estradiol enanthate, estradiol hexahydrobenzoate, estradiol phenylpropionate, estradiol undecanoate, estradiol valerate, estrone, ethinylestradiol, mestranol) and the like.
   (ii) antiestrogen
      ormeloxifene, mifepristone, Org-33628 and the like.
   (iii) spermatocide ushercell and the like.
(17) others
   (i) T cell inhibitors
   (ii) inosine monophosphate dehydrogenase (IMPDH) inhibitor mycophenolate mofetil and the like.
   (iii) adhesion molecule inhibitor
      ISIS-2302, selectin inhibitor ELAM-1, VCAM-1, ICAM-1 and the like.
   (iv) thalidomide
   (v) cathepsin inhibitor
   (vi) matrix metalloprotease (MMPs) inhibitor
      V-85546 and the like.
   (vii) glucose-6-phosphate dehydrogenase inhibitor
   (viii) Dihydroorotate dehydrogenase (DHODH) inhibitor
   (ix) phosphodiesterase IV(PDE IV) inhibitor
      roflumilast, CG-1088 and the like.
   (x) phospholipase A₂ inhibitor
   (xi) iNOS inhibitor
      VAS-203 and the like.
   (xii) microtubule stimulating drug
      paclitaxel and the like.
   (xiii) microtuble inhibitor
      reumacon and the like.
   (xiv) MHC class II antagonist
   (xv) prostacyclin agonist
      iloprost and the like.
   (xvi) CD4 antagonist
      zanolimumab and the like.
   (xvii) CD23 antagonist
   (xviii) LTB4 receptor antagonist
      DW-1305 and the like.
   (xix) 5-lipoxygenase inhibitor
      zileuton and the like.
   (xx) cholinesterase inhibitor
      galanthamine and the like.
   (xxi) tyrosine kinase inhibitor
      Tyk2 inhibitor (WO2010142752) and the like.
   (xxii) cathepsin B inhibitor
   (xxiii) adenosine deaminase inhibitor
      pentostatin and the like.
   (xxiv) osteogenesis stimulator
   (xxv) dipeptidylpeptidase inhibitor
   (xxvi) collagen agonist
   (xxvii) capsaicin cream
   (xxviii) hyaluronic acid derivative
      synvisc (hylan G-F 20), orthovisc and the like.
   (xxix) glucosamine sulfate
   (xxx) amiprilose
   (xxxi) CD-20 inhibitor
      rituximab, ibritumomab, tositumomab, ofatumumab and the like.
   (xxxii) BAFF inhibitor
      belimumab, tabalumab, atacicept, A-623 and the like.
   (xxxiii) CD52 inhibitor
      alemtuzumab and the like.
   (xxxiv) IL-17 inhibitor
      secukinumab (AIN-457), LY-2439821, AMG827 and the like

Other concomitant drugs besides the above-mentioned include , for example, antibacterial agent, antifungal agent, antiprotozoal agent, antibiotic, antitussive and expectorant drug, sedative, anesthetic, antiulcer drug, antiarrhythmic agent, hypotensive diuretic drug, anticoagulant, tranquilizer, antipsychotic, antitumor drug, hypolipidemic drug, muscle relaxant, antiepileptic drug, antidepressant, antiallergic drug, cardiac stimulants, therapeutic drug for arrhythmia, vasodilator, vasoconstrictor, hypotensive diuretic, therapeutic drug for diabetes, antinarcotic, vitamin, vitamin derivative, antiasthmatic, therapeutic agent for pollakisuria/anischuria, antipruritic drug, therapeutic agent for atopic dermatitis, therapeutic agent for allergic rhinitis, hypertensor, endotoxin-antagonist or -antibody, signal transduction inhibitor, inhibitor of inflammatory mediator activity, antibody to inhibit inflammatory mediator activity, inhibitor of anti-inflammatory mediator activity, antibody to inhibit anti-inflammatory mediator activity and the like. Specific examples thereof include the following.
(1) Antibacterial agent
   (i) sulfa drug
      sulfamethizole, sulfisoxazole, sulfamonomethoxine, sulfamethizole, salazosulfapyridine, silver sulfadiazine and the like.
   (ii) quinolone antibacterial agent
      nalidixic acid, pipemidic acid trihydrate, enoxacin, norfloxacin, ofloxacin, tosufloxacin tosylate, ciprofloxacin hydrochloride, lomefloxacin hydrochloride, sparfloxacin, fleroxacin and the like.
   (iii) antiphthisic
      isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
   (iv) antiacidfast bacterium drug
      diaphenylsulfone, rifampicin and the like.
   (v) antiviral drug
      idoxuridine, acyclovir, vidarabine, gancyclovir and the like.
   (vi) anti-HIV agent
      zidovudine, didanosine, zalcitabine, indinavir sulfate ethanolate, ritonavir and the like.
   (vii) antispirochetele
   (viii) antibiotic
      tetracycline hydrochloride, ampicillin, piperacillin, gentamicin, dibekacin, kanendomycin, lividomycin, tobramycin, amikacin, fradiomycin, sisomicin, tetracycline, oxytetracycline, rolitetracycline, doxycycline, ticarcillin, cephalothin, cephapirin, cephaloridine, cefaclor cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt a salt thereof, griseofulvin, lankacidin-group [Journal of Antibiotics (J. Antibiotics), 38, 877-885(1985)], azole compound [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone, fluconazole, itraconazole and the like] and the like.
(2) antifungal agent
   (i) polyethylene antibiotic (e.g., amphotericin B, nystatin, trichomycin)
   (ii) griseofulvin, pyrrolnitrin and the like
   (iii) cytosine metabolism antagonist (e.g., flucytosine)
   (iv) imidazole derivative (e.g., econazole, clotrimazole, miconazole nitrate, bifonazole, croconazole)
   (v) triazole derivative (e.g., fluconazole, itraconazole)
   (vi) thiocarbamic acid derivative (e.g., trinaphthol) and the like.
(3) antiprotozoal agent
   metronidazole, tinidazole, diethylcarbamazine citrate, quinine hydrochloride, quinine sulfate and the like.
(4) antitussive and expectorant drug
   ephedrine hydrochloride, noscapine hydrochloride, codeine phosphate, dihydrocodeine phosphate, isoproterenol hydrochloride, methylephedrine hydrochloride, alloclamide, chlophedianol, picoperidamine, cloperastine, protokylol, isoproterenol, salbutamol, terputaline, oxypetebanol, morphine hydrochloride, dextropethorfan hydrobromide, oxycodone hydrochloride, dimorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.
(5) sedative
   chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.
(6) anesthetic
   (6-1) local anesthetic
      cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine and the like.
   (6-2) general anesthetic
      (i) inhalation anesthetic (e.g., ether, halothane, nitrous oxide, isoflurane, enflurane),
      (ii) intravenous anesthetic (e.g., ketamine hydrochloride, droperidol, thiopental sodium, thiamylal sodium, pentobarbital) and the like.
(7) antiulcer drug
   histidine hydrochloride, lansoprazole, metoclopramide, pirenzepine, cimetidine, ranitidine, famotidine, urogastrine, oxethazaine, proglumide, omeprazole, sucralfate, sulpiride, cetraxate, gefarnate, aldioxa, teprenone, prostaglandin and the like.
(8) antiarrhythmic agent
   (i) sodium channel blocker (e.g., quinidine, procainamide, disopyramide, ajmaline, lidocaine, mexiletine, phenytoin),
   (ii) β-blocker (e.g., propranolol, alprenolol, bufetolol hydrochloride, oxprenolol, atenolol, acebutolol, metoprolol, bisoprolol, pindolol, carteolol, arotinolol hydrochloride),
   (iii) potassium channel blocker (e.g., amiodarone),
   (iv) calcium channel blocker (e.g., verapamil, diltiazem) and the like.
(9) hypotensive diuretic drug
   hexamethonium bromide, clonidine hydrochloride, hydrochlorothiazide, trichlormethiazide, furosemide, ethacrynic acid, bumetanide, mefruside, azosemide, spironolactone, potassium canrenoate, triamterene, amiloride, acetazolamide, D-mannitol, isosorbide, aminophylline and the like.
(10) anticoagulant
   heparin sodium, sodium citrate, activated protein C, tissue factor pathway inhibitor antithrombin III, dalteparin sodium, warfarin potassium, argatroban, gabexate, ozagrel sodium, ethyl icosapentate, beraprost sodium, alprostadil, ticlopidine hydrochloride, pentoxifylline, dipyridamole, tisokinase, urokinase, streptokinase and the like.
(11) tranquilizer
   diazepam, lorazepam, oxazepam, chlordiazepoxide, medazepam, oxazolam, cloxazolam, clotiazepam, bromazepam, etizolam, fludiazepam, hydroxyzine and the like.
(12) antipsychotic
   chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine and the like.
(13) antitumor drug
   6-O-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, zusulfan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestrol, chlormadinone acetate, leuprorelin acetate, buserelin acetate and the like.
(14) hypolipidemic drug
   clofibrate, ethyl 2-chloro-3-[4-(2-methyl-2-phenylpropoxy)phenyl]propionate [Chem. Pharm. Bull, 38, 2792-2796 (1990)], pravastatin, simvastatin, probucol, bezafibrate, clinofibrate, nicomol, cholestyramine, dextran sulfate sodium and the like.
(15) muscle relaxant
   pridinol, tubocurarine, pancuronium, tolperisone hydrochloride, chlorphenesin carbamate, baclofen, chlormezanone, mephenesin, chlorzoxazone, eperisone, tizanidine and the like.
(16) antiepileptic drug
   phenytoin, ethosuximide, acetazolamide, chlordiazepoxide, tripethadione, carbamazepine, phenobarbital, primidone, sulthiame, sodium valproate, clonazepam, diazepam, nitrazepam and the like.
(17) antidepressant
   imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.
(18) antiallergic drug
   diphenhydramine, chlorpheniramine, tripelennamine, metodilamine, clemizole, diphenylpyraline, methoxyphenamine, sodium cromoglicate, tranilast, repirinast, amlexanox, ibudilast, ketotifen, terfenadine, mequitazine, azelastine hydrochloride, epinastine, ozagrel hydrochloride, pranlukast hydrate, seratrodast and the like.
(19) cardiac stimulants
   trans-π-oxocamphor terephyllol, aminophylline, etilefrine, dopamine, dobutamine, denopamine, vesinarine, amrinone, pimobendan, ubidecarenone, digitoxin, digoxin, methyldigoxin, lanatoside C, G-strophanthin and the like.
(20) vasodilator
   oxyfedrine, diltiazem, tolazoline, hexobendine, bamethan, clonidine, methyldopa, guanabenz and the like.
(21) vasoconstrictor
   dopamine, dobutamine denopamine and the like.
(22) hypotensive diuretic
   hexamethonium bromide, pentolinium, mecamylamine, ecarazine, clonidine, diltiazem, nifedipine and the like.
(23) therapeutic drug for diabetes
   tolbutamide, chlorpropamide, acetohexamide, glibenclamide, tolazamide, acarbose, epalrestat, troglitazone, glucagon, glymidine, glipuzide, phenformin, buformin, metformin and the like.
(24) antinarcotic
   levallorphan, nalorphine, naloxone or a salt thereof and the like.
(25) liposoluble vitamins
   (i) vitamin A: vitamin A₁, vitamin A₂ and retinol palmitate
   (ii) vitamin D: vitamin D₁, D₂, D₃, D₄and D₅
   (iii) vitamin E: α-tocopherol, β-tocopherol, γ-tocopherol, δ-tocopherol, dl-α-tocopherol nicotinate
   (iv) vitamin K: vitamin K₁, K₂, K₃and K₄
   (v) folic acid (vitamin M) and the like.
(26) vitamin derivative
   various derivatives of vitamins, for example, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like, and the like.
(27) antiasthmatic
   isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, ipratropium bromide, oxitropium bromide, flutropium bromide, theophylline, aminophylline, sodium cromoglicate, tranilast, repirinast, amlexanox, ibudilast, ketotifen, terfenadine, mequitazine, azelastine, epinastine, ozagrel hydrochloride, pranlkast hydrate, seratrodast, dexamethasone, prednisolone, hydrocortisone, hydrocortisone sodium succinate, beclometasone dipropionate and the like.
(28) therapeutic agent for pollakisuria/anischuria flavoxate hydrochloride and the like.
(29) therapeutic agent for atopic dermatitis sodium cromoglicate and the like.
(30) therapeutic agent for allergic rhinitis
   sodium cromoglicate, chlorpheniramine maleate, alimemazine tartrate, clemastine fumarate, homochlorcyclizine hydrochloride, fexofenadine, mequitazine and the like.
(31) hypertensor
   dopamine, dobutamine, denopamine, digitoxin, digoxin, methyldigoxin, lanatoside C, G-strophanthin and the like.
(32) others
   hydroxycam, diacerein, megestrol acetate, nicergoline, prostaglandins and the like.

For combined use, the administration time of the compound of the present invention and the concomitant drug is not restricted, and the compound of the present invention or the concomitant drug can be administered to an administration subject simultaneously, or may be administered at different times. The dosage of the concomitant drug may be determined according to the dose clinically used, and can be appropriately selected depending on an administration subject, administration route, disease, combination and the like.

The administration form of the combined use is not particularly limited, and the compound of the present invention and a concomitant drug only need to be combined on administration. Examples of such administration mode include the following:
(1) administration of a single preparation obtained by simultaneously processing the compound of the present invention and the concomitant drug, (2) simultaneous administration of two kinds of preparations of the compound of the present invention and the concomitant drug, which have been separately produced, by the same administration route, (3) administration of two kinds of preparations of the compound of the present invention and the concomitant drug, which have been separately produced, by the same administration route in a staggered manner, (4) simultaneous administration of two kinds of preparations of the compound of the present invention and the concomitant drug, which have been separately produced, by different administration routes, (5) administration of two kinds of preparations of the compound of the present invention and the concomitant drug, which have been separately produced, by different administration routes in a staggered manner (e.g., administration in the order of the compound of the present invention and the concomitant drug, or in the reverse order) and the like.

The mixing ratio of the compound of the present invention and a concomitant drug in the combination agent of the present invention can be appropriately selected based on the subject of administration, administration route, disease and the like.

For example, while the content of the compound of the present invention in the combination agent of the present invention varies depending on the preparation form, it is generally about 0.01 - 100 wt%, preferably about 0.1 - 50 wt%, more preferably about 0.5 - 20 wt%, of the whole preparation.

The content of the concomitant drug in the combination agent of the present invention varies depending on the preparation form, and generally about 0.01 to 100% by weight, preferably about 0.1 to 50% by weight, further preferably about 0.5 to 20% by weight, of the entire preparation.

While the content of the additive such as a carrier and the like in the combination agent of the present invention varies depending on the form of a preparation, it is generally about 1 to 99.99% by weight, preferably about 10 to 90% by weight, based on the preparation.

When the compound of the present invention and the concomitant drug are separately prepared, the same content may be adopted.

The dose of the combination agent varies depending on the kind of the compound of the present invention, administration route, symptom, age of patients and the like. For example, for oral administration to patients (body weight about 60 kg) with psoriasis, rheumatoid arthritis, inflammatory bowel disease, Sjogren's syndrome, Behcet's disease, multiple sclerosis or systemic lupus erythematosus, about 0.1 mg/kg body weight - about 50 mg/kg body weight, preferably about 1 mg/kg body weight - 30 mg/kg body weight, of compound (I) can be administered once to several portions per day.

The dose of the pharmaceutical composition of the present invention as a sustained-release preparation varies depending on the kind and content of compound (I), dosage form, period of sustained drug release, subject animal of administration (e.g., mammals such as mouse, rat, hamster, guinea pig, rabbit, cat, dog, bovine, horse, swine, sheep, monkey, human etc.), and administration object. For example, for application by parenteral administration, about 0.1 to about 100 mg of compound (I) needs to be released from the administered preparation per 1 week.

Any amount of the concomitant drug can be adopted as long as the side effects do not cause a problem. The daily dosage in terms of the concomitant drug varies depending on the severity, age, sex, body weight, sensitivity difference of the subject, administration period, interval, and nature, pharmacology, kind of the pharmaceutical preparation, kind of effective ingredient, and the like, and not particularly restricted, and the amount of a drug is, in the case of oral administration for example, generally about 0.001 to 2000 mg, preferably about 0.01 to 500 mg, further preferably about 0.1 to 100 mg, per 1 kg of a mammal and this is generally administered once to 4-times, divided in a day.

When the combination agent of the present invention is administered, the compound of the present invention and the concomitant drug can be administered simultaneously, or may be administered in a staggered manner. When administered at a time interval, the interval varies depending on the effective ingredient, dosage form and administration method, and, for example, when the concomitant drug is administered first, a method in which the compound of the present invention is administered within time range of from 1 minute to 3 days, preferably from 10 minutes to 1 day, more preferably from 15 minutes to 1 hour, after administration of the concomitant drug is an example. When the compound of the present invention is administered first, a method in which the concomitant drug is administered within time range of from 1 minute to 1 day, preferably from 10 minutes to 6 hours, more preferably from 15 minutes to 1 hour after administration of the compound of the present invention is an example.

### Examples

The present invention is explained in detail in the following by referring to Examples, Experimental Examples and Formulation Examples, which are not to be construed as limitative, and the invention may be changed within the scope of the present invention.

In the following Examples, the "room temperature" generally means about 10°C to about 35°C. The ratios indicated for mixed solvents are volume mixing ratios, unless otherwise specified. % means wt%, unless otherwise specified.

In silica gel column chromatography, NH means use of aminopropylsilane-bound silica gel. In HPLC (high performance liquid chromatography), C18 means use of octadecyl-bound silica gel. The ratios of elution solvents are volume mixing ratios, unless otherwise specified.

In Examples, the following abbreviations are used.
BSA: bovine serum albumin
DMSO: dimethyl sulfoxide
DTT: dithiothreitol
EDTA: ethylenediaminetetraacetic acid
EGTA: glycoletherdiaminetetraacetic acid
HEPES: 4-(2-hydroxyethyl)-1-piperazine ethanesulfonic acid M: mol concentration
SFC: Supercritical fluid chromatography

¹H NMR (protone nuclear magnetic resonance spectrum) was measured by Fourier-transform type NMR. For the analysis, ACD/SpecManager (trade name) and the like were used. Peaks with very mild protons such as a hydroxy group, an amino group and the like are not described.

MS (mass spectrum) was measured by LC/MS (liquid chromatography mass spectrometer). As ionization method, ESI (Electro Spray Ionization) method or APCI (Atomospheric Pressure Chemical Ionization) method was used. The data indicates those found. Generally, a molecular ion peak is observed. In the case of a salt, a molecular ion peak or fragment ion peak of free form is generally observed.

### Example 1

### (3S)-3-cyclopropyl-1-(2-((5-(morpholin-4-yl)pyridin-2-yl)amino)pyridin-4-yl)-2-oxopyrrolidine-3-carbonitrile

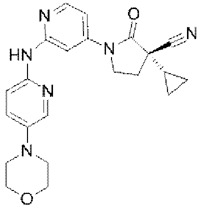

### A) ethyl cyano(cyclopropyl)acetate

To a solution of cyclopropylacetonitrile (75 g) and diethyl carbonate (220 mL) in N,N-dimethylformamide (1000 mL) was added potassium tert-butoxide (260 g), and the mixture was stirred overnight at 50°C. The reaction mixture was cooled to 0°C, 2M hydrochloric acid (1000 mL) was added thereto, and the mixture was extracted with ethyl acetate. The obtained organic layer was washed successively with water and saturated brine, and dried over anhydrous magnesium sulfate, and the solvent was evaporated under reduced pressure. The residue was distilled (boiling point 60°C, 0.5 mmHg) to give the title compound (130 g) .
¹H NMR (400MHz, CDCl₃)δ0.49-0.64 (2H, m), 0.71-0.80 (2H, m), 1.34 (3H, t, J = 7.1 Hz), 1.36-1.44 (1H, m), 3.23 (1H, d, J = 7.6 Hz), 4.29 (2H, q, J = 7.1 Hz).

### B) ethyl 4-(((benzyloxy)carbonyl)amino)-2-cyano-2-cyclopropylbutanoate

To a solution of ethyl cyano(cyclopropyl)acetate obtained in Step A of Example 1 (20 g) and benzyl (2-bromoethyl)carbamate (35 g) in N,N-dimethylformamide (300 mL) was added potassium carbonate (40 g), and the mixture was stirred overnight at room temperature. To the reaction mixture was added water, and the mixture was extracted with ethyl acetate. The obtained organic layer was washed successively with water and saturated brine, and dried over anhydrous magnesium sulfate, and the solvent was evaporated under reduced pressure. The residue was purified by silica gel column chromatography (hexane/ethyl acetate) to give the title compound (30 g).
¹H NMR (400 MHz, DMSO-d₆) δ0.38-0.50 (2H, m), 0.54-0.63 (1H, m), 0.63-0.73 (1H, m), 1.23 (3H, t, J = 7.1 Hz), 1.28-1.38 (1H, m), 1.93-1.94 (1H, m), 2.00-2.09 (1H, m), 2.16-2.26 (1H, m), 3.00-3.14 (1H, m), 3.15-3.28 (1H, m), 4.16-4.27 (2H, m), 4.98-5.04 (2H, m), 7.26-7.41 (6H, m).

### C) 3-cyclopropyl-2-oxopyrrolidine-3-carbonitrile

To a solution of ethyl 4-(((benzyloxy)carbonyl)amino)-2-cyano-2-cyclopropylbutanoate obtained in Step B of Example 1 (17 g) in tetrahydrofuran (400 mL) was added sodium hydride (60% in mineral oil, 2.4 g) under ice bath, and the mixture was stirred at room temperature for 5 hr. To the reaction mixture was added water, and the mixture was extracted with ethyl acetate. The obtained organic layer was washed with saturated aqueous ammonium chloride solution and saturated brine, and dried over anhydrous magnesium sulfate, and the solvent was evaporated under reduced pressure. The residue was purified by silica gel column chromatography (hexane/ethyl acetate) to give the title compound (6.4 g).
¹H NMR (400 MHz, DMSO-d₆) δ0.40-0.57 (3H, m), 0.59-0.68 (1H, m), 1.31 (1H, tt, J = 8.2, 5.2 Hz), 2.20 (1H, dt, J = 13.1, 5.7 Hz), 2.42-2.47 (1H, m), 3.27 (2H, dd, J = 7.3, 5.9 Hz), 8.31 (1H, brs).

### D) (3S)-cyclopropyl-2-oxopyrrolidine-3-carbonitrile

3-Cyclopropyl-2-oxopyrrolidine-3-carbonitrile obtained in Step C of Example 1 (34 g) was resolved by HPLC (column: CHIRALPAK IC, 30 mmID×250 mmL, manufactured by Daicel Chemical Industries, mobile phase: carbon dioxide/2-propanol/acetonitrile = 820/90/90) to give the title compound (16 g: longer retention time).
>99% ee (HPLC (column: CHIRALPAK IC, 4.6 mmID×150 mmL, manufactured by Daicel Chemical Industries, mobile phase: carbon dioxide/2-propanol = 700/300, flow rate: 4.0 mL/min, retention time: 4.50 min))

### E) (3S)-1-(2-bromopyridin-4-yl)-3-cyclopropyl-2-oxopyrrolidine-3-carbonitrile

To a solution of (3S)-cyclopropyl-2-oxopyrrolidine-3-carbonitrile obtained in Step D of Example 1 (2.7 g) in N,N-dimethylformamide (30 mL) was added sodium hydride (60% in mineral oil, 860 mg), and the mixture was stirred at 0°C for 10 min. To the reaction mixture was added a solution of 2-bromo-4-fluoropyridine (3.5 g) in N,N-dimethylformamide (15 mL) at 0°C, and the mixture was stirred was stirred at room temperature for 5 hr. To the reaction mixture was added water, and the mixture was extracted with ethyl acetate. The obtained organic layer was washed with saturated brine, and dried over anhydrous magnesium sulfate, and the solvent was evaporated under reduced pressure to give the title compound (4.2 g).
¹H NMR (400 MHz, DMSO-d₆) δ0.45-0.58 (2H, m), 0.59-0.73 (2H, m), 1.47-1.55 (1H, m), 2.34-2.44 (1H, m), 2.60-2.71 (1H, m), 3.94-4.00 (2H, m), 7.76 (1H, dd, J = 5.7, 2.1 Hz), 7.94 (1H, d, J = 2.0 Hz), 8.38 (1H, d, J = 5.6 Hz).

### F) (3S)-3-cyclopropyl-1-(2-((5-(morpholin-4-yl)pyridin-2-yl)amino)pyridin-4-yl)-2-oxopyrrolidine-3-carbonitrile

To a solution of (3S)-1-(2-bromopyridin-4-yl)-3-cyclopropyl-2-oxopyrrolidine-3-carbonitrile obtained in Step E of Example 1 (200 mg) in 2-methyl-2-propanol (2.0 mL) were added 5-(morpholin-4-yl)pyridin-2-amine (120 mg), potassium carbonate (180 mg), 2-(dicyclohexylphosphino)-3,6-dimethoxy-2',4',6'-triisopropyl-1,1'-biphenyl (140 mg) and tris(dibenzylideneacetone)dipalladium(0) (60 mg) under argon atmosphere, and the mixture was stirred overnight at 100°C. To the reaction mixture was added saturated aqueous sodium bicarbonate solution, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate), and recrystallized (diisopropyl ether/ethyl acetate) to give the title compound (92 mg).
¹H NMR (400 MHz, DMSO-d₆) δ0.48-0.57 (2H, m), 0.59-0.64 (1H, m), 0.66-0.73 (1H, m), 1.41-1.58 (1H, m), 2.29-2.41 (1H, m), 2.61-2.74 (1H, m), 3.01-3.11 (4H, m), 3.67-3.78 (4H, m), 3.86-3.97 (2H, m), 7.21 (1H, dd, J = 5.7, 2.1 Hz), 7.40 (1H, dd, J = 9.2, 3.1 Hz), 7.64 (1H, d, J = 9.0 Hz), 7.92 (2H, d, J = 2.4 Hz), 8.14 (1H, d, J = 5.6 Hz), 9.47 (1H, s).
MS (ESI+) : [M+H]⁺ 405.4.

### Example 2

### (3S)-1-(2-aminopyridin-4-yl)-3-cyclopropyl-2-oxopyrrolidine-3-carbonitrile

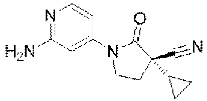

A mixture of (3S)-cyclopropyl-2-oxopyrrolidine-3-carbonitrile obtained in Step D of Example 1 (5.4 g), 4-iodopyridin-2-amine (7.2 g), N,N'-dimethylethane-1,2-diamine (1.5 mL), potassium carbonate (9.0 g) and copper(I) iodide (2.5 g) in 1,2-dimethoxyethane (60 mL) was stirred in a microwave reactor at 130°C for 1 hr. The insoluble substance was removed through Celite, and the solvent was evaporated under reduced pressure. The residue was purified by silica gel column chromatography (NH, ethyl acetate/methanol) to give the title compound (7.7 g).
¹H NMR (400 MHz, DMSO-d₆) δ0.46-0.55 (2H, m), 0.57-0.63 (1H, m), 0.64-0.72 (1H, m), 1.44-1.54 (1H, m), 2.27-2.37 (1H, m), 2.56-2.67 (1H, m), 3.81-3.91 (2H, m), 6.01 (2H, s), 6.74-6.84 (2H, m), 7.80-7.92 (1H, m).
MS (ESI+) : [M+H]⁺ 243.1.

### Example 3

### (3S)-3-cyclopropyl-1-(2-((5-(2-hydroxypropan-2-yl)pyridin-2-yl)amino)pyridin-4-yl)-2-oxopyrrolidine-3-carbonitrile

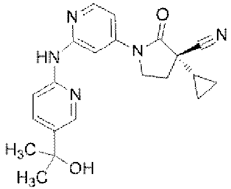

To a solution of (3S)-1-(2-aminopyridin-4-yl)-3-cyclopropyl-2-oxopyrrolidine-3-carbonitrile obtained in Example 2 (150 mg) in 2-methyl-2-propanol (3.0 mL) were added 2-(6-chloropyridin-3-yl)propan-2-ol (130 mg), potassium carbonate (170 mg), 2-(dicyclohexylphosphino)-3,6-dimethoxy-2',4',6'-triisopropyl-1,1'-biphenyl (17 mg) and chloro[2-(dicyclohexylphosphino)-3,6-dimethoxy-2',4',6'-triisopropyl-1,1'-biphenyl][2-(2-aminoethyl)phenyl]palladium(II) (25 mg) under argon atmosphere, and the mixture was stirred overnight at 100°C. The reaction mixture was cooled to 0°C, saturated aqueous sodium bicarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (hexane/ethyl acetate), and recrystallized (hexane/ethyl acetate) to give the title compound (160 mg).
¹H NMR (400MHz, DMSO-d₆)δ0.40-0.76 (4H, m), 1.44 (6H, s), 1.47-1.58 (1H, m), 2.27-2.44 (1H, m), 2.59-2.72 (1H, m), 3.82-3.97 (2H, m), 5.05 (1H, s), 7.28 (1H, dd, J = 5.7, 2.1 Hz), 7.60 (1H, d, J = 8.8 Hz), 7.69-7.76 (1H, m), 8.07 (1H, d, J = 1.7 Hz), 8.18 (1H, d, J = 5.9 Hz), 8.31 (1H, d, J = 2.2 Hz), 9.65 (1H, s).
MS (ESI+) : [M+H]⁺ 378.2.

### Example 4

### tert-butyl 3-((4-((3S)-3-cyano-3-cyclopropyl-2-oxopyrrolidin-1-yl)pyridin-2-yl)amino)-1-methyl-1H-pyrazole-5-carboxylate

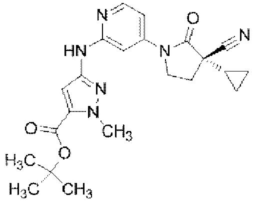

### A) tert-butyl 3-(2,5-dimethyl-1H-pyrrol-1-yl)-1-methyl-1H-pyrazole-5-carboxylate

To a solution of 3-(2,5-dimethyl-1H-pyrrol-1-yl)-1-methyl-1H-pyrazole (6.4 g) in tetrahydrofuran (70 mL) was added dropwise n-butyllithium (1.6 M hexane solution, 27 mL) at -78°C, and the mixture was stirred at the same temperature for 1 hr. To the reaction mixture was added dropwise di-tert-butyl dicarbonate (11 mL) at -78°C, and the mixture was stirred for 2 hr while gradually raising the temperature to room temperature. To the reaction mixture was added saturated aqueous ammonium chloride solution, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (7.9 g).
¹H NMR (400 MHz, DMSO-d₆) δ1.56 (9H, s), 1.94-2.13 (6H, m), 4.09 (3H, s), 5.77 (2H, s), 6.84 (1H, s).
MS (ESI+) : [M+H]⁺ 276.1.

### B) tert-butyl 3-amino-1-methyl-1H-pyrazole-5-carboxylate

To a solution of tert-butyl 3-(2,5-dimethyl-1H-pyrrol-1-yl)-1-methyl-1H-pyrazole-5-carboxylate obtained in Step A of Example 4 (5.3 g) in a mixed solvent of ethanol (30 mL) and water (15 mL) were added hydroxylamine hydrochloride (8.0 g) and potassium hydroxide (4.3 g), and the mixture was stirred at 100°C for 14 hr. The ethanol was evaporated under reduced pressure, saturated aqueous sodium bicarbonate solution was added thereto, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (NH, hexane/ethyl acetate) to give the title compound (2.5 g).
¹H NMR (400 MHz, DMSO-d₆) δ1.50 (9H, s), 3.81 (3H, s), 4.76 (2H, s), 5.87 (1H, s).

### C) tert-butyl 3-((4-((3S)-3-cyano-3-cyclopropyl-2-oxopyrrolidin-1-yl)pyridin-2-yl)amino)-1-methyl-1H-pyrazole-5-carboxylate

To a solution of tert-butyl 3-amino-1-methyl-1H-pyrazole-5-carboxylate obtained in Step B of Example 4 (100 mg) and (3S)-1-(2-bromopyridin-4-yl)-3-cyclopropyl-2-oxopyrrolidine-3-carbonitrile obtained in Step E of Example 1 (160 mg) in tetrahydrofuran (2.0 mL) were added potassium carbonate (140 mg), 2-(dicyclohexylphosphino)-3,6-dimethoxy-2',4',6'-triisopropyl-1,1'-biphenyl (14 mg) and chloro[2-(dicyclohexylphosphino)-3,6-dimethoxy-2',4',6'-triisopropyl-1,1'-biphenyl][2-(2-aminoethyl)phenyl]palladium(II) (20 mg) under argon atmosphere, and the mixture was stirred overnight at 70°C. The reaction mixture was cooled to 0°C, saturated aqueous sodium bicarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate) to give the title compound (140 mg).
¹H NMR (400 MHz, DMSO-d₆) δ0.47-0.58 (2H, m), 0.59-0.65 (1H, m), 0.66-0.72 (1H, m), 1.45-1.63 (10H, m), 2.31-2.42 (1H, m), 2.60-2.70 (1H, m), 3.85-3.93 (2H, m), 3.97 (3H, s), 6.95 (1H, s), 7.10 (1H, dd, J = 5.9, 2.0 Hz), 7.41 (1H, d, J = 1.7 Hz), 8.13 (1H, d, J = 5.6 Hz), 9.54 (1H, s).
MS (ESI+) : [M+H]⁺ 423.2.

### Example 5

### 3-((4-((3S)-3-cyano-3-cyclopropyl-2-oxopyrrolidin-1-yl)pyridin-2-yl)amino)-N,1-dimethyl-1H-pyrazole-5-carboxamide

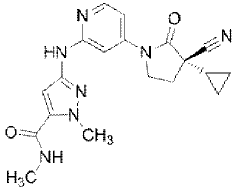

### A) 3-((4-((3S)-3-cyano-3-cyclopropyl-2-oxopyrrolidin-1-yl)pyridin-2-yl)amino)-1-methyl-1H-pyrazole-5-carboxylic acid hydrochloride

To a solution of tert-butyl 3-((4-((3S)-3-cyano-3-cyclopropyl-2-oxopyrrolidin-1-yl)pyridin-2-yl)amino)-1-methyl-1H-pyrazole-5-carboxylate obtained in Step C of Example 4 (180 mg) in ethyl acetate (2.0 mL) was added 4 M hydrogen chloride ethyl acetate solution (16 mL), and the mixture was stirred overnight at 50°C, and the solvent was evaporated under reduced pressure to give the crude title compound (170 mg).
¹H NMR (400 MHz, DMSO-d₆) δ0.49-0.60 (2H, m), 0.60-0.73 (2H, m), 1.54 (1H, tt, J = 8.1, 5.1 Hz), 2.40 (1H, ddd, J = 12.9, 6.9, 3.7 Hz), 2.64-2.72 (1H, m), 3.54 (1H, brs), 3.96-4.02 (2H, m), 4.08 (3H, s), 6.75 (1H, brs), 7.32 (1H, d, J = 6.1 Hz), 7.65 (1H, brs), 8.20 (1H, d, J = 6.8 Hz), 10.34-11.28 (1H, m),
13.64 (1H, s).
MS (ESI+) : [M+H]⁺ 367.2.

### B) 3-((4-((3S)-3-cyano-3-cyclopropyl-2-oxopyrrolidin-1-yl)pyridin-2-yl)amino)-N,1-dimethyl-1H-pyrazole-5-carboxamide

To a solution of 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 (170 mg) in N,N-dimethylformamide (2.0 mL) were added 1-ethyl-3-(3-dimethylaminopropyl)carbodiimide (130 mg), 1-hydroxybenzotriazole (170 mg), triethylamine (240 µL), 4-dimethylaminopyridine (3.0 mg) and methylamine hydrochloride (70 mg), and the mixture was stirred overnight at room temperature. The reaction mixture was cooled to 0°C, saturated aqueous sodium bicarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate), and recrystallized (hexane/ethyl acetate) to give the title compound (78 mg).
¹H NMR (400 MHz, DMSO-d₆) δ0.45-0.58 (2H, m), 0.58-0.65 (1H, m), 0.65-0.74 (1H, m), 1.46-1.56 (1H, m), 2.28-2.42 (1H, m), 2.60-2.70 (1H, m), 2.73 (3H, d, J = 4.6 Hz), 3.86-3.94 (2H, m), 3.96 (3H, s), 6.98 (1H, s), 7.13 (1H, dd, J = 5.9, 2.0 Hz), 7.39 (1H, d, J = 1.7 Hz), 8.11 (1H, d, J = 5.6 Hz), 8.41-8.47 (1H, m), 9.48 (1H, s).
MS(ESI+): [M+H]⁺ 380.2.

### Example 6

### (3S)-3-cyclopropyl-1-(2-((4-(hydroxymethyl)pyridin-2-yl)amino)pyridin-4-yl)-2-oxopyrrolidine-3-carbonitrile

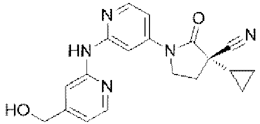

To a solution of (3S)-1-(2-bromopyridin-4-yl)-3-cyclopropyl-2-oxopyrrolidine-3-carbonitrile obtained in Step E of Example 1 (100 mg) in 2-methyl-2-propanol (6.0 mL) were added (2-aminopyridin-4-yl)methanol (41 mg), potassium carbonate (90 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 100°C. To the reaction mixture was added ethyl acetate, the insoluble substance was removed through Celite, and the solvent was evaporated under reduced pressure. The residue was purified by silica gel column chromatography (NH, ethyl acetate/methanol) to give the title compound (58 mg). MS (ESI+) : [M+H]⁺ 350.2.

### Example 7

### (3S)-3-cyclopropyl-1-(2-((1,5-dimethyl-1H-pyrazol-3-yl)amino)pyridin-4-yl)-2-oxopyrrolidine-3-carbonitrile hydrochloride

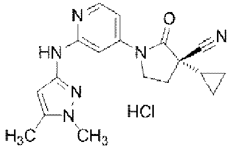

To a solution of (3S)-1-(2-bromopyridin-4-yl)-3-cyclopropyl-2-oxopyrrolidine-3-carbonitrile obtained in Step E of Example 1 (100 mg) in 2-methyl-2-propanol (2.0 mL) were added 1,5-dimethyl-1H-pyrazol-3-amine (40 mg), potassium carbonate (90 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 100°C. To the reaction mixture was added saturated aqueous sodium bicarbonate solution, and the mixture was extracted with ethyl acetate. The obtained organic layer was washed successively with water and saturated brine, and dried over anhydrous magnesium sulfate, and the solvent was evaporated under reduced pressure. The residue was purified by HPLC (C18, mobile phase: water/acetonitrile (containing 5 mM NH₄HCO₃)), to the obtained fraction was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure. To a solution of the residue in ethanol (2.0 mL) was added 1 M hydrochloric acid (0.15 mL), the mixture was stirred at room temperature for 5 min, and the solvent was evaporated under reduced pressure to give the title compound (10 mg).
MS (ESI+) : [M+H]⁺ 337.2.

### Example 8

### (3S)-3-cyclopropyl-1-(2-((5-methyl-1,2-oxazol-3-yl)amino)pyridin-4-yl)-2-oxopyrrolidine-3-carbonitrile

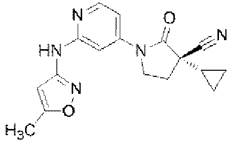

To a solution of (3S)-1-(2-bromopyridin-4-yl)-3-cyclopropyl-2-oxopyrrolidine-3-carbonitrile obtained in Step E of Example 1 (100 mg) in tetrahydrofuran (2.0 mL) were added 5-methyl-1,2-oxazol-3-amine (39 mg), cesium carbonate (210 mg), 4,5-bis(diphenylphosphino)-9,9-dimethylxanthene (15 mg) and tris(dibenzylideneacetone)dipalladium(0) (12 mg) under argon atmosphere, and the mixture was stirred overnight at 100°C. To the reaction mixture was added saturated aqueous sodium bicarbonate solution, and the mixture was extracted with ethyl acetate. The obtained organic layer was washed successively with water and saturated brine, and dried over anhydrous magnesium sulfate, and the solvent was evaporated under reduced pressure. The residue was purified by silica gel column chromatography (NH, hexane/ethyl acetate) to give the title compound (9.0 mg).
MS(ESI+): [M+H]⁺ 324.1.

### Example 9

### (3S)-3-cyclopropyl-1-(2-((3-(1-hydroxy-2-methylpropan-2-yl)-1,2-oxazol-5-yl)amino)pyridin-4-yl)-2-oxopyrrolidine-3-carbonitrile

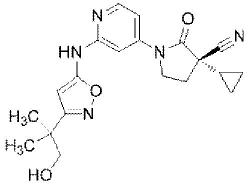

To a solution of (3S)-1-(2-bromopyridin-4-yl)-3-cyclopropyl-2-oxopyrrolidine-3-carbonitrile obtained in Step E of Example 1 (200 mg) in 2-methyl-2-butanol (4.0 mL) were added 2-(5-amino-1,2-oxazol-3-yl)-2-methylpropan-1-ol (120 mg), potassium carbonate (180 mg), 2-(dicyclohexylphosphino)-3,6-dimethoxy-2',4',6'-triisopropyl-1,1'-biphenyl (18 mg) and chloro[2-(dicyclohexylphosphino)-3,6-dimethoxy-2',4',6'-triisopropyl-1,1'-biphenyl][2-(2-aminoethyl)phenyl]palladium(II) (26 mg) under argon atmosphere, and the mixture was stirred overnight at 70°C. To the reaction mixture was added saturated aqueous sodium bicarbonate solution, and the mixture was extracted with ethyl acetate. The obtained organic layer was washed successively with water and saturated brine, and dried over anhydrous magnesium sulfate, and the solvent was evaporated under reduced pressure. The residue was purified by silica gel column chromatography (NH, hexane/ethyl acetate) to give the title compound (85 mg). MS(ESI+): [M+H]⁺ 382.2.

### Example 10

### (3S)-3-cyclopropyl-1-(2-((pyridin-2-yl)amino)pyridin-4-yl)-2-oxopyrrolidine-3-carbonitrile

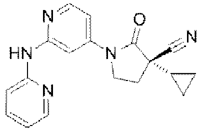

A mixture of (3S)-1-(2-bromopyridin-4-yl)-3-cyclopropyl-2-oxopyrrolidine-3-carbonitrile obtained in Step E of Example 1 (24 mg), 2-aminopyridine (23 mg), cesium carbonate (52 mg), 2-(dicyclohexylphosphino)-3,6-dimethoxy-2',4',6'-triisopropyl-1,1'-biphenyl (2.0 mg), chloro[2-(dicyclohexylphosphino)-3,6-dimethoxy-2',4',6'-triisopropyl-1,1'-biphenyl][2-(2-aminoethyl)phenyl]palladium(II) (3.0 mg) and 2-methyl-2-butanol (1.0 mL) was stirred in a microwave reactor at 120°C for 1 hr. To the reaction mixture were added ethyl acetate (2.0 mL) and water (1.0 mL), and the mixture was stirred for 5 min. The organic layer was filtered through Top-Phase Separation Fiter Tube, and the solvent was evaporated at 60°C. The residue was purified by HPLC (C18, mobile phase: acetonitrile/10 mM aqueous ammonium hydrogencarbonate solution), and dried at 60°C to give the title compound. MS (ESI+) : [M+H]⁺ 319.9.

### Examples 11 to 28

The title compounds of Examples 11 to 28 shown in Tables 1 to 3 were obtained using (3S)-1-(2-bromopyridin-4-yl)-3-cyclopropyl-2-oxopyrrolidine-3-carbonitrile obtained in Step E of Example 1 and the reagents corresponding to the compounds of Examples 11 to 28 (these reagents can be produced according to a method known per se), in the same manner as in the method of the method of Example 10. MS in the tables means actual measured value.

**Table 1**

| Ex. No. | IUPAC Name | Structure | Salt | MS | Corresponding Reagent |
|---|---|---|---|---|---|
| 11 | (3S)-3-cyclopropyl-1-(2-((1-methyl-1H-pyrazol-3-yl)amino)pyridin-4-yl)-2-oxopyrrolidine-3-carbonitrile | 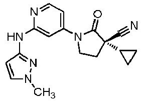 | Free | 322.9 | 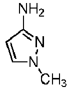 |
| 12 | (3S)-3-cyclopropyl-2-oxo-1-(2-(pyrimidin-2-ylamino)pyridin-4-yl)pyrrolidine-3-carbonitrile | 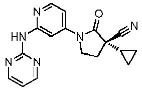 | Free | 320.9 | 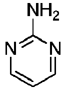 |
| 13 | (3S)-1-(2-anilinopyridin-4-yl)-3-cyclopropyl-2-oxopyrrolidine-3-carbonitrile | 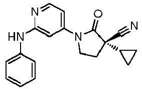 | Free | 318.9 | 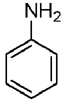 |
| 14 | (3S)-3-cyclopropyl-2-oxo-1-(2-(pyrimidin-4-ylamino)pyridin-4-yl)pyrrolidine-3-carbonitrile | 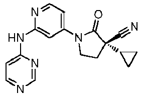 | Free | 320.9 | 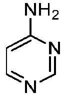 |
| 15 | (3S)-3-cyclopropyl-2-oxo-1-(2-(pyrazin-2-ylamino)pyridin-4-yl)pyrrolidine-3-carbonitrile | 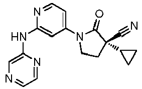 | Free | 320.9 | 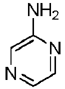 |
| 16 | (3S)-3-cyclopropyl-1-(2-((4-methylpyridin-2-yl)amino)pyridin-4-yl)-2-oxopyrrolidine-3-carbonitrile | 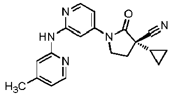 | Free | 333.9 | 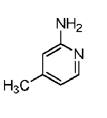 |
| 17 | (3S)-3-cyclopropyl-1-(2-((4-ethylpyridin-2-yl)amino)pyridin-4-yl)-2-oxopyrrolidine-3-carbonitrile | 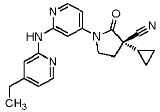 | Free | 347.9 | 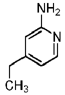 |

**Table 2**

| Ex. No. | IUPAC Name | Structure | Salt | MS | Corresponding Reagent |
|---|---|---|---|---|---|
| 18 | 2-((4-((3S)-3-cyano-3-cyclopropyl-2-oxopyrrolidin-1-yl)pyridin-2-yl)amino)isonicotinamide | 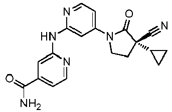 | Free | 362.9 | 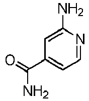 |
| 19 | (3S)-3-cyclopropyl-2-oxo-1-(2-((4-(trifluoromethyl)pyridin-2-yl)amino)pyridin-4-yl)pyrrolidine-3-carbonitrile | 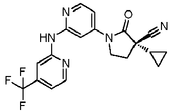 | Free | 387.9 | 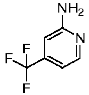 |
| 20 | (3S)-3-cyclopropyl-1-(2-((5-methylpyridin-2-yl)amino)pyridin-4-yl)-2-oxopyrrolidine-3-carbonitrile | 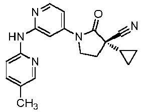 | Free | 333.9 | 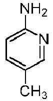 |
| 21 | (3S)-3-cyclopropyl-1-(2-((6-methylpyridin-2-yl)amino)pyridin-4-yl)-2-oxopyrrolidine-3-carbonitrile | 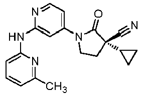 | Free | 333.9 | 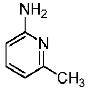 |
| 22 | (3S)-3-cyclopropyl-1-(2-((4-methoxypyridin-2-yl)amino)pyridin-4-yl)-2-oxopyrrolidine-3-carbonitrile | 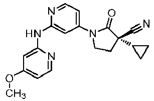 | Free | 349.9 | 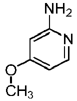 |
| 23 | (3S)-3-cyclopropyl-2-oxo-1-(2-((5-phenylpyridin-2-yl)amino)pyridin-4-yl)pyrrolidine-3-carbonitrile | 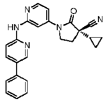 | Free | 396.0 | 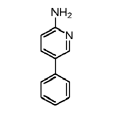 |
| 24 | (3S)-3-cyclopropyl-1-(2-(isoquinolin-3-ylamino)pyridin-4-yl)-2-oxopyrrolidine-3-carbonitrile | 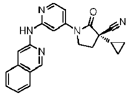 | Free | 369.9 | 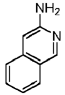 |
| 25 | (3S)-3-cyclopropyl-2-oxo-1-(2-((6-(trifluoromethyl)pyridin-2-yl)amino)pyridin-4-yl)pyrrolidine-3-carbonitrile | 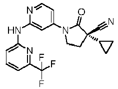 | Free | 387.9 | 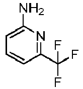 |

**Table 3**

| Ex. No. | IUPAC Name | Structure | Salt | MS | Corresponding Reagent |
|---|---|---|---|---|---|
| 26 | (3S)-3-cyclopropyl-2-oxo-1-(2-(quinolin-2-ylamino)pyridin-4-yl)pyrrolidine-3-carbonitrile | 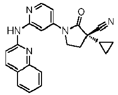 | Free | 369.9 | 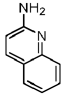 |
| 27 | (3S)-3-cyclopropyl-1-(2-((5-fluoropyridin-2-yl)amino)pyridin-4-yl)-2-oxopyrrolidine-3-carbonitrile | 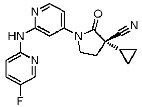 | Free | 337.9 | 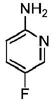 |
| 28 | (3S)-3-cyclopropyl-1-(2-((5-(hydroxymethyl)pyridin-2-yl)amino)pyridin-4-yl)-2-oxopyrrolidine-3-carbonitrile | 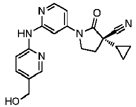 | Free | 349.9 | 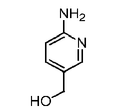 |

### Example 29

### N-(2-((4-((3S)-3-cyano-3-cyclopropyl-2-oxopyrrolidin-1-yl)pyridin-2-yl)amino)pyridin-4-yl)acetamide

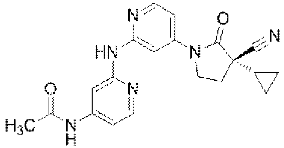

The title compound (94 mg) was obtained using (3S)-1-(2-aminopyridin-4-yl)-3-cyclopropyl-2-oxopyrrolidine-3-carbonitrile obtained in Example 2 (100 mg) and N-(2-bromopyridin-4-yl)acetamide (89 mg), in the same manner as in the method of the method of Example 3.
MS (ESI+) : [M+H]⁺ 377.0.

### Example 30

### N-(6-((4-((3S)-3-cyano-3-cyclopropyl-2-oxopyrrolidin-1-yl)pyridin-2-yl)amino)pyridin-2-yl)acetamide

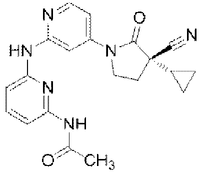

The title compound (110 mg) was obtained using (3S)-1-(2-aminopyridin-4-yl)-3-cyclopropyl-2-oxopyrrolidine-3-carbonitrile obtained in Example 2 (100 mg) and N-(6-bromopyridin-2-yl)acetamide (98 mg), in the same manner as in the method of Example 3.
MS (ESI+) : [M+H]⁺ 377.0.

### Example 31

### (3S)-3-cyclopropyl-1-(2-((1-methyl-5-(morpholin-4-yl)-1H-pyrazol-3-yl)amino)pyridin-4-yl)-2-oxopyrrolidine-3-carbonitrile

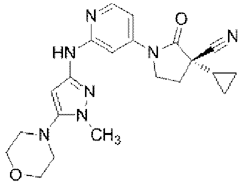

To a solution of (3S)-1-(2-aminopyridin-4-yl)-3-cyclopropyl-2-oxopyrrolidine-3-carbonitrile obtained in Example 2 (200 mg) in 2-methyl-2-propanol (2.0 mL) were added 4-(3-bromo-1-methyl-1H-pyrazol-5-yl)morpholine obtained in Step B of Reference Example 3 (200 mg), potassium carbonate (230 mg), 2-(dicyclohexylphosphino)-3,6-dimethoxy-2',4',6'-triisopropyl-1,1'-biphenyl (22 mg) and chloro[2-(dicyclohexylphosphino)-3,6-dimethoxy-2',4',6'-triisopropyl-1,1'-biphenyl][2-(2-aminoethyl)phenyl]palladium(II) (33 mg) under argon atmosphere, and the mixture was stirred overnight at 100°C. The reaction mixture was cooled to 0°C, saturated aqueous sodium bicarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by HPLC (C18, mobile phase: water/acetonitrile (containing 0.1% TFA)), to the obtained fraction was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure to give the title compound (20 mg).
MS (ESI+) : [M+H]⁺ 408.4.

### Examples 32 to 37

The title compounds of Examples 32 to 37 shown in Table 4 were obtained using (3S)-1-(2-aminopyridin-4-yl)-3-cyclopropyl-2-oxopyrrolidine-3-carbonitrile obtained in Example 2 and the reagents corresponding to the compounds of Examples 32 to 37 (these reagents can be produced according to a method known per se), in the same manner as in the method of Example 31. MS in the tables means actual measured value.

**Table 4**

| Ex. No. | IUPAC Name | Structure | Salt | MS | Corresponding Reagent |
|---|---|---|---|---|---|
| 32 | (3S)-3-cyclopropyl-1-(2-((5-(1-(isopropylsulfonyl)-3-methoxyazetidin-3-yl)-1-methyl-1H-pyrazol-3-yl)amino)pyridin-4-yl)-2-oxopyrrolidine-3-carbonitrile | 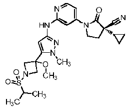 | Free | 514.2 | 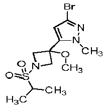 |
| 33 | 1-(3-((4-((3S)-3-cyano-3-cyclopropyl-2-oxopyrrolidin-1-yl)pyridin-2-yl)amino)-1-methyl-1H-pyrazol-5-yl)-4-methylpiperidine-4-carbonitrile | 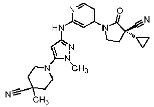 | Free | 445.2 | 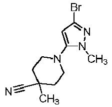 |
| 34 | (3S)-3-cyclopropyl-1-(2-((5-(4, 4-difluoropiperidin-1-yl)-1-methyl-1H-pyrazol-3-yl)amino)pyridin-4-yl)-2-oxopyrrolidine-3-carbonitrile | 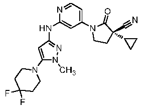 | Free | 442.1 | 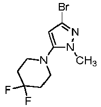 |
| 35 | (3S)-3-cyclopropyl-1-(2-((5-(4-hydroxy-4-(trifluoromethyl)piperidin-1-yl)-1-methyl-1H-pyrazol-3-yl)amino)pyridin-4-yl)-2-oxopyrrolidine-3-carbonitrile | 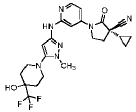 | Free | 490.2 | 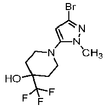 |
| 36 | (3S)-3-cyclopropyl-1-(2-((5-((2R)-2-(hydroxymethyl)pyrrolidin-1-yl)-1-methyl-1H-pyrazol-3-yl)amino)pyridin-4-yl)-2-oxopyrrolidine-3-carbonitrile hydrochloride | 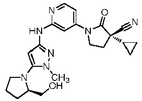 | HCl | 422.3 | 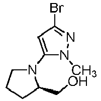 |
| 37 | (3S)-3-cyclopropyl-l-(2-((5-(2-hydroxypropan-2-yl)-1-methyl-1H-pyrazol-3-yl)amino)pyridin-4-yl)-2-oxopyrrolidine-3-carbonitrile hydrochloride | 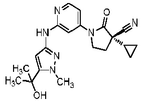 | HCl | 381.2 | 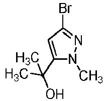 |

### Examples 38 to 44

The title compounds of Examples 38 to 44 shown in Table 5 were obtained using (3S)-1-(2-aminopyridin-4-yl)-3-cyclopropyl-2-oxopyrrolidine-3-carbonitrile obtained in Example 2 and the reagents corresponding to the compounds of Examples 38 to 44 (these reagents can be produced according to a method known per se), in the same manner as in the method of Example 31. MS in the tables means actual measured value.

**Table 5**

| Ex. No. | IUPAC Name | Structure | Salt | MS | Corresponding Reagent |
|---|---|---|---|---|---|
| 38 | (3S)-3-cyclopropyl-1-(2-((6-methoxypyrimidin-4-yl)amino)pyridin-4-yl)-2-oxopyrrolidine-3-carbonitrile | 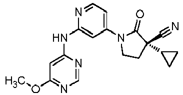 | Free | 351.2 | 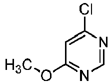 |
| 39 | (3S)-3-cyclopropyl-1-(2-((6-(difluoromethyl)pyrimidin-4-yl)amino)pyridin-4-yl)-2-oxopyrrolidine-3-carbonitrile | 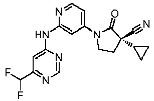 | Free | 371.1 | 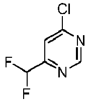 |
| 40 | (3S)-3-cyclopropyl-1-(2-((2-methylpyrimidin-4-yl)amino)pyridin-4-yl)-2-oxopyrrolidine-3-carbonitrile | 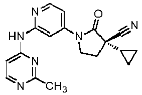 | Free | 335.2 | 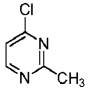 |
| 41 | (3S)-3-cyclopropyl-1-(2-((6-methylpyrimidin-4-yl)amino)pyridin-4-yl)-2-oxopyrrolidine-3-carbonitrile | 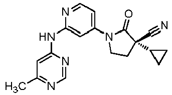 | Free | 335.1 | 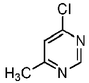 |
| 42 | N-(6-((4-((3S)-3-cyano-3-cyclopropyl-2-oxopyrrolidin-1-yl)pyridin-2-yl)amino)pyrimidin-4-yl)acetamide | 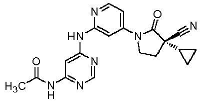 | Free | 378.2 | 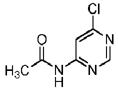 |
| 43 | (3S)-3-cyclopropyl-2-oxo-1-(2-((6-(trifluoromethyl)pyrimidin-4-yl)amino)pyridin-4-yl)pyrrolidine-3-carbonitrile | 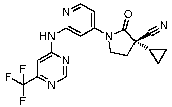 | Free | 389.1 | 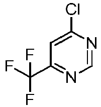 |
| 44 | (3S)-3-cyclopropyl-1-(2-((6-cyclopropylpyrimidin-4-yl)amino)pyridin-4-yl)-2-oxopyrrolidine-3-carbonitrile | 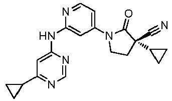 | Free | 361.2 | 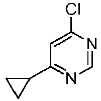 |

### Examples 45 to 70

The title compounds of Examples 45 to 70 shown in Tables 6 to 9 were obtained using (3S)-1-(2-aminopyridin-4-yl)-3-cyclopropyl-2-oxopyrrolidine-3-carbonitrile obtained in Example 2 and the reagents corresponding to the compounds of Examples 45 to 70 (these reagents can be produced according to a method known per se), in the same manner as in the method of Example 3 or a method analogous thereto. MS in the tables means actual measured value.

**Table 6**

| Ex. No. | IUPAC Name | Structure | Salt | MS | Corresponding Reagent |
|---|---|---|---|---|---|
| 45 | (3S)-3-cyclopropyl-1-(2-((4-(4-hydroxytetrahydro-2H-pyran-4-yl)pyridin-2-yl)amino)pyridin-4-yl)-2-oxopyrrolidine-3-carbonitrile | 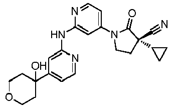 | Free | 420.1 | 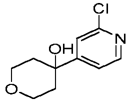 |
| 46 | (3S)-3-cyclopropyl-2-oxo-1-(2-((4-(tetrahydro-2H-pyran-4-yloxy)pyridin-2-yl)amino)pyridin-4-yl)pyrrolidine-3-carbonitrile | 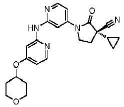 | Free | 420.1 | 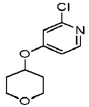 |
| 47 | (3S)-3-cyclopropyl-1-(2-((4-(2-hydroxy-2-methylpropoxy)pyridin-2-yl)amino)pyridin-4-yl)-2-oxopyrrolidine-3-carbonitrile | 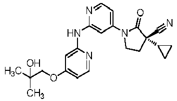 | Free | 408.1 | 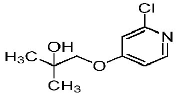 |
| 48 | (3S)-3-cyclopropyl-1-(2-((4-(2-methoxyethoxy)pyridin-2-yl)amino)pyridin-4-yl)-2-oxopyrrolidine-3-carbonitrile | 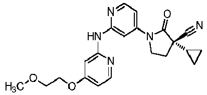 | Free | 394.0 | 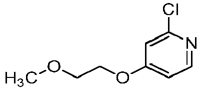 |
| 49 | 2-((4-((3S)-3-cyano-3-cyclopropyl-2-oxopyrrolidin-1-yl)pyridin-2-yl)amino)-N-(tetrahydro-2H-pyran-4-yl)isonicotinamide | 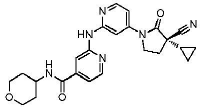 | Free | 447.1 | 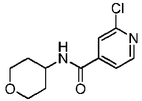 |
| 50 | 2-((4-((3S)-3-cyano-3-cyclopropyl-2-oxopyrrolidin-1-yl)pyridin-2-yl)amino)-N-(2-hydroxyethyl)isonicotinamide | 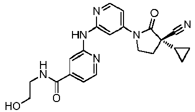 | Free | 407.0 | 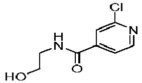 |
| 51 | (3S)-3-cyclopropyl-1-(2-((4-(morpholin-4-ylcarbonyl)pyridin-2-yl)amino)pyridin-4-yl)-2-oxopyrrolidine-3-carbonitrile | 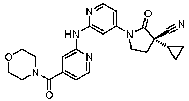 | Free | 433.0 | 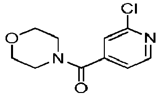 |

**Table 7**

| Ex. No. | IUPAC Name | Structure | Salt | MS | Corresponding Reagent |
|---|---|---|---|---|---|
| 52 | (3S)-3-cyclopropyl-2-oxo-1-(2-((4-(2-oxopyrrolidin-1-yl)pyridin-2-yl)amino)pyridin-4-yl)pyrrolidine-3-carbonitrile | 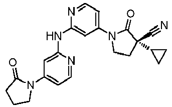 | Free | 403.0 | 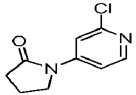 |
| 53 | 2-((4-((3S)-3-cyano-3-cyclopropyl-2-oxopyrrolidin-1-yl)pyridin-2-yl)amino)isonicotinonitrile | 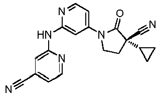 | Free | 344.9 | 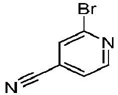 |
| 54 | (3S)-3-cyclopropyl-1-(2-((5-(4-hydroxytetrahydro-2H-pyran-4-yl)pyridin-2-yl)amino)pyridin-4-yl)-2-oxopyrrolidine-3-carbonitrile | 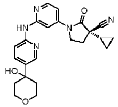 | Free | 420.1 | 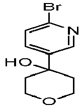 |
| 55 | (3S)-3-cyclopropyl-2-oxo-1-(2-((5-(tetrahydro-2H-pyran-4-yloxy)pyridin-2-yl)amino)pyridin-4-yl)pyrrolidine-3-carbonitrile | 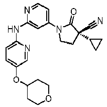 | Free | 420.1 | 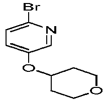 |
| 56 | (3S)-3-cyclopropyl-1-(2-((5-(2-hydroxy-2-methylpropoxy)pyridin-2-yl)amino)pyridin-4-yl)-2-oxopyrrolidine-3-carbonitrile | 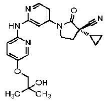 | Free | 408.1 | 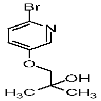 |
| 57 | (3S)-3-cyclopropyl-1-(2-((5-(2-methoxyethoxy)pyridin-2-yl)amino)pyridin-4-yl)-2-oxopyrrolidine-3-carbonitrile | 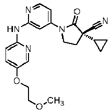 | Free | 394.0 | 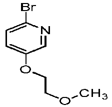 |
| 58 | N-(2-((6-((4-((3S)-3-cyano-3-cyclopropyl-2-oxopyrrolidin-1-yl)pyridin-2-yl)amino)pyridin-3-yl)oxy)ethyl)acetamide | 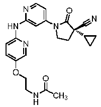 | Free | 421.1 | 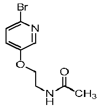 |
| 59 | 2-((6-((4-((3S)-3-cyano-3-cyclopropyl-2-oxopyrrolidin-1-yl)pyridin-2-yl)amino)pyridin-3-yl)oxy)-N-methylacetamide | 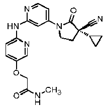 | Free | 407.1 | 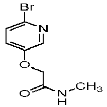 |

**Table 8**

| Ex. No. | IUPAC Name | Structure | Salt | MS | Corresponding Reagent |
|---|---|---|---|---|---|
| 60 | (3S)-1-(2-((5-(4-acetylpiperazin-1-yl)pyridin-2-yl)amino)pyridin-4-yl)-3-cyclopropyl-2-oxopyrrolidine-3-carbonitrile | 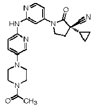 | Free | 446.1 | 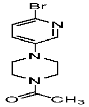 |
| 61 | (3S)-3-cyclopropyl-1-(2-((5-(4-methylpiperazin-1-yl)pyrazin-2-yl)amino)pyridin-4-yl)-2-oxopyrrolidine-3-carbonitrile | 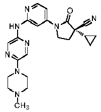 | Free | 419.1 | 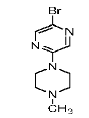 |
| 62 | (3S)-3-cyclopropyl-1-(2-((5-(morpholin-4-yl)pyrazin-2-yl)amino)pyridin-4-yl)-2-oxopyrrolidine-3-carbonitrile | 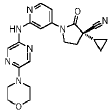 | Free | 406.0 | 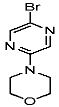 |
| 63 | (3S)-3-cyclopropyl-1-(2-((5-(hydroxymethyl)pyrazin-2-yl)amino)pyridin-4-yl)-2-oxopyrrolidine-3-carbonitrile | 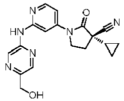 | Free | 350.9 | 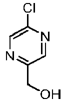 |
| 64 | (3S)-3-cyclopropyl-1-(2-((5-(4, 4-difluoro-1-hydroxycyclohexyl)pyridin-2-yl)amino)pyridin-4-yl)-2-oxopyrrolidine-3-carbonitrile | 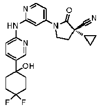 | Free | 454.1 | 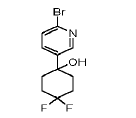 |
| 65 | (3S)-3-cyclopropyl-1-(2-((5-(4, 4-difluoropiperidin-1-yl)pyrazin-2-yl)amino)pyridin-4-yl)-2-oxopyrrolidine-3-carbonitrile | 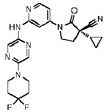 | Free | 440.0 | 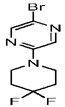 |
| 66 | N-(6-((4-((3S)-3-cyano-3-cyclopropyl-2-oxopyrrolidin-1-yl)pyridin-2-yl)amino)pyridin-3-yl)acetamide | 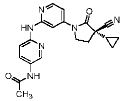 | Free | 376.9 | 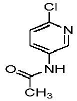 |
| 67 | 6-((4-((3S)-3-cyano-3-cyclopropyl-2-oxopyrrolidin-1-yl)pyridin-2-yl)amino)nicotinonitrile | 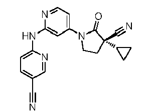 | Free | 344.9 | 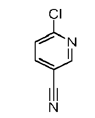 |

**Table 9**

| Ex. No. | IUPAC Name | Structure | Salt | MS | Corresponding Reagent |
|---|---|---|---|---|---|
| 68 | 6-((4-((3S)-3-cyano-3-cyclopropyl-2-oxopyrrolidin-1-yl)pyridin-2-yl)amino)-N-cyclopropylpyridine-3-sulfonamide | 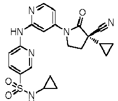 | Free | 439.0 | 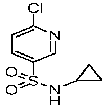 |
| 69 | (3S)-3-cyclopropyl-1-(2-((6-(morpholin-4-yl)pyrimidin-4-yl)amino)pyridin-4-yl)-2-oxopyrrolidine-3-carbonitrile | 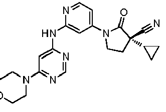 | Free | 406.1 | 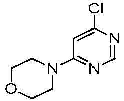 |
| 70 | (3S)-3-cyclopropyl-1-(2-((5-(morpholin-4-ylmethyl)pyrazin-2-yl)amino)pyridin-4-yl)-2-oxopyrrolidine-3-carbonitrile | 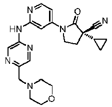 | Free | 420.1 | 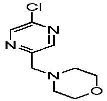 |

### Example 71

### tert-butyl 6-((4-((3S)-3-cyano-3-cyclopropyl-2-oxopyrrolidin-1-yl)pyridin-2-yl)amino)nicotinate

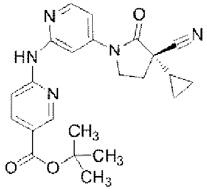

The title compound (800 mg) was obtained using (3S)-1-(2-aminopyridin-4-yl)-3-cyclopropyl-2-oxopyrrolidine-3-carbonitrile obtained in Example 2 (500 mg) and tert-butyl 6-bromonicotinate (640 mg), in the same manner as in the method of Example 3.
MS (ESI+) : [M+H]⁺ 420.4.

### Example 72

### 6-((4-((3S)-3-cyano-3-cyclopropyl-2-oxopyrrolidin-1-yl)pyridin-2-yl)amino)nicotinamide hydrochloride

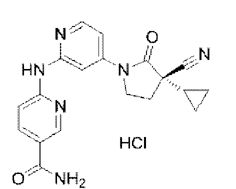

### A) 6-((4-((3S)-3-cyano-3-cyclopropyl-2-oxopyrrolidin-1-yl)pyridin-2-yl)amino)nicotinic acid hydrochloride

The crude title compound (820 mg) was obtained using tert-butyl 6-((4-((3S)-3-cyano-3-cyclopropyl-2-oxopyrrolidin-1-yl)pyridin-2-yl)amino)nicotinate obtained in Example 71 (800 mg), in the same manner as in the method of Step A of Example 5.
MS (ESI+) : [M+H]⁺ 364.3.

### B) 6-((4-((3S)-3-cyano-3-cyclopropyl-2-oxopyrrolidin-1-yl)pyridin-2-yl)amino)nicotinamide hydrochloride

To a solution of the crude 6-((4-((3S)-3-cyano-3-cyclopropyl-2-oxopyrrolidin-1-yl)pyridin-2-yl)amino)nicotinic acid hydrochloride obtained in Step A of Example 72 (130 mg) in acetonitrile (2.0 mL) were added 1-ethyl-3-(3-dimethylaminopropyl)carbodiimide (100 mg), 1-hydroxybenzotriazole ammonium salt (130 mg), triethylamine (0.46 mL) and ammonium chloride (88 mg), and the mixture was stirred overnight at 50°C. To the reaction mixture was added water, and the precipitate was collected by filtration, and purified by HPLC (C18, mobile phase: water/acetonitrile (containing 0.1% TFA)). To the obtained fraction was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure. To a solution of the residue in ethanol (2.0 mL) was added 1 M hydrochloric acid (0.30 mL), and the mixture was stirred at room temperature for 5 min, and the solvent was evaporated under reduced pressure to give the title compound (19 mg).
MS(ESI+): [M+H]⁺ 363.0.

### Example 73

### 6-((4-((3S)-3-cyano-3-cyclopropyl-2-oxopyrrolidin-1-yl)pyridin-2-yl)amino)-N-methylnicotinamide

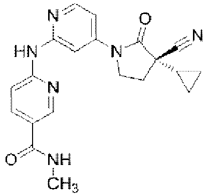

To a solution of 6-((4-((3S)-3-cyano-3-cyclopropyl-2-oxopyrrolidin-1-yl)pyridin-2-yl)amino)nicotinic acid hydrochloride obtained in Step A of Example 72 (100 mg) in N,N-dimethylformamide (2.0 mL) were added 1-ethyl-3-(3-dimethylaminopropyl)carbodiimide (120 mg), 1-hydroxybenzotriazole (100 mg), triethylamine (0.35 mL), 4-dimethylaminopyridine (3.0 mg) and methylamine hydrochloride (51 mg), and the mixture was stirred overnight at room temperature, and then at 50°C for 2 hr. The reaction mixture was cooled to 0°C, saturated aqueous sodium bicarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate) to give the title compound (31 mg). MS (ESI+) : [M+H]⁺ 377.3.

### Examples 74 to 76

The title compounds of Examples 74 to 76 shown in Table 10 were obtained using 6-((4-((3S)-3-cyano-3-cyclopropyl-2-oxopyrrolidin-1-yl)pyridin-2-yl)amino)nicotinic acid hydrochloride obtained in Step A of Example 72 and the reagents corresponding to the compounds of Examples 74 to 76 (these reagents can be produced according to a method known per se), in the same manner as in the method of Example 73. MS in the tables means actual measured value.

**Table 10**

| Ex. No. | IUPAC Name | Structure | Salt | MS | Corresponding Reagent |
|---|---|---|---|---|---|
| 74 | 6-((4-((3S)-3-cyano-3-cyclopropyl-2-oxopyrrolidin-1-yl)pyridin-2-yl)amino)-N,N-dimethylnicotinamide | 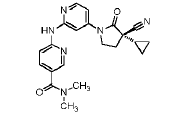 | Free | 391.3 | 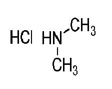 |
| 75 | (3S)-3-cyclopropyl-1-(2-((5-((4,4-difluoropiperidin-1-yl)carbonyl)pyridin-2-yl)amino)pyridin-4-yl)-2-oxopyrrolidine-3-carbonitrile | 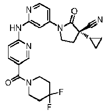 | Free | 467.2 | 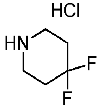 |
| 76 | (3S)-3-cyclopropyl-1-(2-((5-((4-hydroxy-4-methylpiperidin-1-yl)carbonyl)pyridin-2-yl)amino)pyridin-4-yl)-2-oxopyrrolidine-3-carbonitrile | 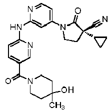 | Free | 461.2 | 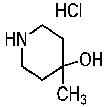 |

### Example 77

### 3-((4-((3S)-3-cyano-3-cyclopropyl-2-oxopyrrolidin-1-yl)pyridin-2-yl)amino)-N,N,1-trimethyl-1H-pyrazole-5-carboxamide

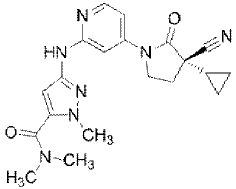

The title compound (12 mg) was obtained using 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 (110 mg) and dimethylamine hydrochloride (56 mg), in the same manner as in the method of Step B of Example 5.
MS (ESI+) : [M+H]⁺ 394.2.

### Example 78

### (3S,3'S)-1,1'-(iminodipyridine-2,4-diyl)bis(3-cyclopropyl-2-oxopyrrolidine-3-carbonitrile)

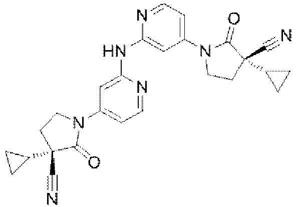

The title compound (61 mg) was obtained using (3S)-1-(2-bromopyridin-4-yl)-3-cyclopropyl-2-oxopyrrolidine-3-carbonitrile obtained in Step E of Example 1 (100 mg) and (3S)-1-(2-aminopyridin-4-yl)-3-cyclopropyl-2-oxopyrrolidine-3-carbonitrile obtained in Example 2 (80 mg), in the same manner as in the method of Example 3.
MS (ESI+) : [M+H]⁺ 468.1.

### Example 79

### methyl 6-((4-((3S)-3-cyano-3-cyclopropyl-2-oxopyrrolidin-1-yl)pyridin-2-yl)amino)nicotinate

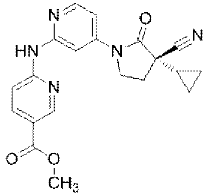

To a solution of (3S)-1-(2-aminopyridin-4-yl)-3-cyclopropyl-2-oxopyrrolidine-3-carbonitrile obtained in Example 2 (100 mg) in 2-methyl-2-propanol (2.0 mL) were added methyl 6-bromonicotinate (110 mg), cesium carbonate (270 mg), 2-(dicyclohexylphosphino)-3,6-dimethoxy-2',4',6'-triisopropyl-1,1'-biphenyl (89 mg) and tris(dibenzylideneacetone)dipalladium(0) (38 mg) under argon atmosphere, and the mixture was stirred overnight at 100°C. To the reaction mixture was added saturated aqueous sodium bicarbonate solution, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (hexane/ethyl acetate) to give the title compound (26 mg).
MS (ESI+) : [M+H]⁺ 378.3.

### Examples 80 to 91

The title compounds of Examples 80 to 91 shown in Tables 11 and 12 were obtained using (3S)-1-(2-aminopyridin-4-yl)-3-cyclopropyl-2-oxopyrrolidine-3-carbonitrile obtained in Example 2 and the reagents corresponding to the compounds of Examples 80 to 91 (these reagents can be produced according to a method known per se), in the same manner as in the method of Example 3 or a method analogous thereto. MS in the tables means actual measured value.

**Table 11**

| Ex. No. | IUPAC Name | Structure | Salt | MS | Corresponding Reagent |
|---|---|---|---|---|---|
| 80 | (3S)-1-(2-((5-(2-cyanopropan-2-yl)pyridin-2-yl)amino)pyridin-4-yl)-3-cyclopropyl-2-oxopyrrolidine-3-carbonitrile | 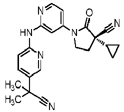 | Free | 387.2 | 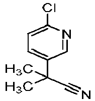 |
| 81 | (3S)-3-cyclopropyl-1-(2-((4-(methoxymethyl)pyridin-2-yl)amino)pyridin-4-yl)-2-oxopyrrolidine-3-carbonitrile hydrochloride | 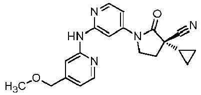 | HCl | 364.2 | 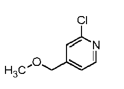 |
| 82 | 2-((4-((3S)-3-cyano-3-cyclopropyl-2-oxopyrrolidin-1-yl)pyridin-2-yl)amino)-N-methylisonicotinamide | 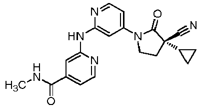 | Free | 377.2 | 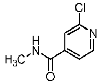 |
| 83 | (3S)-3-cyclopropyl-1-(2-((5-(1,1-dioxidothiomorpholin-4-yl)pyridin-2-yl)amino)pyridin-4-yl)-2-oxopyrrolidine-3-carbonitrile | 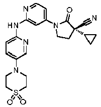 | Free | 453.2 | 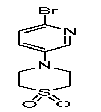 |
| 84 | (3S)-3-cyclopropyl-1-(2-((4-(2-hydroxypropan-2-yl)pyridin-2-yl)amino)pyridin-4-yl)-2-oxopyrrolidine-3-carbonitrile | 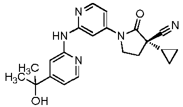 | Free | 378.2 | 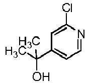 |
| 85 | N-((2-((4-((3S)-3-cyano-3-cyclopropyl-2-oxopyrrolidin-1-yl)pyridin-2-yl)amino)pyridin-4-yl)methyl)acetamide | 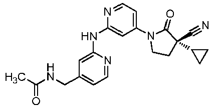 | Free | 391.2 | 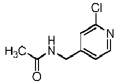 |
| 86 | 2-(6-((4-((3S)-3-cyano-3-cyclopropyl-2-oxopyrrolidin-1-yl)pyridin-2-yl)amino)pyridin-3-yl)-N,2-dimethylpropanamide | 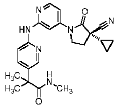 | Free | 419.2 | 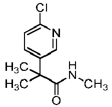 |

**Table 12**

| Ex. No. | IUPAC Name | Structure | Salt | MS | Corresponding Reagent |
|---|---|---|---|---|---|
| 87 | (3S)-1-(2-((5-(1-cyanocyclobutyl)pyridin-2-yl)amino)pyridin-4-yl)-3-cyclopropyl-2-oxopyrrolidine-3-carbonitrile | 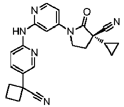 | Free | 399.2 | 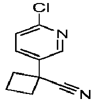 |
| 88 | (3S)-3-cyclopropyl-1-(2-((5-(2-methoxypropan-2-yl)pyridin-2-yl)amino)pyridin-4-yl)-2-oxopyrrolidine-3-carbonitrile | 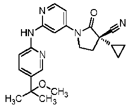 | Free | 392.2 | 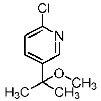 |
| 89 | (3S)-3-cyclopropyl-1-(2-((5-(1-hydroxy-2-methylpropan-2-yl)pyridin-2-yl)amino)pyridin-4-yl)-2-oxopyrrolidine-3-carbonitrile | 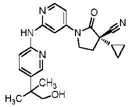 | Free | 392.2 | 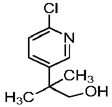 |
| 90 | N-((2-((4-((3S)-3-cyano-3-cyclopropyl-2-oxopyrrolidin-1-yl)pyridin-2-yl)amino)pyridin-4-yl)methyl)-N-methylacetamide | 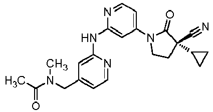 | Free | 405.2 | 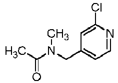 |
| 91 | N-((2-((4-((3S)-3-cyano-3-cyclopropyl-2-oxopyrrolidin-1-yl)pyridin-2-yl)amino)pyridin-4-yl)methyl)methanesulfonamide | 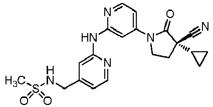 | Free | 427.2 | 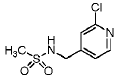 |

### Example 92

### (3S)-3-cyclopropyl-2-oxo-1-(2-(pyridazin-3-ylamino)pyridin-4-yl)pyrrolidine-3-carbonitrile

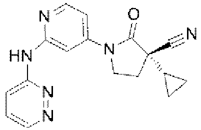

To a solution of (3S)-1-(2-aminopyridin-4-yl)-3-cyclopropyl-2-oxopyrrolidine-3-carbonitrile obtained in Example 2 (100 mg) in 2-methyl-2-propanol (2.0 mL) were added 3-chloropyridazine (52 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred overnight at 100°C. The reaction mixture was cooled to 0°C, saturated aqueous sodium bicarbonate solution was added thereto, and the mixture was extracted with ethyl acetate. The obtained organic layer was washed with water and saturated brine, and dried over anhydrous magnesium sulfate, and the solvent was evaporated under reduced pressure. The obtained residue was purified by HPLC (C18, mobile phase: water/acetonitrile (containing 0.1% TFA)). To the obtained fraction was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure to give the title compound (20 mg) .
MS (ESI+) : [M+H]⁺ 320.9.

### Examples 93 to 97

The title compounds of Examples 93 to 97 shown in Table 13 were obtained using (3S)-1-(2-aminopyridin-4-yl)-3-cyclopropyl-2-oxopyrrolidine-3-carbonitrile obtained in Example 2 and the reagents corresponding to the compounds of Examples 93 to 97 (these reagents can be produced according to a method known per se), in the same manner as in the method of Example 92 or a method analogous thereto. MS in the tables means actual measured value.

**Table 13**

| Ex. No. | IUPAC Name | Structure | Salt | MS | Corresponding Reagent |
|---|---|---|---|---|---|
| 93 | (3S)-3-cyclopropyl-1-(2-((4-(morpholin-4-yl)pyridin-2-yl)amino)pyridin-4-yl)-2-oxopyrrolidine-3-carbonitrile | 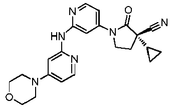 | Free | 405.2 | 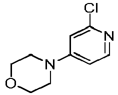 |
| 94 | 2-(6-((4-((3S)-3-cyano-3-cyclopropyl-2-oxopyrrolidin-1-yl)pyridin-2-yl)amino)pyridin-3-yl)-2-methylpropanamide | 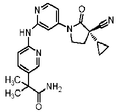 | Free | 405.2 | 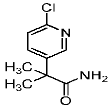 |
| 95 | 2-(6-((4-((3S)-3-cyano-3-cyclopropyl-2-oxopyrrolidin-1-yl)pyridin-2-yl)amino)pyridin-3-yl)acetamide | 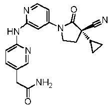 | Free | 377.4 | 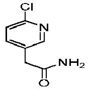 |
| 96 | (3S)-3-cyclopropyl-1-(2-((1-ethyl-5-(morpholin-4-yl)-1H-pyrazol-3-yl)amino)pyridin-4-yl)-2-oxopyrrolidine-3-carbonitrile | 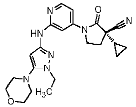 | Free | 422.2 | 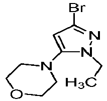 |
| 97 | (3S)-3-cyclopropyl-1-(2-((5-(1-hydroxycyclobutyl)-1-methyl-1H-pyrazol-3-yl)amino)pyridin-4-yl)-2-oxopyrrolidine-3-carbonitrile hydrochloride | 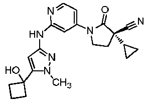 | HCl | 393.2 | 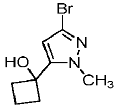 |

### Example 98

### 2-(3-((4-((3S)-3-cyano-3-cyclopropyl-2-oxopyrrolidin-1-yl)pyridin-2-yl)amino)-1H-pyrazol-1-yl)acetamide hydrochloride

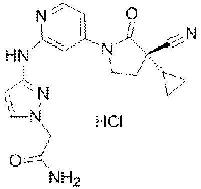

### A) tert-butyl (3-((4-((3S)-3-cyano-3-cyclopropyl-2-oxopyrrolidin-1-yl)pyridin-2-yl)amino)-1H-pyrazol-1-yl)acetate

The title compound (390 mg) was obtained using (3S)-1-(2-bromopyridin-4-yl)-3-cyclopropyl-2-oxopyrrolidine-3-carbonitrile obtained in Step E of Example 1 (300 mg) and tert-butyl (3-amino-1H-pyrazol-1-yl)acetate (210 mg), in the same manner as in the method of Example 6.
MS (ESI+) : [M+H]⁺ 423.2.

### B) (3-((4-((3S)-3-cyano-3-cyclopropyl-2-oxopyrrolidin-1-yl)pyridin-2-yl)amino)-1H-pyrazol-1-yl)acetic acid hydrochloride

The title compound (330 mg) was obtained using tert-butyl (3-((4-((3S)-3-cyano-3-cyclopropyl-2-oxopyrrolidin-1-yl)pyridin-2-yl)amino)-1H-pyrazol-1-yl)acetate obtained in Step A of Example 98 (390 mg), in the same manner as in the method of Step A of Example 5.
MS (ESI+) : [M+H]⁺ 367.1.

### C) 2-(3-((4-((3S)-3-cyano-3-cyclopropyl-2-oxopyrrolidin-1-yl)pyridin-2-yl)amino)-1H-pyrazol-1-yl)acetamide hydrochloride

To a solution of (3-((4-((3S)-3-cyano-3-cyclopropyl-2-oxopyrrolidin-1-yl)pyridin-2-yl)amino)-1H-pyrazol-1-yl)acetic acid hydrochloride obtained in Step B of Example 98 (100 mg) in N,N-dimethylformamide (2.0 mL) were added 1-ethyl-3-(3-dimethylaminopropyl)carbodiimide (77 mg), 1-hydroxybenzotriazole ammonium salt (100 mg) and triethylamine (0.14 mL), and the mixture was stirred overnight at room temperature, and then at 50°C for 5 hr. The reaction mixture was cooled to 0°C, saturated aqueous sodium bicarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, ethyl acetate/methanol). The obtained crude product was purified by HPLC (C18, mobile phase: water/acetonitrile (containing 5 mM NH₄HCO₃)), to the obtained fraction was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure. To a solution of the residue in ethanol (2.0 mL) was added 1 M hydrochloric acid (0.10 mL), and the mixture was stirred at room temperature for 5 min, and the solvent was evaporated under reduced pressure to give the title compound (9.0 mg).
MS (ESI+) : [M+H]⁺ 366.2.

### Example 99

### 2-(3-((4-((3S)-3-cyano-3-cyclopropyl-2-oxopyrrolidin-1-yl)pyridin-2-yl)amino)-1H-pyrazol-1-yl)-N-methylacetamide hydrochloride

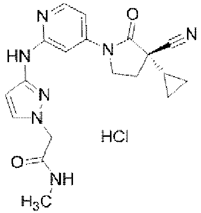

To a solution of (3-((4-((3S)-3-cyano-3-cyclopropyl-2-oxopyrrolidin-1-yl)pyridin-2-yl)amino)-1H-pyrazol-1-yl)acetic acid hydrochloride obtained in Step B of Example 98 (100 mg) in N,N-dimethylformamide (2.0 mL) were added 1-ethyl-3-(3-dimethylaminopropyl)carbodiimide (77 mg), 1-hydroxybenzotriazole (100 mg), methylamine hydrochloride (42 mg) and triethylamine (0.14 mL), and the mixture was stirred overnight at room temperature, and then at 50°C for 5 hr. The reaction mixture was cooled to 0°C, saturated aqueous sodium bicarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, ethyl acetate/methanol). The obtained crude product was purified by HPLC (C18, mobile phase: water/acetonitrile (containing 5 mM NH₄HCO₃)), to the obtained fraction was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure. To a solution of the residue in ethanol (2.0 mL) was added 1 M hydrochloric acid (0.10 mL), and the mixture was stirred at room temperature for 5 min, and the solvent was evaporated under reduced pressure to give the title compound (14 mg).
MS (ESI+) : [M+H]⁺ 380.2.

### Example 100

### 2-(3-((4-((3S)-3-cyano-3-cyclopropyl-2-oxopyrrolidin-1-yl)pyridin-2-yl)amino)-1H-pyrazol-1-yl)-N,N-dimethylacetamide hydrochloride

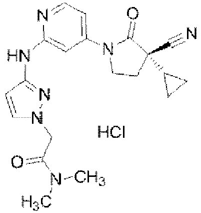

The title compound (10 mg) was obtained using (3-((4-((3S)-3-cyano-3-cyclopropyl-2-oxopyrrolidin-1-yl)pyridin-2-yl)amino)-1H-pyrazol-1-yl)acetic acid hydrochloride obtained in Step B of Example 98 (100 mg) and dimethylamine hydrochloride (51 mg), in the same manner as in the method of Example 99.
MS (ESI+) : [M+H]⁺ 394.2.

### Example 101

### (3S)-3-cyclopropyl-1-(2-((3-(2-hydroxypropan-2-yl)-1,2-oxazol-5-yl)amino)pyridin-4-yl)-2-oxopyrrolidine-3-carbonitrile

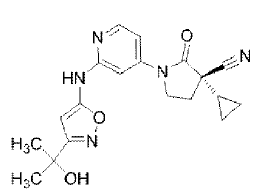

To a solution of 2-(5-amino-1,2-oxazol-3-yl)propan-2-ol obtained in Step B of Reference Example 30 (120 mg) and (3S)-1-(2-bromopyridin-4-yl)-3-cyclopropyl-2-oxopyrrolidine-3-carbonitrile obtained in Step E of Example 1 (120 mg) in tetrahydrofuran (3.0 mL) were added potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred overnight at 100°C. To the reaction mixture was added water, and the mixture was extracted with ethyl acetate. The obtained organic layer was washed with saturated brine, and dried over anhydrous magnesium sulfate, and the solvent was evaporated under reduced pressure. The obtained residue was purified by HPLC (C18, mobile phase: water/acetonitrile (containing 0.1% TFA)), to the obtained fraction was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure to give the title compound (8.0 mg).
MS (ESI+) : [M+H]⁺ 368.2.

### Example 102

### (3S)-3-cyclopropyl-1-(2-((5-(1-hydroxy-2-methylpropan-2-yl)-1-methyl-1H-pyrazol-3-yl)amino)pyridin-4-yl)-2-oxopyrrolidine-3-carbonitrile

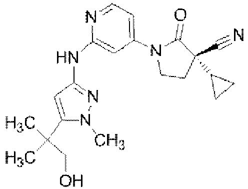

To a solution of 2-(3-amino-1-methyl-1H-pyrazol-5-yl)-2-methylpropan-1-ol obtained in Reference Example 27 (91 mg) and (3S)-1-(2-bromopyridin-4-yl)-3-cyclopropyl-2-oxopyrrolidine-3-carbonitrile obtained in Step E of Example 1 (150 mg) in cyclopentyl methyl ether (3.0 mL) were added potassium carbonate (140 mg), 4,5-bis(diphenylphosphino)-9,9-dimethylxanthene (4.0 mg) and tris(dibenzylideneacetone)dipalladium(0) (18 mg) under argon atmosphere, and the mixture was stirred overnight at 100°C. To the reaction mixture was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (NH, hexane/ethyl acetate). The obtained crude product was purified by HPLC (C18, mobile phase: water/acetonitrile (containing 0.1% TFA)), to the obtained fraction was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure to give the title compound (8.0 mg).
MS (ESI+) : [M+H]⁺ 395.2.

### Example 103

### (3S)-3-cyclopropyl-1-(2-((5-(3,6-dihydro-2H-pyran-4-yl)pyridin-2-yl)amino)pyridin-4-yl)-2-oxopyrrolidine-3-carbonitrile

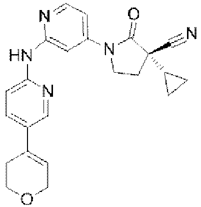

To a solution of 5-(3,6-dihydro-2H-pyran-4-yl)pyridin-2-amine (95 mg) and (3S)-1-(2-bromopyridin-4-yl)-3-cyclopropyl-2-oxopyrrolidine-3-carbonitrile obtained in Step E of Example 1 (150 mg) in 2-methyl-2-propanol (2.0 mL) were added cesium carbonate (320 mg), 2-(dicyclohexylphosphino)-3,6-dimethoxy-2',4',6'-triisopropyl-1,1'-biphenyl (13 mg) and chloro[2-(dicyclohexylphosphino)-3,6-dimethoxy-2',4',6'-triisopropyl-1,1'-biphenyl][2-(2-aminoethyl)phenyl]palladium(II) (39 mg) under argon atmosphere, and the mixture was stirred overnight at 100°C. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (NH, hexane/ethyl acetate). The obtained crude was purified by HPLC (C18, mobile phase: water/acetonitrile (containing 0.1% TFA)), to the obtained fraction was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure. The residue was recrystallized (methanol) to give the title compound (16 mg).
MS (ESI+) : [M+H]⁺ 402.4.

### Example 104

### (3S)-3-cyclopropyl-2-oxo-1-(2-((5-(tetrahydro-2H-pyran-4-yl)pyridin-2-yl)amino)pyridin-4-yl)pyrrolidine-3-carbonitrile

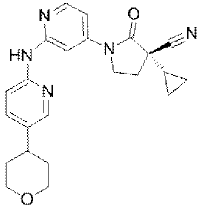

To a solution of 5-(tetrahydro-2H-pyran-4-yl)pyridin-2-amine (64 mg) and (3S)-1-(2-bromopyridin-4-yl)-3-cyclopropyl-2-oxopyrrolidine-3-carbonitrile obtained in Step E of Example 1 (100 mg) in 2-methyl-2-propanol (2.0 mL) were added potassium carbonate (90 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 100°C. The insoluble substance was removed through Celite, and the solvent was evaporated under reduced pressure. The residue was purified by HPLC (C18, mobile phase: water/acetonitrile (containing 0.1% TFA)), to the obtained fraction was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure to give the title compound (10 mg).
MS(ESI+): [M+H]⁺ 404.4.

### Example 105

### (3S)-1-(2-((1'-acetyl-1',2',3',6'-tetrahydro-3,4'-bipyridin-6-yl)amino)pyridin-4-yl)-3-cyclopropyl-2-oxopyrrolidine-3-carbonitrile

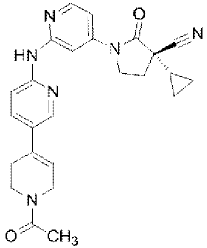

### A) tert-butyl 6-((4-((3S)-3-cyano-3-cyclopropyl-2-oxopyrrolidin-1-yl)pyridin-2-yl)amino)-3',6'-dihydro-3,4'-bipyridine-1'(2'H)-carboxylate

The title compound (90 mg) was obtained using (3S)-1-(2-bromopyridin-4-yl)-3-cyclopropyl-2-oxopyrrolidine-3-carbonitrile obtained in Step E of Example 1 (100 mg) and tert-butyl 6-amino-3',6'-dihydro-3,4'-bipyridine-1'(2'H)-carboxylate (99 mg) under argon atmosphere, in the same manner as in the method of Example 104.
MS (ESI+) : [M+H]⁺ 501.5.

### B) (3S)-1-(2-((1'-acetyl-1',2',3',6'-tetrahydro-3,4'-bipyridin-6-yl)amino)pyridin-4-yl)-3-cyclopropyl-2-oxopyrrolidine-3-carbonitrile

To tert-butyl 6-((4-((3S)-3-cyano-3-cyclopropyl-2-oxopyrrolidin-1-yl)pyridin-2-yl)amino)-3',6'-dihydro-3,4'-bipyridine-1'(2'H)-carboxylate obtained in Step A of Example 105 (90 mg) was added 4 M hydrogen chloride ethyl acetate solution (8.0 mL), the mixture was stirred at room temperature for 3 hr, and the solvent was evaporated under reduced pressure. To a solution of the residue in tetrahydrofuran (1.0 mL) were added triethylamine (0.13 mL) and acetic anhydride (0.020 mL), and the mixture was stirred overnight at room temperature. To the reaction mixture was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (NH, ethyl acetate/methanol) to give the title compound (27 mg).
MS (ESI+) : [M+H]⁺ 443.4.

### Example 106

### (3S)-1-(2-((5-(1-acetylpiperidin-4-yl)pyridin-2-yl)amino)pyridin-4-yl)-3-cyclopropyl-2-oxopyrrolidine-3-carbonitrile hydrochloride

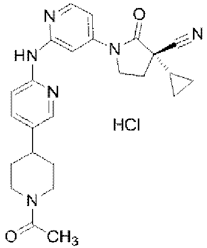

### A) tert-butyl 4-(6-((4-((3S)-3-cyano-3-cyclopropyl-2-oxopyrrolidin-1-yl)pyridin-2-yl)amino)pyridin-3-yl)piperidine-1-carboxylate

The title compound (410 mg) was obtained using (3S)-1-(2-bromopyridin-4-yl)-3-cyclopropyl-2-oxopyrrolidine-3-carbonitrile obtained in Step E of Example 1 (300 mg) and tert-butyl 4-(6-aminopyridin-3-yl)piperidine-1-carboxylate (300 mg), in the same manner as in the method of Step A of Example 105.
MS (ESI+) : [M+H]⁺ 503.5.

### B) (3S)-3-cyclopropyl-2-oxo-1-(2-((5-(piperidin-4-yl)pyridin-2-yl)amino)pyridin-4-yl)pyrrolidine-3-carbonitrile hydrochloride

To tert-butyl 4-(6-((4-((3S)-3-cyano-3-cyclopropyl-2-oxopyrrolidin-1-yl)pyridin-2-yl)amino)pyridin-3-yl)piperidine-1-carboxylate obtained in Step A of Example 106 (410 mg) was added 4 M hydrogen chloride ethyl acetate solution (10 mL), the mixture was stirred at room temperature for 3 hr, and the solvent was evaporated under reduced pressure to give the title compound (330 mg).
MS (ESI+) : [M+H]⁺ 403.4.

### C) (3S)-1-(2-((5-(1-acetylpiperidin-4-yl)pyridin-2-yl)amino)pyridin-4-yl)-3-cyclopropyl-2-oxopyrrolidine-3-carbonitrile hydrochloride

To a solution of (3S)-3-cyclopropyl-2-oxo-1-(2-((5-(piperidin-4-yl)pyridin-2-yl)amino)pyridin-4-yl)pyrrolidine-3-carbonitrile hydrochloride obtained in Step B of Example 106 (100 mg) in tetrahydrofuran (2.0 mL) were added triethylamine (0.13 mL) and acetic anhydride (0.021 mL), and the mixture was stirred overnight at room temperature. To the reaction mixture was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (NH, ethyl acetate/methanol). To a solution of the obtained crude product in ethanol (2.0 mL) was added 1 M hydrochloric acid (0.10 mL), the mixture was stirred at room temperature for 5 min, and the solvent was evaporated under reduced pressure to give the title compound (42 mg).
MS (ESI+) : [M+H]⁺ 445.4

### Example 107

### (2-((4-((3S)-3-cyano-3-cyclopropyl-2-oxopyrrolidin-1-yl)pyridin-2-yl)amino)pyridin-4-yl)acetic acid hydrochloride

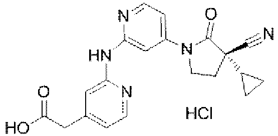

### A) tert-butyl (2-((4-((3S)-3-cyano-3-cyclopropyl-2-oxopyrrolidin-1-yl)pyridin-2-yl)amino)pyridin-4-yl)acetate

The title compound (430 mg) was obtained using (3S)-1-(2-aminopyridin-4-yl)-3-cyclopropyl-2-oxopyrrolidine-3-carbonitrile obtained in Example 2 (300 mg) and tert-butyl (2-bromopyridin-4-yl)acetate (400 mg), in the same manner as in the method of Example 3.
MS (ESI+) : [M+H]⁺434.2.

### B) (2-((4-((3S)-3-cyano-3-cyclopropyl-2-oxopyrrolidin-1-yl)pyridin-2-yl)amino)pyridin-4-yl)acetic acid hydrochloride

The title compound (410 mg) was obtained using tert-butyl (2-((4-((3S)-3-cyano-3-cyclopropyl-2-oxopyrrolidin-1-yl)pyridin-2-yl)amino)pyridin-4-yl)acetate obtained in Step A of Example 107 (430 mg), in the same manner as in the method of Step A of Example 5.
MS (ESI+) : [M+H]⁺ 378.2.

### Example 108

### 2-(2-((4-((3S)-3-cyano-3-cyclopropyl-2-oxopyrrolidin-1-yl)pyridin-2-yl)amino)pyridin-4-yl)acetamide

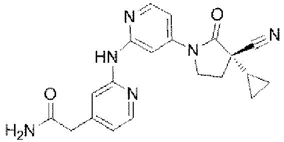

To a solution of (2-((4-((3S)-3-cyano-3-cyclopropyl-2-oxopyrrolidin-1-yl)pyridin-2-yl)amino)pyridin-4-yl)acetic acid hydrochloride obtained in Step B of Example 107 (100 mg) in N,N-dimethylformamide (1.0 mL) were added 1-ethyl-3-(3-dimethylaminopropyl)carbodiimide (75 mg), 1-hydroxybenzotriazole ammonium salt (98 mg) and triethylamine (0.14 mL), and the mixture was stirred overnight at room temperature. The reaction mixture was cooled to 0°C, saturated aqueous sodium bicarbonate solution was added thereto, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (NH, ethyl acetate/methanol) to give the title compound (50 mg).
MS (ESI+) : [M+H]+ 377.2.

### Example 109

### 2-(2-((4-((3S)-3-cyano-3-cyclopropyl-2-oxopyrrolidin-1-yl)pyridin-2-yl)amino)pyridin-4-yl)-N-methylacetamide

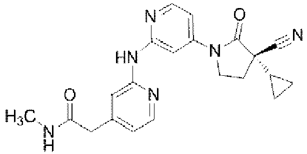

The title compound (53 mg) was obtained using (2-((4-((3S)-3-cyano-3-cyclopropyl-2-oxopyrrolidin-1-yl)pyridin-2-yl)amino)pyridin-4-yl)acetic acid hydrochloride obtained in Step B of Example 107 (100 mg) and methylamine hydrochloride (33 mg), in the same manner as in the method of Step B of Example 5.
MS (ESI+) : [M+H]⁺391.2.

### Example 110

### 2-(2-((4-((3S)-3-cyano-3-cyclopropyl-2-oxopyrrolidin-1-yl)pyridin-2-yl)amino)pyridin-4-yl)-N,N-dimethylacetamide

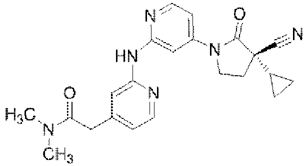

The title compound (50 mg) was obtained using (2-((4-((3S)-3-cyano-3-cyclopropyl-2-oxopyrrolidin-1-yl)pyridin-2-yl)amino)pyridin-4-yl)acetic acid hydrochloride obtained in Step B of Example 107 (100 mg) and dimethylamine hydrochloride (40 mg), in the same manner as in the method of Step B of Example 5.
MS (ESI+) : [M+H]⁺405.2.

### Example 111

### tert-butyl 2-(2-((4-((3S)-3-cyano-3-cyclopropyl-2-oxopyrrolidin-1-yl)pyridin-2-yl)amino)pyridin-4-yl)-2-methylpropanoate

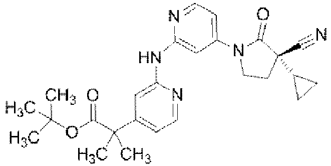

### A) tert-butyl 2-(2-bromopyridin-4-yl)-2-methylpropanoate

To a solution of tert-butyl (2-bromopyridin-4-yl)acetate (690 mg) in tetrahydrofuran (10 mL) was added sodium hydride (60% in mineral oil, 0.23 g) under ice bath, and the mixture was stirred at 0°C for 20 min. To the reaction mixture was added iodomethane (0.40 mL) at 0°C, and the mixture was stirred at room temperature for 3 hr. To the reaction mixture was added saturated aqueous sodium bicarbonate solution, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (510 mg).
MS(ESI+): [M+H]⁺ 300.0.

### B) tert-butyl 2-(2-((4-((3S)-3-cyano-3-cyclopropyl-2-oxopyrrolidin-1-yl)pyridin-2-yl)amino)pyridin-4-yl)-2-methylpropanoate

The title compound (500 mg) was obtained using tert-butyl 2-(2-bromopyridin-4-yl)-2-methylpropanoate obtained in Step A of Example 111 (450 mg) and (3S)-1-(2-aminopyridin-4-yl)-3-cyclopropyl-2-oxopyrrolidine-3-carbonitrile obtained in Example 2 (300 mg), in the same manner as in the method of Example 3.
MS (ESI+) : [M+H]⁺ 462.3.

### Example 112

### 2-(2-((4-((3S)-3-cyano-3-cyclopropyl-2-oxopyrrolidin-1-yl)pyridin-2-yl)amino)pyridin-4-yl)-2-methylpropanoic acid hydrochloride

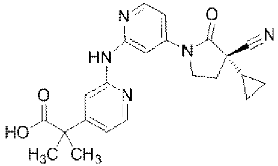

The title compound (480 mg) was obtained using tert-butyl 2-(2-((4-((3S)-3-cyano-3-cyclopropyl-2-oxopyrrolidin-1-yl)pyridin-2-yl)amino)pyridin-4-yl)-2-methylpropanoate obtained in Step B of Example 111 (500 mg), in the same manner as in the method of Step A of Example 5. MS(ESI+): [M+H]⁺ 406.2.

### Example 113

### 2-(2-((4-((3S)-3-cyano-3-cyclopropyl-2-oxopyrrolidin-1-yl)pyridin-2-yl)amino)pyridin-4-yl)-2-methylpropanamide

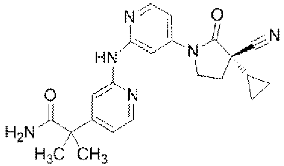

The title compound (50 mg) was obtained using 2-(2-((4-((3S)-3-cyano-3-cyclopropyl-2-oxopyrrolidin-1-yl)pyridin-2-yl)amino)pyridin-4-yl)-2-methylpropanoic acid hydrochloride obtained in Example 112 (100 mg), in the same manner as in the method of Example 108.
MS (ESI+) : [M+H]⁺ 405.2.

### Example 114

### 2-(2-((4-((3S)-3-cyano-3-cyclopropyl-2-oxopyrrolidin-1-yl)pyridin-2-yl)amino)pyridin-4-yl)-N,2-dimethylpropanamide

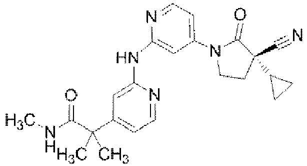

The title compound (50 mg) was obtained using 2-(2-((4-((3S)-3-cyano-3-cyclopropyl-2-oxopyrrolidin-1-yl)pyridin-2-yl)amino)pyridin-4-yl)-2-methylpropanoic acid hydrochloride obtained in Example 112 (100 mg) and methylamine hydrochloride (31 mg), in the same manner as in the method of Step B of Example 5.
MS (ESI+) : [M+H]+ 419.2.

### Example 115

### 2-(2-((4-((3S)-3-cyano-3-cyclopropyl-2-oxopyrrolidin-1-yl)pyridin-2-yl)amino)pyridin-4-yl)-N,N,2-trimethylpropanamide

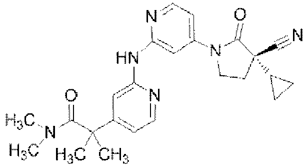

The title compound (37 mg) was obtained using 2-(2-((4-((3S)-3-cyano-3-cyclopropyl-2-oxopyrrolidin-1-yl)pyridin-2-yl)amino)pyridin-4-yl)-2-methylpropanoic acid hydrochloride obtained in Example 112 (100 mg) and dimethylamine hydrochloride (37 mg), in the same manner as in the method of Step B of Example 5.
MS (ESI+) : [M+H]⁺33.2.

### Example 116

### (3S)-3-cyclopropyl-1-(2-((5-(2-methoxypropan-2-yl)-1-methyl-1H-pyrazol-3-yl)amino)pyridin-4-yl)-2-oxopyrrolidine-3-carbonitrile

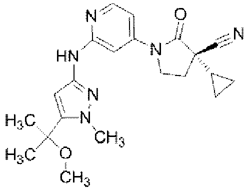

### A) 2-(3-(2,5-dimethyl-1H-pyrrol-1-yl)-1-methyl-1H-pyrazol-5-yl)propan-2-ol

The title compound (1.8 g) was obtained using 3-(2,5-dimethyl-1H-pyrrol-1-yl)-1-methyl-1H-pyrazole (2.0 g) and acetone (3.4 mL), in the same manner as in the method of Step A of Example 4.
MS (ESI+) : [M+H]⁺34.1.

### B) 3-(2,5-dimethyl-1H-pyrrol-1-yl)-5-(2-methoxypropan-2-yl)-1-methyl-1H-pyrazole

To a solution of 2-(3-(2,5-dimethyl-1H-pyrrol-1-yl)-1-methyl-1H-pyrazol-5-yl)propan-2-ol obtained in Step A of Example 116 (380 mg) in N,N-dimethylformamide (7.0 mL) was added sodium hydride (60% in mineral oil, 80 mg), and the mixture was stirred at the same temperature for 20 min. To the reaction mixture was added iodomethane (0.15 mL) at 0°C, and the mixture was stirred for 5.5 hr while gradually raising the temperature to room temperature. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (NH, hexane/ethyl acetate) to give the title compound (320 mg).
MS(ESI+): [M+H]⁺ 248.0.

### C) 5-(2-methoxypropan-2-yl)-1-methyl-1H-pyrazol-3-amine

The title compound (250 mg) was obtained using 3-(2,5-dimethyl-1H-pyrrol-1-yl)-5-(2-methoxypropan-2-yl)-1-methyl-1H-pyrazole obtained in Step B of Example 116 (320 mg), in the same manner as in the method of Step B of Example 4.
MS (ESI+) : [M+H]⁺169.8.

### D) (3S)-3-cyclopropyl-1-(2-((5-(2-methoxypropan-2-yl)-1-methyl-1H-pyrazol-3-yl)amino)pyridin-4-yl)-2-oxopyrrolidine-3-carbonitrile

The title compound (41 mg) was obtained using 5-(2-methoxypropan-2-yl)-1-methyl-1H-pyrazol-3-amine obtained in Step C of Example 116 (99 mg) and (3S)-1-(2-bromopyridin-4-yl)-3-cyclopropyl-2-oxopyrrolidine-3-carbonitrile obtained in Step E of Example 1 (150 mg), in the same manner as in the method of Example 104.
MS (ESI+) : [M+H]⁺395.1.

### Example 117

### (3S)-3-cyclopropyl-1-(2-((5-(4-hydroxytetrahydro-2H-pyran-4-yl)-1-methyl-1H-pyrazol-3-yl)amino)pyridin-4-yl)-2-oxopyrrolidine-3-carbonitrile

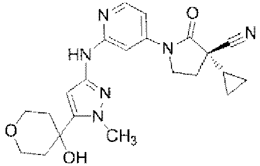

### A) 4-(3-(2,5-dimethyl-1H-pyrrol-1-yl)-1-methyl-1H-pyrazol-5-yl)tetrahydro-2H-pyran-4-ol

The title compound (760 mg) was obtained using 3-(2,5-dimethyl-1H-pyrrol-1-yl)-1-methyl-1H-pyrazole (2.0 g) and tetrahydro-4H-pyran-4-one (1.4 g), in the same manner as in the method of Step A of Example 116.
MS(ESI+): [M+H]⁺275.9.

### B) 4-(3-amino-1-methyl-1H-pyrazol-5-yl)tetrahydro-2H-pyran-4-ol

The title compound (120 mg) was obtained using 4-(3-(2,5-dimethyl-1H-pyrrol-1-yl)-1-methyl-1H-pyrazol-5-yl)tetrahydro-2H-pyran-4-ol obtained in Step A of Example 117 (760 mg), in the same manner as in the method of Step B of Example 4.
¹H NMR (400 MHz, DMSO-d₆) δ1.73-1.88 (4H, m), 3.60-3.75 (7H, m), 4.39 (2H, s), 5.21 (1H, s), 5.27 (1H, s).

### C) (3S)-3-cyclopropyl-1-(2-((5-(4-hydroxytetrahydro-2H-pyran-4-yl)-1-methyl-1H-pyrazol-3-yl)amino)pyridin-4-yl)-2-oxopyrrolidine-3-carbonitrile

The title compound (10 mg) was obtained using 4-(3-amino-1-methyl-1H-pyrazol-5-yl)tetrahydro-2H-pyran-4-ol obtained in Step B of Example 117 (120 mg) and (3S)-1-(2-bromopyridin-4-yl)-3-cyclopropyl-2-oxopyrrolidine-3-carbonitrile obtained in Step E of Example 1 (190 mg), in the same manner as in the method of Step F of Example 1.
MS(ESI+): [M+H]⁺423.2.

### Example 118

### tert-butyl 3-(5-((4-((3S)-3-cyano-3-cyclopropyl-2-oxopyrrolidin-1-yl)pyridin-2-yl)amino)-1-methyl-1H-pyrazol-3-yl)azetidine-1-carboxylate

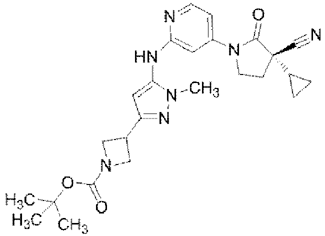

### A) tert-butyl 3-(cyanoacetyl)azetidine-1-carboxylate

To a solution of sodium hydride (60% in mineral oil, 2.8 g) in tetrahydrofuran (40 mL) was added dropwise a solution of 1-tert-butyl 3-methyl azetidine-1,3-dicarboxylate (10 g) and acetonitrile (3.7 mL) in tetrahydrofuran (40 mL) at 70°C under nitrogen atmosphere, and the mixture was stirred overnight at the same temperature. To the reaction mixture was added 1 M hydrochloric acid, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (1.2 g).
MS (ESI-), found: 223.1.

### B) tert-butyl 3-(5-amino-1-methyl-1H-pyrazol-3-yl)azetidine-1-carboxylate

To a solution of tert-butyl 3-(cyanoacetyl)azetidine-1-carboxylate obtained in Step A of Example 118 (1.2 g) in ethanol (10 mL) was added methylhydrazine hydrochloride (0.44 mL) at room temperature, and the mixture was stirred at 70°C for 1 hr. The solvent was evaporated under reduced pressure, and the residue was purified by silica gel column chromatography (NH, hexane/ethyl acetate) to give the title compound (880 mg).
¹H NMR (400MHz, DMSO-d₆)51.31-1.44 (9H, m), 3.44 (3H, s), 3.46-3.58 (1H, m), 3.78 (2H, brs), 3.99-4.15 (2H, m), 5.15 (2H, s), 5.20 (1H, s).

### C) tert-butyl 3-(5-((4-((3S)-3-cyano-3-cyclopropyl-2-oxopyrrolidin-1-yl)pyridin-2-yl)amino)-1-methyl-1H-pyrazol-3-yl)azetidine-1-carboxylate

The title compound (90 mg) was obtained using tert-butyl 3-(5-amino-1-methyl-1H-pyrazol-3-yl)azetidine-1-carboxylate obtained in Step B of Example 118 (99 mg) and (3S)-1-(2-bromopyridin-4-yl)-3-cyclopropyl-2-oxopyrrolidine-3-carbonitrile obtained in Step E of Example 1 (100 mg), in the same manner as in the method of Example 6.
MS (ESI-), found: 476.2.

### Example 119

### (3S)-3-cyclopropyl-1-(2-((1-ethyl-5-(2-hydroxypropan-2-yl)-1H-pyrazol-3-yl)amino)pyridin-4-yl)-2-oxopyrrolidine-3-carbonitrile

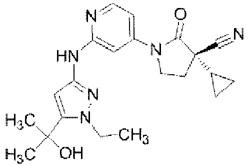

The title compound (91 mg) was obtained using 2-(3-amino-1-ethyl-1H-pyrazol-5-yl)propan-2-ol obtained in Step G of Reference Example 29 (91 mg) and (3S)-1-(2-bromopyridin-4-yl)-3-cyclopropyl-2-oxopyrrolidine-3-carbonitrile obtained in Step E of Example 1 (140 mg), in the same manner as in the method of Example 6.
MS (ESI+) : [M+H]⁺395.2.

### Example 120

### (3S)-3-cyclopropyl-1-(2-((4-fluoro-5-(1-hydroxy-2-methylpropan-2-yl)-1-methyl-1H-pyrazol-3-yl)amino)pyridin-4-yl)-2-oxopyrrolidine-3-carbonitrile

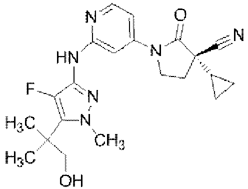

The title compound (28 mg) was obtained using 2-(3-amino-4-fluoro-1-methyl-1H-pyrazol-5-yl)-2-methylpropan-1-ol obtained in Step B of Reference Example 28 (88 mg) and (3S)-1-(2-bromopyridin-4-yl)-3-cyclopropyl-2-oxopyrrolidine-3-carbonitrile obtained in Step E of Example 1 (120 mg), in the same manner as in the method of Step C of Example 4.
MS(ESI+): [M+H]⁺413.2.

### Example 121

### optically active 1-(2-aminopyridin-4-yl)-3-isopropyl-2-oxopyrrolidine-3-carbonitrile

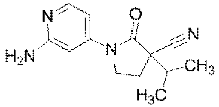

### A) 2-amino-4,5-dihydrofuran-3-carbonitrile

To a solution of malononitrile (6.0 g) and 2-chloroethanol (7.3 g) in methanol (40 mL) was added dropwise a solution of 28% sodium methoxide in methanol (17 mL), and the mixture was stirred at 50°C for 2 hr. The resulting sodium chloride was removed through Celite, and the solvent was evaporated under reduced pressure. Ice was added thereto, and the resulting solid was collected by filtration, and washed with cold water to give the title compound (3.5 g).
¹H NMR(300 MHz, DMSO-d₆)δ 2.59-2.91 (2H, m), 4.17-4.48 (2H, m), 6.93 (2H, brs).

### B) N-(3-cyano-4,5-dihydrofuran-2-yl)-4-fluorobenzamide

To a solution of 2-amino-4,5-dihydrofuran-3-carbonitrile obtained in Step A of Example 121 (3.5 g) in pyridine (8.0 mL) was added dropwise 4-fluorobenzoyl chloride (5.5 g) under ice bath, and the mixture was stirred at room temperature for 3 hr. The solvent was evaporated under reduced pressure, and to the residue was added dropwise saturated aqueous sodium hydrogencarbonate solution under ice bath. The resulting solid was collected by filtration, and washed with water to give the title compound (7.1 g).
¹H NMR(300 MHz, DMSO-d₆) δ 3.00 (2H, t, J = 9.2 Hz), 4.53 (2H, t, J = 9.2 Hz), 7.37 (2H, t, J = 8.9 Hz), 7.88-8.18 (2H, m), 11.16 (1H, s).

### C) 1-(4-fluorobenzoyl)-2-oxopyrrolidine-3-carbonitrile

To a solution of N-(3-cyano-4,5-dihydrofuran-2-yl)-4-fluorobenzamide obtained in Step B of Example 121 (5.0 g) in N,N-dimethylformamide (30 mL) was added sodium iodide (6.5 g), and the mixture was stirred at 150°C for 30 min. The solvent was evaporated under reduced pressure, and to the residue was added ice water. The resulting solid was collected by filtration, and washed with water. The residue was purified by silica gel column chromatography (hexane/ethyl acetate), and the resulting solid was washed with diisopropyl ether to give the title compound (1.3 g).
¹H NMR(300 MHz, DMSO-d₆) δ 2.28-2. 50 (2H, m), 3.74 (1H, td, J = 10.6, 6.8 Hz), 3.84-4.04 (1H, m), 4.43 (1H, dd, J = 11.3, 8.7 Hz), 7.15-7.39 (2H, m), 7.62-7.86 (2H, m).

### D) 1-(4-fluorobenzoyl)-3-isopropyl-2-oxopyrrolidine-3-carbonitrile

To a solution of 1-(4-fluorobenzoyl)-2-oxopyrrolidine-3-carbonitrile obtained in Step C of Example 121 (500 mg) in N,N-dimethylformamide (5.0 mL) was added sodium hydride (60% in mineral oil, 130 mg) under ice bath, and the mixture was stirred at the same temperature for 5 min. To the reaction mixture was added 2-iodopropane (1.1 mL) under ice bath, and the mixture was stirred at 50°C for 1 hr. To the reaction mixture was added 10% aqueous citric acid solution under ice bath, and the mixture was extracted with ethyl acetate. The extract was washed with saturated brine, and dried over anhydrous sodium sulfate, and the solvent was evaporated under reduced pressure. The residue was purified by silica gel column chromatography (hexane/ethyl acetate) to give the title compound (310 mg).
MS (ESI+) : [M+H]⁺275.2.

### E) 3-isopropyl-2-oxopyrrolidine-3-carbonitrile

To a solution of 1-(4-fluorobenzoyl)-3-isopropyl-2-oxopyrrolidine-3-carbonitrile obtained in Step D of Example 121 (280 mg) in tetrahydrofuran (3.0 mL) was added n-octylamine (180 µL), and the mixture was stirred overnight at room temperature. To the reaction mixture was added n-octylamine (170 µL) at room temperature, and the mixture was stirred at 50°C for 5 hr. The reaction mixture was concentrated under reduced pressure, and the residue was purified by silica gel column chromatography (hexane/ethyl acetate) to give the title compound (120 mg).
MS (ESI+) : [M+H]⁺152.9.

### F) optically active 3-isopropyl-2-oxopyrrolidine-3-carbonitrile (shorter retention time)

3-Isopropyl-2-oxopyrrolidine-3-carbonitrile obtained in Step E of Example 121 (2.6 g) was resolved by HPLC (column: CHIRALPAK IC, 50 mmID×500 mmL, manufactured by Daicel Chemical Industries, mobile phase: hexane/2-propanol = 200/800) to give the title compound having (1.2 g) a shorter retention time. >99.9% ee (HPLC (column: CHIRALPAK IC, 4.6 mmID×250 mmL, manufactured by Daicel Chemical Industries, mobile phase: hexane/2-propanol = 200/800, flow rate: 0.5 mL/min, retention time: 12.6 min))

### G) optically active 3-isopropyl-2-oxopyrrolidine-3-carbonitrile (longer retention time)

3-Isopropyl-2-oxopyrrolidine-3-carbonitrile obtained in Step E of Example 121 (2.6 g) was resolved by HPLC (column: CHIRALPAK IC, 50 mmID×500 mmL, manufactured by Daicel Chemical Industries, mobile phase: hexane/2-propanol = 200/800) to give the title compound (1.2 g) having a longer retention time. >99.9% ee (HPLC (column: CHIRALPAK IC, 4.6 mmID×250 mmL, manufactured by Daicel Chemical Industries, mobile phase: hexane/2-propanol = 200/800, flow rate: 0.5 mL/min, retention time: 18.0 min))

### H) optically active 1-(2-aminopyridin-4-yl)-3-isopropyl-2-oxopyrrolidine-3-carbonitrile

The title compound (700 mg) was obtained using optically active 3-isopropyl-2-oxopyrrolidine-3-carbonitrile obtained in Step F of Example 121 (shorter retention time, 500 mg) and 4-iodopyridin-2-amine (660 mg), in the same manner as in the method of Example 2.
MS (ESI+) : [M+H]⁺ 245.1.

### Example 122

### optically active 1-(2-aminopyridin-4-yl)-3-isopropyl-2-oxopyrrolidine-3-carbonitrile

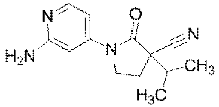

The title compound (730 mg) was obtained using optically active 3-isopropyl-2-oxopyrrolidine-3-carbonitrile obtained in Step G of Example 121 (longer retention time, 500 mg) and 4-iodopyridin-2-amine (660 mg), in the same manner as in the method of Example 2.
MS (ESI+) : [M+H]⁺245.1.

### Example 123

### optically active 3-isopropyl-1-(2-((6-methylpyrimidin-4-yl)amino)pyridin-4-yl)-2-oxopyrrolidine-3-carbonitrile

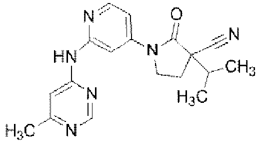

The title compound (61 mg) was obtained using optically active 1-(2-aminopyridin-4-yl)-3-isopropyl-2-oxopyrrolidine-3-carbonitrile obtained in Step H of Example 121 (100 mg) and 4-chloro-6-methylpyrimidine (53 mg), in the same manner as in the method of Example 3.
MS (ESI+) : [M+H]⁺337.2.

### Example 124

### optically active 3-isopropyl-1-(2-((6-methylpyrimidin-4-yl)amino)pyridin-4-yl)-2-oxopyrrolidine-3-carbonitrile

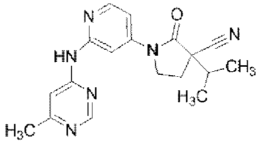

The title compound (58 mg) was obtained using optically active 1-(2-aminopyridin-4-yl)-3-isopropyl-2-oxopyrrolidine-3-carbonitrile obtained in Example 122 (100 mg) and 4-chloro-6-methylpyrimidine (58 mg), in the same manner as in the method of Example 3.
MS (ESI+) : [M+H]⁺337.2.

### Example 125

### N-(4-(3-cyano-3-ethyl-2-oxopyrrolidin-1-yl)pyridin-2-yl)cyclopropanecarboxamide

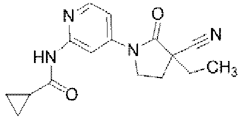

### A) 3-ethyl-1-(4-fluorobenzoyl)-2-oxopyrrolidine-3-carbonitrile

To a solution of 1-(4-fluorobenzoyl)-2-oxopyrrolidine-3-carbonitrile obtained in Step C of Example 121 (1.3 g) in N,N-dimethylformamide (10 mL) was added sodium hydride (60% in mineral oil, 340 mg) under ice bath, and the mixture was stirred at the same temperature for 30 min. To the reaction mixture was added dropwise iodoethane (0.90 mL) under ice bath, and the mixture was stirred at the same temperature for 30 min. To the reaction mixture was added saturated aqueous ammonium chloride solution, and the mixture was extracted with ethyl acetate. The obtained organic layer was washed successively with water and saturated brine, and dried over anhydrous magnesium sulfate, and the solvent was evaporated under reduced pressure. The residue was purified by silica gel column chromatography (hexane/ethyl acetate) to give the title compound (1.1 g).
¹H NMR(300 MHz, DMSO-d₆) δ 1.19 (3H, t, J = 7.6 Hz), 1.82 (1H, dq, J = 14.3, 7.2 Hz), 2.06-2.33 (2H, m), 2.63 (1H, dt, J = 13.3, 6.8 Hz), 4.03 (2H, t, J = 6.8 Hz), 7.04-7.20 (2H, m), 7.52-7.84 (2H, m).

### B) 3-ethyl-2-oxopyrrolidine-3-carbonitrile

To a solution of 3-ethyl-1-(4-fluorobenzoyl)-2-oxopyrrolidine-3-carbonitrile obtained in Step A of Example 125 (1.1 g) in ethanol (15 mL) was added dropwise 28% aqueous ammonia solution (5.0 mL), and the mixture was stirred at room temperature for 2 hr. The solvent was evaporated under reduced pressure, and the residue was purified by silica gel column chromatography (hexane/ethyl acetate). The obtained crude crystals were dissolved in diisopropyl ether, and the remaining solid was removed by filtration. The solvent was evaporated under reduced pressure, and the resulting solid was collected by filtration, and washed with diisopropyl ether to give the title compound (82 mg).
¹H NMR (300MHz, CDCl₃) δ 1.16 (3H, t, J = 7.4 Hz), 1.75 (1H, dq, J = 14.2, 7.4 Hz), 1.99-2.31 (2H, m), 2.62 (1H, ddd, J = 13.0, 7.9, 4.7 Hz), 3.34-3.46 (1H, m), 3.48-3.62 (1H, m), 5.84 (1H, brs).

### C) 1-(2-aminopyridin-4-yl)-3-ethyl-2-oxopyrrolidine-3-carbonitrile

The title compound (310 mg) was obtained using 3-ethyl-2-oxopyrrolidine-3-carbonitrile obtained in Step B of Example 125 (500 mg) and 4-iodopyridin-2-amine (720 mg), in the same manner as in the method of Example 2.
MS(ESI+): [M+H]⁺ 231.2.

### D) N-(4-(3-cyano-3-ethyl-2-oxopyrrolidin-1-yl)pyridin-2-yl)cyclopropanecarboxamide

To a solution of 1-(2-aminopyridin-4-yl)-3-ethyl-2-oxopyrrolidine-3-carbonitrile obtained in Step C of Example 125 (200 mg) in pyridine (3 mL) were added 4-dimethylaminopyridine (110 mg) and cyclopropanecarbonyl chloride (180 mg), and the mixture was stirred overnight at 70°C. To the reaction mixture was added water, and the mixture was extracted with ethyl acetate. The obtained organic layer was washed successively with water and saturated brine, and dried over anhydrous magnesium sulfate, and the solvent was evaporated under reduced pressure. The residue was purified by silica gel column chromatography (hexane/ethyl acetate), and recrystallized (hexane/ethanol) to give the title compound (72 mg).
MS (ESI+) : [M+H]⁺ 299.2.

### Example 126

### 3-ethyl-1-(2-((5-(morpholin-4-yl)pyridin-2-yl)amino)pyridin-4-yl)-2-oxopyrrolidine-3-carbonitrile hydrochloride

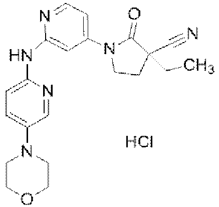

The title compound (16 mg) was obtained using 1-(2-aminopyridin-4-yl)-3-ethyl-2-oxopyrrolidine-3-carbonitrile obtained in Step C of Example 125 (50 mg) and 4-(6-bromopyridin-3-yl)morpholine (58 mg), in the same manner as in the method of Step F of Example 1.
MS (ESI+) : [M+H]⁺93.3.

### Example 127

### 3-cyclopropyl-1-(2-((5-(morpholin-4-yl)pyridin-2-yl)amino)pyridin-4-yl)-2-oxopyrrolidine-3-carbonitrile

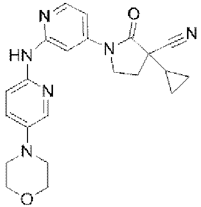

### A) 1-(2-aminopyridin-4-yl)-3-cyclopropyl-2-oxopyrrolidine-3-carbonitrile

The title compound (1.1 g) was obtained using 3-cyclopropyl-2-oxopyrrolidine-3-carbonitrile obtained in Step C of Example 1 (750 mg) and 4-iodopyridin-2-amine (1.0 g), in the same manner as in the method of Example 2.
MS (ESI+) : [M+H]⁺243.2.

### B) 3-cyclopropyl-1-(2-((5-(morpholin-4-yl)pyridin-2-yl)amino)pyridin-4-yl)-2-oxopyrrolidine-3-carbonitrile

The title compound (90 mg) was obtained using 1-(2-aminopyridin-4-yl)-3-cyclopropyl-2-oxopyrrolidine-3-carbonitrile obtained in Step A of Example 127 (200 mg) and 4-(6-bromopyridin-3-yl)morpholine (220 mg), in the same manner as in the method of Step F of Example 1.
MS (ESI+) : [M+H]⁺405.4.

### Example 128

### 3-cyclopropyl-1-(2-((1-methyl-1H-pyrazol-4-yl)amino)pyridin-4-yl)-2-oxopyrrolidine-3-carbonitrile

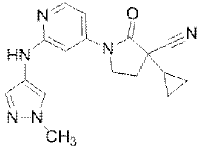

### A) 1-(2-bromopyridin-4-yl)-3-cyclopropyl-2-oxopyrrolidine-3-carbonitrile

The title compound (830 mg) was obtained using 3-cyclopropyl-2-oxopyrrolidine-3-carbonitrile obtained in Step C of Example 1 (500 mg) and 2-bromo-4-fluoropyridine (650 mg), in the same manner as in the method of Step E of Example 1.
¹H NMR (400 MHz, DMSO-d6) δ 0.48-0.57 (2H, m), 0.60-0.72 (2H, m), 1.47-1.58 (1H, m), 2.33-2.43 (1H, m), 2.61-2.70 (1H, m), 3.93-4.01 (2H, m), 7.76 (1H, dd, J = 5.9, 2.0 Hz), 7.94 (1H, d, J = 1.7 Hz), 8.38 (1H, d, J = 5.6 Hz).

### B) 3-cyclopropyl-1-(2-((1-methyl-1H-pyrazol-4-yl)amino)pyridin-4-yl)-2-oxopyrrolidine-3-carbonitrile

The title compound (35 mg) was obtained using 1-(2-bromopyridin-4-yl)-3-cyclopropyl-2-oxopyrrolidine-3-carbonitrile obtained in Step A of Example 128 (100 mg) and 1-methyl-1H-pyrazol-4-amine (35 mg), in the same manner as in the method of Step F of Example 1.
MS(ESI+): [M+H]⁺ 323.3.

### Example 129

### 3-cyclopropyl-1-(2-((1-(1-hydroxy-2-methylpropan-2-yl)-1H-pyrazol-4-yl)amino)pyridin-4-yl)-2-oxopyrrolidine-3-carbonitrile

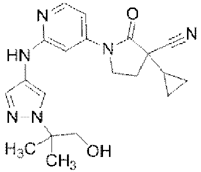

The title compound (45 mg) was obtained using1-(2-bromopyridin-4-yl)-3-cyclopropyl-2-oxopyrrolidine-3-carbonitrile obtained in Step A of Example 128 (100 mg) and 2-(4-amino-1H-pyrazol-1-yl)-2-methylpropan-1-ol (55 mg), in the same manner as in the method of Step F of Example 1.
MS (ESI+) : [M+H]⁺381.4.

### Example 130

### 3-cyclopropyl-1-(2-((1-(1-hydroxy-2-methylpropan-2-yl)-1H-imidazol-4-yl)amino)pyridin-4-yl)-2-oxopyrrolidine-3-carbonitrile

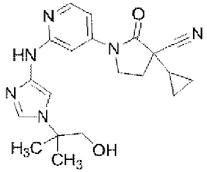

The title compound (21 mg) was obtained using 1-(2-bromopyridin-4-yl)-3-cyclopropyl-2-oxopyrrolidine-3-carbonitrile obtained in Step A of Example 128 (200 mg) and 2-(4-amino-1H-imidazol-1-yl)-2-methylpropan-1-ol (110 mg), in the same manner as in the method of Step F of Example 1.
MS (ESI+) : [M+H]⁺81.4.

### Example 131

### (3S)-3-cyclopropyl-1-(2-((1-(1-hydroxy-2-methylpropan-2-yl)-1H-pyrazol-4-yl)amino)pyridin-4-yl)-2-oxopyrrolidine-3-carbonitrile hydrochloride

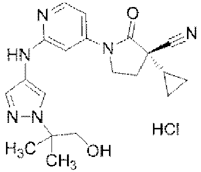

The title compound (90 mg) was obtained using (3S)-1-(2-bromopyridin-4-yl)-3-cyclopropyl-2-oxopyrrolidine-3-carbonitrile obtained in Step E of Example 1 (150 mg) and 2-(4-amino-1H-pyrazol-1-yl)-2-methylpropan-1-ol (84 mg), in the same manner as in the method of Step F of Example 1.
MS (ESI+) : [M+H]⁺81.4.

### Example 132

### (3S)-3-cyclopropyl-1-(2-((1-(1-hydroxy-2-methylpropan-2-yl)-1H-imidazol-4-yl)amino)pyridin-4-yl)-2-oxopyrrolidine-3-carbonitrile

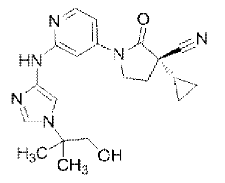

The title compound (10 mg) was obtained using (3S)-1-(2-bromopyridin-4-yl)-3-cyclopropyl-2-oxopyrrolidine-3-carbonitrile obtained in Step E of Example 1 (150 mg) and 2-(4-amino-1H-imidazol-1-yl)-2-methylpropan-1-ol (84 mg), in the same manner as in the method of Step F of Example 1.
MS (ESI+) : [M+H]⁺81.4.

### Example 133

### (3R)-3-cyclopropyl-1-(2-((5-(morpholin-4-yl)pyridin-2-yl)amino)pyridin-4-yl)-2-oxopyrrolidine-3-carbonitrile

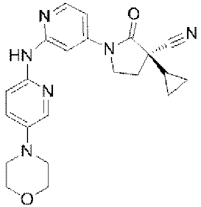

3-cyclopropyl-1-(2-((5-(morpholin-4-yl)pyridin-2-yl)amino)pyridin-4-yl)-2-oxopyrrolidine-3-carbonitrile obtained in Step B of Example 127 (60 mg) was resolved by HPLC (column: CHIRALPAK IA, 20 mmID×250 mmL, manufactured by Daicel Chemical Industries, mobile phase: carbon dioxide/2-propanol/acetonitrile/diethylamine = 600/200/200/3) to give the title compound (27 mg: shorter retention time). >99% ee (HPLC (column: CHIRALPAK IA, 4.6 mmID×150 mmL, manufactured by Daicel Chemical Industries, mobile phase: carbon dioxide/2-propanol/acetonitrile/diethylamine = 600/200/200/3, flow rate: 4.0 mL/min, retention time: 4.82 min) )
MS(ESI+): [M+H]⁺405.4.

### Example 134

### tert-butyl 3-((4-((3S,5R)-3-cyano-3-cyclopropyl-5-methyl-2-oxopyrrolidin-1-yl)pyridin-2-yl)amino)-1-methyl-1H-pyrazole-5-carboxylate

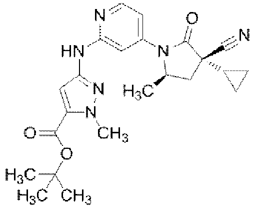

### A) tert-butyl (4R)-4-methyl-1,2,3-oxathiazolidine-3-carboxylate 2,2-dioxide

To a solution of tert-butyl ((2R)-1-hydroxypropan-2-yl)carbamate (10 g) in acetonitrile (100 mL) was added a solution of thionyl chloride (5.2 mL) in acetonitrile (100 mL) under -10°C, and the mixture was stirred at the same temperature for 10 min. To the reaction mixture was added pyridine (18 mL) at the same temperature, and the mixture was stirred at 0°C for 1 hr. To the reaction mixture was added water, and the solvent was evaporated under reduced pressure. The obtained residue was added 1 M hydrochloric acid, and the mixture was extracted with ethyl acetate. The obtained organic layer was washed successively with water and saturated brine, and dried over sodium sulfate, and the solvent was evaporated under reduced pressure.

To the residue was added acetonitrile (100 mL), and the mixture was cooled to 0°C. Ruthenium(III) chloride (50 mg), sodium periodate (18 g) and water (100 mL) were added thereto, and the mixture was stirred at the same temperature for 1 hr. To the reaction mixture was added water, and the mixture was extracted with ethyl acetate. The obtained organic layer was washed successively with saturated aqueous sodium sulfite solution and saturated brine, and dried over magnesium sulfate, and the solvent was evaporated under reduced pressure. The obtained crude crystals were washed with hexane to give the title compound (9.4 g).
¹H NMR (400MHz, CDCl₃) δ 1.51 (3H, d, J = 6.4 Hz), 1.56 (9H, s), 4.20 (1H, dd, J = 9.0, 2.9 Hz), 4.34-4.50 (1H, m), 4.67 (1H, dd, J = 9.0, 5.9 Hz).

### B) ethyl (4R)-4-((tert-butoxycarbonyl)amino)-2-cyano-2-cyclopropylpentanoate

To a solution of ethyl cyano(cyclopropyl)acetate obtained in Step A of Example 1 (6.0 g) in toluene (200 mL) were added tert-butyl (4R)-4-methyl-1,2,3-oxathiazolidine-3-carboxylate 2,2-dioxide obtained in Step A of Example 134 (9.4 g), tetrabutylammonium bromide (1.3 g) and cesium carbonate (19 g), and the mixture was stirred overnight at room temperature. To the reaction mixture was added 1 M hydrochloric acid, and the mixture was extracted with ethyl acetate. The obtained organic layer was washed successively with water and saturated brine, and dried over sodium sulfate, and the solvent was evaporated under reduced pressure. The residue was purified by column chromatography (NH, hexane/ethyl acetate) to give the title compound (8.6 g).
¹H NMR (400 MHz, CDCl₃) δ 0.44-0.84 (4H, m), 1.04-1.63 (15H, m), 1.79-2.43 (3H, m), 3.73-4.56 (4H, m).

### C) (3S,5R)-3-cyclopropyl-5-methyl-2-oxopyrrolidine-3-carbonitrile

To a solution of ethyl (4R)-4-((tert-butoxycarbonyl)amino)-2-cyano-2-cyclopropylpentanoate obtained in Step B of Example 134 (8.6 g) in ethyl acetate (50 mL) was added 4 M hydrogen chloride ethyl acetate solution (69 mL), and the mixture was stirred at room temperature for 3 hr. The solvent was evaporated under reduced pressure, to a solution of the residue in acetonitrile (100 mL) was added potassium carbonate (15 g), and the mixture was stirred overnight at room temperature. The insoluble substance was removed by filtration, and the solvent was evaporated under reduced pressure. The residue was purified by column chromatography (hexane/ethyl acetate) to give the title compound (1.0 g).
¹H NMR (400 MHz, CDCl₃) δ 0.43-0.89 (4H, m), 1.15-1.28 (1H, m), 1.33 (3H, d, J = 6.4 Hz), 2.23 (1H, dd, J = 13.0, 7.6 Hz), 2.50 (1H, dd, J = 13.2, 6.6 Hz), 3.75-3.89 (1H, m), 5.98 (1H, brs).

### D) (3S,5R)-1-(2-bromopyridin-4-yl)-3-cyclopropyl-5-methyl-2-oxopyrrolidine-3-carbonitrile

To a solution of (3S,5R)-3-cyclopropyl-5-methyl-2-oxopyrrolidine-3-carbonitrile obtained in Step C of Example 134 (500 mg) in N,N-dimethylformamide (10 mL) was added sodium hydride (60% in mineral oil, 130 mg) under ice bath. The mixture was stirred at the same temperature for 10 min, and to the reaction mixture was added 2-bromo-4-fluoropyridine (540 mg). The reaction mixture was stirred overnight while allowed to be warmed to room temperature. To the reaction mixture was added water under ice bath, and the resulting crystals were collected by filtration, and washed with water to give the title compound (640 mg).
¹H NMR (400MHz, CDCl₃) δ 0.60-1.00 (4H, m), 1.16-1.35 (1H, m), 1.46 (3H, d, J = 6.4 Hz), 2.36 (1H, dd, J = 13.4, 5.4 Hz), 2.58 (1H, dd, J = 13.3, 7.5 Hz), 4.33-4.52 (1H, m), 7.49 (1H, dd, J = 5.6, 2.0 Hz), 7.69 (1H, d, J = 2.0 Hz), 8.38 (1H, d, J = 5.6 Hz).
MS (ESI+) : [M+H] ⁺320.0.

### E) tert-butyl 3-((4-((3S,5R)-3-cyano-3-cyclopropyl-5-methyl-2-oxopyrrolidin-1-yl)pyridin-2-yl)amino)-1-methyl-1H-pyrazole-5-carboxylate

The title compound (200 mg) was obtained using (3S,5R)-1-(2-bromopyridin-4-yl)-3-cyclopropyl-5-methyl-2-oxopyrrolidine-3-carbonitrile obtained in Step D of Example 134 (150 mg) and tert-butyl 3-amino-1-methyl-1H-pyrazole-5-carboxylate obtained in Step B of Example 4 (110 mg), in the same manner as in the method of Example 3.
MS (ESI+) : [M+H]⁺437.2.

### Example 135

### 3-((4-((3S,5R)-3-cyano-3-cyclopropyl-5-methyl-2-oxopyrrolidin-1-yl)pyridin-2-yl)amino)-1-methyl-1H-pyrazole-5-carboxylic acid hydrochloride

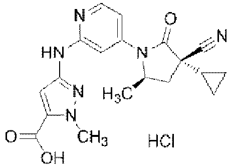

The title compound (130 mg) was obtained using tert-butyl 3-((4-((3S,5R)-3-cyano-3-cyclopropyl-5-methyl-2-oxopyrrolidin-1-yl)pyridin-2-yl)amino)-1-methyl-1H-pyrazole-5-carboxylate obtained in Step E of Example 134 (200 mg), in the same manner as in the method of Step A of Example 5.
MS (ESI+) : [M+H]⁺381.2.

### Example 136

### 3-((4-((3S,5R)-3-cyano-3-cyclopropyl-5-methyl-2-oxopyrrolidin-1-yl)pyridin-2-yl)amino)-N,1-dimethyl-1H-pyrazole-5-carboxamide

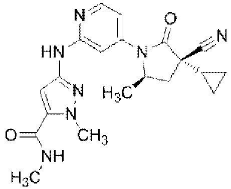

To a solution of 3-((4-((3S,5R)-3-cyano-3-cyclopropyl-5-methyl-2-oxopyrrolidin-1-yl)pyridin-2-yl)amino)-1-methyl-1H-pyrazole-5-carboxylic acid hydrochloride obtained in Example 135 (60 mg) in N,N-dimethylacetamide (2.0 mL) were added N,N-diisopropylethylamine (0.13 mL), O-(7-azabenzotriazol-1-yl)-N,N,N',N'-tetramethyluronium hexafluorophosphorate (110 mg), and methylamine hydrochloride (29 mg), and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate), and recrystallized (hexane/ethyl acetate) to give the title compound (49 mg).
MS (ESI+) : [M+H]⁺394.2.

### Example 137

### 3-((4-((3S,5R)-3-cyano-3-cyclopropyl-5-methyl-2-oxopyrrolidin-1-yl)pyridin-2-yl)amino)-1-methyl-1H-pyrazole-5-carboxamide

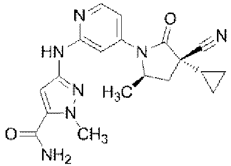

The title compound (36 mg) was obtained using 3-((4-((3S,5R)-3-cyano-3-cyclopropyl-5-methyl-2-oxopyrrolidin-1-yl)pyridin-2-yl)amino)-1-methyl-1H-pyrazole-5-carboxylic acid hydrochloride obtained in Example 135 (60 mg) and ammonium chloride (23 mg), in the same manner as in the method of Example 136.
MS (ESI+) : [M+H]⁺ 380.2.

### Example 138

### 3-((4-((3S)-3-cyano-3-cyclopropyl-2-oxopyrrolidin-1-yl)pyridin-2-yl)amino)-1-methyl-1H-pyrazole-5-carboxamide

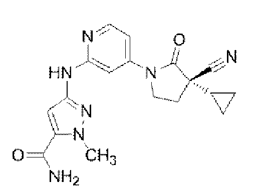

To a solution of 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 (1.1 g) in N,N-dimethylacetamide (10 mL) were added 2-(7-azabenzotriazol-1-yl)-1,1,3,3-hexafluorophosphate (HATU) (2.1 g), diisopropylethylamine (2.4 mL) and ammonium chloride (440 mg), and the mixture was stirred overnight at room temperature. The reaction mixture was cooled to 0°C, saturated aqueous sodium hydrogencarbonate solution was added thereto, and the mixture was extracted with ethyl acetate. The residue was purified by HPLC (C18, mobile phase: water/acetonitrile (containing 5 mM NH₄HCO₃)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (370 mg).
¹H NMR (400 MHz, DMSO-d₆) δ 0.37-0.77 (4H, m), 1.50 (1H, tt, J = 8.2, 5.0 Hz), 2.23-2.44 (1H, m), 2.57-2.72 (1H, m), 3.84-3.92 (2H, m), 3.96 (3H, s), 7.00 (1H, s), 7.13 (1H, dd, J = 5.7, 2. Hz), 7.33-7.53 (2H, m), 7.94 (1H, brs), 8.11 (1H, d, J=5.9Hz), 9.43 (1H, s).
MS (ESI+) : [M+H]⁺366.2.

### Reference Example 1

### ethyl 3,5-dibromo-1H-pyrazole-4-carboxylate

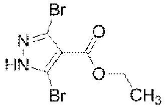

To a solution of ethyl 1H-pyrazole-4-carboxylate (31 g) and sodium acetate (120 g) in a mixed solvent of ethanol (200 mL) and water (300 mL) was added bromine (140 g), and the mixture was stirred at room temperature for 10 hr. To the reaction mixture was added sodium thiosulfate (180 g), and the mixture solvent was evaporated under reduced pressure. The reaction mixture was poured into water, and the mixture was extracted with ethyl acetate. The extract was washed with saturated brine, and dried over anhydrous magnesium sulfate, and the solvent was evaporated under reduced pressure to give the title compound (65 g).
MS (ESI+) : [M+H]⁺296.7.

### Reference Example 2

### ethyl 3,5-dibromo-1-methyl-1H-pyrazole-4-carboxylate

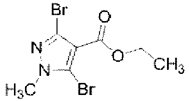

To a solution of ethyl 3,5-dibromo-1H-pyrazole-4-carboxylate obtained in Reference Example 1 (30 g) in tetrahydrofuran (400 mL) was added sodium hydride (60% in mineral oil, 4.2 g) at 0°C. The mixture was stirred under nitrogen atmosphere at 0°C for 1 hr, to the reaction mixture was added iodomethane (41 g) at 0°C, and the mixture was stirred at room temperature for 10 hr. To the reaction mixture was added water, and the solvent was evaporated under reduced pressure. The reaction mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (31 g).
MS (ESI+) : [M+H]⁺310.7.

### Reference Example 3

### 4-(3-bromo-1-methyl-1H-pyrazol-5-yl)morpholine

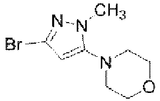

### A) ethyl 3-bromo-1-methyl-5-morpholino-1H-pyrazole-4-carboxylate

A mixture of ethyl 3,5-dibromo-1-methyl-1H-pyrazole-4-carboxylate obtained in Reference Example 2 (10 g) and morpholine (11 g) was stirred at 150°C for 10 hr. The reaction mixture was added to water at room temperature while stirring, and the obtained crystal were washed successively with water and hexane, and dried under reduced pressure to give the title compound (7.8 g).
MS (ESI+) : [M+H]⁺319.8.

### B) 4-(3-bromo-1-methyl-1H-pyrazol-5-yl)morpholine

A mixture of ethyl 3-bromo-1-methyl-5-morpholino-1H-pyrazole-4-carboxylate obtained in Step A of Reference Example 3 (7.7 g), 2 M aqueous sodium hydroxide solution (48 mL) and ethanol (80 mL) was stirred at 60°C for 2 hr 30 min. To the reaction mixture was added conc. sulfuric acid (13 mL) at 0°C, and the mixture was stirred at 60°C for 30 min. The reaction mixture was basified with 8 M aqueous sodium hydroxide solution at 0°C, and the mixture was extracted with ethyl acetate (x3). The extracts were combined, washed with saturated brine, and dried over anhydrous magnesium sulfate, and the solvent was evaporated under reduced pressure to give the title compound (5.9 g).
MS (ESI+): [M+H]⁺245.7.

### Reference Example 4

### 3,5-dibromo-1-methyl-1H-pyrazole

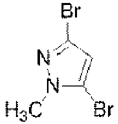

A mixture of ethyl 3,5-dibromo-1-methyl-1H-pyrazole-4-carboxylate obtained in Reference Example 2 (40 g) and 50% aqueous sulfuric acid solution (150 mL, v/v) was stirred at 160°C for 90 min. The reaction mixture was diluted with water (300 mL) at 0°C, and the mixture was extracted with ethyl acetate (500 mL x 2, 300 mL x 1). The extract was washed with saturated brine (300 mL), and dried over anhydrous magnesium sulfate, and the solvent was evaporated under reduced pressure to give the title compound (27 g).
MS (ESI+): [M+H]⁺238.6.

### Reference Example 5

### 2-(3-bromo-1-methyl-1H-pyrazol-5-yl)propan-2-ol

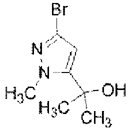

To a solution of 3,5-dibromo-1-methyl-1H-pyrazole obtained in Reference Example 4 (14 g) in tetrahydrofuran (200 mL) was added dropwise n-butyllithium (1.6 M hexane solution, 40 mL) at -78°C. The mixture was stirred at -78°C for 30 min under argon atmosphere, to the reaction mixture was added propan-2-one (5.6 mL), and the mixture was stirred at room temperature for 10 hr. To the reaction mixture was added water (400 mL), and the mixture extracted with ethyl acetate (400 mL, 500 mL). The extract was washed with saturated brine (300 mL), and dried over anhydrous magnesium sulfate, and the solvent was evaporated under reduced pressure. The residue was purified by silica gel column chromatography (hexane/ethyl acetate) to give the title compound (9.1 g).
MS (ESI+) : [M+H]⁺218.8.

### Reference Example 6

### 3-bromo-5-(1-(isopropylsulfonyl)-3-methoxyazetidin-3-yl)-1-methyl-1H-pyrazole

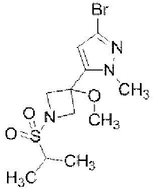

### A) tert-butyl 3-(3-bromo-1-methyl-1H-pyrazol-5-yl)-3-hydroxyazetidine-1-carboxylate

To a solution of 3,5-dibromo-1-methyl-1H-pyrazole obtained in Reference Example 4 (2.0 g) in tetrahydrofuran (20 mL) was added dropwise n-butyllithium (1.6 M hexane solution, 5.7 mL) at -78°C under argon atmosphere. The mixture was stirred at -78°C for 30 min under argon atmosphere, to the reaction mixture was added a solution of tert-butyl 3-oxoazetidine-1-carboxylate (1.9 g) in tetrahydrofuran (5.0 mL), and the mixture was stirred at -78°C for 30 min, and then at room temperature for 3 hr. To the reaction mixture was added water, and the mixture was extracted with ethyl acetate. The extract was washed with saturated brine, and dried over anhydrous magnesium sulfate, and the solvent was evaporated under reduced pressure. The residue was purified by silica gel column chromatography (hexane/ethyl acetate) to give the title compound (2.6 g).
MS (ESI+): [M+H]⁺331.9.

### B) tert-butyl 3-(3-bromo-1-methyl-1H-pyrazol-5-yl)-3-methoxyazetidine-1-carboxylate

To a solution of tert-butyl 3-(3-bromo-1-methyl-1H-pyrazol-5-yl)-3-hydroxyazetidine-1-carboxylate obtained in Step A of Reference Example 6 (2.0 g) in tetrahydrofuran (40 mL) was added sodium hydride (60% in mineral oil, 0.27 g), and the mixture was stirred at 0°C for 30 min. To the reaction mixture was added iodomethane (0.45 mL) at 0°C, and the mixture was stirred at room temperature for 4 hr. Iodomethane (0.45 mL) was added thereto, and the mixture was stirred at 50°C for 2 hr. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (1.8 g).
MS (ESI+) : [M+H]⁺345.9.

### C) 3-bromo-5-(3-methoxyazetidin-3-yl)-1-methyl-1H-pyrazole hydrochloride

To a solution of tert-butyl 3-(3-bromo-1-methyl-1H-pyrazol-5-yl)-3-methoxyazetidine-1-carboxylate obtained in Step B of Reference Example 6 (6.5 g) in ethyl acetate (25 mL) was added 4 M hydrogen chloride ethyl acetate solution (10 mL), and the mixture was stirred at 50°C for 1 hr. Methanol (25 mL) was added thereto, and the mixture was stirred at 50°C for 3 hr. To the reaction mixture was added 4 M hydrogen chloride ethyl acetate solution (5.0 mL), and the mixture was stirred at 50°C for 2 hr, and then overnight at room temperature. The solvent was evaporated under reduced pressure. The residue was washed with diethyl ether to give the title compound (4.8 g).
MS (ESI+) : [M+H]⁺245.8.

### D) 3-bromo-5-(1-(isopropylsulfonyl)-3-methoxyazetidin-3-yl)-1-methyl-1H-pyrazole

To a solution of 3-bromo-5-(3-methoxyazetidin-3-yl)-1-methyl-1H-pyrazole hydrochloride obtained in Step C of Reference Example 6 (400 mg) in tetrahydrofuran (10 mL) were added triethylamine (0.60 mL) and propane-2-sulfonyl chloride (300 mg) at 0°C, and the mixture was stirred at room temperature for 1 hr. Saturated aqueous sodium bicarbonate solution was added thereto, and the mixture was extracted with ethyl acetate. The obtained organic layer was washed with saturated aqueous ammonium chloride solution and saturated brine, and dried over anhydrous magnesium sulfate, and the solvent was evaporated under reduced pressure to give the title compound (490 mg).
MS (ESI+) : [M+H]⁺351.9.

### Reference Examples 7 to 10

The title compounds of Reference Examples 7 to 10 shown in Table 14 were obtained using ethyl 3,5-dibromo-1-methyl-1H-pyrazole-4-carboxylate obtained in Reference Example 2 and the reagents corresponding to the compounds of Reference Examples 7 to 10 (these reagents can be produced according to a method known per se), in the same manner as in the method of Reference Example 3 or a method analogous thereto. MS in the tables means actual measured value.

**Table 14**

| Ref. No. | IUPAC Name | Structure | Salt | MS | Corresponding Reagent |
|---|---|---|---|---|---|
| 7 | 1-(3-bromo-1-methyl-1H-pyrazol-5-yl)-4-methylpiperidine-4-carbonitrile | 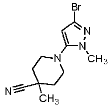 | Free | 282.8 | 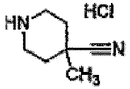 |
| 8 | 1-(3-bromo-1-methyl-1H-pyrazol-5-yl)-4,4-difluoropiperidine | 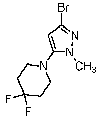 | Free | 279.8 | 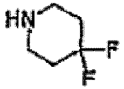 |
| 9 | 1-(3-bromo-1-methyl-1H-pyrazol-5-yl)-4-(trifluoromethyl)piperidin-4-ol | 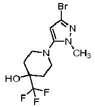 | Free | 327.8 | 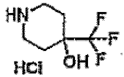 |
| 10 | ((2R)-1-(3-bromo-1-methyl-1H-pyrazol-5-yl)pyrrolidin-2-yl)methanol | 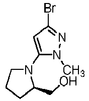 | Free | 260.2 | 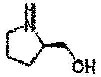 |

### Reference Example 11

### 4-(6-bromopyridin-3-yl)tetrahydro-2H-pyran-4-ol

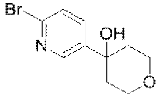

To a suspension of 2-bromo-5-iodopyridine (2.0 g) in tetrahydrofuran (20 mL) and diethyl ether (10 mL) was added dropwise 1.6M n-butyllithium hexane solution (4.4 mL) at -78°C. The mixture was stirred at -78°C for 30 min under argon atmosphere, and a solution of tetrahydro-4H-pyran-4-one (0.65 mL) in tetrahydrofuran (10 mL) was added dropwise thereto at - 78°C. The reaction mixture was slowly warmed to -50°C, aqueous ammonium chloride solution was added thereto, and the mixture was extracted with ethyl acetate. The extract was washed successively with water and saturated brine, and dried over anhydrous sodium sulfate, and the solvent was evaporated under reduced pressure. The residue was purified by silica gel column chromatography (ethyl acetate/hexane) to give the title compound (0.93 g).
MS (ESI+) : [M+H]⁺58.1.

### Reference Example 12

### 4-(6-bromopyridin-3-yl)thiomorpholine 1,1-dioxide

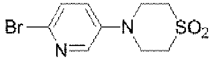

A mixture of 2-bromo-5-iodopyridine (1.0 g), thiomorpholine 1,1-dioxide (0.51 g), tris(dibenzylideneacetone)dipalladium(O) (0.11 g), 4,5-bis(diphenylphosphino)-9,9-dimethylxanthene (0.20 g), sodium tert-butoxide (0.85 g) and toluene (30 mL) was stirred under argon atmosphere at room temperature for 4 days. To the reaction mixture was added water, and the mixture was extracted with ethyl acetate. The extract was washed with water and saturated brine, and dried over anhydrous sodium sulfate, and the solvent was evaporated under reduced pressure. The residue was purified by silica gel column chromatography (ethyl acetate/hexane) to give the title compound (420 mg).
MS (ESI+) : [M+H]⁺ 290.9.

### Reference Example 13

### tert-butyl (2-((6-bromopyridin-3-yl)oxy)ethyl)carbamate

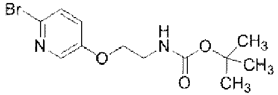

A mixture of 6-bromopyridin-3-ol (1.5 g), tert-butyl (2-bromoethyl)carbamate (2.3 g), cesium carbonate (3.7 g) and N,N-dimethylformamide (30 mL) was stirred at 40°C for 20 hr. To the reaction mixture was added water, and the mixture was extracted with ethyl acetate. The extract was washed with water and saturated brine, and dried over anhydrous sodium sulfate, and the solvent was evaporated under reduced pressure. The residue was purified by silica gel column chromatography (ethyl acetate/hexane) to give the title compound (2.5 g).
MS (ESI+) : [M+H]⁺317.2.

### Reference Example 14

### 2-((6-bromopyridin-3-yl)oxy)ethanamine dihydrochloride

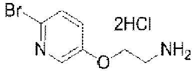

To a solution of tert-butyl (2-((6-bromopyridin-3-yl)oxy)ethyl)carbamate obtained in Reference Example 13 (1.2 g) in ethanol (25 mL) was added 4M hydrogen chloride ethyl acetate solution (10 mL) at room temperature. The mixture was stirred at room temperature for 3 days, and the solvent was evaporated under reduced pressure. To the residue was added ethanol, the solvent was evaporated under reduced pressure, and the precipitated solid was collected by filtration to give the title compound (1.1 g).
MS (ESI+) : [M+H]⁺217.1.

### Reference Example 15

### N-(2-((6-bromopyridin-3-yl)oxy)ethyl)acetamide

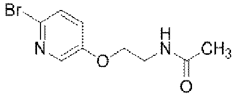

To a mixture of 2-((6-bromopyridin-3-yl)oxy)ethanamine dihydrochloride obtained in Reference Example 14 (1.00 g), pyridine (1.1 mL) and tetrahydrofuran (20 mL) was added dropwise acetyl chloride (0.37 mL) at 0°C. The reaction mixture was stirred at room temperature for 5 hr, aqueous sodium hydrogencarbonate solution was added thereto, and the mixture was extracted with ethyl acetate. The extract was washed with water and saturated brine, and dried over anhydrous sodium sulfate, and the solvent was evaporated under reduced pressure. The precipitated solid was collected by filtration, and washed with diisopropyl ether to give the title compound (0.79 g).
MS(ESI+): [M+H]⁺ 259.0.

### Reference Example 16

### 1-((6-bromopyridin-3-yl)oxy)-2-methylpropan-2-ol

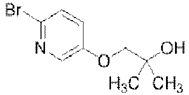

The title compound (1.3 g) was obtained using 6-bromopyridin-3-ol and 2,2-dimethyloxirane, in the same manner as in the method of Reference Example 13.
MS(ESI+): [M+H]⁺ 246.1.

### Reference Example 17

### 1-((2-chloropyridin-4-yl)oxy)-2-methylpropan-2-ol

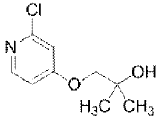

The title compound (400 mg) was obtained using 2-chloropyridin-4-ol and 2,2-dimethyloxirane, in the same manner as in the method of Reference Example 13.
MS (ESI+) : [M+H]⁺ 202.2.

### Reference Example 18

### 4-(2-chloropyridin-4-yl)tetrahydro-2H-pyran-4-ol

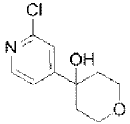

The title compound (1.3 g) was obtained using 2-chloro-4-iodopyridine and tetrahydro-4H-pyran-4-one, in the same manner as in the method of Reference Example 11.
MS (ESI+) : [M+H]⁺214.1.

### Reference Example 19

### 2-bromo-5-(4,4-difluoropiperidin-1-yl)pyrazine

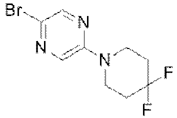

A mixture of 2,5-dibromopyrazine (200 mg), 4,4-difluoropiperidine hydrochloride (130 mg), cesium carbonate (600 mg) and dimethyl sulfoxide (5.0 mL) was stirred overnight at 90°C under nitrogen atmosphere,. To the reaction mixture was added water, and the mixture was extracted with ethyl acetate. The obtained organic layer was washed successively with water and saturated brine, and dried over anhydrous magnesium sulfate, and the solvent was evaporated under reduced pressure. The residue was purified by silica gel column chromatography (hexane/ethyl acetate) to give the title compound (89 mg).
MS (ESI+) : [M+H]⁺ 277.8.

### Reference Example 20

### 2-chloro-5-((4-methylpiperazin-1-yl)methyl)pyrazine

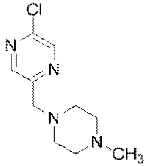

Sodium triacetoxyborohydride (870 mg) was added to a solution of 5-chloropyrazine-2-carbaldehyde (390 mg) and 1-methylpiperazine (0.34 mL) in acetonitrile (10 mL) at 0°C. The reaction mixture was stirred at room temperature for 4 hr. To the reaction mixture was added saturated aqueous sodium hydrogencarbonate solution, and the mixture was extracted with ethyl acetate. The obtained organic layer was washed successively with water and saturated brine, and dried over anhydrous magnesium sulfate, and the solvent was evaporated under reduced pressure. The residue was purified by silica gel column chromatography (NH, hexane/ethyl acetate) to give the title compound (290 mg).
MS (ESI+) : [M+H]⁺ 226.8.

### Reference Example 21

### methyl 3-((4-methoxybenzyl)oxy)-2,2-dimethylpropanoate

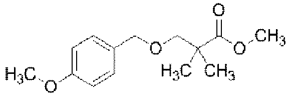

To a solution of methyl 3-hydroxy-2,2-dimethylpropanoate (0.68 mL) in N,N-dimethylformamide (20 mL) was added sodium hydride (60% in mineral oil, 250 mg) at 0°C. The reaction mixture was stirred at 0°C for 30 min under nitrogen atmosphere, and 1-(chloromethyl)-4-methoxybenzene (0.79 mL) and tetrabutylammonium iodide (0.20 g) were added thereto at 0°C. The reaction mixture was warmed to room temperature over 2 hr, and stirred overnight under nitrogen atmosphere. To the reaction mixture was added saturated aqueous ammonium chloride solution, and the mixture was extracted with diethyl ether. The extract was washed with saturated brine, and dried over anhydrous magnesium sulfate, and the solvent was evaporated under reduced pressure. The residue was purified by silica gel column chromatography (ethyl acetate/hexane) to give the title compound (1.2 g).
¹H NMR (300 MHz, CDCl₃) δ 1.14-1.23 (6H, m), 3.41 (2H, s), 3. 67 (3H, s), 3.80 (3H, s), 4.40-4.47 (2H, m), 6.84-6.92 (2H, m), 7.21-7.26 (2H, m).

### Reference Example 22

### 5-((4-methoxybenzyl)oxy)-4,4-dimethyl-3-oxopentanenitrile

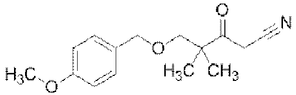

A mixture of sodium hydride (60% in mineral oil, 5.5 g) and tetrahydrofuran (200 mL) was stirred at 70°C for 30 min under nitrogen atmosphere. To the reaction mixture was added a solution of methyl 3-((4-methoxybenzyl)oxy)-2,2-dimethylpropanoate obtained in Reference Example 21 (23 g) and acetonitrile (7.3 mL) in tetrahydrofuran (100 mL) at 70°C. The reaction mixture was stirred overnight at 70°C under nitrogen atmosphere. The solvent was evaporated under reduced pressure. To the reaction mixture was added ethyl acetate, and then 1 M hydrochloric acid was added thereto at room temperature, and the mixture was extracted with ethyl acetate. The extract was washed with saturated brine, and dried over anhydrous magnesium sulfate, and the solvent was evaporated under reduced pressure. The residue was purified by silica gel column chromatography (ethyl acetate/hexane) to give the title compound (17 g).
¹H NMR (300 MHz, CDCl₃) δ 1.17 (6H, s), 3.36 (2H, s), 3.63 (2H, s), 3.82 (3H, s), 4.42 (2H, s), 6.86-6.92 (2H, m), 7.17-7.24 (2H, m).

### Reference Example 23

### 5-(1-((4-methoxybenzyl)oxy)-2-methylpropan-2-yl)-1H-pyrazol-3-amine

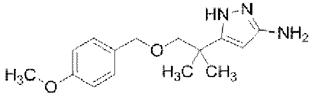

To a solution of 5-((4-methoxybenzyl)oxy)-4,4-dimethyl-3-oxopentanenitrile obtained in Reference Example 22 (17.11 g) in methanol (200 mL) was added hydrazine monohydrate (4.8 mL) at room temperature. The reaction mixture was heated at 70°C for 6 hr under nitrogen atmosphere. The reaction mixture was concentrated under reduced pressure, and the residue was purified by silica gel column chromatography (ethyl acetate/methanol) to give the title compound (12 g).
MS (ESI+) : [M+H]⁺276.3.

### Reference Example 24

### 2-(5-(1-((4-methoxybenzyl)oxy)-2-methylpropan-2-yl)-1H-pyrazol-3-yl)-1H-isoindole-1,3(2H)-dione

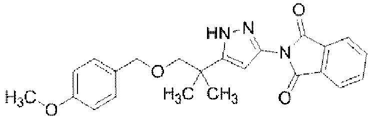

To a solution of 5-(1-((4-methoxybenzyl)oxy)-2-methylpropan-2-yl)-1H-pyrazol-3-amine obtained in Reference Example 23 (12 g) in N,N-dimethylformamide (180 mL) were added phthalic anhydride (6.6 g) and acetic acid (2.6 mL) at room temperature. The reaction mixture was stirred overnight at 90°C under nitrogen atmosphere. To the reaction mixture were added saturated aqueous sodium hydrogencarbonate solution and water at room temperature, and the mixture was extracted with ethyl acetate. The extract was washed with saturated brine, and dried over anhydrous magnesium sulfate, and the solvent was evaporated under reduced pressure. The residue was purified by silica gel column chromatography (ethyl acetate/hexane) to give the title compound (3.6 g).
MS (ESI+) : [M+H]⁺406.3.

### Reference Example 25

### 2-(5-(1-((4-methoxybenzyl)oxy)-2-methylpropan-2-yl)-1-methyl-1H-pyrazol-3-yl)-1H-isoindole-1,3(2H)-dione

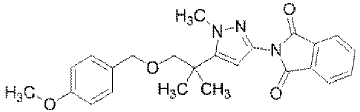

To a solution of 2-(5-(1-((4-methoxybenzyl)oxy)-2-methylpropan-2-yl)-1H-pyrazol-3-yl)-1H-isoindole-1,3(2H)-dione obtained in Reference Example 24 (7.3 g) in tetrahydrofuran (120 mL) was added sodium hydride (60% in mineral oil, 1.1 g) at 0°C. The reaction mixture was stirred at 0°C for 10 min under nitrogen atmosphere, to the reaction mixture was added iodomethane (2.3 mL) at room temperature, and the mixture was stirred at room temperature for 1 hr under nitrogen atmosphere. To the reaction mixture was added saturated aqueous ammonium chloride solution, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (5.0 g).
MS (ESI+) : [M+H]⁺420.3.

### Reference Example 26

### 2-(5-(1-hydroxy-2-methylpropan-2-yl)-1-methyl-1H-pyrazol-3-yl)-1H-isoindole-1,3(2H)-dione

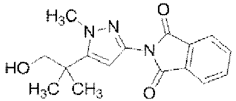

To 2-(5-(1-((4-methoxybenzyl)oxy)-2-methylpropan-2-yl)-1-methyl-1H-pyrazol-3-yl)-1H-isoindole-1,3(2H)-dione obtained in Reference Example 25 (17 g) was added trifluoroacetic acid (64 mL) at 0°C, and the mixture was stirred at the same temperature for 20 min under nitrogen atmosphere. The solvent was evaporated under reduced pressure, and the residue was purified by silica gel column chromatography (ethyl acetate), and the crude product was washed with diisopropyl ether to give the crude title compound (13 g).
MS (ESI+) : [M+H]⁺299.9.

### Reference Example 27

### 2-(3-amino-1-methyl-1H-pyrazol-5-yl)-2-methylpropan-1-ol

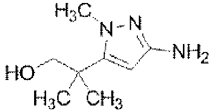

To a solution of the crude 2-(5-(1-hydroxy-2-methylpropan-2-yl)-1-methyl-1H-pyrazol-3-yl)-1H-isoindole-1,3(2H)-dione obtained in Reference Example 26 (1.1 g) in ethanol (7.0 mL) was added hydrazine monohydrate (0.23 mL) at room temperature, and the mixture was stirred at 65°C for 3 hr under nitrogen atmosphere. The insoluble substance was removed by filtration, and the solvent was evaporated under reduced pressure. The residue was purified by silica gel column chromatography (NH, ethyl acetate/methanol), and recrystallized (ethyl acetate/diisopropyl ether) to give the title compound (0.22 g).
MS (ESI+) : [M+H]⁺169.8.

### Reference Example 28

### 2-(3-amino-4-fluoro-1-methyl-1H-pyrazol-5-yl)-2-methylpropan-1-ol

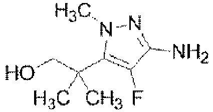

### A) 2-(4-fluoro-5-(1-hydroxy-2-methylpropan-2-yl)-1-methyl-1H-pyrazol-3-yl)-1H-isoindole-1,3(2H)-dione

To a solution of 2-(5-(1-hydroxy-2-methylpropan-2-yl)-1-methyl-1H-pyrazol-3-yl)-1H-isoindole-1,3(2H)-dione obtained in Reference Example 26 (1.0 g) in acetonitrile (10 mL) was added N-fluoro-N'-(chloromethyl)triethylenediamine bis(tetrafluoroborate) (1.6 g) at room temperature, and the mixture was stirred at the same temperature for 3 days. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (130 mg).
MS (ESI+) : [M+H]⁺317.9.

### B) 2-(3-amino-4-fluoro-1-methyl-1H-pyrazol-5-yl)-2-methylpropan-1-ol

The title compound (120 mg) was obtained using 2-(4-fluoro-5-(1-hydroxy-2-methylpropan-2-yl)-1-methyl-1H-pyrazol-3-yl)-1H-isoindole-1,3(2H)-dione obtained in Step A of Reference Example 28 (1.1 g), in the same manner as in the method of Reference Example 27.
MS (ESI+) : [M+H]⁺187.8.

### Reference Example 29

### 2-(3-amino-1-ethyl-1H-pyrazol-5-yl)propan-2-ol

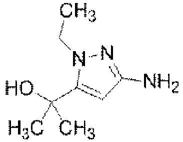

### A) methyl 2-((4-methoxybenzyl)oxy)-2-methylpropanoate

The title compound (23 g) was obtained using methyl 2-hydroxy-2-methylpropanoate (10 g) and 1-(chloromethyl)-4-methoxybenzene (13 mL), in the same manner as in the method of Reference Example 21.
¹H NMR (400MHz, CDCl₃)δ1.51 (6H, s), 3.76 (3H, s), 3.80 (3H, s), 4.39 (2H, s), 6.87 (2H, d, J = 8.8 Hz), 7.31 (2H, d, J = 8.6 Hz).

### B) 4-((4-methoxybenzyl)oxy)-4-methyl-3-oxopentanenitrile

The title compound (19 g) was obtained using methyl 2-((4-methoxybenzyl)oxy)-2-methylpropanoate obtained in Step A of Reference Example 29 (23 g), in the same manner as in the method of Reference Example 22.
¹H NMR (400MHz, CDCl₃)δ1.45 (6H, s), 3.76 (2H, s), 3.82 (3H, s), 4.39 (2H, s), 6.86-6.94 (2H, m), 7.24 (2H, d, J = 8.8 Hz).

### C) 5-(2-((4-methoxybenzyl)oxy)propan-2-yl)-1H-pyrazol-3-amine

The title compound (20 g) was obtained using 4-((4-methoxybenzyl)oxy)-4-methyl-3-oxopentanenitrile obtained in Step B of Reference Example 29 (19 g), in the same manner as in the method of Reference Example 23.
MS(ESI+): [M+H]⁺ 262.1.

### D) 2-(5-(2-((4-methoxybenzyl)oxy)propan-2-yl)-1H-pyrazol-3-yl)-1H-isoindole-1,3(2H)-dione

The title compound (12 g) was obtained using 5-(2-((4-methoxybenzyl)oxy)propan-2-yl)-1H-pyrazol-3-amine obtained in Step C of Reference Example 29 (12 g), in the same manner as in the method of Reference Example 24.
¹H NMR (400MHz, DMSO-d₆)δ1.61 (6H, s), 3.73 (3H, s), 4.19 (2H, s), 6.37 (1H, s), 6.88 (2H, d, J = 8.8 Hz), 7.21 (2H, d, J = 8.6 Hz), 7.82-8.06 (4H, m), 13.14 (1H, brs).

### E) 2-(1-ethyl-5-(2-((4-methoxybenzyl)oxy)propan-2-yl)-1H-pyrazol-3-yl)-1H-isoindole-1,3(2H)-dione

The title compound (0.30 g) was obtained using 2-(5-(2-((4-methoxybenzyl)oxy)propan-2-yl)-1H-pyrazol-3-yl)-1H-isoindole-1,3(2H)-dione obtained in Step D of Reference Example 29 (1.0 g) and iodoethane (0.80 g), in the same manner as in the method of Reference Example 25.
MS(ESI+): [M+H]⁺ 420.2.

### F) 2-(1-ethyl-5-(2-hydroxypropan-2-yl)-1H-pyrazol-3-yl)-1H-isoindole-1,3(2H)-dione

The title compound (0.19 g) was obtained using 2-(1-ethyl-5-(2-((4-methoxybenzyl)oxy)propan-2-yl)-1H-pyrazol-3-yl)-1H-isoindole-1,3(2H)-dione obtained in Step E of Reference Example 29 (0.30 g), in the same manner as in the method of Reference Example 26.
MS(ESI+): [M+H]⁺ 299.9.

### G) 2-(3-amino-1-ethyl-1H-pyrazol-5-yl)propan-2-ol

The title compound (91 mg) was obtained using 2-(1-ethyl-5-(2-hydroxypropan-2-yl)-1H-pyrazol-3-yl)-1H-isoindole-1,3(2H)-dione obtained in Step F of Reference Example 29 (0.19 g), in the same manner as in the method of Reference Example 27.
¹H NMR (400 MHz, CDCl₃) δ1.38-1.43 (3H, m), 1.59 (6H, s), 3.53 (2H, brs), 4.24 (2H, q, J = 7.1 Hz), 5.40 (1H, s).

### Reference Example 30

### 2-(5-amino-1,2-oxazol-3-yl)propan-2-ol

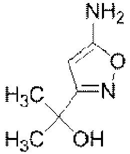

### A) 3-(2-((4-methoxybenzyl)oxy)propan-2-yl)-1,2-oxazol-5-amine

The title compound (360 mg) was obtained using 4-((4-methoxybenzyl)oxy)-4-methyl-3-oxopentanenitrile obtained in Step B of Reference Example 29 (500 mg), hydroxylamine hydrochloride (160 mg) and 2M aqueous sodium hydroxide solution (1.1 mL), in the same manner as in the method of Reference Example 23.
¹H NMR (400MHz, DMSO-d₆)δ1.46 (6H, s), 3.73 (3H, s), 4.18 (2H, s), 4.93 (1H, s), 6.60 (2H, s), 6.87 (2H, d, J = 8.8 Hz), 7.19 (2H, d, J = 8.8 Hz).

### B) 2-(5-amino-1,2-oxazol-3-yl)propan-2-ol

The title compound (70 mg) was obtained using 3-(2-((4-methoxybenzyl)oxy)propan-2-yl)-1,2-oxazol-5-amine obtained in Step A of Reference Example 30 (360 mg), in the same manner as in the method of Reference Example 26.
¹H NMR (400MHz, DMSO-d₆) δ1.34 (6H, s), 4.92 (1H, s), 5.08 (1H, s), 6.45 (2H, s).

### Example 139

### (3S)-3-cyclopropyl-1-(2-((1-methyl-5-phenyl-1H-pyrazol-3-yl)amino)pyridin-4-yl)-2-oxopyrrolidine-3-carbonitrile

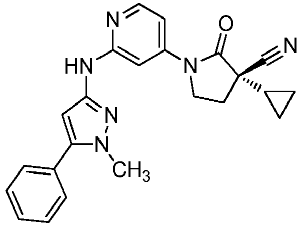

To a solution of (3S)-1-(2-bromopyridin-4-yl)-3-cyclopropyl-2-oxopyrrolidine-3-carbonitrile obtained in Step E of Example 1 (190 mg) in 2-methyl-2-butanol (6.0 mL) were added 1-methyl-5-phenyl-1H-pyrazol-3-amine (110 mg), potassium carbonate (180 mg), 2-(dicyclohexylphosphino)-3,6-dimethoxy-2',4',6'-triisopropyl-1,1'-biphenyl (17 mg) and chloro[2-(dicyclohexylphosphino)-3,6-dimethoxy-2',4',6'-triisopropyl-1,1'-biphenyl][2-(2-aminoethyl)phenyl]palladium(II) (25 mg) under argon atmosphere, and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate) to give the title compound (110 mg).
MS (ESI+) : [M+H]⁺ 399.2.

### Examples 140 to 156

The title compounds of Examples 140 to 156 shown in Table 15 and Table 16 were obtained using (3S)-1-(2-bromopyridin-4-yl)-3-cyclopropyl-2-oxopyrrolidine-3-carbonitrile obtained in Step E of Example 1 and the reagents corresponding to the compounds of Examples 140 to 156 (these reagents can be produced according to a method known per se), in the same manner as in the method of Example 139 or a method analogous thereto. MS in the tables means actual measured value.

**Table 15**

| Ex. No. | IUPAC Name | Structure | Salt | MS | Corresponding Reagent |
|---|---|---|---|---|---|
| 140 | 3-((4-((3S)-3-cyano-3-cyclopropyl-2-oxopyrrolidin-1 yl)pyridin-2-yl)amino)-1-(difluoromethyl)-N-methyl-1H-pyrazole-5-carboxamide | 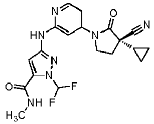 | Free | 416. 1 | 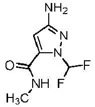 |
| 141 | 3-((4-((3S)-3-cyano-3-cyclopropyl-2-oxopyrrolidin-1 yl)pyridin-2-yl)amino)-1-(difluoromethyl)-N-(oxetan-3-yl)-1H-pyrazole-5-carboxamide | 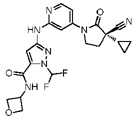 | Free | 458.2 | 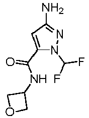 |
| 142 | tert-butyl 3-((4-((3S)-3-cyano-3-cyclopropyl-2-oxopyrrolidin-1-yl)pyridin-2-yl)amino)-1-(difluoromethyl)-1H-pyrazole-5-carboxylate | 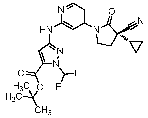 | Free | 459.2 | 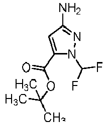 |
| 143 | (3S)-3-cyclopropyl-1-(2-((1-(difluoromethyl)-5-(2-hydroxypropan-2-yl)-1H-pyrazol-3-yl)amino)pyridin-4-yl)-2-oxopyrrolidine-3-carbonitrile | 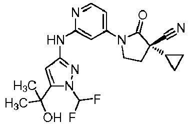 | Free | 417.2 | 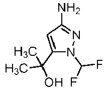 |
| 144 | (3S)-3-cyclopropyl-1-(2-((5-methyl-4-oxo-4,5,6,7-tetrahydropyrazolo[1,5-a]pyrazin-2-yl)amino)pyridin-4-yl)-2-oxopyrrolidine-3-carbonitrile | 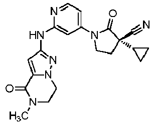 | Free | 392. | 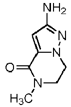 |
| 145 | tert-butyl 3-(3-((4-((3S)-3-cyano-3-cyclopropyl-2-oxopyrrolidin-1-yl)pyridin-2-yl)amino)-1-methyl-1H-pyrazol-5-yl)azetidine-1-carboxylate | 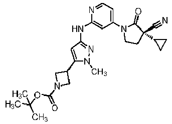 | Free | 478.2 | 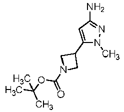 |
| 146 | (3S)-3-cyclopropyl-1-(2-((5-(4-hydroxy-4-methylpiperidin-1-yl)-1-methyl-1H-pyrazol-3-yl)amino)pyridin-4-yl)-2-oxopyrrolidine-3-carbonitrile | 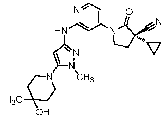 | Free | 436.1 | 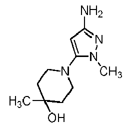 |

**Table 16**

| | | | | | |
|---|---|---|---|---|---|
| 148 | (3S)-3-cyclopropyl-1-(2-((1-methyl-5-(tetrahydro-2H-pyran-4-yl)-1H-pyrazol-3-yl)amino)pyridin-4-yl)-2-oxopyrrolidine-3-carbonitrile | 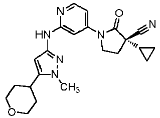 | Free | 407.2 | 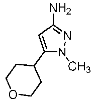 |
| 149 | tert-butyl (3-((4-((3S)-3-cyano-3-cyclopropyl-2-oxopyrrolidin-1-yl)pyridin-2-yl)amino)-1-methyl-1H-pyrazol-5-yl)carbamate | 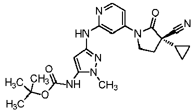 | Free | 438.2 | 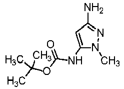 |
| 150 | tert-butyl 4-(3-((4-((3S)-3-cyano-3-cyclopropyl-2-oxopyrrolidin-1-yl)pyridin-2-yl)amino)-1-methyl-1H-pyrazol-5-yl)piperidine-1-carboxylate | 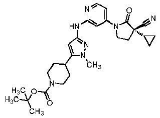 | Free | 505.3 | 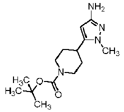 |
| 151 | 3-((4-((3S)-3-cyano-3-cyclopropyl-2-oxopyrrolidin-1-yl)pyridin-2-yl)amino)-1-methyl-1H-pyrazole-5-carbonitrile | 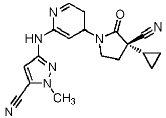 | Free | 348 | 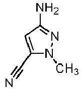 |
| 152 | N-((3-((4-((3S)-3-cyano-3-cyclopropyl-2-oxopyrrolidin-1-yl)pyridin-2-yl)amino)-1-methyl-1H-pyrazol-5-yl)methyl)acetamide | 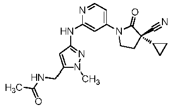 | Free | 394.1 | 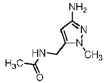 |
| 153 | N-(2-(3-((4-((3S)-3-cyano-3-cyclopropyl-2-oxopyrrolidin-1-yl)pyridin-2-yl)amino)-1-methyl-1H-pyrazol-5-yl)propan-2-yl)acetamide | 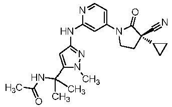 | Free | 422.1 | 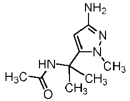 |
| 155 | N-(2-(3-((4-((3S)-3-cyano-3-cyclopropyl-2-oxopyrrolidin-1-yl)pyridin-2-yl)amino)-1-methyl-1H-pyrazol-5-yl)propan-2-yl)-N-methylacetamide | 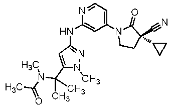 | Free | 436.1 | 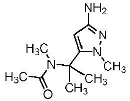 |
| 156 | 1-(3-((4-((3S)-3-cyano-3-cyclopropyl-2-oxopyrrolidin-1 yl)pyridin-2-yl)amino)-1-methyl-1H-pyrazol-5-yl)cyclobutanecarboxamide | 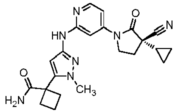 | Free | 420.0 | 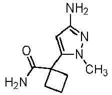 |

### Example 157

### 3-((4-((3S)-3-cyano-3-cyclopropyl-2-oxopyrrolidin-1-yl)pyridin-2-yl)amino)-1-(difluoromethyl)-1H-pyrazole-5-carboxylic acid hydrochloride

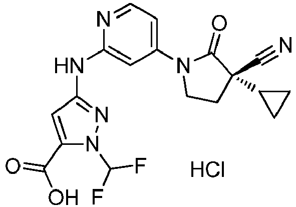

The title compound (210 mg) was obtained using tert-butyl 3-((4-((3S)-3-cyano-3-cyclopropyl-2-oxopyrrolidin-1-yl)pyridin-2-yl)amino)-1-(difluoromethyl)-1H-pyrazole-5-carboxylate obtained in Example 142 (250 mg), in the same manner as in the method of Step A of Example 5.
MS (ESI+) : [M+H]⁺ 403.2.

### Example 158

### 3-((4-((3S)-3-cyano-3-cyclopropyl-2-oxopyrrolidin-1-yl)pyridin-2-yl)amino)-1-(difluoromethyl)-1H-pyrazole-5-carboxamide

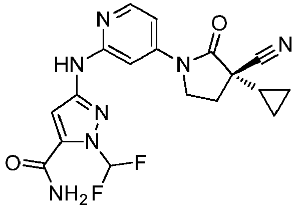

The title compound (73 mg) was obtained using 3-((4-((3S)-3-cyano-3-cyclopropyl-2-oxopyrrolidin-1-yl)pyridin-2-yl)amino)-1-(difluoromethyl)-1H-pyrazole-5-carboxylic acid hydrochloride obtained in Example 157 (180 mg) and ammonium chloride (220 mg), in the same manner as in the method of Example 136.
MS(ESI+): [M+H] + 402.2.

### Example 159

### N-(3-((4-((3S)-3-cyano-3-cyclopropyl-2-oxopyrrolidin-1-yl)pyridin-2-yl)amino)-1-methyl-1H-pyrazol-5-yl)acetamide

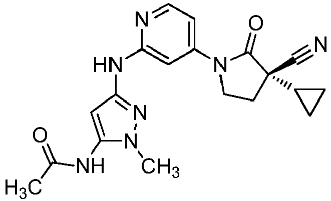

### A) (3S)-1-(2-((5-amino-1-methyl-1H-pyrazol-3-yl)amino)pyridin-4-yl)-3-cyclopropyl-2-oxopyrrolidine-3-carbonitrile dihydrochloride

To tert-butyl (3-((4-((3S)-3-cyano-3-cyclopropyl-2-oxopyrrolidin-1-yl)pyridin-2-yl)amino)-1-methyl-1H-pyrazol-5-yl)carbamate obtained in Example 149 (94 mg) was added 4 M hydrogen chloride ethyl acetate solution (3.0 mL), and the mixture was stirred at room temperature for 2.5 hr, and the solvent was evaporated under reduced pressure to give the crude title compound (89 mg).
MS (ESI+) : [M+H]⁺ 337.9.

### B) N-(3-((4-((3S)-3-cyano-3-cyclopropyl-2-oxopyrrolidin-1-yl)pyridin-2-yl)amino)-1-methyl-1H-pyrazol-5-yl)acetamide

To a solution of (3S)-1-(2-((5-amino-1-methyl-1H-pyrazol-3-yl)amino)pyridin-4-yl)-3-cyclopropyl-2-oxopyrrolidine-3-carbonitrile dihydrochloride obtained in Step A of Example 159 (89 mg) in tetrahydrofuran (2.0 mL) were added triethylamine (0.12 mL) and acetic anhydride (0.020 mL), and the mixture was stirred at room temperature for 15 hr. To the reaction mixture was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (NH, ethyl acetate/methanol). The obtained crude product was purified by HPLC (C18, mobile phase: water/acetonitrile (containing 5 mM ammonium acetate)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (13 mg).
MS (ESI+) : [M+H]⁺380.2.

### Example 160

### (3S)-3-cyclopropyl-1-(2-((1-methyl-5-(piperidin-4-yl)-1H-pyrazol-3-yl)amino)pyridin-4-yl)-2-oxopyrrolidine-3-carbonitrile dihydrochloride

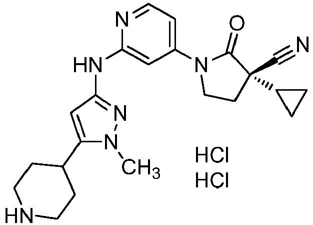

The title compound (250 mg) was obtained using tert-butyl 4-(3-((4-((3S)-3-cyano-3-cyclopropyl-2-oxopyrrolidin-1-yl)pyridin-2-yl)amino)-1-methyl-1H-pyrazol-5-yl)piperidine-1-carboxylate obtained in Example 150 (270 mg), in the same manner as in the method of Step A of Example 159.
MS (ESI+) : [M+H]⁺406.3.

### Example 161

### (3S)-1-(2-((5-(1-acetylpiperidin-4-yl)-1-methyl-1H-pyrazol-3-yl)amino)pyridin-4-yl)-3-cyclopropyl-2-oxopyrrolidine-3-carbonitrile

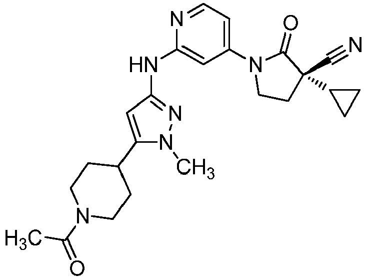

The title compound (82 mg) was obtained using (3S)-3-cyclopropyl-1-(2-((1-methyl-5-(piperidin-4-yl)-1H-pyrazol-3-yl)amino)pyridin-4-yl)-2-oxopyrrolidine-3-carbonitrile dihydrochloride obtained in Example 160 (100 mg), in the same manner as in the method of Step B of Example 159.
MS (ESI+) : [M+H]⁺448.1.

### Example 172

### (3S)-1-(2-((5-(1-acetyl azetidin-3-yl)-1-methyl-1H-pyrazol-3-yl)amino)pyridin-4-yl)-3-cyclopropyl-2-oxopyrrolidine-3-carbonitrile

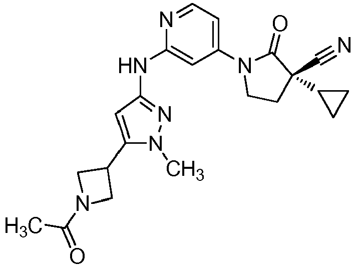

To tert-butyl 3-(3-((4-((3S)-3-cyano-3-cyclopropyl-2-oxopyrrolidin-1-yl)pyridin-2-yl)amino)-1-methyl-1H-pyrazol-5-yl)azetidine-1-carboxylate obtained in Example 145 (75 mg) was added 4 M hydrogen chloride ethyl acetate solution (5.0 mL), and the mixture was stirred overnight at room temperature. The solvent was evaporated under reduced pressure, to a solution of the residue in pyridine (3.0 mL) was added acetic anhydride (0.030 mL), and the mixture was stirred at room temperature for 2 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, ethyl acetate/methanol). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (15 mg).
MS (ESI+) : [M+H]⁺420.2.

### Example 173

### (3S)-3-cyclopropyl-1-(2-((1-methyl-5-(1-(methylsulfonyl)azetidin-3-yl)-1H-pyrazol-3-yl)amino)pyridin-4-yl)-2-oxopyrrolidine-3-carbonitrile

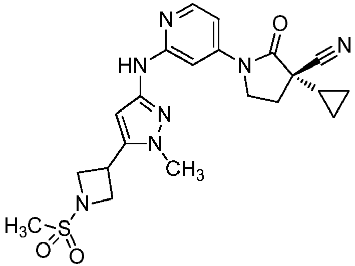

To tert-butyl 3-(3-((4-((3S)-3-cyano-3-cyclopropyl-2-oxopyrrolidin-1-yl)pyridin-2-yl)amino)-1-methyl-1H-pyrazol-5-yl)azetidine-1-carboxylate obtained in Example 145 (75 mg) was added 4 M hydrogen chloride ethyl acetate solution (5.0 mL), and the mixture was stirred overnight at room temperature. The solvent was evaporated under reduced pressure, to a solution of the residue in pyridine (3.0 mL) was added methanesulfonyl chloride (0.024 mL), and the mixture was stirred at room temperature for 2 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate) to give the title compound (34 mg).
MS (ESI+) : [M+H]⁺456.2.

### Example 174

### (3S)-1-(2-((5-(1-(cyclobutylcarbonyl)azetidin-3-yl)-1-methyl-1H-pyrazol-3-yl)amino)pyridin-4-yl)-3-cyclopropyl-2-oxopyrrolidine-3-carbonitrile

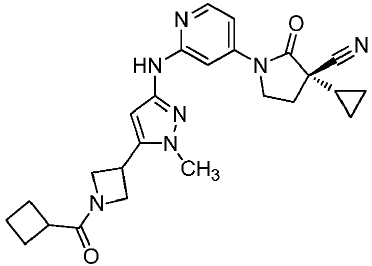

### A) (3S)-1-(2-((5-(azetidin-3-yl)-1-methyl-1H-pyrazol-3-yl)amino)pyridin-4-yl)-3-cyclopropyl-2-oxopyrrolidine-3-carbonitrile dihydrochloride

To tert-butyl 3-(3-((4-((3S)-3-cyano-3-cyclopropyl-2-oxopyrrolidin-1-yl)pyridin-2-yl)amino)-1-methyl-1H-pyrazol-5-yl)azetidine-1-carboxylate obtained in Example 145 (270 mg) was added 4 M hydrogen chloride ethyl acetate solution (5.0 mL), and the mixture was stirred overnight at room temperature. The solvent was evaporated under reduced pressure to give the crude title compound (260 mg).
MS (ESI+) : [M+H]⁺378.2.

### B) (3S)-1-(2-((5-(1-(cyclobutylcarbonyl)azetidin-3-yl)-1-methyl-1H-pyrazol-3-yl)amino)pyridin-4-yl)-3-cyclopropyl-2-oxopyrrolidine-3-carbonitrile

To a solution of the crude (3S)-1-(2-((5-(azetidin-3-yl)-1-methyl-1H-pyrazol-3-yl)amino)pyridin-4-yl)-3-cyclopropyl-2-oxopyrrolidine-3-carbonitrile dihydrochloride obtained in Step A of Example 174 (80 mg) in pyridine (3.0 mL) was added cyclobutanecarbonyl chloride (0.030 mL) under ice-cooling, and the mixture was stirred overnight at room temperature. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate and ethyl acetate/methanol). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (35 mg).
MS (ESI+) : [M+H]⁺460.3.

### Example 175

### (3S)-3-cyclopropyl-1-(2-((5-(1-((3,3-difluorocyclobutyl)carbonyl)azetidin-3-yl)-1-methyl-1H-pyrazol-3-yl)amino)pyridin-4-yl)-2-oxopyrrolidine-3-carbonitrile

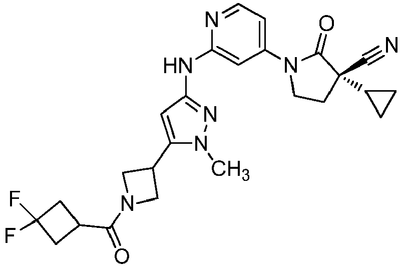

To a solution of (3S)-1-(2-((5-(azetidin-3-yl)-1-methyl-1H-pyrazol-3-yl)amino)pyridin-4-yl)-3-cyclopropyl-2-oxopyrrolidine-3-carbonitrile dihydrochloride obtained in Step A of Example 174 (85 mg) in N,N-dimethylacetamide (5.0 mL) were added triethylamine (260 mL), O-(7-azabenzotriazol-1-yl)-N,N,N',N'-tetramethyluronium hexafluorophosphorate (110 mg), and 3,3-difluorocyclobutanecarboxylic acid (31 mg) under ice-cooling, and the mixture was stirred at room temperature for 3 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate and ethyl acetate/methanol). The obtained crude product was purified by HPLC (C18, mobile phase: water/acetonitrile (containing 5 mM NH₄HCO₃)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure. The residue was purified by column chromatography (NH, hexane/ethyl acetate and ethyl acetate/methanol), and recrystallized (ethyl acetate/diisopropyl ether) to give the title compound (6.2 mg).
MS (ESI+) : [M+H]⁺496.2.

### Example 176

### (3S)-3-cyclopropyl-1-(2-((1-methyl-5-(1-(3,3,3-trifluoropropanoyl)azetidin-3-yl)-1H-pyrazol-3-yl)amino)pyridin-4-yl)-2-oxopyrrolidine-3-carbonitrile

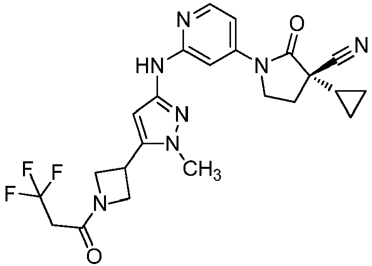

The title compound (15 mg) was obtained using (3S)-1-(2-((5-(azetidin-3-yl)-1-methyl-1H-pyrazol-3-yl)amino)pyridin-4-yl)-3-cyclopropyl-2-oxopyrrolidine-3-carbonitrile dihydrochloride obtained in Step A of Example 174 (85 mg) and 3,3,3-trifluoropropanoic acid (29 mg), in the same manner as in the method of Example 175.
MS (ESI+) : [M+H]⁺488.1.

### Example 177

### optically active 1-(2-((5-(2-hydroxypropan-2-yl)-1-methyl-1H-pyrazol-3-yl)amino)pyridin-4-yl)-3-isopropyl-2-oxopyrrolidine-3-carbonitrile

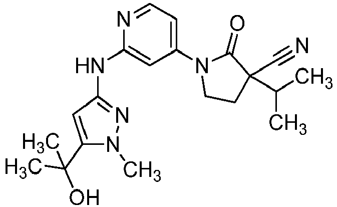

### A) optically active 1-(2-bromopyridin-4-yl)-3-cyclopropyl-2-oxopyrrolidine-3-carbonitrile

To a solution of the optically active 3-isopropyl-2-oxopyrrolidine-3-carbonitrile obtained in Step G of Example 121 (longer retention time) (5.0 g) in N,N-dimethylformamide (50 mL) was added sodium hydride (60% in mineral oil, 1.6 g), and the mixture was stirred at 0°C for 10 min. To the reaction mixture was added a solution of 2-bromo-4-fluoropyridine (6.5 g) in N,N-dimethylformamide (25 mL) at 0°C, and the mixture was stirred at room temperature for 2 hr. The reaction mixture was cooled to 0°C, water was added thereto, and the mixture was extracted with ethyl acetate. The obtained organic layer was washed with saturated brine, and dried over anhydrous magnesium sulfate, and the solvent was evaporated under reduced pressure to give the title compound (4.5 g).
MS (ESI+) : [M+H]⁺308.0.

### B) optically active 1-(2-((5-(2-hydroxypropan-2-yl)-1-methyl-1H-pyrazol-3-yl)amino)pyridin-4-yl)-3-isopropyl-2-oxopyrrolidine-3-carbonitrile

To a solution of the optically active 1-(2-bromopyridin-4-yl)-3-cyclopropyl-2-oxopyrrolidine-3-carbonitrile obtained in Step A of Example 177 (100 mg) in 2-methyl-2-propanol (3.0 mL) were added 2-(3-amino-1-methyl-1H-pyrazol-5-yl)propan-2-ol (76 mg), potassium carbonate (90 mg), 2-(dicyclohexylphosphino)-3,6-dimethoxy-2',4',6'-triisopropyl-1,1'-biphenyl (9 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate and ethyl acetate/methanol). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (40 mg).
MS(ESI+): [M+H] ⁺383.2.

### Example 178

### optically active 3-isopropyl-1-(2-((5-(morpholin-4-yl)pyridin-2-yl)amino)pyridin-4-yl)-2-oxopyrrolidine-3-carbonitrile

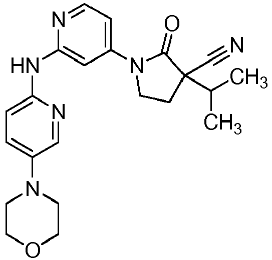

To a solution of the optically active 1-(2-bromopyridin-4-yl)-3-cyclopropyl-2-oxopyrrolidine-3-carbonitrile obtained in Step A of Example 177 (100 mg) in 2-methyl-2-butanol (3.0 mL) were added 5-(morpholin-4-yl)pyridin-2-amine (58 mg), potassium carbonate (90 mg), 2-(dicyclohexylphosphino)-3,6-dimethoxy-2',4',6'-triisopropyl-1,1'-biphenyl (9 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 70°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate) to give the title compound (26 mg).
MS (ESI+) : [M+H] ⁺407.2.

### Example 179

### (3S)-3-cyclopropyl-1-(2-((5-(1,4-oxazepan-4-yl)pyridin-2-yl)amino)pyridin-4-yl)-2-oxopyrrolidine-3-carbonitrile

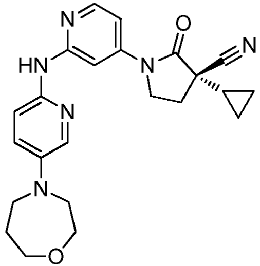

To a solution of (3S)-1-(2-bromopyridin-4-yl)-3-cyclopropyl-2-oxopyrrolidine-3-carbonitrile obtained in Step E of Example 1 (100 mg) in 2-methyl-2-propanol (3.0 mL) were added 5-(1,4-oxazepan-4-yl)pyridin-2-amine (69 mg), potassium carbonate (90 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 90°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (diol, hexane/ethyl acetate) to give the title compound (35 mg).
MS (ESI+) : [M+H] ⁺419.2.

### Examples 180 to 183

The title compounds of Examples 180 to 183 shown in Table 17 were obtained using (3S)-1-(2-bromopyridin-4-yl)-3-cyclopropyl-2-oxopyrrolidine-3-carbonitrile obtained in Step E of Example 1 and the reagents corresponding to the compounds of Examples 180 to 183 (these reagents can be produced according to a method known per se), in the same manner as in the method of Example 179 or a method analogous thereto. MS in the tables means actual measured value.

**Table 17**

| Ex. No. | IUPAC Name | Structure | Salt | MS | Corresponding Reagent |
|---|---|---|---|---|---|
| 180 | (3S)-3-cyclopropyl-1-(2-((5-((3R)-3-hydroxypyrrolidin-1-yl)pyridin-2-yl)amino)pyridin-4-yl)-2-oxopyrrolidine-3-carbonitrile | 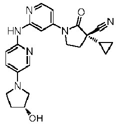 | Free | 405.2 | 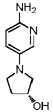 |
| 181 | (3S)-3-cyclopropyl-1-(2-((5-((3S)-3-hydroxypyrrolidin-1-yl)pyridin-2-yl)amino)pyridin-4-yl)-2-oxopyrrolidine-3-carbonitrile | 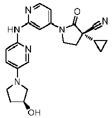 | Free | 405.2 | 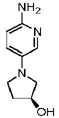 |
| 182 | (3S)-3-cyclopropyl-2-oxo-1-(2-((5-(3-oxomorpholin-4-yl)pyridin-2-yl)amino)pyridin-4-yl)pyrrolidine-3-carbonitrile | 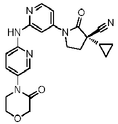 | Free | 419.2 | 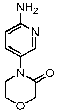 |
| 183 | (3S)-3-cyclopropyl-1-(2-((5-((3R)-3-hydroxypiperidin-1-yl)pyridin-2-yl)amino)pyridin-4-yl)-2-oxopyrrolidine-3-carbonitrile | 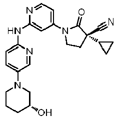 | Free | 419.2 | 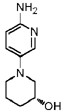 |

### Example 184

### (3S)-3-cyclopropyl-1-(2-((5-(4-hydroxy-4-methylpiperidin-1-yl)pyridin-2-yl)amino)pyridin-4-yl)-2-oxopyrrolidine-3-carbonitrile

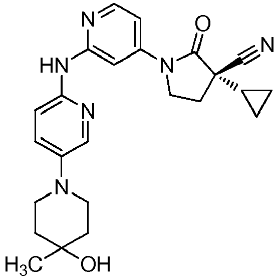

To a solution of (3S)-1-(2-bromopyridin-4-yl)-3-cyclopropyl-2-oxopyrrolidine-3-carbonitrile obtained in Step E of Example 1 (100 mg) in 2-methyl-2-propanol (3.0 mL) were added 1-(6-aminopyridin-3-yl)-4-methylpiperidin-4-ol (75 mg), potassium carbonate (90 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 90°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (diol, hexane/ethyl acetate). The obtained crude product was purified by HPLC (C18, mobile phase: water/acetonitrile (containing 5 mM ammonium acetate)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure. The obtained solid was recrystallized (ethyl acetate) to give the title compound (35 mg).
MS (ESI+) : [M+H] ⁺433.2.

### Example 185

### (3S)-3-cyclopropyl-2-oxo-1-(2-((5-(5-oxo-4,5-dihydro-1,2,4-oxadiazol-3-yl)pyridin-2-yl)amino)pyridin-4-yl)pyrrolidine-3-carbonitrile

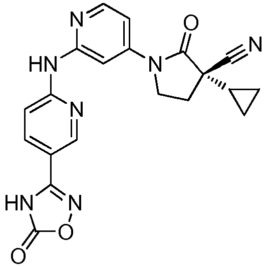

To a solution of (3S)-1-(2-bromopyridin-4-yl)-3-cyclopropyl-2-oxopyrrolidine-3-carbonitrile obtained in Step E of Example 1 (100 mg) in 2-methyl-2-propanol (3.0 mL) were added 3-(6-aminopyridin-3-yl)-1,2,4-oxadiazol-5(4H)-one (87 mg), potassium carbonate (135 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 90°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (ethyl acetate/methanol) to give the title compound (45 mg).
MS (ESI+) : [M+H] ⁺404.2.

### Example 187

### N-(2-((4-((3S)-3-cyano-3-cyclopropyl-2-oxopyrrolidin-1-yl)pyridin-2-yl)amino)-5-fluoropyridin-4-yl)acetamide

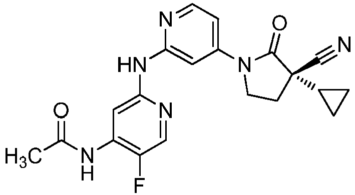

To a solution of (3S)-1-(2-aminopyridin-4-yl)-3-cyclopropyl-2-oxopyrrolidine-3-carbonitrile obtained in Example 2 (100 mg) in 2-methyl-2-butanol (2.0 mL) were added N-(2-chloro-5-fluoropyridin-4-yl)acetamide (78 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate), and recrystallized (diethyl ether/ethyl acetate) to give the title compound (83 mg).
MS(ESI+): [M+H]+ 395.2.

### Examples 188 to 219

The title compounds of Examples 188 to 219 shown in Table 18-Table 21 were obtained using (3S)-1-(2-aminopyridin-4-yl)-3-cyclopropyl-2-oxopyrrolidine-3-carbonitrile obtained in Example 2 and the reagents corresponding to the compounds of Examples 188 to 219 (these reagents can be produced according to a method known per se), in the same manner as in the method of Example 187 or a method analogous thereto. MS in the tables means actual measured value.

**Table 18**

| Ex. No. | IUPAC Name | Structure | Salt | MS | Corresponding Reagent |
|---|---|---|---|---|---|
| 188 | N-(2-((4-((3S)-3-eyano-3-cyclopropyl-2-oxopyrrolidin-1 yl)pyridin-2-yl)amino)pyridin-4-yl)-2-methoxyacetamide | 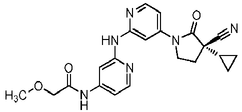 | Free | 407.0 | 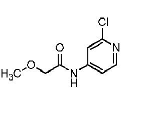 |
| 189 | 2-(6-((4-((3S)-3-cyano-3-cyclopropyl-2-oxopyrrolidin-1 yl)pyridin-2-yl)amino)pyridin-3-yl)-N,N,2-trimethylpropanamide | 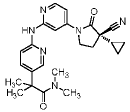 | Free | 433.1 | 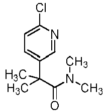 |
| 190 | tert-butyl 6-((4-((3S)-3-cyano-3-cyclopropyl-2-oxopyrrolidin-1-yl)pyridin-2-yl)amino)-1H-pyrrolo[3, 2-c]pyridine-1-carboxylate | 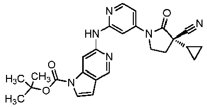 | Free | 459.3 | 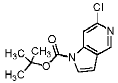 |
| 191 | (3S)-3-cyclopropyl-2-oxo-1-(2-([1,2,4]triazolo[4,3-c]pyrimidin-7-ylamino)pvridin-4-yl)pyrrolidine-3-carbonitrile | 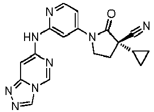 | Free | 361.2 2 | 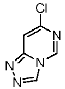 |
| 192 | tert-butyl (2-((4-((3S)-3-cyano-3-cyclopropyl-2-oxopyrrolidin-1-yl)pyridin-2-yl)amino)pyridin-4-yl)carbamate | 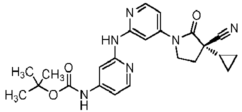 | Free | 435.1 | 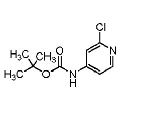 |
| 194 | (3S)-3-cyclopropyl-1-(2-((5-(2-methyl-1-(morpholin-4-yl)-1-oxopropan-2-yl)pyridin-2-yl)amino)pyridin-4-yl)-2-oxopyrrolidine-3-carbonitrile | 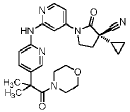 | Free | 475.2 | 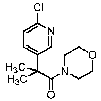 |
| 195 | N-(2-((4-((3S)-3-cyano-3-cyclopropyl-2-oxopyrrolidin-1-yl)pyridin-2-yl)amino)pyridin-4-yl)cyclopropanecarboxamide | 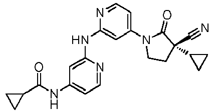 | Free | 403.2 | 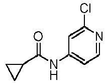 |

**Table 19**

| | | | | | |
|---|---|---|---|---|---|
| 196 | 2-(6-((4-((3S)-3-cyano-3-cyclopropyl-2-oxopyrrolidin-1-yl)pyridin-2-yl)amino)pyridin-3-yl)-N,2-dimethyl-N-(oxetan-3-yl)propanamide | 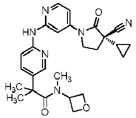 | Free | 475.2 | 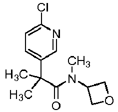 |
| 197 | methyl (2-((4-((3S)-3-cyano-3-cyclopropyl-2-oxopyrrolidin-1-yl)pyridin-2-yl)amino)pyridin-4-yl)carbamate | 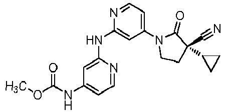 | Free | 393.2 | 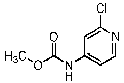 |
| 198 | (3S)-1-(2-((5-(1-(azetidin-1-ylcarbonyl)cyclopropyl)pyridin 2-yl)amino)pyridin-4-yl)-3-cyclopropyl-2-oxopyrrolidine-3-carbonitrile | 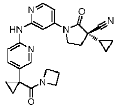 | Free | 443.1 | 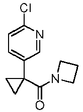 |
| 199 | 1-(6-((4-((3S)-3-cyano-3-cyclopropyl-2-oxopyrrolidin-1-yl)pyridin-2-yl)amino)pyridin-3-yl)-N-(2,2,2-trifluoroethyl)cyclopropanecar boxamide | 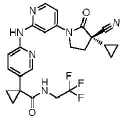 | Free | 485.1 | 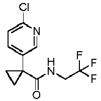 |
| 200 | 1-(6-((4-((3S)-3-cyano-3-cyclopropyl-2-oxopyrrolidin-1-yl)pyridin-2-yl)amino)pyridin-3-yl)-N-cyclopropyl-N-methylcyclopropanecarboxamide | 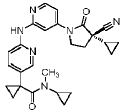 | Free | 457.2 | 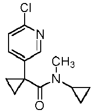 |
| 201 | (3S)-3-cyclopropyl-1-(2-((5-(1-((3,3-difluoroazetidin-1-yl)carbonyl)cyclopropyl)pyridi n-2-yl)amino)pyridin-4-yl)-2-oxopyrrolidine-3-carbonitrile | 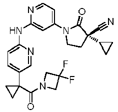 | Free | 479.2 | 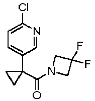 |
| 202 | 1-(6-((4-((3S)-3-cyano-3-cyclopropyl-2-oxopyrrolidin-1-yl)pyridin-2-yl)amino)pyridin-3-yl)-N-methylcyclopropanecarboxamide | 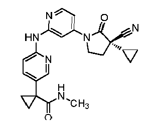 | Free | 417.0 | 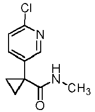 |
| 203 | 1-(6-((4-((3S)-3-cyano-3-cyclopropyl-2-oxopyrrolidin-1-yl)pyridin-2-yl)amino)pyridin-3-yl)cyclopropanecarboxamide | 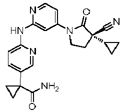 | Free | 403.0 | 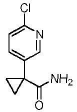 |
| 204 | 5-((4-((3S)-3-cyano-3-cyclopropyl-2-oxopyrrolidin-1-yl)pyridin-2-yl)amino)-N,N,4-trimethylnicotinamide | 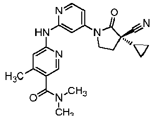 | Free | 405.2 | 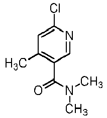 |

**Table 20**

| | | | | | |
|---|---|---|---|---|---|
| 205 | 4-chloro-6-((4-((3S)-3-cyano-3-cyclopropyl-2-oxopyrrolidin 1-yl)pyridin-2-yl)amino)-N,N-dimethylnicotinamide | 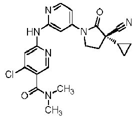 | Free | 425.2 | 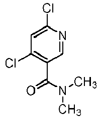 |
| 206 | (3S)-3-cyclopropyl-1-(2-((4-methyl-5-(morpholin-4-ylcarbonyl)pyridin-2-yl)amino)pyridin-4-yl)-2-oxopyrrolidine-3-carbonitrile | 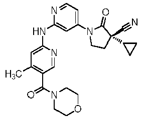 | Free | 447.2 | 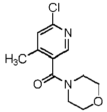 |
| 207 | (3S)-3-cyclopropyl-1-(2-((5-((4-hydroxy-4-methylpiperidin-1-yl)carbonyl)-4-methylpyridin-2-yl)amino)pyridin-4-yl)-2-oxopyrrolidine-3-carbonitrile | 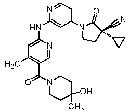 | Free | 475.3 | 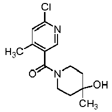 |
| 208 | (3S)-3-cyclopropyl-1-(2-((5-(((3R)-3-hydroxypyrrolidin-1-yl)carbonyl)-4-methylpyridin-2-yl)amino)pyridin-4-yl)-2-oxopyrrolidine-3-carbonitrile | 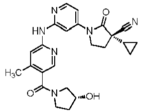 | Free | 447.2 | 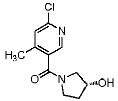 |
| 209 | (3S)-3-cyclopropyl-1-(2-((5-(((3S)-3-hydroxypyrrolidin-1-yl)carbonyl)-4-methylpyridin-2-yl)amino)pyridin-4-yl)-2-oxopyrrolidine-3-carbonitrile | 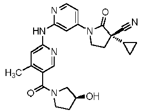 | Free | 447.2 | 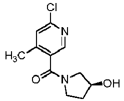 |
| 211 | 1-(6-((4-((3S)-3-cyano-3-cyclopropyl-2-oxopyrrolidin-1-yl)pyridin-2-yl)amino)pyridin-3-yl)-N-(oxetan-3-yl)cyclopentanecarboxamide | 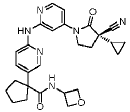 | Free | 487.1 | 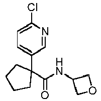 |
| 212 | 1-(6-((4-((3S)-3-cyano-3-cyclopropyl-2-oxopyrrolidin-1 yl)pyridin-2-yl)amino)pyridin-3-yl)-N-(2-methoxyethyl)cyclopropanecarbo xamide | 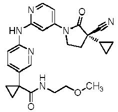 | Free | 461.3 | 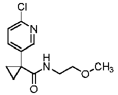 |
| 213 | 6-((4-((3S)-3-cyano-3-cyclopropyl-2-oxopyrrolidin-1 yl)pyridin-2-yl)amino)-4-methyl-N-((3R)-tetrahydrofuran-3-yl)nicotinamide | 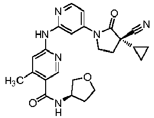 | Free | 447.2 | 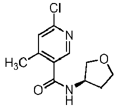 |
| 214 | 5-((4-((3S)-3-cyano-3-cyclopropyl-2-oxopyrrolidin-1 yl)pyridin-2-yl)amino)-4-methyl-N-((3S)-tetrahydrofuran-3-yl)nicotinamide | 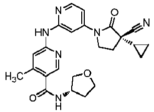 | Free | 447.2 | 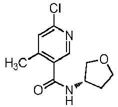 |

**Table 21**

| | | | | | |
|---|---|---|---|---|---|
| 215 | (3S)-3-cyclopropyl-1-(2-((5-(((3R)-3-hydroxypiperidin-1-yl)carbonyl)-4-methylpyridin-2-yl)amino)pyridin-4-yl)-2-oxopyrrolidine-3-carbonitrile | 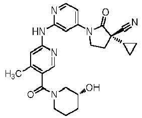 | Free | 461.2 | 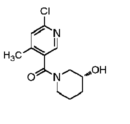 |
| 216 | (3S)-3-cyclopropyl-1-(2-((5-(((3S)-3-hydroxypiperidin-1-yl)carbonyl)-4-methylpyridin-2-yl)amino)pyridin-4-yl)-2-oxopyrrolidine-3-carbonitrile | 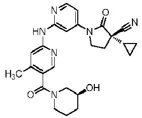 | Free | 461.2 | 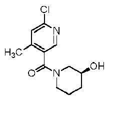 |
| 217 | (3S)-3-cyclopropyl-1-(2-((5-(1-(((3R)-3-hydroyxypyrrolidin-1-yl)carbonyl)cyclopropyl)pyridi n-2-yl)amino)pyridin-4-yl)-2-oxopyrrolidine-3-carbonitrile | 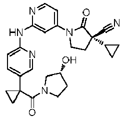 | Free | 473.2 | 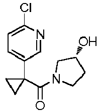 |
| 218 | (3S)-3-cyclopropyl-1-(2-((5-(1-(((3S)-3-hydroxypyrrolidin-1-yl)carbonyl)cyclopropyl)pyridi n-2-yl)amino)pyridin-4-yl)-2-oxopyrrolidine-3-carbonitrile | 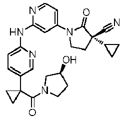 | Free | 473.2 | 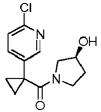 |
| 219 | 1-(5-((4-((3S)-3-cyano-3-cyclopropyl-2-oxopyrrolidin-1-yl)pyridin-2-yl)amino)pyridin-3-yl)-N-methylcyclopentanecarboxamide | 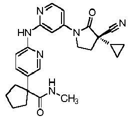 | Free | 445.1 | 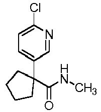 |

### Example 220

### (3S)-1-(2-((4-aminopyridin-2-yl)amino)pyridin-4-yl)-3-cyclopropyl-2-oxopyrrolidine-3-carbonitrile dihydrochloride

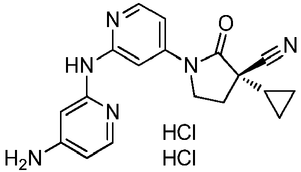

The title compound (180 mg) was obtained using tert-butyl (2-((4-((3S)-3-cyano-3-cyclopropyl-2-oxopyrrolidin-1-yl)pyridin-2-yl)amino)pyridin-4-yl)carbamate obtained in Example 192 (220 mg), in the same manner as in the method of Step A of Example 5.
MS (ESI+) : [M+H]⁺ 335.2.

### Example 221

### N-(2-((4-((3S)-3-cyano-3-cyclopropyl-2-oxopyrrolidin-1-yl)pyridin-2-yl)amino)pyridin-4-yl)-2-hydroxyacetamide

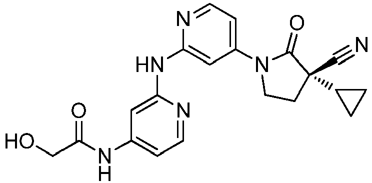

To a solution of (3S)-1-(2-((4-aminopyridin-2-yl)amino)pyridin-4-yl)-3-cyclopropyl-2-oxopyrrolidine-3-carbonitrile dihydrochloride obtained in Example 220 (70 mg) in N,N-dimethylformamide (1.0 mL) were added triethylamine (0.024 mL), 2-chloro-2-oxoethyl acetate (23 mg) and N,N-dimethyl-4-aminopyridine (21 mg), and the mixture was stirred overnight at room temperature. To the reaction mixture was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure. To a solution of the residue in a mixed solvent of tetrahydrofuran/2-methyl-2-butanol (V/V=1/1, 4.0 mL) was added potassium carbonate (48 mg), and the mixture at 80°C for 48 hr was stirred. To the reaction mixture was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure. The obtained residue was purified by silica gel column chromatography (NH, ethyl acetate/methanol) to give the title compound (12 mg).
MS(ESI+): [M+H]+ 393.2.

### Example 222

### N-(2-((4-((3S)-3-cyano-3-cyclopropyl-2-oxopyrrolidin-1-yl)pyridin-2-yl)amino)pyridin-4-yl)-3,3,3-trifluoropropanamide

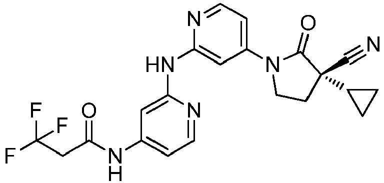

To a solution of (3S)-1-(2-((4-aminopyridin-2-yl)amino)pyridin-4-yl)-3-cyclopropyl-2-oxopyrrolidine-3-carbonitrile dihydrochloride obtained in Example 220 (100 mg) in N,N-dimethylformamide (1.0 mL) were added triethylamine (0.17 mL), O-(7-azabenzotriazol-1-yl)-N,N,N',N'-tetramethyluronium hexafluorophosphorate (280 mg), N,N-dimethyl-4-aminopyridine (3.0 mg) and 3,3,3-trifluoropropanoic acid (94 mg), and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (23 mg).
MS(ESI+): [M+H]+ 445.2.

### Example 223

### 2-cyano-N-(2-((4-((3S)-3-cyano-3-cyclopropyl-2-oxopyrrolidin-1-yl)pyridin-2-yl)amino)pyridin-4-yl)acetamide

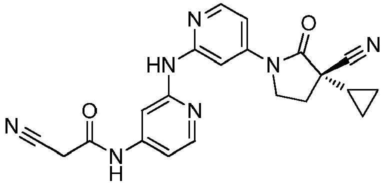

The title compound (27 mg) was obtained using (3S)-1-(2-((4-aminopyridin-2-yl)amino)pyridin-4-yl)-3-cyclopropyl-2-oxopyrrolidine-3-carbonitrile dihydrochloride obtained in Example 220 (90 mg) and cyanoacetic acid (21 mg), in the same manner as in the method of Example 222.
MS(ESI+): [M+H]⁺ 402.2.

### Example 224

### 5-((4-((3S)-3-cyano-3-cyclopropyl-2-oxopyrrolidin-1-yl)pyridin-2-yl)amino)-N,N-dimethylpyrazine-2-carboxamide

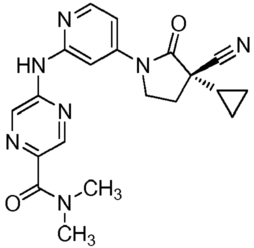

To a solution of (3S)-1-(2-aminopyridin-4-yl)-3-cyclopropyl-2-oxopyrrolidine-3-carbonitrile obtained in Example 2 (100 mg) in 2-methyl-2-butanol (2.0 mL) were added 5-chloro-N,N-dimethylpyrazine-2-carboxamide (110 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (4.0 mg).
MS (ESI+) : [M+H]⁺392.0.

### Example 225

### 5-((4-((3S)-3-cyano-3-cyclopropyl-2-oxopyrrolidin-1-yl)pyridin-2-yl)amino)-N-methylpyrazine-2-carboxamide

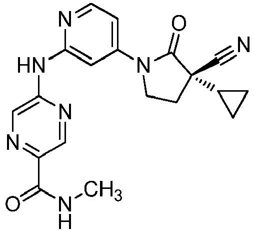

The title compound (38 mg) was obtained using (3S)-1-(2-aminopyridin-4-yl)-3-cyclopropyl-2-oxopyrrolidine-3-carbonitrile obtained in Example 2 (100 mg) and 5-chloro-N-methylpyrazine-2-carboxamide (78 mg) under argon atmosphere, in the same manner as in the method of Example 224. MS (ESI+) : [M+H]⁺378.0.

### Example 226

### 1-(6-((4-((3S)-3-cyano-3-cyclopropyl-2-oxopyrrolidin-1-yl)pyridin-2-yl)amino)pyridin-3-yl)-N-methylcyclobutanecarboxamide

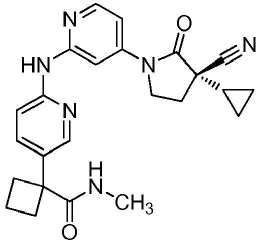

The title compound (29 mg) was obtained using (3S)-1-(2-aminopyridin-4-yl)-3-cyclopropyl-2-oxopyrrolidine-3-carbonitrile obtained in Example 2 (72 mg) and 1-(6-chloropyridin-3-yl)-N-methylcyclobutanecarboxamide (67 mg) under argon atmosphere, in the same manner as in the method of Example 224.
MS (ESI+) : [M+H] ⁺431.1.

### Example 227

### 6-((4-((3S)-3-cyano-3-cyclopropyl-2-oxopyrrolidin-1-yl)pyridin-2-yl)amino)-N,4-dimethylnicotinamide

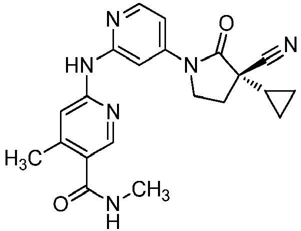

To a solution of (3S)-1-(2-aminopyridin-4-yl)-3-cyclopropyl-2-oxopyrrolidine-3-carbonitrile obtained in Example 2 (140 mg) in 2-methyl-2-butanol (4.0 mL) were added 6-chloro-N,4-dimethylnicotinamide (110 mg), potassium carbonate (170 mg), 2-(dicyclohexylphosphino)-3,6-dimethoxy-2',4',6'-triisopropyl-1,1'-biphenyl (16 mg) and chloro[2-(dicyclohexylphosphino)-3,6-dimethoxy-2',4',6'-triisopropyl-1,1'-biphenyl][2-(2-aminoethyl)phenyl]palladium(II) (24 mg) under argon atmosphere, and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate). The obtained crude product was purified by HPLC (C18, mobile phase: water/acetonitrile (containing 5 mM ammonium acetate)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (35 mg).
¹H NMR (400 MHz, DMSO-d₆) δ0.53 (2H, qd, J = 9.4, 4.2 Hz), 0.62 (1H, dd, J = 8.4, 5.0 Hz), 0.69 (1H, dt, J = 8.7, 4.5 Hz), 1.46-1.61 (1H, m), 2.30-2.41 (4H, m), 2.61-2.70 (1H, m), 2.74 (3H, d, J = 4.4 Hz), 3.89-4.04 (2H, m), 7.29 (1H, dd, J = 5.7, 1.8 Hz), 7.55 (1H, s), 8.13 (1H, d, J = 1.5 Hz), 8.19-8.30 (3H, m), 9.87 (1H, s).
MS (ESI+) : [M+H] ⁺391.2.

### Example 228

### 2-(6-((4-((3S)-3-cyano-3-cyclopropyl-2-oxopyrrolidin-1-yl)pyridin-2-yl)amino)pyridin-3-yl)-N-(2-hydroxyethyl)-N,2-dimethylpropanamide

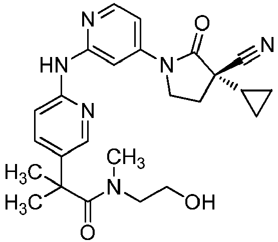

To a solution of (3S)-1-(2-aminopyridin-4-yl)-3-cyclopropyl-2-oxopyrrolidine-3-carbonitrile obtained in Example 2 (80 mg) in 2-methyl-2-butanol (2.0 mL) were added 2-(6-chloropyridin-3-yl)-N-(2-hydroxyethyl)-N,2-dimethylpropanamide (85 mg), potassium carbonate (90 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate). The obtained crude product was purified by silica gel column chromatography (diol, hexane/ethyl acetate) to give the title compound (55 mg).
MS(ESI+): [M+H]+ 463.2.

### Examples 229 to 232

The title compounds of Examples 229 to 232 shown in Table 22 were obtained using (3S)-1-(2-aminopyridin-4-yl)-3-cyclopropyl-2-oxopyrrolidine-3-carbonitrile obtained in Example 2 and the reagents corresponding to the compounds of Examples 229 to 232 (these reagents can be produced according to a method known per se), in the same manner as in the method of Example 228 or a method analogous thereto. MS in the tables means actual measured value.

**Table 22**

| Ex. No. | IUPAC Name | Structure | Salt | MS | Corresponding Reagent |
|---|---|---|---|---|---|
| 229 | 2-(6-((4-((3S)-3-cyano-3-cyclopropyl-2-oxopyrrolidin-1 yl)pyridin-2-yl)amino)pyridin-3-yl)-N-(2-hydroxyethyl)-2-methylpropanamide | 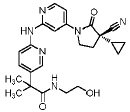 | Free | 449.2 | 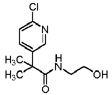 |
| 230 | 1-(6-((4-((3S)-3-cyano-3-cyclopropyl-2-oxopyrrolidin-1 yl)pyridin-2-yl)amino)pyridin-3-yl)-N,N-dimethylcyclopropanecarboxamid e | 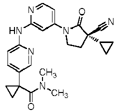 | Free | 431.1 | 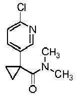 |
| 231 | 1-(6-((4-((3S)-3-cyano-3-cyclopropyl-2-oxopyrrolidin-1 yl)pyridin-2-yl)amino)pyridin-3-yl)cyclopentanecarboxamide | 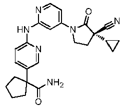 | Free | 431.1 | 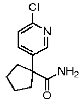 |
| 232 | 6-((4-((3S)-3-cyano-3-cyclopropyl-2-oxopyrrolidin-1 yl)pyridin-2-yl)amino)-N-(1-hydroxy-2-methylpropan-2-yl)-N-methylnicotinamide | 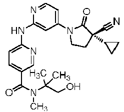 | Free | 449.1 | 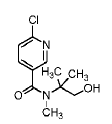 |

### Example 233

### 1-(6-((4-((3S)-3-cyano-3-cyclopropyl-2-oxopyrrolidin-1-yl)pyridin-2-yl)amino)pyridin-3-yl)cyclobutanecarboxamide

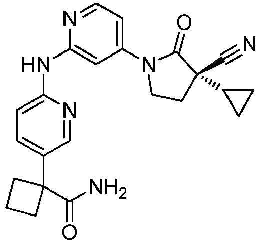

To a solution of (3S)-1-(2-aminopyridin-4-yl)-3-cyclopropyl-2-oxopyrrolidine-3-carbonitrile obtained in Example 2 (100 mg) in 2-methyl-2-butanol (2.0 mL) were added 1-(6-chloropyridin-3-yl)cyclobutanecarboxamide (87 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate). The obtained crude product was purified by silica gel column chromatography (diol, hexane/ethyl acetate). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (36 mg). MS (ESI+) : [M+H]⁺417.0.

### Example 234

### (3S)-3-cyclopropyl-1-(2-((5-(1-(3-oxa-8-azabicyclo[3.2.1]oct- 8-ylcarbonyl)cyclopropyl)pyridin-2-yl)amino)pyridin-4-yl)-2- oxopyrrolidine-3-carbonitrile

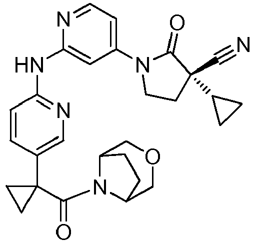

To a solution of (3S)-1-(2-aminopyridin-4-yl)-3-cyclopropyl-2-oxopyrrolidine-3-carbonitrile obtained in Example 2 (100 mg) in 2-methyl-2-butanol (2.0 mL) were added (1-(6-chloropyridin-3-yl)cyclopropyl)(3-oxa-8-azabicyclo[3.2.1]oct-8-yl)methanone (120 mg), potassium carbonate (120 mg), 2-(dicyclohexylphosphino)-3,6-dimethoxy-2',4',6'-triisopropyl-1,1'-biphenyl (12 mg) and chloro[2-(dicyclohexylphosphino)-3,6-dimethoxy-2',4',6'-triisopropyl-1,1'-biphenyl][2-(2-aminoethyl)phenyl]palladium(II) (17 mg) under argon atmosphere, and the mixture was stirred at 100°C for 9 hr. To the reaction mixture were added potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (17 mg), and the mixture was stirred at 100°C for 15 hr. To the reaction mixture were added potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (17 mg), and the mixture was stirred at 100°C for 9 hr. To the reaction mixture were added potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg), and the mixture was stirred at 100°C for 12 hr. To the reaction mixture were added saturated aqueous sodium hydrogencarbonate solution and ethyl acetate, and the insoluble substance was removed through Celite. The filtrate was extracted with ethyl acetate, the obtained organic layer was washed with water and saturated brine, and dried over anhydrous magnesium sulfate, and the solvent was evaporated under reduced pressure. The residue was purified by silica gel column chromatography (NH, hexane/ethyl acetate) to give the title compound (42 mg).
MS (ESI+) : [M+H]⁺499.3.

### Example 235

### 1-(6-((4-((3S)-3-cyano-3-cyclopropyl-2-oxopyrrolidin-1-yl)pyridin-2-yl)amino)pyridin-3-yl)-N-(oxetan-3-yl)cyclopropanecarboxamide

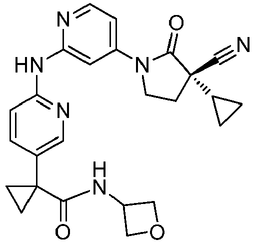

To a solution of (3S)-1-(2-aminopyridin-4-yl)-3-cyclopropyl-2-oxopyrrolidine-3-carbonitrile obtained in Example 2 (100 mg) in 2-methyl-2-butanol (2.0 mL) were added 1-(6-chloropyridin-3-yl)-N-(oxetan-3-yl)cyclopropanecarboxamide (100 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (17 mg) under argon atmosphere, and the mixture was stirred at 100°C for 15 hr. To the reaction mixture were added potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (17 mg), and the mixture was stirred at 100°C for 10 hr. To the reaction mixture were added 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (17 mg), and the mixture was stirred at 100°C for 60 hr. To the reaction mixture were added saturated aqueous sodium hydrogencarbonate solution and ethyl acetate, and the insoluble substance was removed through Celite. The filtrate was extracted with ethyl acetate, and the obtained organic layer was washed with water and saturated brine, and dried over anhydrous magnesium sulfate, and the solvent was evaporated under reduced pressure. The residue was purified by silica gel column chromatography (NH, hexane/ethyl acetate) to give the title compound (8.0 mg).
MS(ESI+): [M+H]⁺459.1.

### Example 236

### 4-(6-((4-((3S)-3-cyano-3-cyclopropyl-2-oxopyrrolidin-1-yl)pyridin-2-yl)amino)pyridin-3-yl)-N-methyltetrahydro-2H-pyran-4-carboxamide

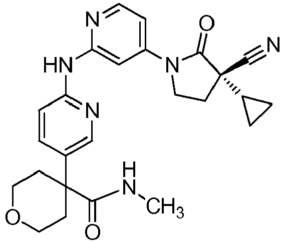

To a solution of (3S)-1-(2-aminopyridin-4-yl)-3-cyclopropyl-2-oxopyrrolidine-3-carbonitrile obtained in Example 2 (59 mg) in 2-methyl-2-butanol (2.0 mL) were added 4-(6-chloropyridin-3-yl)-N-methyltetrahydro-2H-pyran-4-carboxamide (63 mg), potassium carbonate (68 mg), 2-(dicyclohexylphosphino)-3,6-dimethoxy-2',4',6'-triisopropyl-1,1'-biphenyl (6.8 mg) and chloro[2-(dicyclohexylphosphino)-3,6-dimethoxy-2',4',6'-triisopropyl-1,1'-biphenyl][2-(2-aminoethyl)phenyl]palladium(II) (20 mg) under argon atmosphere, and the mixture was stirred at 100°C for 9 hr. To the reaction mixture were added potassium carbonate (68 mg), 2-(dicyclohexylphosphino)-3,6-dimethoxy-2',4',6'-triisopropyl-1,1'-biphenyl (13 mg) and chloro[2-(dicyclohexylphosphino)-3,6-dimethoxy-2',4',6'-triisopropyl-1,1'-biphenyl][2-(2-aminoethyl)phenyl]palladium(II) (20 mg), and the mixture was stirred at 100°C for 60 hr. To the reaction mixture were added saturated aqueous sodium hydrogencarbonate solution and ethyl acetate, and the insoluble substance was removed through Celite. The filtrate was extracted with ethyl acetate, and the obtained organic layer was washed with water and saturated brine, and dried over anhydrous magnesium sulfate, and the solvent was evaporated under reduced pressure. The residue was purified by silica gel column chromatography (NH, hexane/ethyl acetate). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (4.0 mg).
MS (ESI+) : [M+H]⁺461.1.

### Example 237

### (3S)-3-cyclopropyl-2-oxo-1-(2-(1H-pyrrolo[3,2-c]pyridin-6-ylamino)pyridin-4-yl)pyrrolidine-3-carbonitrile

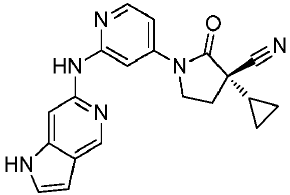

To tert-butyl 6-((4-((3S)-3-cyano-3-cyclopropyl-2-oxopyrrolidin-1-yl)pyridin-2-yl)amino)-1H-pyrrolo[3,2-c]pyridine-1-carboxylate (100 mg) obtained in Example 190 was added 4 M hydrogen chloride ethyl acetate solution (5.0 mL), the mixture was stirred overnight at 50°C, and the solvent was evaporated under reduced pressure. To the residue was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure. The obtained solid was recrystallized (ethyl acetate/hexane) to give the title compound (77 mg).
MS (ESI+) : [M+H]⁺359.0.

### Example 239

### 3-((4-((3S)-3-cyano-3-cyclopropyl-2-oxopyrrolidin-1-yl)pyridin-2-yl)amino)-N-(2,2-difluoroethyl)-1-methyl-1H-pyrazole-5-carboxamide

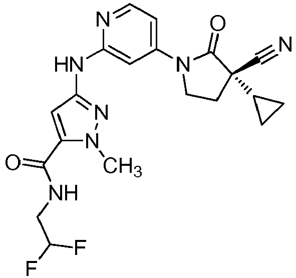

To a solution of 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 (100 mg) in N,N-dimethylformamide (2.0 mL) were added N,N-diisopropylethylamine (0.13 mL), O-(7-azabenzotriazol-1-yl)-N,N,N',N'-tetramethyluronium hexafluorophosphorate (190 mg), and 2,2-difluoroethanamine (0.060 mL), and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate), and recrystallized (hexane/ethyl acetate) to give the title compound (95 mg).
MS(ESI+): [M+H]+ 430.2.

### Examples 240 to 244

The title compounds of Examples 240 to 244 shown in Table 23 were obtained using 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 and the reagents corresponding to the compounds of Examples 240 to 244 (these reagents can be produced according to a method known per se), in the same manner as in the method of Example 239 or a method analogous thereto. MS in the tables means actual measured value.

**Table 23**

| Ex. No. | IUPAC Name | Structure | Salt | MS | Corresponding Reagent |
|---|---|---|---|---|---|
| 240 | 3-((4-((3S)-3-cyano-3-cyclopropyl-2-oxopyrrolidin-1 yl)pyridin-2-yl)amino)-N-cyclobutyl-1-methyl-1H-pyrazole-5-carboxamide | 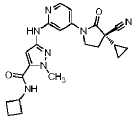 | Free | 420.3 | 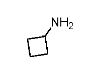 |
| 241 | 3-((4-((3S)-3-cyano-3-cyclopropyl-2-oxopyrrolidin-1 yl)pyridin-2-yl)amino)-1-methyl-N-((2R)-tetrahydrofuran-2-ylmethyl)-1H-pyrazole-5-carboxamide | 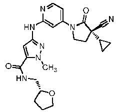 | Free | 450.1 | 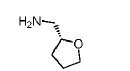 |
| 242 | 3-((4-((3S)-3-cyano-3-cyclopropyl-2-oxopyrrolidin-1 yl)pyridin-2-yl)amino)-1-methyl-N-((2S)-tetrahydrofuran-2-ylmethyl)-1H-pyrazole-5-carboxamide | 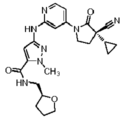 | Free | 450.1 | 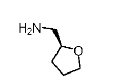 |
| 243 | (3S)-3-cyclopropyl-1-(2-((1-methyl-5-(1,4-o_{Y}azepan-4-ylcarbonyl)-1H-pyrazol-3-yl)amino)pyridin-4-yl)-2-oxopyrrolidine-3-carbonitrile | 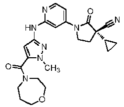 | Free | 450.1 | 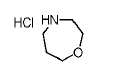 |
| 244 | (3S)-3-cyclopropyl-1-(2-((1-methyl-5-(6-oxa-3-azabicyclo[3.1.1]hept-3-ylcarbonyl)-1H-pyrazol-3-yl)amino)pyridin-4-yl)-2-oxopyrrolidine-3-carbonitrile | 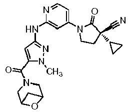 | Free | 448.1 | 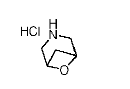 |

### Example 245

### (3S)-3-cyclopropyl-1-(2-((1-methyl-5-(3-oxa-8-azabicyclo[3.2.1]oct-8-ylcarbonyl)-1H-pyrazol-3-yl)amino)pyridin-4-yl)-2-oxopyrrolidine-3-carbonitrile

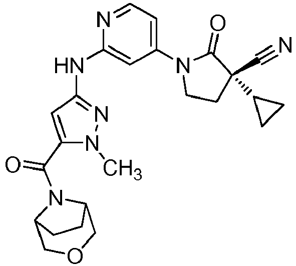

To a solution of 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 (80 mg) in N,N-dimethylacetamide (2.0 mL) were added triethylamine (0.17 mL), 1.7 M propane phosphonic acid anhydride ethyl acetate solution (0.35 mL), N,N-dimethyl-4-aminopyridine (2.4 mg) and 3-oxa-8-azabicyclo[3.2.1]octane hydrochloride (30 mg), and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure. The residue was purified by column chromatography (NH, hexane/ethyl acetate) to give the title compound (4.1 mg).
MS(ESI+): [M+H]+ 462.1.

### Example 246

### 3-((4-((3S)-3-cyano-3-cyclopropyl-2-oxopyrrolidin-1-yl)pyridin-2-yl)amino)-N-(2-hydroxy-2-methylpropyl)-1-methyl-1H-pyrazole-5-carboxamide

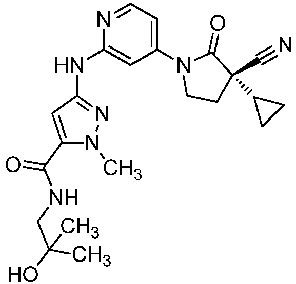

To a solution of 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 (100 mg) in N,N-dimethylformamide (1.0 mL) were added triethylamine (0.17 mL), 1.7 M propane phosphonic acid anhydride ethyl acetate solution (0.44 mL), N,N-dimethyl-4-aminopyridine (3.0 mg) and 1-amino-2-methylpropan-2-ol (66 mg), and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (48 mg).
MS(ESI+): [M+H]+ 438.2.

### Examples 247 to 274

The title compounds of Examples 247 to 274 shown in Table 24-Table 27 were obtained using 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 and the reagents corresponding to the compounds of Examples 247 to 274 (these reagents can be produced according to a method known per se), in the same manner as in the method of Example 246 or a method analogous thereto. MS in the tables means actual measured value.

**Table 24**

| Ex. No. | IUPAC Name | Structure | Salt | MS | Corresponding Reagent |
|---|---|---|---|---|---|
| 247 | 3-((4-((3S)-3-cyano-3-cyclopropyl-2-oxopyrrolidin-1-yl)pyridin-2-yl)amino)-1-methyl-N-(oxetan-3-ylmethyl)-1H-pyrazole-5-carboxamide | 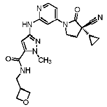 | Free | 436.2 | 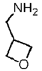 |
| 248 | 3-((4-((3S)-3-cyano-3-cyclopropyl-2-oxopyrrolidin-1-yl)pyridin-2-yl)amino)-1-methyl-N-(oxetan-3-yl)-1H-pyrazole-5-carboxamide | 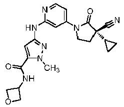 | Free | 422.1 | 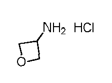 |
| 249 | 3-((4-((3S)-3-cyano-3-cyclopropyl-2-oxopyrrolidin-1-yl)pyridin-2-yl)amino)-N-(3,3-difluorocyclobutyl)-1-methyl-1H-pyrazole-5-carboxamide | 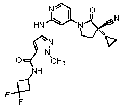 | Free | 456.1 | 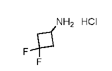 |
| 250 | 3-((4-((3S)-3-cyano-3-cyclopropyl-2-oxopyrrolidin-1-yl)pyridin-2-yl)amino)-1-methyl-N-(tetrahydro-2H-pyran-4-yl)-1H-pyrazole-5-carboxamide | 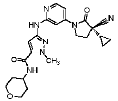 | Free | 450.2 | 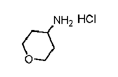 |
| 251 | 3-((4-((3S)-3-cyano-3-cyclopropyl-2-oxopyrrolidin-1-yl)pyridin-2-yl)amino)-1-methyl-N-((3S)-tetrahydrofuran-3-yl)-1H-pyrazole-5-carboxamide | 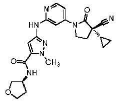 | Free | 436.1 | 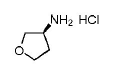 |
| 252 | 3-((4-((3S)-3-cyano-3-cyclopropyl-2-oxopyrrolidin-1-yl)pyridin-2-yl)amino)-1-methyl-N-((3R)-tetrahydrofuran-3-yl)-1H-pyrazole-5-carboxamide | 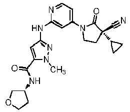 | Free | 436.1 | 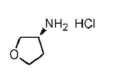 |
| 253 | N-(1-cyanocyclobutyl)-3-((4-((3S)-3-cyano-3-cyclopropyl-2-oxopyrrolidin-1-yl)pyridin-2-yl)amino)-1-methyl-1H-pyrazole-5-carboxamide | 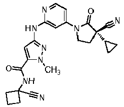 | Free | 445.2 | 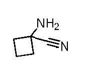 |
| 254 | 3-((4-((3S)-3-cyano-3-cyclopropyl-2-oxopyrrolidin-1-yl)pyridin-2-yl)amino)-N-(1,1 dioxidotetrahydro-2H-thiopyran-4-yl)-1-methyl-1H-pyrazole-5-carboxamide | 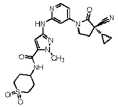 | Free | 498.3 | 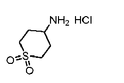 |

**Table 25**

| | | | | | |
|---|---|---|---|---|---|
| 255 | (3S)-3-cyclopropyl-1-(2-((1-methyl-5-(pyrrolidin-1-ylcarbonyl)-1H-pyrazol-3-yl)amino)pyridin-4-yl)-2-oxopyrrolidine-3-carbonitrile | 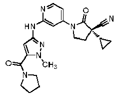 | Free | 420.2 | 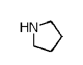 |
| 256 | (3S)-3-cyclopropyl-1-(2-((5-((3,3-difluoroazetidin-1-yl)carbonyl)-1-methyl-1H-pyrazol-3-yl)amino)pyridin-4-yl)-2-oxopyrrolidine-3-carbonitrile | 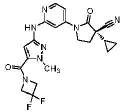 | Free | 442. 2 | 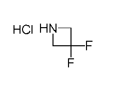 |
| 257 | (3S)-3-cyclopropyl-1-(2-((1-methyl-5-(morpholin-4-ylcarbonyl)-1H-pyrazol-3-yl)amino)pyridin-4-yl)-2-oxopyrrolidine-3-carbonitrile | 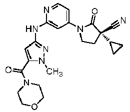 | Free | 436.2 | 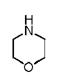 |
| 258 | (3S)-3-cyclopropyl-1-(2-((1-methyl-5-(8-oxa-3-azabicyclo[3.2.1]oct-3-ylcarbonyl)-1H-pyrazol-3-yl)amino)pyridin-4-yl)-2-oxopyrrolidine-3-carbonitrile | 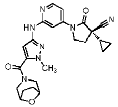 | Free | 462.1 | 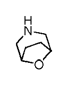 |
| 259 | (3S)-3-cyclopropyl-1-(2-((1-methyl-5-(2-oxa-6-azaspiro[3.4]oct-6-ylcarbonyl)-1H-pyrazol-3-yl)amino)pyridin-4-yl)-2-oxopyrrolidine-3-carbonitrile | 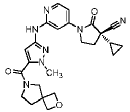 | Free | 462.1 | 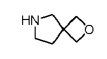 |
| 260 | (3S)-3-cyclopropyl-1-(2-((5-((3-fluoroazetidin-1-yl)carbonyl)-1-methyl-1H-pyrazol-3-yl)amino)pyridin-4-yl)-2-oxopyrrolidine-3-carbonitrile | 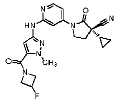 | Free | 424.2 | 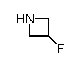 |
| 261 | (3S)-3-cyclopropyl-1-(2-((5-(((3R)-3-fluoropyrrolidin-1-yl)carbonyl)-1-methyl-1H-pyrazol-3-yl)amino)pyridin-4-yl)-2-oxopyrrolidine-3-carbonitrile | 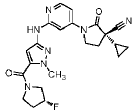 | Free | 438.2 | 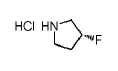 |
| 262 | (3S)-3-cyclopropyl-1-(2-((5-(((3S)-3-fluoropyrrolidin-1-yl)carbonyl)-1-methyl-1H-pyrazol-3-yl)amino)pyridin-4-yl)-2-oxopyrrolidine-3-carbonitrile | 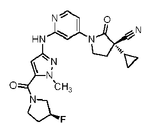 | Free | 438.2 | 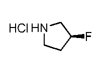 |
| 263 | (3S)-1-(2-((5-(3-azabicyclo[3.1.0]hex-3-ylcarbonyl)-1-methyl-1H-pyrazol-3-yl)amino)pyridin-4-yl)-3-cyclopropyl-2-oxopyrrolidine-3-carbonitrile | 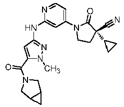 | Free | 432.2 | 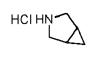 |

**Table 26**

| | | | | | |
|---|---|---|---|---|---|
| 264 | 3-((4-((3S)-3-cyano-3-cyclopropyl-2-oxopyrrolidin-1 yl)pyridin-2-yl)amino)-N,1-dimethyl-N-(oxetan-3-yl)-1H-pyrazole-5-carboxamide | 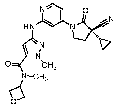 | Free | 436.2 | 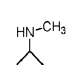 |
| 265 | (3S)-3-cyclopropyl-1-(2-((5-(1,3-dihydro-2H-pyrrolo[3,4-c]pyridin-2-ylcarbonyl)-1-methyl-1H-pyrazol-3-yl)amino)pyridin-4-yl)-2-oxopyrrolidine-3-carbonitrile | 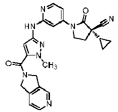 | Free | 469.2 | 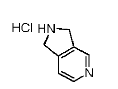 |
| 266 | (3S)-3-cyclopropyl-1-(2-((5-(5,7-dihydro-6H-pyrrolo[3,4-b]pyridin-5-ylcarbonyl)-1-methyl-1H-pyrazol-3-yl)amino)pyridin-4-yl)-2-oxopyrrolidine-3-carbonitrile | 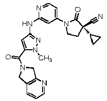 | Free | 469.2 | 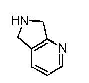 |
| 267 | (3S)-3-cyclopropyl-1-(2-((5-(5,7-dihydro-6H-pyrrolo[3,4-d]pyrimidin-5-ylcarbonyl)-1-methyl-1H-pyrazol-3-yl)amino)pyridin-4-yl)-2-oxopyrrolidine-3-carbonitrile | 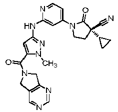 | Free | 470.2 | 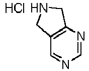 |
| 268 | (3S)-3-cyclopropyl-1-(2-((5-((3,3-difluoropyrrolidin-1-yl)carbonyl)-1-methyl-1H-pyrazol-3-yl)amino)pyridin-4-yl)-2-oxopyrrolidine-3-carbonitrile | 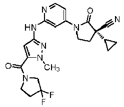 | Free | 456.1 | 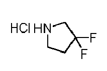 |
| 269 | (3S)-3-cyclopropyl-1-(2-((5-((3-methoxyazetidin-1-yl)carbonyl)-1-methyl-1H-pyrazol-3-yl)amino)pyridin-4-yl)-2-oxopyrrolidine-3-carbonitrile | 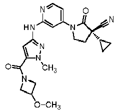 | Free | 436.1 | 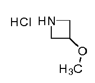 |
| 270 | (3S)-3-cyclopropyl-1-(2-((5-((4-methoxypiperidin-1-yl)carbonyl)-1-methyl-1H-pyrazol-3-yl)amino)pyridin-4-yl)-2-oxopyrrolidine-3-carbonitrile | 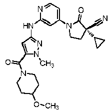 | Free | 464.1 | 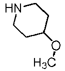 |
| 271 | (3S)-1-(2-((5-(azetidin-1-ylcarbonyl)-1-methyl-1H-pyrazol-3-yl)amino)pyridin-4-yl)-3-cyclopropyl-2-oxopyrrolidine-3-carbonitrile | 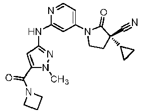 | Free | 406.0 | 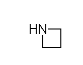 |
| 272 | (3S)-3-cyclopropyl-1-(2-((1-methyl-5-(piperidin-1-ylcarbonyl)-1H-pyrazol-3-yl)amino)pyridin-4-yl)-2-oxopyrrolidine-3-carbonitrile | 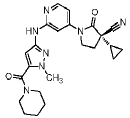 | Free | 434.1 | 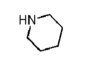 |

**Table 27**

| | | | | | |
|---|---|---|---|---|---|
| 273 | 3-((4-((3S)-3-cyano-3-cyclopropyl-2-oxopyrrolidin-1 yl)pyridin-2-yl)amino)-N,1-dimethyl-N-(tetrahydro-2H-pyran-4-yl)-1H-pyrazole-5-carboxamide | 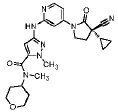 | Free | 464.1 | 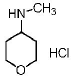 |
| 274 | 3-((4-((3S)-3-cyano-3-cyclopropyl-2-oxopyrrolidin-1 yl)pyridin-2-yl)amino)-1-methyl-N-(3-methyloxetan-3-yl)-1H-pyrazole-5-carboxamide | 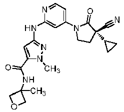 | Free | 436.2 | 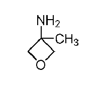 |

### Example 275

### 3-((4-((3S)-3-cyano-3-cyclopropyl-2-oxopyrrolidin-1-yl)pyridin-2-yl)amino)-1-methyl-N-(2,2,2-trifluoroethyl)-1H-pyrazole-5-carboxamide

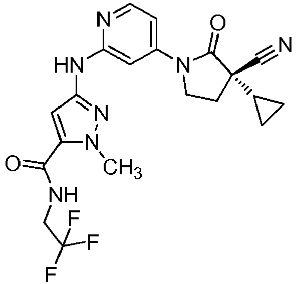

To a solution of 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 (100 mg) in N,N-dimethylformamide (2.0 mL) were added 1-ethyl-3-(3-dimethylaminopropyl)carbodiimide (0.13 mL), 1-hydroxybenzotriazole (100 mg), N,N-dimethyl-4-aminopyridine (1.5 mg), 2,2,2-trifluoroethanamine (0.059 mL) and triethylamine (0.17 mL), and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (53 mg).
MS(ESI+): [M+H]+ 448.2.

### Examples 276 to 278

The title compounds of Examples 276 to 278 shown in Table 28 were obtained using 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 and the reagents corresponding to the compounds of Examples 276 to 278 (these reagents can be produced according to a method known per se), in the same manner as in the method of Example 275 or a method analogous thereto. MS in the tables means actual measured value.

**Table 28**

| Ex. No. | IUPAC Name | Structure | Salt | MS | Corresponding Reagent |
|---|---|---|---|---|---|
| 276 | 3-((4-((3S)-3-cyano-3-cyclopropyl-2-oxopyrrolidin-1 yl)pyridin-2-yl)amino)-N-cyclopropyl-1-methyl-1H-pyrazole-5-carboxamide | 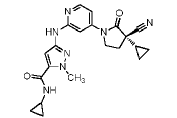 | Free | 406.2 | 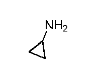 |
| 277 | 3-((4-((3S)-3-cyano-3-cyclopropyl-2-oxopyrrolidin-1 yl)pyridin-2-yl)amino)-N-(2-methoxyethyl)-1-methyl-1H-pyrazole-5-carboxamide | 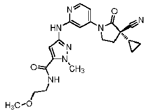 | Free | 424.1 | 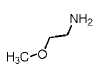 |
| 278 | 3-((4-((3S)-3-cyano-3-cyclopropyl-2-oxopyrrolidin-1 yl)pyridin-2-yl)amino)-N-(cyclopropylmethyl)-1-methyl-1H-pyrazole-5-carboxamide | 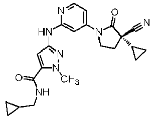 | Free | 420.3 3 | 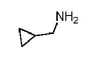 |

### Example 279

### 3-((4-((3S)-3-cyano-3-cyclopropyl-2-oxopyrrolidin-1-yl)pyridin-2-yl)amino)-N-ethyl-1-methyl-1H-pyrazole-5-carboxamide

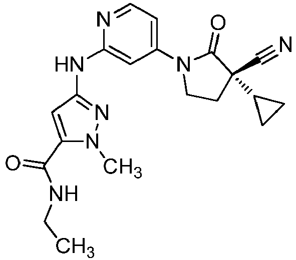

To a solution of 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 (100 mg) in N,N-dimethylformamide (1.0 mL) were added 1-ethyl-3-(3-dimethylaminopropyl)carbodiimide (0.088 mL), 1-hydroxybenzotriazole (76 mg), N,N-dimethyl-4-aminopyridine (1.5 mg), 2M ethylamine tetrahydrofuran solution (0.62 mL) and triethylamine (0.10 mL), and the mixture was stirred overnight at room temperature. To the reaction mixture were added 1.7 M propane phosphonic acid anhydride ethyl acetate solution (0.29 mL) and ethylamine hydrochloride (100 mg), and the mixture was stirred at 50°C for 3 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (59 mg).
MS (ESI+) : [M+H]+ 394.2.

### Example 280

### 6-((4-((3S)-3-cyano-3-cyclopropyl-2-oxopyrrolidin-1-yl)pyridin-2-yl)amino)-N-(2-hydroxy-2-methylpropyl)-N-methylnicotinamide

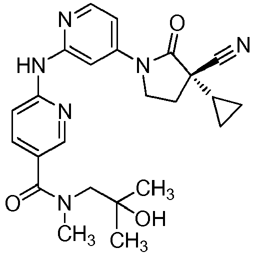

To a solution of the crude 6-((4-((3S)-3-cyano-3-cyclopropyl-2-oxopyrrolidin-1-yl)pyridin-2-yl)amino)nicotinic acid hydrochloride obtained in Step A of Example 72 (100 mg) in acetonitrile (3.0 mL) were added 2-methyl-6-nitrobenzoic anhydride (100 mg), 2-methyl-1-(methylamino)propan-2-ol (39 mg), N,N-dimethyl-4-aminopyridine (37 mg) and triethylamine (0.10 mL), and the mixture was stirred for 3 hr, and then overnight at 50°C. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure. The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (24 mg).
MS(ESI+): [M+H]+ 449.1.

### Example 281

### (3S)-3-cyclopropyl-1-(2-((5-(morpholin-4-ylcarbonyl)pyridin-2-yl)amino)pyridin-4-yl)-2-oxopyrrolidine-3-carbonitrile

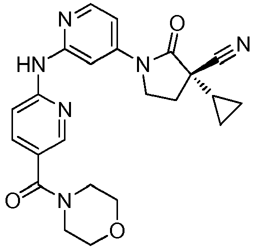

### A) 6-((4-((3S)-3-cyano-3-cyclopropyl-2-oxopyrrolidin-1-yl)pyridin-2-yl)amino)nicotinic acid trihydrochloride

To a solution of tert-butyl 6-((4-((3S)-3-cyano-3-cyclopropyl-2-oxopyrrolidin-1-yl)pyridin-2-yl)amino)nicotinate obtained in Example 71 (1.5 g) in tetrahydrofuran (10 mL) was added 4 M hydrogen chloride ethyl acetate solution (15 mL), the mixture was stirred overnight at 50°C, and the solvent was evaporated under reduced pressure to give the crude title compound (1.4 g).
MS (ESI+) : [M+H] ⁺364.0.

### B) (3S)-3-cyclopropyl-1-(2-((5-(morpholin-4-ylcarbonyl)pyridin-2-yl)amino)pyridin-4-yl)-2-oxopyrrolidine-3-carbonitrile

To a solution of 6-((4-((3S)-3-cyano-3-cyclopropyl-2-oxopyrrolidin-1-yl)pyridin-2-yl)amino)nicotinic acid trihydrochloride obtained in Step A of Example 281 (100 mg) in N,N-dimethylformamide (1.0 mL) were added triethylamine (0.18 mL), 1.7 M propane phosphonic acid anhydride ethyl acetate solution (0.37 mL), N,N-dimethyl-4-aminopyridine (2.6 mg) and morpholine (0.028 mL), and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (69 mg).
¹H NMR (400 MHz, CDCl₃) δ 0.67-0.85 (4H, m), 1.22-1.35 (2H, m), 2.36-2.46 (1H, m), 2.65-2.76 (1H, m), 3.58-3.81 (8H, m), 3.91-4.03 (2H, m), 7.28 (1H, dd, J = 5.9, 2.0 Hz), 7.51-7.55 (1H, m), 7.69-7.76 (2H, m), 8.02 (1H, d, J = 1.7 Hz), 8.26 (1H, d, J = 5.9 Hz), 8.40 (1H, dd, J = 2.2, 0.7 Hz).
MS(ESI+): [M+H]⁺ 433.1.

### Examples 282 to 298

The title compounds of Examples 282 to 298 shown in Table 29-Table 30 were obtained using 6-((4-((3S)-3-cyano-3-cyclopropyl-2-oxopyrrolidin-1-yl)pyridin-2-yl)amino)nicotinic acid trihydrochloride obtained in Step A of Example 281 and the reagents corresponding to the compounds of Examples 282 to 298 (these reagents can be produced according to a method known per se), in the same manner as in the method of Step B of Example 281 or a method analogous thereto. MS in the tables means actual measured value.

**Table 29**

| Ex. No. | IUPAC Name | Structure | Salt | MS | Corresponding Reagent |
|---|---|---|---|---|---|
| 282 | 6-((4-((3S)-3-cyano-3-cyclopropyl-2-oxopyrrolidin-1-yl)pyridin-2-yl)amino)-N-(2-methoxyethyl)-N-methylnicotinamide | 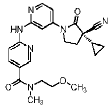 | Free | 435.1 | 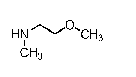 |
| 283 | 6-((4-((3S)-3-cyano-3-cyclopropyl-2-oxopyrrolidin-1-yl)pyridin-2-yl)amino)-N-(2-cyanoethyl)-N-methylnicotinamide | 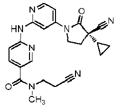 | Free | 430.1 | 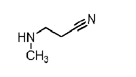 |
| 284 | 6-((4-((3S)-3-cyano-3-cyclopropyl-2-oxopyrrolidin-1 yl)pyridin-2-yl)amino)-N-methyl-N-(oxetan-3-yl)nicotinamide | 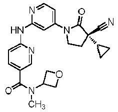 | Free | 433.1 | 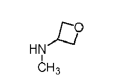 |
| 285 | 6-((4-((3S)-3-cyano-3-cyclopropyl-2-oxopyrrolidin-1 yl)pyridin-2-yl)amino)-N-(tetrahydro-2H-pyran-4-yl)nicotinamide | 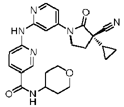 | Free | 447.1 | 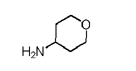 |
| 286 | 6-((4-((3S)-3-cyano-3-cyclopropyl-2-oxopyrrolidin-1 yl)pyridin-2-yl)amino)-N-(2-methoxyethyl)nicotinamide | 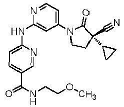 | Free | 421.1 | 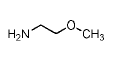 |
| 287 | 6-((4-((3S)-3-cyano-3-cyclopropyl-2-oxopyrrolidin-1 yl)pyridin-2-yl)amino)-N-(2-cyanoethyl)nicotinamide | 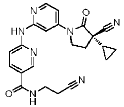 | Free | 416.0 | 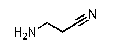 |
| 288 | 6-((4-((3S)-3-cyano-3-cyclopropyl-2-oxopyrrolidin-1 yl)pyridin-2-yl)amino)-N-cyclopropyl-N-methylnicotinamide | 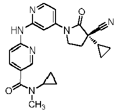 | Free | 417.0 | 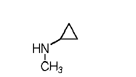 |
| 289 | 6-((4-((3S)-3-cyano-3-cyclopropyl-2-oxopyrrolidin-1 yl)pyridin-2-yl)amino)-N-cyclopropylnicotinamide | 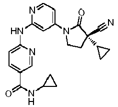 | Free | 403.2 | 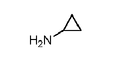 |

**Table 30**

| | | | | | |
|---|---|---|---|---|---|
| 290 | (3S)-3-cyclopropyl-2-oxo-1-(2-((5-((4-(trifluoromethyl)piperidin-1-yl)carbonyl)pyridin-2-yl)amino)pyridin-4-yl)pyrrolidine-3-carbonitrile | 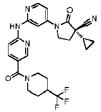 | Free | 499.3 | 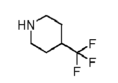 |
| 291 | (3S)-3-cyclopropyl-1-(2-((5-((3,3-difluoroazetidin-1-yl)carbonyl)pyridin-2-yl)amino)pyridin-4-yl)-2-oxopyrrolidine-3-carbonitrile | 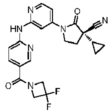 | Free | 439.1 | 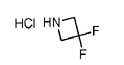 |
| 292 | (3S)-1-(2-((5-(azetidin-1-ylcarbonyl)pyridin-2-yl)amino)pyridin-4-yl)-3-cyclopropyl-2-oxopyrrolidine-3-carbonitrile | 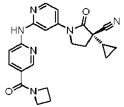 | Free | 403.2 | 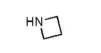 |
| 293 | (3S)-3-cyclopropyl-1-(2-((5-((4-methoxypiperidin-1-yl)carbonyl)pyridin-2-yl)amino)pyridin-4-yl)-2-oxopyrrolidine-3-carbonitrile | 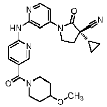 | Free | 461.2 | 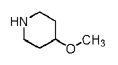 |
| 294 | (3S)-3-cyclopropyl-1-(2-((5-((3-methoxyazetidin-1-yl)carbonyl)pyridin-2-yl)amino)pyridin-4-yl)-2-oxopyrrolidine-3-carbonitrile | 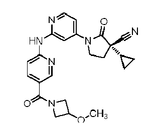 | Free | 433.2 | 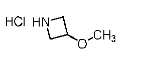 |
| 295 | (3S)-3-cyclopropyl-1-(2-((5-(6-oxa-3-azabicyclo[3.1.1]hept-3-ylcarbonyl)pyridin-2-yl)amino)pyridin-4-yl)-2-oxopyrrolidine-3-carbonitrile | 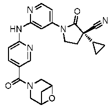 | Free | 445.1 | 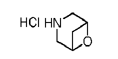 |
| 296 | 6-((4-((3S)-3-cyano-3-cyclopropyl-2-oxopyrrolidin-1 yl)pyridin-2-yl)amino)-N-methyl-N-(tetrahydro-2H-pyran-4-yl)nicotinamide | 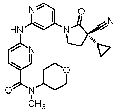 | Free | 461.2 | 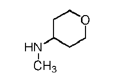 |
| 297 | (3S)-3-cyclopropyl-1-(2-((5-(((3S)-3-methoxypyrrolidin-1-yl)carbonyl)pyridin-2-yl)amino)pyridin-4-yl)-2-oxopyrrolidine-3-carbonitrile | 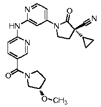 | Free | 447.1 | 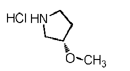 |
| 298 | (3S)-3-cyclopropyl-1-(2-((5-(((3R)-3-methoxypyrrolidin-1-yl)carbonyl)pyridin-2-yl)amino)pyridin-4-yl)-2-oxopyrrolidine-3-carbonitrile | 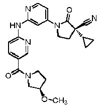 | Free | 447.1 | 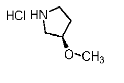 |

### Example 299

### (3S)-3-cyclopropyl-1-(2-((5-(1,4-oxazepan-4-ylcarbonyl)pyridin-2-yl)amino)pyridin-4-yl)-2-oxopyrrolidine-3-carbonitrile

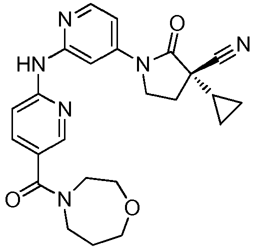

To a solution of 6-((4-((3S)-3-cyano-3-cyclopropyl-2-oxopyrrolidin-1-yl)pyridin-2-yl)amino)nicotinic acid trihydrochloride obtained in Step A of Example 281 (100 mg) in N,N-dimethylacetamide (5.0 mL) were added triethylamine (0.15 mL), 0-(7-azabenzotriazol-1-yl)-N,N,N',N'-tetramethyluronium hexafluorophosphorate (160 mg) and 1,4-oxazepane hydrochloride (44 mg), and the mixture was stirred at room temperature for 3 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate), and recrystallized (diisopropyl ether) to give the title compound (17 mg).
¹H NMR (400 MHz, DMSO-d₆) δ 0.47-0.74 (4H, m), 1.46-1.58 (1H, m), 1.75-1.91 (2H, m), 2.30-2.44 (2H, m), 2.64-2.74 (1H, m), 3.49-3.80 (7H, m), 3.89-4.01 (2H, m), 7.29-7.37 (1H, m), 7.70-7.78 (1H, m), 7.78-7.85 (1H, m), 8.00-8.07 (1H, m), 8.18-8.26 (1H, m), 8.30 (1H, s), 10.01 (1H, s).
MS (ESI+) : [M+H]⁺ 447.3.

### Example 300

### (3S)-3-cyclopropyl-1-(2-((5-(3-oxa-8-azabicyclo[3.2.1]oct-8-ylcarbonyl)pyridin-2-yl)amino)pyridin-4-yl)-2-oxopyrrolidine-3-carbonitrile

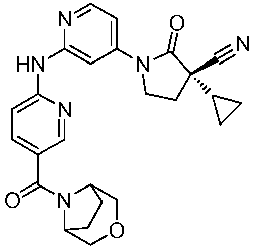

To a solution of 6-((4-((3S)-3-cyano-3-cyclopropyl-2-oxopyrrolidin-1-yl)pyridin-2-yl)amino)nicotinic acid trihydrochloride obtained in Step A of Example 281 (400 mg) in N,N-dimethylformamide (4.0 mL) were added 3-oxa-8-azabicyclo[3.2.1]octane hydrochloride (250 mg), N,N-diisopropylethylamine (0.39 mL) and O-(7-azabenzotriazol-1-yl)-N,N,N',N'-tetramethyluronium hexafluorophosphorate (480 mg), and the mixture was stirred at room temperature for 3 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (380 mg).
¹H NMR (400 MHz, DMSO-d₆) δ 0.41-0.74 (4H, m), 1.52 (1H, tt, J = 8.2, 5.0 Hz), 1.88 (4H, brs), 2.24-2.46 (1H, m), 2.59-2.74 (1H, m), 3.60 (2H, brs), 3.65-3.74 (2H, m), 3.86-3.99 (2H, m), 4.05-4.64 (2H, m), 7.35 (1H, dd, J = 5.9, 2.0 Hz), 7.82 (2H, d, J = 1.5 Hz), 8.04 (1H, d, J = 1.7 Hz), 8.24 (1H, d, J = 5.9 Hz), 8.40 (1H, t, J = 1.5 Hz), 10.07 (1H, s).
MS (ESI+) : [M+H]⁺ 459.2.

### Example 301

### (3S)-3-cyclopropyl-1-(2-((5-(((3S or 3R)-3-hydroxy-3-methylpiperidin-1-yl)carbonyl)pyridin-2-yl)amino)pyridin-4-yl)-2-oxopyrrolidine-3-carbonitrile

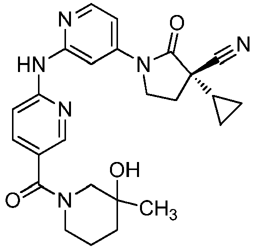

### A) (6-bromopyridin-3-yl)(3-hydroxy-3-methylpiperidin-1-yl)methanone

To a solution of 6-bromonicotinic acid (400 mg) in ethyl acetate (8.0 mL) were added N,N-diisopropylethylamine (1.0 mL), 1.7 M propane phosphonic acid anhydride ethyl acetate solution (1.7 mL) and 3-methylpiperidin-3-ol (230 mg), and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (280 mg).
MS(ESI+): [M+H]⁺ 298.8.

### B) optically active (6-bromopyridin-3-yl)(3-hydroxy-3-methylpiperidin-1-yl)methanone (shorter retention time)

(6-Bromopyridin-3-yl)(3-hydroxy-3-methylpiperidin-1-yl)methanone obtained in Step A of Example 301 (420 mg) was resolved by HPLC (column: CHIRALPAK AD, 50 mmID×500 mmL, manufactured by Daicel Chemical Industries, mobile phase: hexane/ethanol = 300/700) to give the title compound (190 mg) having a shorter retention time.
>99.9% ee (HPLC (column: CHIRALPAK AD, 4.6 mmID×250 mmL, manufactured by Daicel Chemical Industries, mobile phase: hexane/ethanol = 300/700, flow rate: 0.5 mL/min, retention time: 16.2 min))

### C) optically active (6-bromopyridin-3-yl)(3-hydroxy-3-methylpiperidin-1-yl)methanone (longer retention time)

(6-Bromopyridin-3-yl)(3-hydroxy-3-methylpiperidin-1-yl)methanone obtained in Step A of Example 301 (420 mg) was resolved by HPLC (column: CHIRALPAK AD, 50 mmID×500 mmL, manufactured by Daicel Chemical Industries, mobile phase: hexane/ethanol = 300/700) to give the title compound (180 mg) having a longer retention time.
>99.9% ee (HPLC (column: CHIRALPAK AD, 4.6 mmID×250 mmL, manufactured by Daicel Chemical Industries, mobile phase: hexane/ethanol = 300/700, flow rate: 0.5 mL/min, retention time: 20.1 min))

### D) (3S)-3-cyclopropyl-1-(2-((5-(((3S or 3R)-3-hydroxy-3-methylpiperidin-1-yl)carbonyl)pyridin-2-yl)amino)pyridin-4-yl)-2-oxopyrrolidine-3-carbonitrile

To a solution of (3S)-1-(2-aminopyridin-4-yl)-3-cyclopropyl-2-oxopyrrolidine-3-carbonitrile obtained in Example 2 (100 mg) in 2-methyl-2-butanol (2.0 mL) were added the optically active (6-bromopyridin-3-yl)(3-hydroxy-3-methylpiperidin-1-yl)methanone (shorter retention time) obtained in Step B of Example 301 (120 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was recrystallized (methanol/tetrahydrofuran) to give the title compound (65 mg).
MS(ESI+): [M+H]⁺ 461.1.

### Example 302

### (3S)-3-cyclopropyl-1-(2-((5-(((3S or 3R)-3-hydroxy-3-methylpiperidin-1-yl)carbonyl)pyridin-2-yl)amino)pyridin-4-yl)-2-oxopyrrolidine-3-carbonitrile

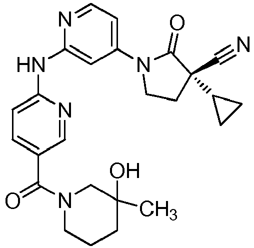

The title compound (89 mg) was obtained using (3S)-1-(2-aminopyridin-4-yl)-3-cyclopropyl-2-oxopyrrolidine-3-carbonitrile obtained in Example 2 (100 mg) and optically active (6-bromopyridin-3-yl)(3-hydroxy-3-methylpiperidin-1-yl)methanone obtained in Step C of Example 301 (longer retention time) (99 mg), in the same manner as in the method of Step D of Example 301.
MS(ESI+): [M+H]⁺ 461.1.

### Examples 304 to 309

The title compounds of Examples 304 to 309 shown in Table 31 were obtained using (3S)-1-(2-aminopyridin-4-yl)-3-cyclopropyl-2-oxopyrrolidine-3-carbonitrile obtained in Example 2 and the reagents corresponding to the compounds of Examples 304 to 309 (these reagents can be produced according to a method known per se), in the same manner as in the method of Step D of Example 301 or a method analogous thereto. MS in the tables means actual measured value.

**Table 31**

| Ex. No. | IUPAC Name | Structure | Salt | MS | Corresponding Reagent |
|---|---|---|---|---|---|
| 304 | (3S)-3-cyclopropyl-1-(2-((6-methoxypyridin-2-yl)amino)pyridin-4-yl)-2-oxopyrrolidine-3-carbonitrile | 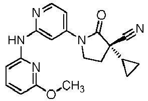 | Free | 350.0 | 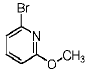 |
| 305 | (3S)-1-(2-((5-(4-acetylpiperazin-1-yl)pyridin-2-yl)amino)pyridin-4-yl)-3-cyclopropyl-2-oxopyrrolidine-3-carbonitrile hydrochloride | 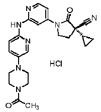 | HCl | 446.2 | 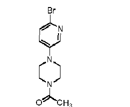 |
| 306 | 6-((4-((3S)-3-cyano-3-cyclopropyl-2-oxopyrrolidin-1-yl)pyridin-2-yl)amino)-N-(2-hydroxy-2-methylpropyl)nicotinamide | 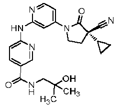 | Free | 435.1 | 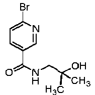 |
| 307 | 6-((4-((3S)-3-cyano-3-cyclopropyl-2-oxopyrrolidin-1-yl)pyridin-2-yl)amino)pyridine-2-carboxamide | 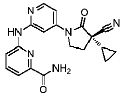 | Free | 363.2 | 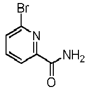 |
| 308 | 6-((4-((3S)-3-cyano-3-cyclopropyl-2-oxopyrrolidin-1-yl)pyridin-2-yl)amino)-N-methylpyridine-2-carboxamide | 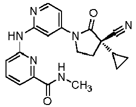 | Free | 377.2 | 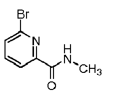 |
| 309 | 6-((4-((3S)-3-cyano-3-cyclopropyl-2-oxopyrrolidin-1-yl)pyridin-2-yl)amino)-N,N-dimethylpyridine-2-carboxamide | 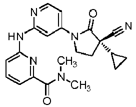 | Free | 391.2 | 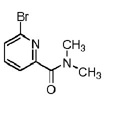 |

### Example 310

### (3S)-3-cyclopropyl-1-(2-((5-((4-hydroxy-4-methylpiperidin-1-yl)methyl)pyridin-2-yl)amino)pyridin-4-yl)-2-oxopyrrolidine-3-carbonitrile

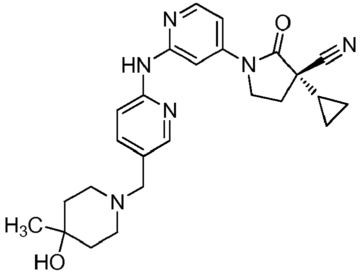

To a solution of (3S)-1-(2-aminopyridin-4-yl)-3-cyclopropyl-2-oxopyrrolidine-3-carbonitrile obtained in Example 2 (100 mg) in 2-methyl-2-butanol (4.0 mL) were added 1-((6-bromopyridin-3-yl)methyl)-4-methylpiperidin-4-ol (120 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (55 mg).
MS(ESI+): [M+H] + 447.1.

### Example 311

### (3S)-3-cyclopropyl-1-(2-((5-((4-hydroxy-4-(trifluoromethyl)piperidin-1-yl)carbonyl)pyridin-2-yl)amino)pyridin-4-yl)-2-oxopyrrolidine-3-carbonitrile

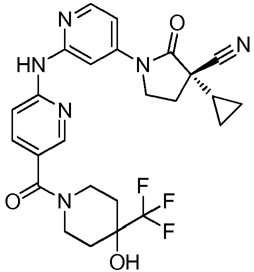

To a solution of (3S)-1-(2-aminopyridin-4-yl)-3-cyclopropyl-2-oxopyrrolidine-3-carbonitrile obtained in Example 2 (100 mg) in 2-methyl-2-butanol (2.0 mL) were added (6-bromopyridin-3-yl)(4-hydroxy-4-(trifluoromethyl)piperidin-1-yl)methanone (160 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred at 100°C for 16 hr. To the reaction mixture were added 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg), and the mixture was stirred at 100°C for 6 hr. To the reaction mixture was added saturated aqueous sodium hydrogencarbonate solution, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate). The obtained crude product was purified by silica gel column chromatography (diol, hexane/ethyl acetate) to give the title compound (66 mg).
MS (ESI+) : [M+H]⁺515.2.

### Example 312

### 2-(3-((4-((3S)-3-cyano-3-cyclopropyl-2-oxopyrrolidin-1-yl)pyridin-2-yl)amino)-1-methyl-1H-pyrazol-5-yl)-2-methylpropanamide

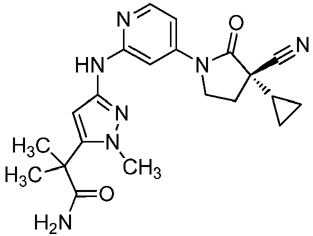

To a solution of (3S)-1-(2-bromopyridin-4-yl)-3-cyclopropyl-2-oxopyrrolidine-3-carbonitrile obtained in Step E of Example 1 (120 mg) in 2-methyl-2-butanol (2.0 mL) were added 2-(3-amino-1-methyl-1H-pyrazol-5-yl)-2-methylpropanamide (86 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred at 100°C for 6 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate). The obtained crude product was purified by silica gel column chromatography (diol, ethyl acetate/methanol) to give the title compound (29 mg).
MS(ESI+): [M+H]⁺ 408.1.

### Example 313

### 2-(3-((4-((3S)-3-cyano-3-cyclopropyl-2-oxopyrrolidin-1-yl)pyridin-2-yl)amino)-1-methyl-1H-pyrazol-5-yl)-N-(2,2,2-trifluoroethyl)acetamide

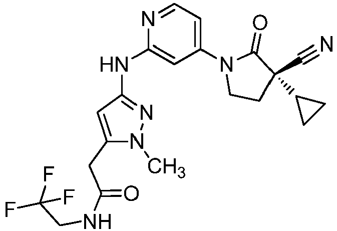

To a solution of (3S)-1-(2-bromopyridin-4-yl)-3-cyclopropyl-2-oxopyrrolidine-3-carbonitrile obtained in Step E of Example 1 (100 mg) in 2-methyl-2-butanol (2.0 mL) were added 2-(3-amino-1-methyl-1H-pyrazol-5-yl)-N-(2,2,2-trifluoroethyl)acetamide (110 mg), potassium carbonate (90 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred at 100°C for 6 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate). The obtained crude product was purified by silica gel column chromatography (diol, hexane/ethyl acetate) to give the title compound (48 mg).
MS (ESI+) : [M+H]⁺ 462.1.

### Example 314

### 1-(3-((4-((3S)-3-cyano-3-cyclopropyl-2-oxopyrrolidin-1-yl)pyridin-2-yl)amino)-1-methyl-1H-pyrazol-5-yl)cyclopropanecarboxamide

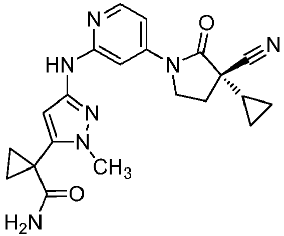

To a solution of (3S)-1-(2-bromopyridin-4-yl)-3-cyclopropyl-2-oxopyrrolidine-3-carbonitrile obtained in Step E of Example 1 (100 mg) in 2-methyl-2-butanol (2.0 mL) were added 1-(3-amino-1-methyl-1H-pyrazol-5-yl)cyclopropanecarboxamide (130 mg), potassium carbonate (90 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred at 100°C for 6 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate and ethyl acetate/methanol). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (7.4 mg).
MS (ESI+) : [M+H]⁺ 406.0.

### Example 315

### 1-(3-((4-((3S)-3-cyano-3-cyclopropyl-2-oxopyrrolidin-1-yl)pyridin-2-yl)amino)-1-methyl-1H-pyrazol-5-yl)cyclopentanecarboxamide

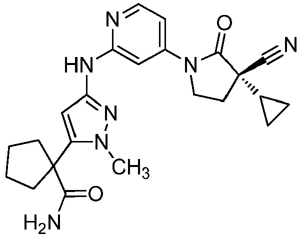

To a solution of (3S)-1-(2-bromopyridin-4-yl)-3-cyclopropyl-2-oxopyrrolidine-3-carbonitrile obtained in Step E of Example 1 (120 mg) in 2-methyl-2-butanol (2.0 mL) were added 1-(3-amino-1-methyl-1H-pyrazol-5-yl)cyclopentanecarboxamide (200 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred at 100°C for 6 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate and ethyl acetate/methanol). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (67 mg).
MS (ESI+) : [M+H]⁺ + 34.1.

### Example 316

### 4-(6-((4-((3S)-3-cyano-3-cyclopropyl-2-oxopyrrolidin-1-yl)pyridin-2-yl)amino)pyridin-3-yl)tetrahydro-2H-pyran-4-carboxamide

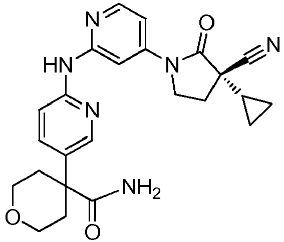

To a solution of (3S)-1-(2-aminopyridin-4-yl)-3-cyclopropyl-2-oxopyrrolidine-3-carbonitrile obtained in Example 2 (130 mg) in 2-methyl-2-butanol (3.0 mL) were added 4-(6-chloropyridin-3-yl)tetrahydro-2H-pyran-4-carboxamide (130 mg), potassium carbonate (220 mg), 2-(dicyclohexylphosphino)-3,6-dimethoxy-2',4',6'-triisopropyl-1,1'-biphenyl (29 mg) and chloro[2-(dicyclohexylphosphino)-3,6-dimethoxy-2',4',6'-triisopropyl-1,1'-biphenyl][2-(2-aminoethyl)phenyl]palladium(II) (20 mg) under argon atmosphere, and the mixture was stirred at 100°C for 40 hr. To the reaction mixture were added saturated aqueous sodium hydrogencarbonate solution and ethyl acetate, and the insoluble substance was removed through Celite. The filtrate was extracted with ethyl acetate, the obtained organic layer was washed with water and saturated brine, and dried over anhydrous magnesium sulfate, and the solvent was evaporated under reduced pressure. The residue was purified by silica gel column chromatography (NH, hexane/ethyl acetate). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure to give the title compound (5.0 mg).
MS (ESI+) : [M+H]⁺447.4.

### Example 317

### (3S)-3-cyclopropyl-1-(2-((4-methyl-5-(3-oxa-8-azabicyclo[3.2.1]oct-8-ylcarbonyl)pyridin-2-yl)amino)pyridin-4-yl)-2-oxopyrrolidine-3-carbonitrile

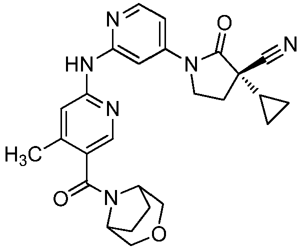

To a solution of (3S)-1-(2-aminopyridin-4-yl)-3-cyclopropyl-2-oxopyrrolidine-3-carbonitrile obtained in Example 2 (100 mg) in 2-methyl-2-propanol (2.0 mL) were added (6-chloro-4-methylpyridin-3-yl)(3-oxa-8-azabicyclo[3.2.1]oct-8-yl)methanone (130 mg), potassium carbonate (110 mg), 2-(dicyclohexylphosphino)-3,6-dimethoxy-2',4',6'-triisopropyl-1,1'-biphenyl (11 mg) and chloro[2-(dicyclohexylphosphino)-3,6-dimethoxy-2',4',6'-triisopropyl-1,1'-biphenyl][2-(2-aminoethyl)phenyl]palladium(II) (16 mg) under argon atmosphere, and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate), and recrystallized (ethyl acetate) to give the title compound (12 mg).
MS (ESI+) : [M+H]⁺473.2.

### Example 318

### 6-((4-((3S)-3-cyano-3-cyclopropyl-2-oxopyrrolidin-1-yl)pyridin-2-yl)amino)-N,4-dimethyl-N-((3S)-tetrahydrofuran-3-yl)nicotinamide

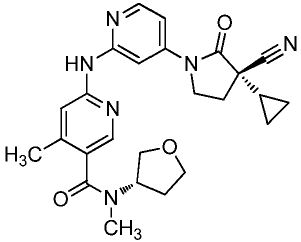

The title compound (64 mg) was obtained using (3S)-1-(2-aminopyridin-4-yl)-3-cyclopropyl-2-oxopyrrolidine-3-carbonitrile obtained in Example 2 (90 mg) and 6-chloro-N,4-dimethyl-N-((3S)-tetrahydrofuran-3-yl)nicotinamide (110 mg), in the same manner as in the method of Example 317. MS (ESI+) : [M+H]⁺461.2.

### Example 319

### 6-((4-((3S)-3-cyano-3-cyclopropyl-2-oxopyrrolidin-1-yl)pyridin-2-yl)amino)-N,4-dimethyl-N-((3R)-tetrahydrofuran-3-yl)nicotinamide

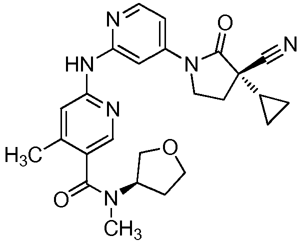

The title compound (78 mg) was obtained using (3S)-1-(2-aminopyridin-4-yl)-3-cyclopropyl-2-oxopyrrolidine-3-carbonitrile obtained in Example 2 (90 mg) and 6-chloro-N,4-dimethyl-N-((3R)-tetrahydrofuran-3-yl)nicotinamide (110 mg), in the same manner as in the method of Example 317.
MS (ESI+) : [M+H]⁺461.2.

### Example 320

### 6-((4-((3S)-3-cyano-3-cyclopropyl-2-oxopyrrolidin-1-yl)pyridin-2-yl)amino)-4-methylnicotinic acid dihydrochloride

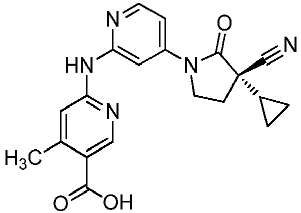

### A) tert-butyl 6-chloro-4-methylnicotinate

To a solution of 6-chloro-4-methylnicotinic acid (200 mg) in toluene (5.0 mL) was added N,N-dimethylformamide di-tert-butylacetal (2.8 mL) under argon atmosphere, and the mixture was stirred overnight at 100°C. The solvent was evaporated under reduced pressure, and the residue was purified by column chromatography (hexane/ethyl acetate) to give the title compound (110 mg).
MS (ESI+) : [M+H]⁺227.8.

### B) tert-butyl 6-((4-((3S)-3-cyano-3-cyclopropyl-2-oxopyrrolidin-1-yl)pyridin-2-yl)amino)-4-methylnicotinate

The title compound (120 mg) was obtained using (3S)-1-(2-aminopyridin-4-yl)-3-cyclopropyl-2-oxopyrrolidine-3-carbonitrile obtained in Example 2 (90 mg) and tert-butyl 6-chloro-4-methylnicotinate obtained in Step A of Example 320 (100 mg), in the same manner as in the method of Example 317. MS (ESI+) : [M+H]⁺434.2.

### C) 6-((4-((3S)-3-cyano-3-cyclopropyl-2-oxopyrrolidin-1-yl)pyridin-2-yl)amino)-4-methylnicotinic acid dihydrochloride

The title compound (100 mg) was obtained using tert-butyl 6-((4-((3S)-3-cyano-3-cyclopropyl-2-oxopyrrolidin-1-yl)pyridin-2-yl)amino)-4-methylnicotinate obtained in Step B of Example 320 (120 mg), in the same manner as in the method of Step A of Example 5.
MS (ESI+) : [M+H]⁺434.2.

### Example 321

### 6-((4-((3S)-3-cyano-3-cyclopropyl-2-oxopyrrolidin-1-yl)pyridin-2-yl)amino)-4-methylnicotinamide

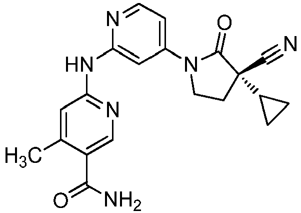

The title compound (7.0 mg) was obtained using 6-((4-((3S)-3-cyano-3-cyclopropyl-2-oxopyrrolidin-1-yl)pyridin-2-yl)amino)-4-methylnicotinic acid dihydrochloride obtained in Step C of Example 320 (100 mg) and ammonium chloride (30 mg), in the same manner as in the method of Example 246.
MS (ESI+) : [M+H]⁺ 377.0.

### Example 322

### 3-((4-((3S)-3-cyano-3-cyclopropyl-2-oxopyrrolidin-1-yl)pyridin-2-yl)amino)-N,1-dimethyl-N-((3S)-tetrahydrofuran-3-yl)-1H-pyrazole-5-carboxamide

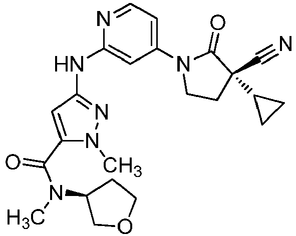

The title compound (26 mg) was obtained using 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 (50 mg) and (3S)-N-methyltetrahydrofuran-3-amine hydrochloride (51 mg), in the same manner as in the method of Example 246.
MS (ESI+) : [M+H]⁺450.1.

### Example 323

### 3-((4-((3S)-3-cyano-3-cyclopropyl-2-oxopyrrolidin-1-yl)pyridin-2-yl)amino)-N,1-dimethyl-N-((3R)-tetrahydrofuran-3-yl)-1H-pyrazole-5-carboxamide

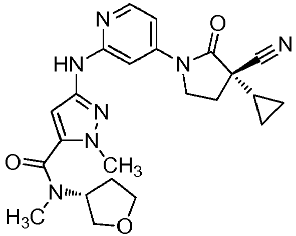

The title compound (32 mg) was obtained using 3-((4-((3S)-3-cyano-3-cyclopropyl-2-oxopyrrolidin-1-yl)pyridin-2-yl)amino)-1-methyl-1H-pyrazole-5-carboxylic acid hydrochloride obtained in Step A of Example 5 (50 mg) and (3R)-N-methyltetrahydrofuran-3-amine hydrochloride (51 mg), in the same manner as in the method of Example 246.
MS (ESI+) : [M+H]⁺450.1.

### Example 324

### (3S)-3-cyclopropyl-1-(2-((5-((3S)-3-hydroxypiperidin-1-yl)pyridin-2-yl)amino)pyridin-4-yl)-2-oxopyrrolidine-3-carbonitrile

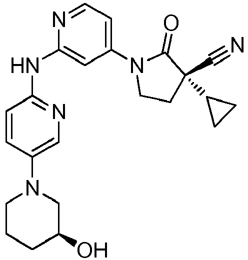

To a solution of (3S)-1-(2-bromopyridin-4-yl)-3-cyclopropyl-2-oxopyrrolidine-3-carbonitrile obtained in Step E of Example 1 (100 mg) in 2-methyl-2-propanol (2.0 mL) were added (3S)-1-(6-aminopyridin-3-yl)piperidin-3-ol (95 mg), potassium carbonate (90 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 90°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (diol, hexane/ethyl acetate). The obtained crude product was purified by HPLC (C18, mobile phase: water/acetonitrile (containing 0.1% TFA)), to the obtained fraction was added saturated aqueous sodium hydrogencarbonate solution, and the mixture was extracted with ethyl acetate. The obtained organic layer was washed with saturated brine, and dried over anhydrous magnesium sulfate, and the solvent was evaporated under reduced pressure. The obtained residue was recrystallized (ethyl acetate/hexane) to give the title compound (62 mg). MS (ESI+) : [M+H]⁺419.2.

### Example 325

### (3S)-3-cyclopropyl-1-(2-((5-(8-oxa-3-azabicyclo[3.2.1]oct-3-yl)pyridin-2-yl)amino)pyridin-4-yl)-2-oxopyrrolidine-3-carbonitrile

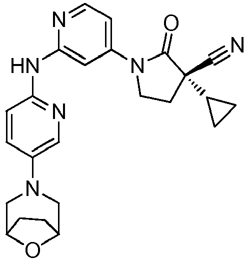

To a solution of (3S)-1-(2-bromopyridin-4-yl)-3-cyclopropyl-2-oxopyrrolidine-3-carbonitrile obtained in Step E of Example 1 (100 mg) in 2-methyl-2-propanol (2.0 mL) were added 5-(3-oxa-8-azabicyclo[3.2.1]oct-8-yl)pyridin-2-amine (74 mg), potassium carbonate (90 mg), 2-(dicyclohexylphosphino)-3,6-dimethoxy-2',4',6'-triisopropyl-1,1'-biphenyl (9.0 mg) and chloro[2-(dicyclohexylphosphino)-3,6-dimethoxy-2',4',6'-triisopropyl-1,1'-biphenyl][2-(2-aminoethyl)phenyl]palladium(II) (13 mg) under argon atmosphere, and the mixture was stirred overnight at 90°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate), and recrystallized (ethyl acetate/hexane) to give the title compound (26 mg) .
MS (ESI+) : [M+H]⁺431.1.

### Example 326

### (3S)-3-cyclopropyl-1-(2-((5-(6-oxa-3-azabicyclo[3.1.1]hept-3-yl)pyridin-2-yl)amino)pyridin-4-yl)-2-oxopyrrolidine-3-carbonitrile

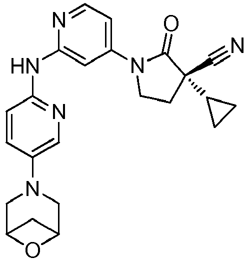

The title compound (45 mg) was obtained using (3S)-1-(2-bromopyridin-4-yl)-3-cyclopropyl-2-oxopyrrolidine-3-carbonitrile obtained in Step E of Example 1 (190 mg) and 5-(6-oxa-3-azabicyclo[3.1.1]hept-3-yl)pyridin-2-amine (130 mg), in the same manner as in the method of Example 325.
MS (ESI+) : [M+H]⁺417.1.

### Example 327

### (3S)-3-cyclopropyl-1-(2-((5-(3-oxa-8-azabicyclo[3.2.1]oct-8-yl)pyridin-2-yl)amino)pyridin-4-yl)-2-oxopyrrolidine-3-carbonitrile

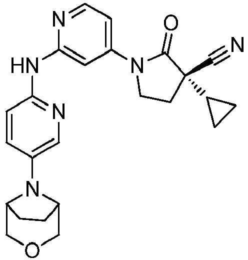

The title compound (44 mg) was obtained using (3S)-1-(2-bromopyridin-4-yl)-3-cyclopropyl-2-oxopyrrolidine-3-carbonitrile obtained in Step E of Example 1 (110 mg) and 5-(8-oxa-3-azabicyclo[3.2.1]oct-3-yl)pyridin-2-amine (83 mg), in the same manner as in the method of Example 325.
MS (ESI+) : [M+H]⁺ 431.1.

### Reference Example 31

### 3-amino-1-(difluoromethyl)-N-methyl-1H-pyrazole-5-carboxamide

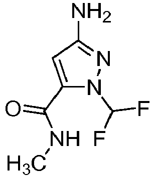

### A) ethyl 3-nitro-1H-pyrazole-5-carboxylate

To a solution of 3-nitro-1H-pyrazole-5-carboxylic acid (730 mg) in ethanol (15 mL) was added thionyl chloride (0.51 mL), and the mixture was stirred at 80°C for 3 hr. The reaction mixture was evaporated under reduced pressure to give the crude title compound (700 mg).
MS (ESI-), found: 184.0.

### B) ethyl 1-(difluoromethyl)-3-nitro-1H-pyrazole-5-carboxylate

To a solution of ethyl 3-nitro-1H-pyrazole-5-carboxylate obtained in Step A of Reference Example 31 (500 mg) in N,N-dimethylformamide (5.0 mL) were added cesium carbonate (2.6 g) and sodium chlorodifluoroacetate (1.2 g), and the mixture was stirred overnight at 80°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (hexane/ethyl acetate) to give the title compound (82 mg).
¹H NMR (400 MHz, DMSO-d₆) δ1.35 (3H, t, J = 7.1 Hz), 4.40 (2H, q, J = 7.1Hz), 7.75 (1H, s), 8.28 (1H, t, J = 60.0 Hz).

### C) 1-(difluoromethyl)-3-nitro-1H-pyrazole-5-carboxylic acid

To a solution of ethyl 1-(difluoromethyl)-3-nitro-1H-pyrazole-5-carboxylate obtained in Step B of Reference Example 31 (530 mg) in ethanol (8.0 mL) was added 1M aqueous sodium hydroxide solution (4.5 mL), and the mixture was stirred overnight at room temperature. The reaction mixture was cooled to 0°C, neutralized with 1M hydrochloric acid, and extracted with ethyl acetate. The obtained organic layer was washed with water and saturated brine, and dried over anhydrous magnesium sulfate, and the solvent was evaporated under reduced pressure to give the crude title compound (500 mg).
MS (ESI-), found: 206.0.

### D) 1-(difluoromethyl)-N-methyl-3-nitro-1H-pyrazole-5-carboxamide

To a solution of 1-(difluoromethyl)-3-nitro-1H-pyrazole-5-carboxylic acid obtained in Step C of Reference Example 31 (200 mg) in ethyl acetate (1.0 mL) were added triethylamine (0.40 mL), 1.7 M propane phosphonic acid anhydride ethyl acetate solution (1.1 mL) and methylamine hydrochloride (65 mg), and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (hexane/ethyl acetate) to give the title compound (130 mg).
1H NMR (400 MHz, DMSO-d₆) δ2.82 (3H, s), 7.74 (1H, s), 8.49 (1H, t, J = 60.0 Hz), 9.10 (1H, brs).

### E) 3-amino-1-(difluoromethyl)-N-methyl-1H-pyrazole-5-carboxamide

To a solution of 1-(difluoromethyl)-N-methyl-3-nitro-1H-pyrazole-5-carboxamide obtained in Step D of Reference Example 31 (130 mg) in ethanol (6.0 mL) was added 10% palladium-carbon (64 mg), and the mixture was stirred overnight at room temperature under hydrogen atmosphere (normal pressure). The palladium on carbon was removed through Celite, and the solvent was evaporated under reduced pressure to give the title compound (120 mg).
MS (ESI-), found: 189.1.

### Reference Example 32

### 3-amino-1-(difluoromethyl)-N-(oxetan-3-yl)-1H-pyrazole-5-carboxamide

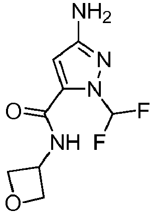

### A) 1-(difluoromethyl)-3-nitro-N-(oxetan-3-yl)-1H-pyrazole-5-carboxamide

The title compound (30 mg) was obtained using 1-(difluoromethyl)-3-nitro-1H-pyrazole-5-carboxylic acid obtained in Step C of Reference Example 31 (100 mg) and oxetan-3-amine (53 mg), in the same manner as in the method of Step D of Reference Example 31.
MS (ESI-), found: 261.0.

### B) 3-amino-1-(difluoromethyl)-N-(oxetan-3-yl)-1H-pyrazole-5-carboxamide

The title compound (30 mg) was obtained using 1-(difluoromethyl)-3-nitro-N-(oxetan-3-yl)-1H-pyrazole-5-carboxamide obtained in Step A of Reference Example 32 (30 mg), in the same manner as in the method of Step E of Reference Example 31.
MS (ESI-), found: 231.1.

### Reference Example 33

### tert-butyl 3-amino-1-(difluoromethyl)-1H-pyrazole-5-carboxylate

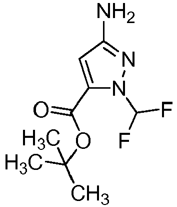

### A) tert-butyl 1-(difluoromethyl)-3-nitro-1H-pyrazole-5-carboxylate

To a solution of 1-(difluoromethyl)-3-nitro-1H-pyrazole-5-carboxylic acid obtained in Step C of Reference Example 31 (400 mg) in toluene (4.0 mL) was added N,N-dimethylformamide di-tert-butylacetal (4.6 mL) under argon atmosphere, and the mixture was stirred overnight at 100°C. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (hexane/ethyl acetate) to give the title compound (280 mg).
¹H NMR (400 MHz, DMSO-d₆) δ 1.57 (9H, s), 7.68 (1H, s), 8.25 (1H, t, J = 60.0 Hz).

### B) tert-butyl 3-amino-1-(difluoromethyl)-1H-pyrazole-5-carboxylate

The title compound (240 mg) was obtained using tert-butyl 1-(difluoromethyl)-3-nitro-1H-pyrazole-5-carboxylate obtained in Step A of Reference Example 33 (280 mg), in the same manner as in the method of Step E of Reference Example 31.
¹H NMR (400 MHz, DMSO-d₆) δ1.52 (9H, s), 5.46 (2H, s), 6.19 (1H, s), 7.86 (1H, t, J = 60.0 Hz).

### Reference Example 34

### 2-(3-amino-1-(difluoromethyl)-1H-pyrazol-5-yl)propan-2-ol

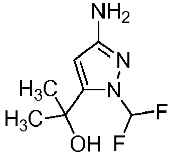

### A) 2-(1-(difluoromethyl)-5-(2-((4-methoxybenzyl)oxy)propan-2-yl)-1H-pyrazol-3-yl)-1H-isoindole-1,3(2H)-dione

To a solution of 2-(5-(2-((4-methoxybenzyl)oxy)propan-2-yl)-1H-pyrazol-3-yl)-1H-isoindole-1,3(2H)-dione obtained in Step D of Reference Example 29 (1.5 g) in N,N-dimethylformamide (15 mL) was added sodium hydride (60% in mineral oil, 0.19 g), and the mixture was stirred at 0°C for 5 min, chlorodifluoromethane was added thereto, and stirred at the same temperature for 4 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (hexane/ethyl acetate) to give the title compound (150 mg).
¹H NMR (400 MHz, DMSO-d₆) δ1.70 (6H, s), 3.75 (3H, s), 6.62 (1H, s), 6.87-6.97 (2H, m), 7.26 (2H, d, J = 8.6 Hz), 7.91-7.97 (2H, m), 7.98-8.03 (2H, m), 8.08 (1H, t, J = 60.0 Hz).

### B) 2-(1-(difluoromethyl)-5-(2-hydroxypropan-2-yl)-1H-pyrazol-3-yl)-1H-isoindole-1,3(2H)-dione

The title compound (74 mg) was obtained using 2-(1-(difluoromethyl)-5-(2-((4-methoxybenzyl)oxy)propan-2-yl)-1H-pyrazol-3-yl)-1H-isoindole-1,3(2H)-dione obtained in Step A of Reference Example 34 (150 mg), in the same manner as in the method of Reference Example 26.
MS (ESI+) : [M+H]⁺322.1.

### C) 2-(3-amino-1-(difluoromethyl)-1H-pyrazol-5-yl)propan-2-ol

The title compound (33 mg) was obtained using 2-(1-(difluoromethyl)-5-(2-hydroxypropan-2-yl)-1H-pyrazol-3-yl)-1H-isoindole-1,3(2H)-dione obtained in Step B of Reference Example 34 (74 mg), in the same manner as in the method of Reference Example 27.
MS (ESI+) : [M+H]⁺192.1.

### Reference Example 36

### tert-butyl 3-(3-amino-1-methyl-1H-pyrazol-5-yl)azetidine-1-carboxylate

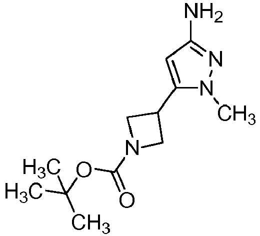

### A) tert-butyl 3-(cyanoacetyl)azetidine-1-carboxylate

To a solution of sodium hydride (60% in mineral oil, 3.3 g) in tetrahydrofuran (50 mL) was added dropwise a solution of 1-tert-butyl 3-methyl azetidine-1,3-dicarboxylate (15 g) and acetonitrile (4.4 mL) in tetrahydrofuran (50 mL) at 70°C under nitrogen atmosphere, and the mixture was stirred overnight at 70°C. The reaction mixture was allowed to be cooled to room temperature, neutralized with 1M hydrochloric acid, and extracted with ethyl acetate. The obtained organic layer was washed with water and saturated brine, and dried over anhydrous magnesium sulfate, and the solvent was evaporated under reduced pressure. The residue was purified by column chromatography (hexane/ethyl acetate) to give the title compound (1.8 g).
¹H NMR (400 MHz, CDCl₃) δ1.44 (9H, s), 3.50 (2H, s), 3.69 (1H, tt, J = 8.4, 6.4 Hz), 4.03-4.27 (4H, m).

### B) tert-butyl 3-(3-amino-1H-pyrazol-5-yl)azetidine-1-carboxylate

To a solution of tert-butyl 3-(cyanoacetyl)azetidine-1-carboxylate obtained in Step A of Reference Example 36 (1.8 g) in methanol (30 mL) was added hydrazine monohydrate (0.59 mL) at room temperature under nitrogen atmosphere. The reaction mixture was heated at 70°C for 3 hr. The solvent was evaporated under reduced pressure, and the residue was purified by silica gel column chromatography (ethyl acetate/methanol) to give the title compound (0.52 g).
MS (ESI-), found: 237.1.

### C) tert-butyl 3-(3-(1,3-dioxo-1,3-dihydro-2H-isoindol-2-yl)-1H-pyrazol-5-yl)azetidine-1-carboxylate

To a solution of tert-butyl 3-(3-amino-1H-pyrazol-5-yl)azetidine-1-carboxylate obtained in Step B of Reference Example 36 (520 mg) in cyclopentyl methyl ether (20 mL) was added phthalic anhydride (0.35 g) under nitrogen atmosphere, and the mixture was stirred overnight at 100°C. The solvent was evaporated under reduced pressure, and the residue was purified by silica gel column chromatography (ethyl acetate/hexane) to give the title compound (0.58 g).
MS (ESI-), found: 367.2.

### D) tert-butyl 3-(3-(1,3-dioxo-1,3-dihydro-2H-isoindol-2-yl)-1-methyl-1H-pyrazol-5-yl)azetidine-1-carboxylate

To a solution of tert-butyl 3-(3-(1,3-dioxo-1,3-dihydro-2H-isoindol-2-yl)-1H-pyrazol-5-yl)azetidine-1-carboxylate obtained in Step C of Reference Example 36 (0.58 g) in tetrahydrofuran (10 mL) was added sodium hydride (60% in mineral oil, 95 mg) at 0°C under nitrogen atmosphere. The reaction mixture was stirred at the same temperature for 10 min. To the reaction mixture was added methyl iodide (0.20 mL) at room temperature, and the mixture was stirred at the same temperature for 1 hr. To the reaction mixture was added saturated aqueous ammonium chloride solution, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (280 mg).
¹H NMR (400 MHz, CDCl₃) δ1.46 (9H, s), 3.80 (3H, s), 3.81-3.88 (1H, m), 4.06 (2H, dd, J = 8.3, 6.4 Hz), 4.27-4.43 (2H, m), 6.38 (1H, d, J = 0.5 Hz), 7.72-7.84 (2H, m), 7.96 (2H, dd, J = 5.5, 3.1 Hz).

### E) tert-butyl 3-(3-amino-1-methyl-1H-pyrazol-5-yl)azetidine-1-carboxylate

To a solution of tert-butyl 3-(3-(1,3-dioxo-1,3-dihydro-2H-isoindol-2-yl)-1-methyl-1H-pyrazol-5-yl)azetidine-1-carboxylate obtained in Step D of Reference Example 36 (0.28 g) in ethanol (7.0 mL) was added hydrazine monohydrate (0.072 mL) at room temperature under nitrogen atmosphere, and the mixture was stirred at 65°C for 3 hr. The insoluble substance was removed by filtration, and the solvent was evaporated under reduced pressure. The residue was purified by silica gel column chromatography (NH, hexane/ethyl acetate) to give the title compound (0.18 g).
¹H NMR (400 MHz, CDCl₃) δ1.45 (9H, s), 3.54 (3H, s), 3.58 (2H, brs), 3.63-3.75 (1H, m), 3.93 (2H, dd, J = 8.2, 6.5 Hz), 4.23-4.31 (2H, m), 5.58 (1H, s).

### Reference Example 37

### tert-butyl 4-(3-amino-1-methyl-1H-pyrazol-5-yl)piperidine-1-carboxylate

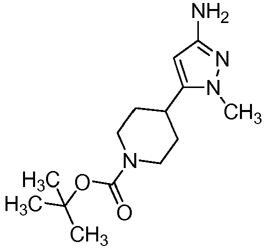

### A) tert-butyl 4-(cyanoacetyl)piperidine-1-carboxylate

The title compound (2.8 g) was obtained using 1-tert-butyl 4-methyl piperidine-1,4-dicarboxylate (5.0 g), in the same manner as in the method of Step A of Reference Example 36.
MS (ESI-), found: 251.1.

### B) tert-butyl 4-(3-amino-1H-pyrazol-5-yl)piperidine-1-carboxylate

The title compound (3.3 g) was obtained using tert-butyl 4-(cyanoacetyl)piperidine-1-carboxylate obtained in Step A of Reference Example 37 (2.8 g), in the same manner as in the method of Step B of Reference Example 36.
MS (ESI-), found: 265.1.

### C) tert-butyl 4-(3-(1,3-dioxo-1,3-dihydro-2H-isoindol-2-yl)-1H-pyrazol-5-yl)piperidine-1-carboxylate

The crude title compound (4.2 g) was obtained using tert-butyl 4-(3-amino-1H-pyrazol-5-yl)piperidine-1-carboxylate obtained in Step B of Reference Example 37 (3.3 g), in the same manner as in the method of Step C of Reference Example 36.
MS (ESI-), found: 395.2.

### D) tert-butyl 4-(3-(1,3-dioxo-1,3-dihydro-2H-isoindol-2-yl)-1-methyl-1H-pyrazol-5-yl)piperidine-1-carboxylate

The title compound (0.74 g) was obtained using tert-butyl 4-(3-(1,3-dioxo-1,3-dihydro-2H-isoindol-2-yl)-1H-pyrazol-5-yl)piperidine-1-carboxylate obtained in Step C of Reference Example 37 (2.0 g), in the same manner as in the method of Step D of Reference Example 36.
MS (ESI+) : [M+H]⁺ 411.2.

### E) tert-butyl 4-(3-amino-1-methyl-1H-pyrazol-5-yl)piperidine-1-carboxylate

The title compound (0.42 g) was obtained using tert-butyl 4-(3-(1,3-dioxo-1,3-dihydro-2H-isoindol-2-yl)-1-methyl-1H-pyrazol-5-yl)piperidine-1-carboxylate obtained in Step D of Reference Example 37 (0.74 g), in the same manner as in the method of Step E of Reference Example 36.
MS (ESI+): [M+H]⁺ 281.2

### Reference Example 38

### 1-methyl-5-(tetrahydro-2H-pyran-4-yl)-1H-pyrazol-3-amine

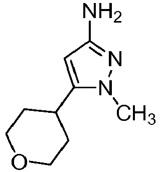

### A) 3-oxo-3-(tetrahydro-2H-pyran-4-yl)propanenitrile

The title compound (6.3 g) was obtained using methyl tetrahydro-2H-pyran-4-carboxylate (10 g), in the same manner as in the method of Step A of Reference Example 36.
¹H NMR (400MHz, CDCl₃) δ1.65-1.91 (4H, m), 2.82 (1H, tt, J = 11.2, 4.0 Hz), 3.46 (2H, td, J = 11.6, 2.6 Hz), 3.52 (2H, s), 3.97-4.11 (2H, m).

### B) 5-(tetrahydro-2H-pyran-4-yl)-1H-pyrazol-3-amine

The title compound (8.6 g) was obtained using 3-oxo-3-(tetrahydro-2H-pyran-4-yl)propanenitrile obtained in Step A of Reference Example 38 (6.2 g), in the same manner as in the method of Step B of Reference Example 36.
¹H NMR (400MHz, CDCl₃) δ 1.56-1.90 (4H, m), 2.81 (1H, tt, J = 11.5, 4.2 Hz), 3.36-3.59 (4H, m), 4.03 (2H, ddd, J = 11 . 6, 4. 1, 2.0 Hz), 5.47 (1H, d, J = 0.5 Hz).

### C) 2-(5-(tetrahydro-2H-pyran-4-yl)-1H-pyrazol-3-yl)-1H-isoindole-1,3(2H)-dione

The title compound (11 g) was obtained using 5-(tetrahydro-2H-pyran-4-yl)-1H-pyrazol-3-amine obtained in Step B of Reference Example 38 (6.8 g), in the same manner as in the method of Step C of Reference Example 36.
¹H NMR (400MHz, DMSO-d₆)δ1.42-1.72 (4H, m), 1.79-1.90 (1H, m), 3.37-3. 50 (2H, m), 3.91 (2H, dd, J = 10.0, 4.4 Hz), 6.38 (1H, s), 7.32-7.68 (2H, m), 7.80 (1H, dd, J = 7.7, 1.1 Hz), 7.86-8.03 (1H, m), 10.71 (1H, brs).

### D) 2-(1-methyl-5-(tetrahydro-2H-pyran-4-yl)-1H-pyrazol-3-yl)-1H-isoindole-1,3(2H)-dione

The title compound (0.87 g) was obtained using 2-(5-(tetrahydro-2H-pyran-4-yl)-1H-pyrazol-3-yl)-1H-isoindole-1,3(2H)-dione obtained in Step C of Reference Example 38 (2.0 g), in the same manner as in the method of Step D of Reference Example 36.
MS (ESI+): [M+H]⁺ 312.1.

### E) 1-methyl-5-(tetrahydro-2H-pyran-4-yl)-1H-pyrazol-3-amine

The title compound (0.10 g) was obtained using 2-(1-methyl-5-(tetrahydro-2H-pyran-4-yl)-1H-pyrazol-3-yl)-1H-isoindole-1,3(2H)-dione obtained in Step D of Reference Example 38 (0.87 g), in the same manner as in the method of Step E of Reference Example 36.
¹H NMR (400 MHz, CDCl₃) δ1.54-1.92 (4H, m), 2.59-2.84 (1H, m), 3.40-3.56 (2H, m), 3.63 (3H, s), 4.05 (2H, dd, J = 10.6, 4.5 Hz), 5.41 (1H, s).

### Reference Example 39

### 1-(3-amino-1-methyl-1H-pyrazol-5-yl)-4-methylpiperidin-4-ol

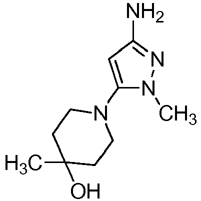

### A) ethyl 3-bromo-5-(4-hydroxy-4-methylpiperidin-1-yl)-1-methyl-1H-pyrazole-4-carboxylate

To a solution of ethyl 3,5-dibromo-1-methyl-1H-pyrazole-4-carboxylate obtained in Reference Example 2 (26 g) in N-methyl-2-pyrrolidone (80 mL) was added 4-methylpiperidin-4-ol (8.0 g) under nitrogen atmosphere, and the mixture was stirred at 160°C for 8 hr. The reaction mixture was allowed to be cooled to room temperature, water was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (hexane/ethyl acetate) to give the title compound (13 g).
MS (ESI+): [M+H]⁺ 346.2.

### B) 1-(3-bromo-1-methyl-1H-pyrazol-5-yl)-4-methylpiperidin-4-ol

A mixture of ethyl 3-bromo-5-(4-hydroxy-4-methylpiperidin-1-yl)-1-methyl-1H-pyrazole-4-carboxylate obtained in Step A of Reference Example 39 (14 g), 2 M aqueous sodium hydroxide solution (99 mL) and ethanol (150 mL) was stirred at 70°C for 4 hr. To the reaction mixture was added conc. sulfuric acid (25 mL) at 0°C, and the mixture was stirred at 60°C 1 hr. The reaction mixture was basified with 8 M aqueous sodium hydroxide solution at 0°C, and extracted with ethyl acetate (x2). The extracts were combined, washed with saturated brine, and dried over anhydrous magnesium sulfate, and the solvent was evaporated under reduced pressure to give the title compound (10 g).
MS (ESI+): [M+H]⁺ 274.2.

### C) 1-(3-amino-1-methyl-1H-pyrazol-5-yl)-4-methylpiperidin-4-ol

To a solution of 1-(3-bromo-1-methyl-1H-pyrazol-5-yl)-4-methylpiperidin-4-ol obtained in Step B of Reference Example 39 (600 mg) in 1,2-dimethoxyethane (20 mL) were added 1,1-diphenylmethanimine (0.48 g), potassium phosphate (1.1 g), tris(dibenzylideneacetone)dipalladium(0) (100 mg) and di-tert-butyl(2',4',6'-triisopropylbiphenyl-2-yl)phosphine (140 mg), under argon atmosphere, and the mixture was stirred at 100°C for 9 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. To a solution of the residue in tetrahydrofuran (30 mL) was added 1M hydrochloric acid (4.4 mL), and the mixture was stirred overnight at room temperature. To the reaction mixture was added saturated aqueous sodium hydrogencarbonate solution, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate) to give the title compound (120 mg).
MS (ESI+) : [M+H]⁺ 210.8.

### Reference Example 40

### 2-(3-amino-1-methyl-1H-pyrazol-5-yl)-2-methylpropanamide

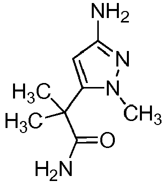

### A) 3-(2,5-dimethyl-1H-pyrrol-1-yl)-1-methyl-1H-pyrazole-5-carbaldehyde

To a solution of 3-(2,5-dimethyl-1H-pyrrol-1-yl)-1-methyl-1H-pyrazole (8.0 g) in tetrahydrofuran (80 mL) was added dropwise n-butyllithium (1.6 M hexane solution, 34 mL) at -78°C, and the mixture was stirred at the same temperature for 1 hr. To the reaction mixture was added dropwise N,N-dimethylformamide (5.3 mL) at -78°C, and the mixture was stirred overnight while gradually raising the temperature to room temperature. To the reaction mixture was added saturated aqueous ammonium chloride solution, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (7.9 g).
MS (ESI+) : [M+H]⁺ 203.8.

### B) (3-(2,5-dimethyl-1H-pyrrol-1-yl)-1-methyl-1H-pyrazol-5-yl)methanol

To a solution of 3-(2,5-dimethyl-1H-pyrrol-1-yl)-1-methyl-1H-pyrazole-5-carbaldehyde obtained in Step A of Reference Example 40 (2.9 g) in tetrahydrofuran (30 mL) was added sodium borohydride (0.68 g) under ice-cooling, and the mixture was stirred at room temperature for 4 hr. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (2.7 g).
MS (ESI+) : [M+H]⁺ 205.8.

### C) (3-(2,5-dimethyl-1H-pyrrol-1-yl)-1-methyl-1H-pyrazol-5-yl)acetonitrile

To a solution of (3-(2,5-dimethyl-1H-pyrrol-1-yl)-1-methyl-1H-pyrazol-5-yl)methanol obtained in Step B of Reference Example 40 (2.1 g), acetone cyanohydrin (1.1 mL) and tri-n-butyl phosphate (3.1 mL) in tetrahydrofuran (50 mL) was added (E)-diazene-1,2-diylbis(piperidin-1-ylmethanone) (3.2 g), and the mixture was stirred at room temperature for 7 hr. To the reaction mixture were added acetone cyanohydrin (1.1 mL), tri-n-butyl phosphate (3.1 mL) and (E)-diazene-1,2-diylbis(piperidin-1-ylmethanone) (3.2 g), and the mixture was stirred at room temperature for 2 hr. To the reaction mixture was added hexane (150 mL), and the insoluble substance was removed through Celite. The solvent was evaporated under reduced pressure, and the residue was purified by silica gel column chromatography (hexane/ethyl acetate) to give the title compound (1.5 g).
MS(ESI+): [M+H]⁺ 214.9.

### D) 2-(3-(2,5-dimethyl-1H-pyrrol-1-yl)-1-methyl-1H-pyrazol-5-yl)-2-methylpropanenitrile

To a solution of (3-(2,5-dimethyl-1H-pyrrol-1-yl)-1-methyl-1H-pyrazol-5-yl)acetonitrile obtained in Step C of Reference Example 40 (800 mg) in N,N-dimethylformamide (10 mL) was added sodium hydride (60% in mineral oil, 330 mg) under ice-cooling, and the mixture was stirred at the same temperature for 30 min. To the reaction mixture was added methyl iodide (0.70 mL), and the mixture was stirred overnight at room temperature. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (280 mg).
MS(ESI+): [M+H]⁺ 242.9.

### E) 2-(3-(2,5-dimethyl-1H-pyrrol-1-yl)-1-methyl-1H-pyrazol-5-yl)-2-methylpropanamide

To a solution of 2-(3-(2,5-dimethyl-1H-pyrrol-1-yl)-1-methyl-1H-pyrazol-5-yl)-2-methylpropanenitrile obtained in Step D of Reference Example 40 (270 mg) in dimethyl sulfoxide (5.0 mL) were added potassium carbonate (460 mg) and 35% aqueous hydrogen peroxide (0.29 mL), and the mixture was stirred overnight at room temperature. To the reaction mixture was added saturated aqueous sodium thiosulfate solution, and the mixture was extracted with ethyl acetate. The obtained organic layer was washed with saturated brine, and dried over anhydrous magnesium sulfate, and the solvent was evaporated under reduced pressure. The residue was recrystallized (ethyl acetate) to give the title compound (200 mg).
MS(ESI+): [M+H]⁺ 260.9.

### F) 2-(3-amino-1-methyl-1H-pyrazol-5-yl)-2-methylpropanamide

To a solution of 2-(3-(2,5-dimethyl-1H-pyrrol-1-yl)-1-methyl-1H-pyrazol-5-yl)-2-methylpropanamide obtained in Step E of Reference Example 40 (190 mg) in ethanol (5.0 mL) were added hydroxylamine hydrochloride (300 mg) and 8M aqueous potassium hydroxide solution (0.37 mL), and the mixture was stirred at 100°C for 16 hr. The solvent was evaporated under reduced pressure, and the obtained residue was purified by silica gel column chromatography (NH, hexane/ethyl acetate) to give the title compound (90 mg).
MS(ESI+): [M+H] + 182.8.

### Reference Examples 41 to 43

The title compounds of Reference Examples 41 to 43 shown in Table 32 were obtained using (3-(2,5-dimethyl-1H-pyrrol-1-yl)-1-methyl-1H-pyrazol-5-yl)acetonitrile obtained in Step C of Reference Example 40 and the reagents corresponding to the compounds of Reference Examples 41 to 43 (these reagents can be produced according to a method known per se), in the same manner as in the method of Reference Example 40 or a method analogous thereto. MS in the tables means actual measured value.

**Table 32**

| Ref. No. | IUPAC Name | Structure | Salt | MS | Corresponding Reagent |
|---|---|---|---|---|---|
| 41 | 1-(3-amino-1-methyl-1H-pyrazol-5-yl)cyclopropanecarboxamide | 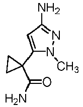 | Free | 180.8 | 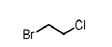 |
| 42 | 1-(3-amino-1-methyl-1H-pyrazol-5-yl)cyclobutanecarboxamide | 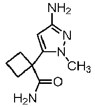 | Free | 194.8 | 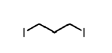 |
| 43 | 1-(3-amino-1-methyl-1H-pyrazol-5-yl)cyclopentanecarboxamide | 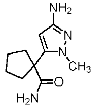 | Free | 208.8 | 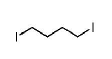 |

### Reference Example 44

### 2-(3-amino-1-methyl-1H-pyrazol-5-yl)-N-(2,2,2-trifluoroethyl)acetamide

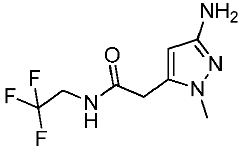

### A) (3-(2,5-dimethyl-1H-pyrrol-1-yl)-1-methyl-1H-pyrazol-5-yl)acetic acid

To a solution of (3-(2,5-dimethyl-1H-pyrrol-1-yl)-1-methyl-1H-pyrazol-5-yl)acetonitrile obtained in Step C of Reference Example 40 (300 mg) in ethanol (6.0 mL) was added 8M aqueous sodium hydroxide solution (1.8 mL), and the mixture was stirred at 80°C for 2 hr. The reaction mixture was cooled to 0°C, neutralized with 6M hydrochloric acid, and extracted with ethyl acetate. The obtained organic layer was washed with water and saturated brine, and dried over anhydrous magnesium sulfate, and the solvent was evaporated under reduced pressure to give the crude title compound (310 mg).
MS(ESI+): [M+H]⁺ 233.8.

### B) 2-(3-(2,5-dimethyl-1H-pyrrol-1-yl)-1-methyl-1H-pyrazol-5-yl)-N-(2,2,2-trifluoroethyl)acetamide

To a solution of (3-(2,5-dimethyl-1H-pyrrol-1-yl)-1-methyl-1H-pyrazol-5-yl)acetic acid obtained in Step A of Reference Example 44 (400 mg) in N,N-dimethylformamide (5.0 mL) were added triethylamine (1.2 mL), 1.7 M propane phosphonic acid anhydride ethyl acetate solution (1.5 mL) and 2,2,2-trifluoroethanamine (0.20 mL), and the mixture was stirred at room temperature for 4 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (hexane/ethyl acetate) to give the title compound (360 mg).
MS (ESI+) : [M+H]⁺ 314.9.

### C) 2-(3-amino-1-methyl-1H-pyrazol-5-yl)-N-(2,2,2-trifluoroethyl)acetamide

The title compound (200 mg) was obtained using 2-(3-(2,5-dimethyl-1H-pyrrol-1-yl)-1-methyl-1H-pyrazol-5-yl)-N-(2,2,2-trifluoroethyl)acetamide obtained in Step B of Reference Example 44 (360 mg), in the same manner as in the method of Step F of Reference Example 40.
MS (ESI+) : [M+H]⁺ 236.8.

### Reference Example 45

### 3-amino-1-methyl-1H-pyrazole-5-carbonitrile

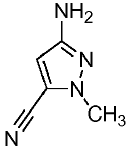

### A) 1-(3-(2,5-dimethyl-1H-pyrrol-1-yl)-1-methyl-1H-pyrazol-5-yl)-N-hydroxymethanimine

To a solution of 3-(2,5-dimethyl-1H-pyrrol-1-yl)-1-methyl-1H-pyrazole-5-carbaldehyde obtained in Step A of Reference Example 40 (1.0 g) in methanol (10 mL) were added potassium carbonate (820 mg) and hydroxylamine hydrochloride (410 mg), and the mixture was stirred at room temperature for 6 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure to give the crude title compound (1.0 g). MS (ESI+) : [M+H]⁺ 218.9.

### B) 5-((hydroxyimino)methyl)-1-methyl-1H-pyrazol-3-amine

The title compound (500 mg) was obtained using the crude 1-(3-(2,5-dimethyl-1H-pyrrol-1-yl)-1-methyl-1H-pyrazol-5-yl)-N-hydroxymethanimine obtained in Step A of Reference Example 45 (720 mg), in the same manner as in the method of Step F of Reference Example 40.
MS (ESI+) : [M+H]⁺140.8.

### C) 3-amino-1-methyl-1H-pyrazole-5-carbonitrile

To a solution of 5-((hydroxyimino)methyl)-1-methyl-1H-pyrazol-3-amine obtained in Step B of Reference Example 45 (720 mg) in acetonitrile (30 mL) was added thionyl chloride (0.45 mL), and the mixture was stirred at room temperature for 2 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by column chromatography (hexane/ethyl acetate) to give the title compound (175 mg).
MS (ESI+) : [M+H]⁺ 122.7.

### Reference Example 46

### N-((3-amino-1-methyl-1H-pyrazol-5-yl)methyl)acetamide

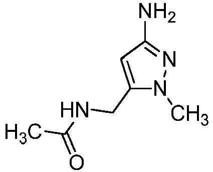

### A) 5-(azidomethyl)-3-(2,5-dimethyl-1H-pyrrol-1-yl)-1-methyl-1H-pyrazole

To a solution of (3-(2,5-dimethyl-1H-pyrrol-1-yl)-1-methyl-1H-pyrazol-5-yl)methanol obtained in Step B of Reference Example 40 (740 mg) in tetrahydrofuran (15 mL) were added diphenyl phosphorazidate (1.2 g) and diazabicycloundecene (0.65 mL), and the mixture was stirred at room temperature for 16 hr. To the reaction mixture was added water, and the mixture was extracted with ethyl acetate. The obtained organic layer was washed with water and saturated brine, and dried over anhydrous magnesium sulfate, and the solvent was evaporated under reduced pressure. The residue was purified by column chromatography (hexane/ethyl acetate) to give the title compound (730 mg).
MS (ESI+) : [M+H]⁺ 230.9.

### B) 1-(3-(2,5-dimethyl-1H-pyrrol-1-yl)-1-methyl-1H-pyrazol-5-yl)methanamine

To a solution of 5-(azidomethyl)-3-(2,5-dimethyl-1H-pyrrol-1-yl)-1-methyl-1H-pyrazole obtained in Step A of Reference Example 46 (470 mg) in tetrahydrofuran (12 mL) was added lithium aluminium hydride (93 mg), and the mixture was stirred at room temperature for 4 hr. To the reaction mixture was added sodium sulfate decahydrate (1.3 g), and the mixture was stirred at room temperature for 2 hr. The insoluble substance was removed through Celite, and the solvent was evaporated under reduced pressure to give the title compound (440 mg).
MS(ESI+): [M+H]⁺ 204.8.

### C) N-((3-(2,5-dimethyl-1H-pyrrol-1-yl)-1-methyl-1H-pyrazol-5-yl)methyl)acetamide

To a solution of 1-(3-(2,5-dimethyl-1H-pyrrol-1-yl)-1-methyl-1H-pyrazol-5-yl)methanamine obtained in Step B of Reference Example 46 (300 mg) in acetic anhydride (3.0 mL) were added triethylamine (0.61 mL) and N,N-dimethyl-4-aminopyridine (18 mg), and the mixture was stirred at room temperature for 2 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by column chromatography (hexane/ethyl acetate) to give the title compound (240 mg).
MS (ESI+) : [M+H]⁺ 246.9.

### D) N-((3-amino-1-methyl-1H-pyrazol-5-yl)methyl)acetamide

The title compound (74 mg) was obtained using N-((3-(2,5-dimethyl-1H-pyrrol-1-yl)-1-methyl-1H-pyrazol-5-yl)methyl)acetamide obtained in Step C of Reference Example 46 (230 mg), in the same manner as in the method of Step F of Reference Example 40.
MS (ESI+) : [M+H]⁺ 168.8.

### Reference Example 47

### 2-(3-amino-1-methyl-1H-pyrazol-5-yl)propan-2-ol

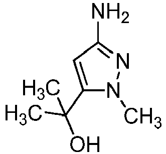

The title compound (1.1 g) was obtained using 2-(3-(2,5-dimethyl-1H-pyrrol-1-yl)-1-methyl-1H-pyrazol-5-yl)propan-2-ol obtained in Step A of Example 116 (1.4 g), in the same manner as in the method of Step F of Reference Example 40.
MS (ESI+) : [M+H]⁺ 156.1.

### Reference Example 48

### N-(2-(3-amino-1-methyl-1H-pyrazol-5-yl)propan-2-yl)acetamide

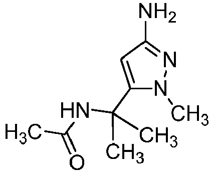

### A) N-(2-(3-bromo-1-methyl-1H-pyrazol-5-yl)propan-2-yl)acetamide

To a solution of 2-(3-bromo-1-methyl-1H-pyrazol-5-yl)propan-2-ol obtained in Reference Example 5 (5.0 g) in acetonitrile (50 mL) was added sulfuric acid (3.7 mL) under ice-cooling, and the mixture was stirred at room temperature for 15 hr. The reaction mixture was neutralized with 2M aqueous sodium hydroxide solution (35 mL), and extracted with ethyl acetate. The obtained organic layer was washed with water and saturated brine, and dried over anhydrous magnesium sulfate, and the solvent was evaporated under reduced pressure. The residue was recrystallized (toluene/diisopropyl ether) to give the crude title compound (4.9 g).
MS (ESI+) : [M+H]⁺260.8.

### B) N-(2-(3-amino-1-methyl-1H-pyrazol-5-yl)propan-2-yl)acetamide

A mixture of N-(2-(3-bromo-1-methyl-1H-pyrazol-5-yl)propan-2-yl)acetamide obtained in Step A of Reference Example 48 (1.0 g), 28% aqueous ammonia solution (3.8 mL), (4R)-4-hydroxy-L-proline (0.20 g), potassium carbonate (1.6 g) and copper(I) iodide (0.15 g) in dimethyl sulfoxide (0.50 mL) was stirred at 135°C for 2 hr in a microwave reactor. The solvent was evaporated under reduced pressure, and the residue was purified by silica gel column chromatography (NH, ethyl acetate/methanol) to give the title compound (0.49 g).
MS (ESI+) : [M+H]⁺ 197.8.

### Reference Example 49

### N-(2-(3-amino-1-methyl-1H-pyrazol-5-yl)propan-2-yl)-N-methylacetamide

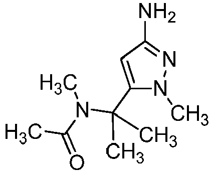

To a solution of N-(2-(3-bromo-1-methyl-1H-pyrazol-5-yl)propan-2-yl)acetamide obtained in Step A of Reference Example 48 (450 mg) in tetrahydrofuran (10 mL) was added sodium hydride (60% in mineral oil, 76 mg) under ice-cooling, and the mixture was stirred for 30 min. To the reaction mixture was added methyl iodide (370 mg), and the mixture was stirred at room temperature for 15 hr. To the reaction mixture was added water, and the mixture was extracted with ethyl acetate. The obtained organic layer was washed with saturated brine, and dried over anhydrous magnesium sulfate, and the solvent was evaporated under reduced pressure. To a solution (3.2 mL) of the obtained residue in 28% aqueous ammonia was added a solution of (4R)-4-hydroxy-L-proline (91 mg), potassium carbonate (0.72 g) and copper(I) iodide (66 mg) in dimethyl sulfoxide (5.0 mL), and the mixture was stirred at 135°C for 3 hr in a microwave reactor. The reaction mixture was evaporated under reduced pressure. The residue was purified by silica gel column chromatography (NH, hexane/ethyl acetate and ethyl acetate/methanol) to give the title compound (0.24 g).
MS (ESI+) : [M+H]⁺ 211.8.

### Reference Example 50

### 5-(1,4-oxazepan-4-yl)pyridin-2-amine

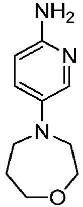

### A) 4-(6-nitropyridin-3-yl)-1,4-oxazepane

To a solution of 5-bromo-2-nitropyridine (1.0 g) in dimethyl sulfoxide (10 mL) were added 1,4-oxazepane (0.50 g), potassium carbonate (1.0 g) and tetrabutylammonium iodide (0.18 g), and the mixture was stirred overnight at 80°C. To the reaction mixture was added water, and the mixture was extracted with ethyl acetate. The obtained organic layer was washed with water and saturated brine, and dried over anhydrous magnesium sulfate, and the solvent was evaporated under reduced pressure. The residue was purified by column chromatography (NH, hexane/ethyl acetate) to give the title compound (520 mg).
MS (ESI+) : [M+H]⁺ 223.8.

### B) 5-(1,4-oxazepan-4-yl)pyridin-2-amine

To a solution of 4-(6-nitropyridin-3-yl)-1,4-oxazepane obtained in Step A of Reference Example 50 (520 mg) in methanol (5.0 mL) was added 10% palladium-carbon (124 mg), and the mixture was stirred at room temperature for 3 hr under hydrogen atmosphere (normal pressure). The palladium on carbon was removed through Celite, and the solvent was evaporated under reduced pressure to give the title compound (450 mg).
MS (ESI+) : [M+H]⁺ 193.8.

### Reference Examples 51 to 53

The title compounds of Reference Examples 51 to 53 shown in Table 33 were obtained using 5-bromo-2-nitropyridine and the reagents corresponding to the compounds of Reference Examples 51 to 53 (these reagents can be produced according to a method known per se), in the same manner as in the method of Reference Example 50 or a method analogous thereto. MS in the tables means actual measured value.

**Table 33**

| Ref. No. | IUPAC Name | Structure | Salt | MS | Corresponding Reagent |
|---|---|---|---|---|---|
| 51 | (3R)-1-(6-aminopyridin-3-yl)pyrrolidin-3-ol | 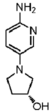 | Free | 179.8 | 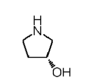 |
| 52 | (3S)-1-(5-aminopyridin-3-yl)pyrrolidin-3-ol | 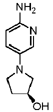 | Free | 179.8 | 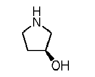 |
| 53 | (3R)-1-(5-aminopyridin-3-yl)piperidin-3-ol | 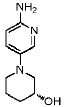 | Free | 193.8 | 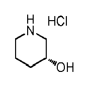 |

### Reference Example 54

### 3-(6-aminopyridin-3-yl)-1,2,4-oxadiazol-5(4H)-one

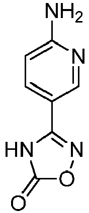

### A) tert-butyl (5-(5-oxo-4,5-dihydro-1,2,4-oxadiazol-3-yl)pyridin-2-yl)carbamate

To a solution of tert-butyl (5-cyanopyridin-2-yl)carbamate (600 mg) in dimethyl sulfoxide (10 mL) were added hydroxylamine hydrochloride (950 mg) and sodium carbonate (1.5 g), and the mixture was stirred at 60°C for 5 hr. To the reaction mixture was added water, and the mixture was extracted with ethyl acetate. The obtained organic layer was washed with water and saturated brine, and dried over anhydrous magnesium sulfate, and the solvent was evaporated under reduced pressure. To a solution of the obtained residue in tetrahydrofuran (10 mL) were added diazabicycloundecene (0.83 mL) and 1,1'-carbonyldiimidazole (890 mg), and the mixture was stirred at 60°C for 2 hr. The reaction mixture was neutralized with 2M hydrochloric acid, and extracted with ethyl acetate. The obtained organic layer was washed with water and saturated brine, and dried over anhydrous magnesium sulfate, and the solvent was evaporated under reduced pressure to give the crude title compound (450 mg).
¹H NMR (400MHz, DMSO-d₆)δ 1.49 (9H, s), 7.98 (1H, dd, J = 9.0, 0.7 Hz), 8.12 (1H, dd, J = 8.8, 2.4 Hz), 8.64 (1H, dd, J = 2.3, 0.9 Hz), 10.29 (1H, s), 12.97 (1H, brs).

### B) 3-(6-aminopyridin-3-yl)-1,2,4-oxadiazol-5(4H)-one

To the crude tert-butyl (5-(5-oxo-4,5-dihydro-1,2,4-oxadiazol-3-yl)pyridin-2-yl)carbamate obtained in Step A of Reference Example 54 (450 mg) was added 4 M hydrogen chloride ethyl acetate solution (5.0 mL), and the mixture was stirred overnight at room temperature, and the solvent was evaporated under reduced pressure to give the title compound (340 mg).
MS (ESI-), found: 177.0.

### Reference Example 55

### N-(2-chloro-5-fluoropyridin-4-yl)acetamide

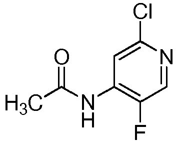

To a solution of 2-chloro-5-fluoropyridin-4-amine (500 mg) in acetic anhydride (5.0 mL) was added N,N-dimethyl-4-aminopyridine (4.2 mg), and the mixture was stirred at 80°C for 4 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (hexane/ethyl acetate) to give the title compound (440 mg).
MS (ESI+) : [M+H]⁺ 189.0.

### Reference Example 56

### N-(2-chloropyridin-4-yl)-2-methoxyacetamide

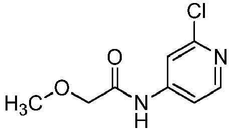

To a solution of 4-amino-2-chloropyridine (1.0 g) in tetrahydrofuran (10 mL) were added triethylamine (1.6 mL) and methoxyacetyl chloride (0.78 mL), and the mixture was stirred at 60°C for 5 hr. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate) to give the title compound (490 mg).
MS(ESI+): [M+H]⁺ 201.0.

### Reference Example 57

### N-(2-chloropyridin-4-yl)cyclopropanecarboxamide

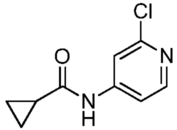

To a solution of 4-amino-2-chloropyridine (1.0 g) in tetrahydrofuran (20 mL) were added triethylamine (2.2 mL), N,N-dimethyl-4-aminopyridine (48 mg) and cyclopropanecarbonyl chloride (0.86 mL) under ice-cooling, and the mixture was stirred overnight at room temperature. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (NH, hexane/ethyl acetate) to give the title compound (1.4 g). MS(ESI+): [M+H]⁺ 201.0.

### Reference Example 58

### methyl (2-chloropyridin-4-yl)carbamate

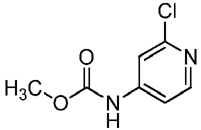

To a solution of 4-bromo-2-chloropyridine (3.0 g) in toluene (30 mL) were added methyl carbamate (1.3 g), palladium(II) acetate (0.18 g), dicyclohexyl(2',4',6'-triisopropylbiphenyl-2-yl)phosphine (0.74 g) and cesium carbonate (10 g), and the mixture was stirred overnight at 100°C. The reaction mixture was cooled to 0°C, saturated aqueous sodium hydrogencarbonate solution was added thereto, and the mixture was extracted with ethyl acetate. The obtained organic layer was washed with water and saturated brine, and dried over anhydrous magnesium sulfate, and the solvent was evaporated under reduced pressure. The residue was purified by silica gel column chromatography (hexane/ethyl acetate) to give the title compound (660 mg).
¹H NMR (400 MHz, DMSO-d₆) δ 3.72 (3H, s), 7.39 (1H, dd, J = 5.7, 1.8 Hz), 7.54 (1H, d, J = 1.7 Hz), 8.21 (1H, d, J = 5.6 Hz), 10.40 (1H, s).

### Reference Example 59

### tert-butyl 6-chloro-1H-pyrrolo[3,2-c]pyridine-1-carboxylate

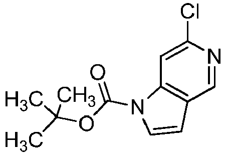

To a solution of 6-chloro-1H-pyrrolo[3,2-c]pyridine (350 mg) in acetonitrile (10 mL) were added N,N-dimethyl-4-aminopyridine (340 mg) and di-tert-butyl dicarbonate (0.64 mL), and the mixture was stirred at room temperature for 2 hr. The solvent was evaporated under reduced pressure. The residue was purified by silica gel column chromatography (hexane/ethyl acetate) to give the title compound (560 mg).
¹H NMR (400 MHz, DMSO-d₆) δ 1.64 (9H, s), 6.88 (1H, dd, J = 3.8, 0.9 Hz), 7.79 (1H, d, J = 3.7 Hz), 7.93 (1H, s), 8.74 (1H, d, J = 0.7 Hz).

### Reference Example 60

### tert-butyl 6-chloro-2,3-dihydro-1H-pyrrolo[3,2-c]pyridine-1-carboxylate

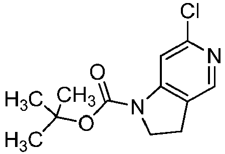

The title compound (400 mg) was obtained using 6-chloro-2,3-dihydro-1H-pyrrolo[3,2-c]pyridine (250 mg), in the same manner as in the method of Reference Example 59.
MS (ESI+) : [M+H]⁺ 255.1.

### Reference Example 61

### 6-bromo-N-(2-hydroxy-2-methylpropyl)nicotinamide

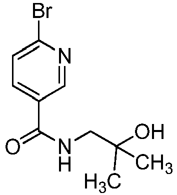

To a solution of 6-bromonicotinic acid (400 mg) in ethyl acetate (8.0 mL) were added N,N-diisopropylethylamine (1.0 mL), 1.7 M propane phosphonic acid anhydride ethyl acetate solution (1.7 mL) and 1-amino-2-methylpropan-2-ol (210 mg), and the mixture was stirred at room temperature for 4 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (270 mg).
MS (ESI+) : [M+H]⁺ 272.8.

### Reference Example 62

### (6-bromopyridin-3-yl)(4-hydroxy-4-(trifluoromethyl)piperidin-1-yl)methanone

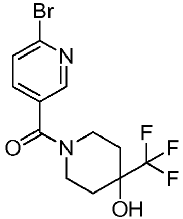

To a solution of 6-bromonicotinic acid (480 mg) in ethyl acetate (10 mL) were added N,N-diisopropylethylamine (1.2 mL), 1.7 M propane phosphonic acid anhydride ethyl acetate solution (2.1 mL) and 4-(trifluoromethyl)piperidin-4-ol (400 mg), and the mixture was stirred at room temperature for 15 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (650 mg).
MS (ESI+) : [M+H]⁺ 352.8.

### Reference Example 63

### 1-((6-bromopyridin-3-yl)methyl)-4-methylpiperidin-4-ol

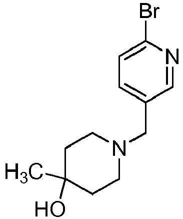

To a solution of 6-bromonicotinaldehyde (460 mg) and 4-methylpiperidin-4-ol (430 mg) in acetonitrile (10 mL) was added sodium triacetoxyborohydride (1.0 g) under ice-cooling, and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (430 mg).
MS (ESI+) : [M+H]⁺ 284.8.

### Reference Example 64

### 6-bromo-N-(1-hydroxy-2-methylpropan-2-yl)-N-methylnicotinamide

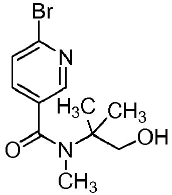

To a solution of 6-bromonicotinic acid (240 mg) in ethyl acetate (10 mL) were added N,N-diisopropylethylamine (0.62 mL), 1.7 M propane phosphonic acid anhydride ethyl acetate solution (1.0 mL) and 2-methyl-2-(methylamino)propan-1-ol (120 mg), and the mixture was stirred at room temperature for 16 hr, and then at 50°C for 2 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (42 mg).
MS (ESI+) : [M+H]⁺ 286.8.

### Reference Example 65

### 2-(6-chloropyridin-3-yl)-N,N,2-trimethylpropanamide

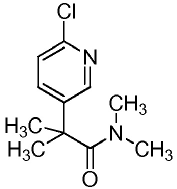

### A) ethyl 2-(6-chloropyridin-3-yl)-2-methylpropanoate

To a solution of ethyl (6-chloropyridin-3-yl)acetate (8.2 g) in N,N-dimethylformamide (50 mL) was added sodium hydride (60% in mineral oil, 2.4 g) under ice-cooling, and the mixture was stirred for 30 min. To the reaction mixture was added methyl iodide (7.7 mL), and the mixture was stirred at room temperature for 2 hr. To the reaction mixture was added methyl iodide (3.8 mL), and the mixture was stirred at 40°C for 15 hr. The reaction mixture was cooled to 0°C, N,N-dimethylformamide (15 mL) and sodium hydride (60% in mineral oil, 1.2 g) were added thereto, and the mixture was stirred at room temperature for 30 min. To the reaction mixture was added methyl iodide (2.5 mL), and the mixture was stirred at room temperature for 2 hr. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (4.4 g).
MS (ESI+) : [M+H]⁺ 227.8.

### B) 2-(6-chloropyridin-3-yl)-2-methylpropanoic acid

To a solution of ethyl 2-(6-chloropyridin-3-yl)-2-methylpropanoate obtained in Step A of Reference Example 65 (4.4 g) in ethanol (80 mL) was added 2M aqueous sodium hydroxide solution (39 mL), and the mixture was stirred at 50°C for 2 hr. The solvent was evaporated under reduced pressure, neutralized with 2M hydrochloric acid (39 mL), and extracted with ethyl acetate. The obtained organic layer was washed with water and saturated brine, and dried over anhydrous magnesium sulfate, and the solvent was evaporated under reduced pressure to give the title compound (4.0 g).
MS (ESI+) : [M+H]⁺ 199.7.

### C) 2-(6-chloropyridin-3-yl)-N,N,2-trimethylpropanamide

To a solution of 2-(6-chloropyridin-3-yl)-2-methylpropanoic acid obtained in Step B of Reference Example 65 (310 mg) in ethyl acetate (8.0 mL) were added N,N-diisopropylethylamine (0.82 mL), 1.7 M propane phosphonic acid anhydride ethyl acetate solution (1.9 mL) and dimethylamine hydrochloride (260 mg), and the mixture was stirred overnight at room temperature. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (160 mg).
MS (ESI+) : [M+H]⁺ 226.8.

### Reference Examples 66 to 69

The title compounds of Reference Examples 66 to 69 shown in Table 34 were obtained using 2-(6-chloropyridin-3-yl)-2-methylpropanoic acid obtained in Step B of Reference Example 65 and the reagents corresponding to the compounds of Reference Examples 66 to 69 (these reagents can be produced according to a method known per se), in the same manner as in the method of Step C of Reference Example 65 or a method analogous thereto. MS in the tables means actual measured value.

**Table 34**

| Ref. No. | IUPAC Name | Structure | Salt | MS | Corresponding Reagent |
|---|---|---|---|---|---|
| 66 | 2-(6-chloropyridin-3-yl)-N-(2-hydroxyethyl)-2-methylpropanamide | 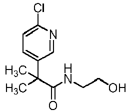 | Free | 242.8 | 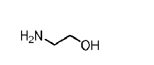 |
| 67 | 2-(6-chloropyridin-3-yl)-N-(2-hydroxyethyl)-N,2-dimethylpropanamide | 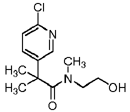 | Free | 256.9 | 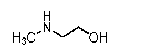 |
| 68 | 2-(6-chloropyridin-3-yl)-N,2-dimethyl-N-(oxetan-3-yl)propanamide | 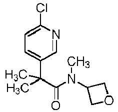 | Free | 268.9 | 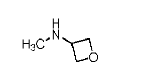 |
| 69 | 2-(6-chloropyridin-3-yl)-2-methyl-1-(morpholin-4-yl)propan-1-one | 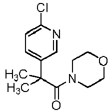 | Free | 268.9 | 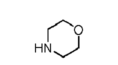 |

### Reference Examples 70 to 78

The title compounds of Reference Examples 70 to 78 shown in Table 35-Table 36 were obtained using 1-(6-chloropyridin-3-yl)cyclopropanecarboxylic acid and the reagents corresponding to the compounds of Reference Examples 70 to 78 (these reagents can be produced according to a method known per se), in the same manner as in the method of Step C of Reference Example 65 or a method analogous thereto. MS in the tables means actual measured value.

**Table 35**

| Ref. No. | IUPAC Name | Structure | Salt | MS | Corresponding Reagent |
|---|---|---|---|---|---|
| 70 | 1-(5-chloropyridin-3-yl)-N-methylcyclopropanecarboxamide | 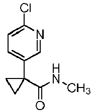 | Free | 210.8 | 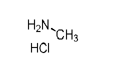 |
| 71 | 1-(5-chloropyridin-3-yl)-N,N-dimethylcyclopropanecarboxamid e | 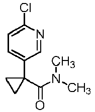 | Free | 224.8 | 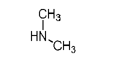 |
| 72 | azetidin-1-yl(1-(5-chloropyridin-3-yl)cyclopropyl)methanone | 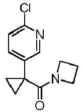 | Free | 236.8 | 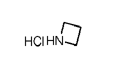 |
| 73 | (1-(5-chloropyridin-3-yl)cyclopropyl)(3-oxa-8-azabicyclo[3.2.1]oct-8-yl)methanone | 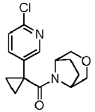 | Free | 293.1 | 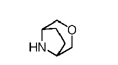 |
| 74 | 1-(5-chloropyridin-3-yl)-N-cyclopropyl-N-methylcyclopropanecarboxamide | 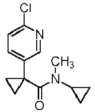 | Free | 250.8 | 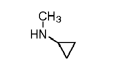 |
| 75 | (1-(5-chloropyridin-3-yl)cyclopropyl)(3,3-difluoroazetidin-1-yl)methanone | 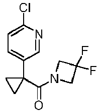 | Free | 273.0 | 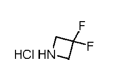 |
| 76 | 1-(6-chloropyridin-3-yl)-N-(2-methoxyethyl)cyclopropanecarbo xamide | 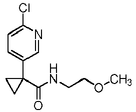 | Free | 254.8 | 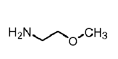 |
| 77 | 1-(5-chloropyridin-3-yl)-N-(2,2,2-trifluoroethyl)cyclopropanecar boxamide | 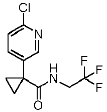 | Free | 278.8 | 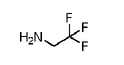 |

**Table 36**

| | | | | | |
|---|---|---|---|---|---|
| 78 | 1-(5-chloropyridin-3-yl)-N-(oxetan-3-yl)cyclopropanecarboxamide | 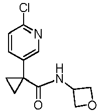 | Free | 253.0 | 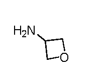 |

### Reference Example 79 1-(6-chloropyridin-3-yl)cyclopropanecarboxamide

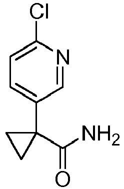

To a solution of 1-(6-chloropyridin-3-yl)cyclopropanecarboxylic acid (200 mg) in N,N-dimethylacetamide (4.0 mL) were added 2-(7-azabenzotriazol-1-yl)-1,1,3,3-hexafluorophosphate (HATU) (0.77 g), diisopropylethylamine (0.88 mL) and ammonium chloride (160 mg), and the mixture was stirred overnight at room temperature. The reaction mixture was cooled to 0°C, saturated aqueous sodium hydrogencarbonate solution was added thereto, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (170 mg).
MS(ESI+): [M+H]⁺ 196.7.

### Reference Examples 80 to 81

The title compounds of Reference Examples 80 to 81 shown in Table 37 were obtained using 1-(6-chloropyridin-3-yl)cyclopropanecarboxylic acid and the reagents corresponding to the compounds of Reference Examples 80 to 81 (these reagents can be produced according to a method known per se), in the same manner as in the method of Reference Example 79 or a method analogous thereto. MS in the tables means actual measured value.

**Table 37**

| Ref. No. | IUPAC Name | Structure | Salt | MS | Corresponding Reagent |
|---|---|---|---|---|---|
| 80 | (1-(5-chloropyridin-3-yl)cyclopropyl)((3S)-3-hydroxypyrrolidin-1-yl)methanone | 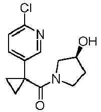 | Free | 266.8 | 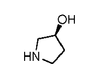 |
| 81 | (1-(5-chloropyridin-3-yl)cyclopropyl)((3R)-3-hydroxypyrrolidin-1-yl)methanone | 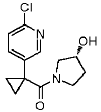 | Free | 266.8 | 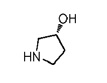 |

### Reference Example 82

### 1-(6-chloropyridin-3-yl)cyclobutanecarboxamide

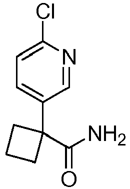

To a solution of 1-(6-chloropyridin-3-yl)cyclobutanecarboxylic acid (170 mg) in N,N-dimethylacetamide (4.0 mL) were added 2-(7-azabenzotriazol-1-yl)-1,1,3,3-hexafluorophosphate (HATU) (0.61 g), diisopropylethylamine (0.70 mL) and ammonium chloride (130 mg), and the mixture was stirred overnight at room temperature. The reaction mixture was cooled to 0°C, saturated aqueous sodium hydrogencarbonate solution was added thereto, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (96 mg).
MS(ESI+): [M+H]⁺ 210.8.

### Reference Example 83

### 1-(6-chloropyridin-3-yl)-N-methylcyclobutanecarboxamide

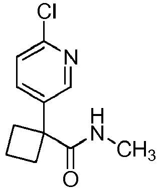

To a solution of 1-(6-chloropyridin-3-yl)cyclobutanecarboxylic acid (170 mg) in ethyl acetate (5.0 mL) were added N,N-diisopropylethylamine (0.70 mL), 1.7 M propane phosphonic acid anhydride ethyl acetate solution (0.71 mL) and methylamine hydrochloride (65 mg), and the mixture was stirred at room temperature for 10 hr. To the reaction mixture were added N,N-dimethylformamide (1.0 mL), N,N-diisopropylethylamine (0.70 mL), 1.7 M propane phosphonic acid anhydride ethyl acetate solution (0.71 mL), methylamine hydrochloride (65 mg) and 40% methylamine-methanol (0.040 mL), and the mixture was stirred at room temperature for 2 hr. To the reaction mixture was added saturated aqueous sodium hydrogencarbonate solution, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (68 mg).
MS (ESI+) : [M+H]⁺ 225.0.

### Reference Example 84

### 1-(6-chloropyridin-3-yl)cyclopentanecarboxamide

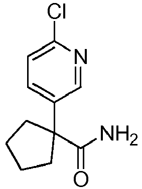

### A) ethyl 1-(6-chloropyridin-3-yl)cyclopentanecarboxylate

To a solution of ethyl (6-chloropyridin-3-yl)acetate (2.4 g) in N,N-dimethylformamide (18 mL) was added sodium hydride (60% in mineral oil, 1.2 g) under ice-cooling, and the mixture was stirred for 40 min. To the reaction mixture was added 1,4-diiodobutane (4.3 mL), and the mixture was stirred at room temperature for 2 hr. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (0.75 g).
MS (ESI+) : [M+H]⁺ 253.8.

### B) 1-(6-chloropyridin-3-yl)cyclopentanecarboxylic acid

To a solution of ethyl 1-(6-chloropyridin-3-yl)cyclopentanecarboxylate obtained in Step A of Reference Example 84 (0.74 g) in ethanol (10 mL) was added 2M aqueous sodium hydroxide solution (5.8 mL), and the mixture was stirred at room temperature for 4 hr, and then at 60°C for 2 hr. To the reaction mixture was added 2M aqueous sodium hydroxide solution (2.9 mL), and the mixture was stirred at 60°C for 2 hr. The solvent was evaporated under reduced pressure, 2N hydrochloric acid (8.7 mL) was added thereto, and the mixture was stirred for 30 min under ice-cooling. The precipitate was collected by filtration, and dried to give the title compound (0.44 g).
MS (ESI+) : [M+H]⁺ 225.8.

### C) 1-(6-chloropyridin-3-yl)cyclopentanecarboxamide

To a solution of 1-(6-chloropyridin-3-yl)cyclopentanecarboxylic acid obtained in Step B of Reference Example 84 (150 mg) in N,N-dimethylacetamide (3.0 mL) were added 2-(7-azabenzotriazol-1-yl)-1,1,3,3-hexafluorophosphate (HATU) (0.51 g), diisopropylethylamine (0.58 mL) and ammonium chloride (110 mg), and the mixture was stirred at room temperature for 5 hr. The reaction mixture was cooled to 0°C, saturated aqueous sodium hydrogencarbonate solution was added thereto, and the mixture was extracted with ethyl acetate. The obtained organic layer was washed successively with water and saturated brine, and dried over magnesium sulfate, and the solvent was evaporated under reduced pressure. The residue was purified by column chromatography (NH, hexane/ethyl acetate) to give the title compound (99 mg).
MS (ESI+) : [M+H]⁺ 224.8.

### Reference Example 85

### 1-(6-chloropyridin-3-yl)-N-methylcyclopentanecarboxamide

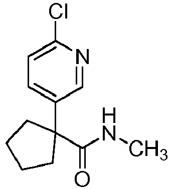

The title compound (100 mg) was obtained using 1-(6-chloropyridin-3-yl)cyclopentanecarboxylic acid obtained in Step B of Reference Example 84 (140 mg) and methylamine hydrochloride (63 mg), in the same manner as in the method of Step C of Reference Example 84.
MS(ESI+): [M+H]⁺ 238.8.

### Reference Example 86

### 1-(6-chloropyridin-3-yl)-N-(oxetan-3-yl)cyclopentanecarboxamide

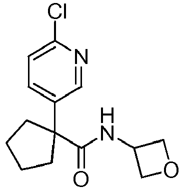

The title compound (140 mg) was obtained using 1-(6-chloropyridin-3-yl)cyclopentanecarboxylic acid obtained in Step B of Reference Example 84 (150 mg) and oxetan-3-amine (58 mg), in the same manner as in the method of Step C of Reference Example 84.
MS(ESI+): [M+H]⁺ 280.8.

### Reference Example 87

### 6-chloro-N,N,4-trimethylnicotinamide

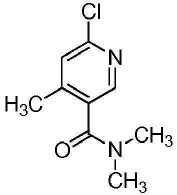

To a solution of 6-chloro-4-methylnicotinic acid (220 mg) in N,N-dimethylacetamide (4.0 mL) were added 2-(7-azabenzotriazol-1-yl)-1,1,3,3-hexafluorophosphate (HATU) (0.73 g), diisopropylethylamine (0.45 mL) and dimethylamine hydrochloride (160 mg), and the mixture was stirred at room temperature for 5 hr. The reaction mixture was cooled to 0°C, saturated aqueous sodium hydrogencarbonate solution was added thereto, and the mixture was extracted with ethyl acetate. The obtained organic layer was washed successively with water and saturated brine, and dried over magnesium sulfate, and the solvent was evaporated under reduced pressure. The residue was purified by column chromatography (hexane/ethyl acetate) to give the title compound (200 mg).
MS (ESI+) : [M+H]⁺ 198.7.

### Reference Examples 88 to 96

The title compounds of Reference Examples 88 to 96 shown in Table 38-Table 39 were obtained using 6-chloro-4-methylnicotinic acid and the reagents corresponding to the compounds of Reference Examples 88 to 96 (these reagents can be produced according to a method known per se), in the same manner as in the method of Reference Example 87 or a method analogous thereto. MS in the tables means actual measured value.

**Table 38**

| Ref.No. | IUPAC Name | Structure | Salt | MS | Corresponding Reagent |
|---|---|---|---|---|---|
| 88 | (6-chloro-4-methylpyridin-3-yl)(morpholin-4-yl)methanone | 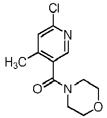 | Free | 240.8 | 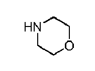 |
| 89 | (6-chloro-4-methylpyridin-3-yl)(4-hydroxy-4-methylpiperidin-1-yl)methanone | 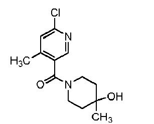 | Free | 268.8 | 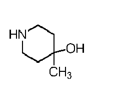 |
| 90 | (6-chloro-4-methylpyridin-3-yl)((3R)-3-hydroxypyrrolidin-1-yl)methanone | 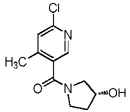 | Free | 240.8 | 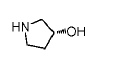 |
| 91 | (6-chloro-4-methylpyridin-3-yl)((3S)-3-hydroxypyrrolidin-1-yl)methanone | 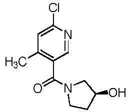 | Free | 240.8 | 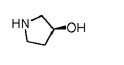 |
| 92 | 5-chloro-4-methyl-N-((3R)-tetrahydrofuran-3-yl)nicotinamide | 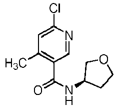 | Free | 240.8 | 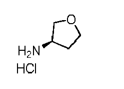 |
| 93 | 6-chloro-4-methyl-N-((3S)-tetrahydrofuran-3-yl)nicotinamide | 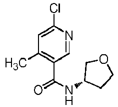 | Free | 240.8 | 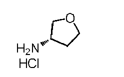 |
| 94 | (6-chloro-4-methylpyridin-3-yl)((3R)-3-hydroxypiperidin-1-yl)methanone | 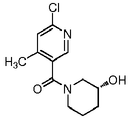 | Free | 254.8 | 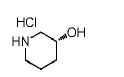 |
| 95 | (6-chloro-4-methylpyridin-3-yl)((3S)-3-hydroxypiperidin-1-yl)methanone | 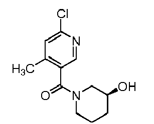 | Free | 254.8 | 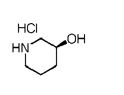 |

**Table 39**

| | | | | | |
|---|---|---|---|---|---|
| 96 | 6-chloro-N,4-dimethylnicotinamide | 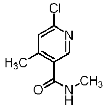 | Free | 185.0 | 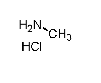 |

### Reference Example 97

### 4,6-dichloro-N,N-dimethylnicotinamide

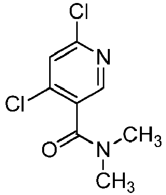

To a solution of 4,6-dichloronicotinic acid (300 mg) in N,N-dimethylacetamide (4.0 mL) were added 2-(7-azabenzotriazol-1-yl)-1,1,3,3-hexafluorophosphate (HATU) (0.89 g), diisopropylethylamine (0.55 mL) and dimethylamine hydrochloride (190 mg), and the mixture was stirred overnight at room temperature. The reaction mixture was cooled to 0°C, saturated aqueous sodium hydrogencarbonate solution was added thereto, and the mixture was extracted with ethyl acetate. The obtained organic layer was washed successively with water and saturated brine, and dried over magnesium sulfate, and the solvent was evaporated under reduced pressure. The residue was purified by column chromatography (hexane/ethyl acetate) to give the title compound (240 mg).
MS (ESI+) : [M+H]⁺ 218.7.

### Reference Example 98

### 4-(6-chloropyridin-3-yl)-N-methyltetrahydro-2H-pyran-4-carboxamide

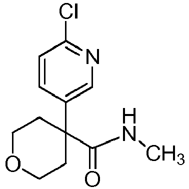

### A) ethyl 4-(6-chloropyridin-3-yl)tetrahydro-2H-pyran-4-carboxylate

To a solution of ethyl (6-chloropyridin-3-yl)acetate (1.0 g) in N,N-dimethylformamide (20 mL) was added sodium hydride (60% in mineral oil, 0.40 g) under ice-cooling, and the mixture was stirred for 40 min. To the reaction mixture was added 1-bromo-2-(2-bromoethoxy)ethane (2.3 g), and the mixture was stirred at 0°C for 3 hr. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by silica gel column chromatography (hexane/ethyl acetate) to give the title compound (0.82 g).
MS(ESI+): [M+H]⁺ 270.1.

### B) 4-(6-chloropyridin-3-yl)tetrahydro-2H-pyran-4-carboxylic acid

To a solution of ethyl 4-(6-chloropyridin-3-yl)tetrahydro-2H-pyran-4-carboxylate obtained in Step A of Reference Example 98 (0.82 g) in ethanol (10 mL) was added 2M aqueous sodium hydroxide solution (6.1 mL), and the mixture was stirred at room temperature for 4 hr. The solvent was evaporated under reduced pressure, 2M hydrochloric acid was added thereto under ice-cooling, and the mixture was stirred for 1 hr. The precipitate was collected by filtration, and dried to give the title compound (0.56 g).
MS (ESI+) : [M+H]⁺ 242.0.

### C) 4-(6-chloropyridin-3-yl)-N-methyltetrahydro-2H-pyran-4-carboxamide

To a solution of 4-(6-chloropyridin-3-yl)tetrahydro-2H-pyran-4-carboxylic acid obtained in Step B of Reference Example 98 (180 mg) in N,N-dimethylformamide (3.0 mL) were added N,N-diisopropylethylamine (580 mg), 1.7 M propane phosphonic acid anhydride ethyl acetate solution (0.88 mL) and methylamine hydrochloride (150 mg), and the mixture was stirred at room temperature for 15 hr. The reaction mixture was cooled to 0°C, saturated aqueous sodium hydrogencarbonate solution was added thereto, and the mixture was extracted with ethyl acetate. The obtained organic layer was washed successively with water and saturated brine, and dried over magnesium sulfate, and the solvent was evaporated under reduced pressure. The residue was purified by column chromatography (hexane/ethyl acetate) to give the title compound (65 mg).
MS (ESI+) : [M+H]⁺ 255.1.

### Reference Example 99

### 4-(6-chloropyridin-3-yl)tetrahydro-2H-pyran-4-carboxamide

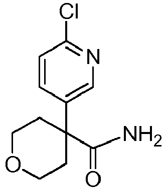

To a solution of 4-(6-chloropyridin-3-yl)tetrahydro-2H-pyran-4-carboxylic acid obtained in Step B of Reference Example 98 (180 mg) in N,N-dimethylformamide (3.0 mL) were added 2-(7-azabenzotriazol-1-yl)-1,1,3,3-hexafluorophosphate (HATU) (0.71 g), diisopropylethylamine (0.78 mL) and ammonium chloride (120 mg), and the mixture was stirred overnight at room temperature. The reaction mixture was cooled to 0°C, saturated aqueous sodium hydrogencarbonate solution was added thereto, and the mixture was extracted with ethyl acetate. The residue was recrystallized (hexane/ethyl acetate) to give the title compound (130 mg).
MS(ESI+): [M+H]⁺ 241.0.

### Reference Example 100

### (6-chloro-4-methylpyridin-3-yl)(3-oxa-8-azabicyclo[3.2.1]oct-8-yl)methanone

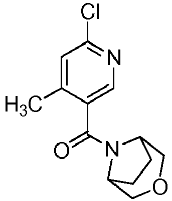

To a solution of 6-chloro-4-methylnicotinic acid (200 mg) in N,N-dimethylacetamide (4.0 mL) were added 2-(7-azabenzotriazol-1-yl)-1,1,3,3-hexafluorophosphate (HATU) (0.67 g), diisopropylethylamine (0.51 mL) and 3-oxa-8-azabicyclo[3.2.1]octane hydrochloride (230 mg), and the mixture was stirred overnight at room temperature. The reaction mixture was cooled to 0°C, saturated aqueous sodium hydrogencarbonate solution was added thereto, and the mixture was extracted with ethyl acetate. The obtained organic layer was washed successively with water and saturated brine, and dried over magnesium sulfate, and the solvent was evaporated under reduced pressure. The residue was purified by column chromatography (hexane/ethyl acetate) to give the title compound (270 mg).
¹H NMR (400 MHz, DMSO-d₆) δ 1.75-1.98 (4H, m), 2.29 (3H, s), 3.41-3.55 (2H, m), 3.61 (1H, brs), 3.66 (2H, s), 4.56 (1H, brs), 7.53 (1H, d, J = 0.7 Hz), 8.34 (1H, s).

### Reference Example 101

### 6-chloro-N,4-dimethyl-N-((3S)-tetrahydrofuran-3-yl)nicotinamide

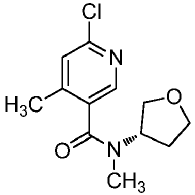

### A) 6-chloro-4-methyl-N-((3S)-tetrahydrofuran-3-yl)nicotinamide

The title compound (480 mg) was obtained using 6-chloro-4-methylnicotinic acid (370 mg) and (3S)-tetrahydrofuran-3-amine hydrochloride (320 mg), in the same manner as in the method of Reference Example 100.
¹H NMR (400 MHz, DMSO-d₆) δ 1.78-1.91 (1H, m), 2.15 (1H, dq, J = 12.7, 7.7 Hz), 2.35 (3H, s), 3.58 (1H, dd, J = 9.0, 3.9 Hz), 3.71 (1H, td, J = 8.1, 5.5 Hz), 3.76-3.88 (2H, m), 4.31-4.52 (1H, m), 7.48 (1H, s), 8.32 (1H, s), 8.75 (1H, d, J = 6.4 Hz). B) 6-chloro-N,4-dimethyl-N-((3S)-tetrahydrofuran-3-yl)nicotinamide

To a solution of 6-chloro-4-methyl-N-((3S)-tetrahydrofuran-3-yl)nicotinamide obtained in Step A of Reference Example 101 (480 mg) in N,N-dimethylformamide (4.0 mL) was added sodium hydride (60% in mineral oil, 57 mg) under ice-cooling, and the mixture was stirred for 30 min. To the reaction mixture was added methyl iodide (0.16 mL), and the mixture was stirred at room temperature for 3 hr. To the reaction mixture was added water, and the mixture was extracted with ethyl acetate. The obtained organic layer was washed with saturated brine, and dried over anhydrous magnesium sulfate, and the solvent was evaporated under reduced pressure. The residue was purified by column chromatography (NH, hexane/ethyl acetate) to give the title compound (230 mg).
MS (ESI+) : [M+H]⁺ 254.8.

### Reference Example 102

### 6-chloro-N,4-dimethyl-N-((3R)-tetrahydrofuran-3-yl)nicotinamide

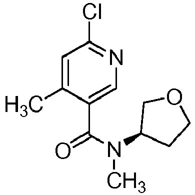

### A) 6-chloro-4-methyl-N-((3R)-tetrahydrofuran-3-yl)nicotinamide

The title compound (450 mg) was obtained using 6-chloro-4-methylnicotinic acid (500 mg) and (3R)-tetrahydrofuran-3-amine hydrochloride (430 mg), in the same manner as in the method of Reference Example 100.
¹H NMR (400 MHz, DMSO-d₆) δ 1.79-1.93 (1H, m), 2.15 (1H, dq, J = 12.7, 7.7 Hz), 2.35 (3H, s), 3.58 (1H, dd, J = 8.9, 3.8 Hz), 3.70 (1H, td, J = 8.2, 5.6 Hz), 3.77-3.93 (2H, m), 4.35-4.51 (1H, m), 7.48 (1H, s), 8.32 (1H, s), 8.75 (1H, d, J = 6.4 Hz). B) 6-chloro-N,4-dimethyl-N-((3R)-tetrahydrofuran-3-yl)nicotinamide

The title compound (300 mg) was obtained using 6-chloro-4-methyl-N-((3R)-tetrahydrofuran-3-yl)nicotinamide obtained in Step A of Reference Example 102 (450 mg) under ice-cooling, in the same manner as in the method of Step B of Reference

### Example 101.

MS (ESI+) : [M+H]⁺ 254.8.

### Reference Examples 103 to 106

The title compounds of Reference Examples 103 to 106 shown in Table 40 were obtained using 5-bromo-2-nitropyridine and the reagents corresponding to the compounds of Reference Examples 103 to 106 (these reagents can be produced according to a method known per se), in the same manner as in the method of Reference Example 50 or a method analogous thereto. MS in the tables means actual measured value.

**Table 40**

| Ref.No. | IUPAC Name | Structure | Salt | MS | Corresponding Reagent |
|---|---|---|---|---|---|
| 103 | (3S)-1-(5-aminopyridin-3-yl)piperidin-3-ol | 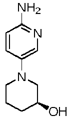 | Free | 193.8 | 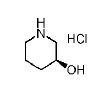 |
| 104 | 5-(8-oxa-3-azabicyclo[3.2.1]oct-3-yl)pyridin-2-amine | 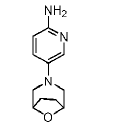 | Free | 205.9 | 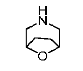 |
| 105 | 5-(6-oxa-3-azabicyclo[3.1.1]hept-3-yl)pyridin-2-amine | 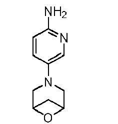 | Free | 191.9 | 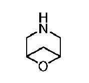 |
| 106 | 5-(3-oxa-8-azabicyclo[3.2.1]oct-8-yl)pyridin-2-amine | 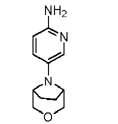 | Free | 205.9 | 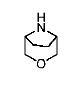 |

### Example 328

### 1-(4-((3S)-3-cyano-3-cyclopropyl-2-oxopyrrolidin-1-yl)pyridin-2-yl)-3-(pyridin-2-ylmethyl)urea

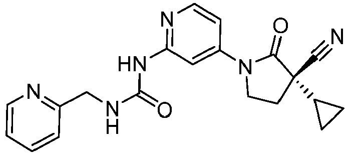

To a solution of (3S)-1-(2-aminopyridin-4-yl)-3-cyclopropyl-2-oxopyrrolidine-3-carbonitrile obtained in Example 2 (100 mg) in tetrahydrofuran (3 mL) was successively added triethylamine (0.069 mL) and 2,2,2-trichloroethyl chloroformate (0.068 mL) under ice bath, and the mixture was stirred at room temperature for 3 hr. To the reaction mixture was added saturated aqueous sodium hydrogencarbonate solution, and the mixture was extracted with ethyl acetate. The obtained organic layer was washed with saturated brine, and dried over magnesium sulfate, and the solvent was evaporated under reduced pressure.

To a solution of the residue in N,N-dimethyl sulfoxide (3 mL) were added diisopropylethylamine (0.087 mL) and 2-(aminomethyl)pyridine (0.045 mL), and the mixture was stirred at 80°C for 4 hr. To the reaction mixture was added saturated aqueous sodium hydrogencarbonate solution, and the mixture was extracted with ethyl acetate. The obtained organic layer was washed with saturated brine, and dried over magnesium sulfate, and the solvent was evaporated under reduced pressure. The residue was purified by column chromatography (NH, ethyl acetate/methanol) to give the title compound (13 mg).
MS(ESI+): [M+H]⁺ 377.1

### Examples 329 to 345

The title compounds of Examples 329 to 345 shown in Table 41-Table 42 were obtained using (3S)-1-(2-aminopyridin-4-yl)-3-cyclopropyl-2-oxopyrrolidine-3-carbonitrile obtained in Example 2 and the reagents corresponding to the compounds of Examples 329 to 345 (these reagents can be produced according to a method known per se), in the same manner as in the method of Example 328 or a method analogous thereto. MS in the tables means actual measured value.

**Table 41**

| Ex. No. | IUPAC Name | Structure | Salt | MS | Corresponding Reagent |
|---|---|---|---|---|---|
| 329 | 1-(4-((3S)-3-cyano-3-cyclopropyl-2-oxopyrrolidin-1-yl)pyridin-2-yl)-3-phenylurea | 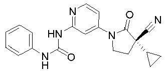 | free | 362.1 | 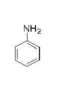 |
| 330 | 1-benzyl-3-(4-((3S)-3-cyano-3-cyclopropyl-2-oxopyrrolidin-1-yl)pyridin-2-yl)urea | 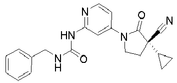 | free | 376.1 | 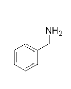 |
| 331 | 1-(4-((3S)-3-cyano-3-cyclopropyl-2-oxopyrrolidin-1-yl)l)yridin-2-yl)-3-(pyridin-3-ylmethyl)urea | 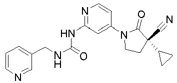 | free | 377.1 | 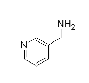 |
| 332 | 1-(4-((3S)-3-cyano-3-cyclopropyl-2-oxopyrrolidin-1-yl)pyridin-2-yl)-3-(pyridin-4-ylmethyl)urea | 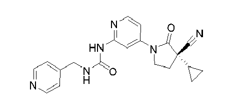 | free | 377.1 | 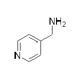 |
| 333 | 1-(4-((3S)-3-cyano-3-cyclopropyl-2-oxopyrrolidin-1-yl)pyridin-2-yl)-3-(2-phenylethyl)urea | 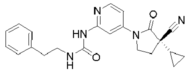 | free | 390.1 | 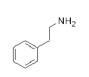 |
| 334 | 1-(1,3-benzoxazol-2-ylmethyl)-3-(4-((3S)-3-cyano-3-cyclopropyl-2-oxopyrrolidin-1-yl)pyridin-2-yl)urea | 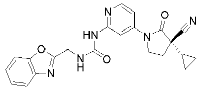 | free | 417.1 | 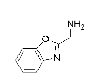 |
| 335 | 1-(2-(1H-benzimidazol-2-yl)ethyl)-3-(4-((3S)-3-cyano-3-cyclopropyl-2-oxopyrrolidin-1-yl)pyridin-2-yl)urea | 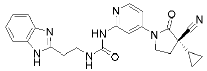 | free | 430.1 | 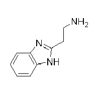 |
| 336 | 1-(4-((3S)-3-cyano-3-cyclopropyl-2-oxopyrrolidin-1-yl)l)yridin-2-yl)-3-(2-(1H-imidazol-4-yl)ethyl)urea | 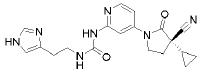 | free | 380.2 | 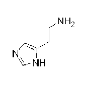 |
| 337 | 1-(1-benzofuran-2-ylmethyl)-3-(4-((3S)-3-cyano-3-cyclopropyl-2-oxopyrrolidin-1-yl)pyridin-2-yl)urea | 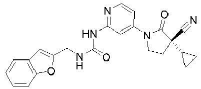 | free | 416.1 | 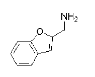 |

**Table 42**

| Ex. No. | IUPAC Name | Structure | Salt | MS | Corresponding Reagent |
|---|---|---|---|---|---|
| 338 | 1-(4-((3S)-3-cyano-3-cyclopropyl-2-oxopyrrolidin-1-yl)pyridin-2-yl)-3-(2-(3-oxopiperazin-1-yl)ethyl)urea | 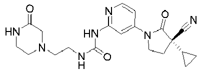 | free | 412.2 | 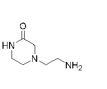 |
| 339 | 1-(4-((3S)-3-cyano-3-cyclopropyl-2-oxopyrrolidin-1-yl)pyridin-2-yl)-3-cyclohexylurea | 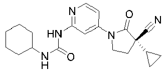 | free | 368.2 | 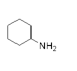 |
| 340 | 1-(4-((3S)-3-cyano-3-cyclopropyl-2-oxopyrrolidin-1-yl)pyridin-2-yl)-3-(tetrahydro-2H-pyran-4-yl)urea | 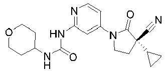 | free | 370.2 | 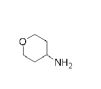 |
| 341 | 1-(4-((3S)-3-cyano-3-cyclopropyl-2-oxopyrrolidin-1-yl)pyridin-2-yl)-3-((1S)-1-phenylethyl)urea | 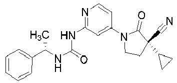 | free | 390.1 | 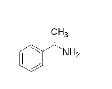 |
| 342 | 1-(4-((3S)-3-cyano-3-cyclopropyl-2-oxopyrrolidin-1-yl)pyridin-2-yl)-3-((1R)-1-phenylethyl)urea | 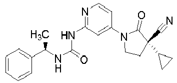 | free | 390.2 | 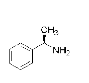 |
| 343 | 1-(4-((3S)-3-cyano-3-cyclopropyl-2-oxopyrrolidin-1-yl)pyridin-2-yl)-3-(2-hydroxybenzyl)urea | 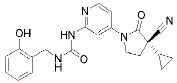 | free | 392.1 | 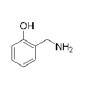 |
| 344 | 1-(4-((3S)-3-cyano-3-cyclopropyl-2-oxopyrrolidin-1-yl)pyridin-2-yl)-3-(3-hydroxybenzyl)urea | 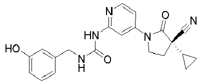 | free | 392.1 | 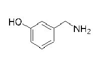 |
| 345 | 1-(4-((3S)-3-cyano-3-cyclopropyl-2-oxopyrrolidin-1-yl)pyridin-2-yl)-3-ethylurea | 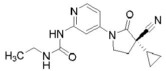 | free | 314.2 | 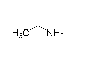 |

### Example 346

### N-(4-((3S)-3-cyano-3-cyclopropyl-2-oxopyrrolidin-1-yl)pyridin-2-yl)-4-fluorobenzamide

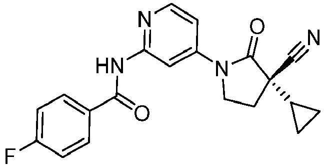

To a solution of (3S)-1-(2-aminopyridin-4-yl)-3-cyclopropyl-2-oxopyrrolidine-3-carbonitrile obtained in Example 2 (100 mg) in N,N-dimethylformamide (2 mL) were added triethylamine (0.086 mL), 4-fluorobenzoic acid (64 mg) and HATU (190 mg), and the mixture was stirred overnight at room temperature. To the reaction mixture was added saturated aqueous sodium hydrogencarbonate solution, and the mixture was extracted with ethyl acetate. The obtained organic layer was washed with saturated brine, and dried over magnesium sulfate, and the solvent was evaporated under reduced pressure. The residue was purified by column chromatography (NH, hexane/ethyl acetate) to give the title compound (32 mg).
MS (ESI+) : [M+H]⁺ 365.1

### Examples 347 to 349

The title compounds of Examples 347 to 349 shown in Table 43 were obtained using (3S)-1-(2-aminopyridin-4-yl)-3-cyclopropyl-2-oxopyrrolidine-3-carbonitrile obtained in Example 2 and the reagents corresponding to the compounds of Examples 347 to 349 (these reagents can be produced according to a method known per se), in the same manner as in the method of Example 346 or a method analogous thereto. MS in the tables means actual measured value.

**Table 43**

| Ex. No. | IUPAC Name | Structure | Salt | MS | Corresponding Reagent |
|---|---|---|---|---|---|
| 347 | N-(4-((3S)-3-cyano-3-cyclopropyl-2-oxopyrrolidin-1-yl)pyridin-2-yl)-1-methyl-1H-pyrazole-4-carboxamide | 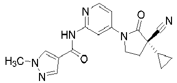 | free | 351.1 | 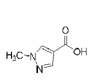 |
| 348 | N-(4-((3S)-3-cyano-3-cyclopropyl-2-oxopyrrolidin-1-yl)pyridin-2-yl)-3-phenylpropanamide | 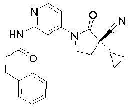 | free | 375.1 | 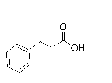 |
| 349 | N-(4-((3S)-3-cyano-3-cyclopropyl-2-oxopyrrolidin-1-yl)pyridin-2-yl)-2-(morpholin-4-yl)acetamide | 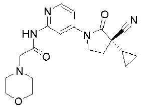 | free | 370.1 | 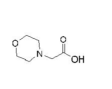 |

### Examples 350 to 362

The title compounds of Examples 350 to 362 shown in Table 44-Table 45 were obtained using 6-((4-((3S)-3-cyano-3-cyclopropyl-2-oxopyrrolidin-1-yl)pyridin-2-yl)amino)-4-methylnicotinic acid dihydrochloride obtained in Step C of Example 320 and the reagents corresponding to the compounds of Examples 350 to 362 (these reagents can be produced according to a method known per se), in the same manner as in the method of Example 246 or a method analogous thereto. MS in the tables means actual measured value.

**Table 44**

| Ex. No. | IUPAC Name | Structure | Salt | MS | Corresponding Reagent |
|---|---|---|---|---|---|
| 350 | 6-((4-((3S)-3-eyano-3-cyclopropyl-2-oxopyrrolidin-1-yl)pyridin-2-yl)amino)-N-ethyl-4-methylnicotinamide | 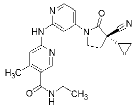 | free | 405.0 | 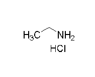 |
| 351 | (3S)-3-cyclopropyl-1-(2-((5-((3-methoxyazetidin-1-yl)carbonyl)-4-methylpyridin-2-yl)amino)pyridin-4-yl)-2-oxopyrrolidine-3-carbonitrile | 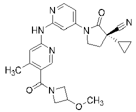 | free | 447.0 | 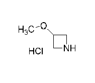 |
| 352 | (3S)-3-cyclopropyl-1-(2-((5-(((3R)-3-methoxypyrrolidin-1-yl)carbonyl)-4-methylpyridin-2-yl)amino)pyridin-4-yl)-2-oxopyrrolidine-3-carbonitrile | 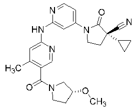 | free | 461.1 | 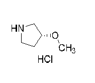 |
| 353 | (3S)-3-cyclol)ropyl-l-(2-((5-(((3S)-3-methoxypyrrolidin-1-yl)carbonyl)-4-methylpyridin-2-yl)amino)pyridin-4-yl)-2-oxopyrrolidine-3-carbonitrile | 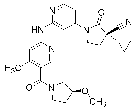 | free | 461.1 | 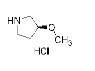 |
| 354 | 5-((4-((3S)-3-cyano-3-cyclopropyl-2-oxopyrrolidin-1-yl)pyridin-2-yl)amino)-N-(2-methoxyethyl)-4-methylnicotinamide | 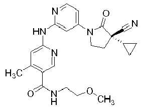 | free | 435.0 | 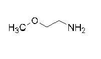 |
| 355 | 6-((4-((3S)-3-cyano-3-cyclopropyl-2-oxopyrrolidin-1-yl)pyridin-2-yl)amino)-N-(2-(dimethylamino)ethyl)-4-methylnicotinamide | 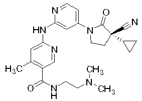 | free | 448.1 | 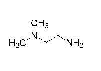 |
| 356 | 5-((4-((3S)-3-cyano-3-cyclopropyl-2-oxopyrrolidin-1-yl)pyridin-2-yl)amino)-4-methyl-N-(1-methyl-1H-pyrazol-4-yl)nicotinamide | 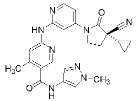 | free | 457.1 | 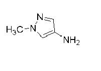 |
| 357 | (3S)-3-cyclopropyl-1-(2-((4-methyl-5-((4-methylpiperazin-1-yl)carbonyl)pyridin-2-yl)amino)pyridin-4-yl)-2-oxopyrrolidine-3-carbonitrile | 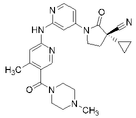 | free | 460.1 | 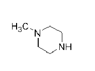 |
| 358 | 6-((4-((3S)-3-eyano-3-cyclopropyl-2-oxopyrrolidin-1-yl)pyridin-2-yl)amino)-N-(2-(dimethylamino)ethyl)-N,4-dimethylnicotinamide | 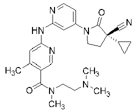 | free | 462.1 | 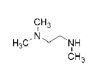 |

**Table 45**

| Ex. No. | IUPAC Name | Structure | Salt | MS | Corresponding Reagent |
|---|---|---|---|---|---|
| 359 | 6-((4-((3S)-3-cyano-3-cyclopropyl-2-oxopyrrolidin-1-yl)pyridin-2-yl)amino)-N-cyclohexyl-4-methylnicotinamide | 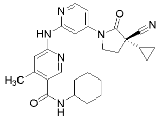 | free | 459.2 | 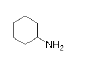 |
| 360 | 6-((4-((3S)-3-cyano-3-cyclopropyl-2-oxopyrrolidin-1-yl)pyridin-2-yl)amino)-N,4-dimethyl-N-((1S, 2S)-(methylamino)cyclohexyl)nicotinamid e | 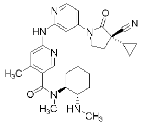 | free | 502.4 | 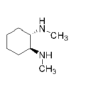 |
| 361 | N-((1SR, 2RS)-2-aminocyclohexyl)-6-((4-((3S)-3-cyano-3-cyclopropyl-2-oxopyrrolidin-1-yl)pyridin-2-yl)amino)-4-methylnicotinamide | 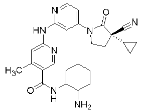 | free | 474.2 | 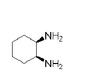 |
| 362 | 6-((4-((3S)-3-cyano-3-cyclopropyl-2-oxopyrrolidin-1-yl)pyridin-2-yl)amino)-N,4-dimethyl-N-((1R, 2R)-2-(methylamino)cyclohexyl)nicotinamid e | 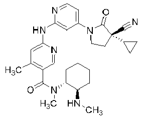 | free | 502.3 | 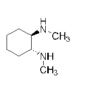 |

### Example 363

### 6-((4-((3S)-3-cyano-3-cyclopropyl-2-oxopyrrolidin-1-yl)pyridin-2-yl)amino)-4-methyl-N-(pyrrolidin-3-yl)nicotinamide hydrochloride

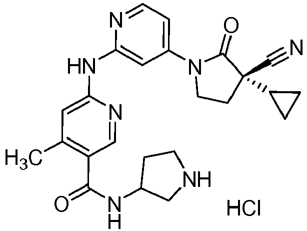

To a solution of 6-((4-((3S)-3-cyano-3-cyclopropyl-2-oxopyrrolidin-1-yl)pyridin-2-yl)amino)-4-methylnicotinic acid dihydrochloride obtained in Step C of Example 320 (100 mg) in N,N-dimethylacetamide (3 mL) were added tert-butyl 3-aminopyrrolidine-1-carboxylate (41 mg), diisopropylethylamine (0.10 mL) and HATU (130 mg), and the mixture was stirred overnight at room temperature. To the reaction mixture was added saturated aqueous sodium hydrogencarbonate solution, and the mixture was extracted with ethyl acetate. The obtained organic layer was washed with saturated brine, and dried over magnesium sulfate, and the solvent was evaporated under reduced pressure. The residue was purified by column chromatography (NH, ethyl acetate). To the residue was added 4 M hydrogen chloride ethyl acetate (3 mL) solution, and the mixture was stirred for 5 min, and the solvent was evaporated under reduced pressure. The residue was solidified (hexane/ethyl acetate) to give the title compound (100 mg). MS (ESI+) : [M+H]⁺ 446.1

### Example 364

### N-((1RS,2RS)-2-aminocyclohexyl)-6-((4-((3S)-3-cyano-3-cyclopropyl-2-oxopyrrolidin-1-yl)pyridin-2-yl)amino)-4-methylnicotinamide hydrochloride

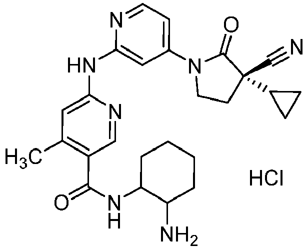

The title compound (110 mg) was obtained using 6-((4-((3S)-3-cyano-3-cyclopropyl-2-oxopyrrolidin-1-yl)pyridin-2-yl)amino)-4-methylnicotinic acid dihydrochloride obtained in Step C of Example 320 (100 mg) and tert-butyl ((1RS, 2RS)-2-aminocyclohexyl)carbamate (52 mg), in the same manner as in the method of Example 363.
MS(ESI+): [M+H]⁺ 474.1

### Example 365

### 6-((4-((3S)-3-cyano-3-cyclopropyl-2-oxopyrrolidin-1-yl)pyridin-2-yl)amino)-N,N-dimethyl-4-(trifluoromethyl)nicotinamide

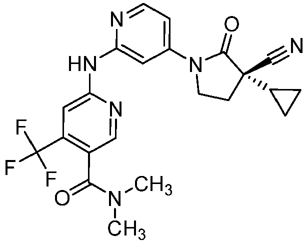

### A) 6-chloro-N,N-dimethyl-4-(trifluoromethyl)nicotinamide

To a solution of 6-chloro-4-(trifluoromethyl)nicotinic acid (100 mg) in N,N-dimethylacetamide (20 mL) were added dimethylamine hydrochloride (54 mg), HATU (250 mg) and N,N-diisopropylethylamine (0.16 mL), and the mixture was stirred overnight at room temperature. To the reaction mixture was added saturated aqueous sodium bicarbonate solution, and the mixture was extracted with ethyl acetate. The obtained organic layer was washed with water and saturated brine, and dried over magnesium sulfate, and the solvent was evaporated under reduced pressure. The residue was purified by column chromatography (hexane/ethyl acetate) to give the title compound (110 mg).
MS (ESI+) : [M+H]⁺ 252.8

### B) 6-((4-((3S)-3-cyano-3-cyclopropyl-2-oxopyrrolidin-1-yl)pyridin-2-yl)amino)-N,N-dimethyl-4-(trifluoromethyl)nicotinamide

The title compound (98 mg) was obtained using 6-chloro-N,N-dimethyl-4-(trifluoromethyl)nicotinamide obtained in Step A of Example 365 (100 mg) and (3S)-1-(2-aminopyridin-4-yl)-3-cyclopropyl-2-oxopyrrolidine-3-carbonitrile obtained in Example 2 (87 mg), in the same manner as in the method of Example 31.
MS (ESI+) : [M+H]⁺ 459.1

### Example 366

### 6-((4-((3S)-3-cyano-3-cyclopropyl-2-oxopyrrolidin-1-yl)pyridin-2-yl)amino)-4-(trifluoromethyl)nicotinamide

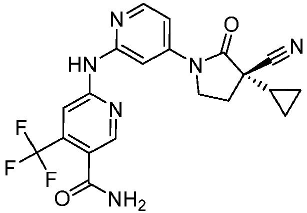

### A) tert-butyl 6-chloro-4-(trifluoromethyl)nicotinate

To a solution of 6-chloro-4-(trifluoromethyl)nicotinic acid (300 mg) in toluene (5.0 mL) was added N,N-dimethylformamide di-tert-butyl acetal (1.1 mL), and the mixture was stirred at 100°C for 3 days. To the reaction mixture was added N,N-dimethylformamide di-tert-butyl acetal (0.94 mL), and the mixture was stirred at 100°C for 2 hr. The solvent was evaporated under reduced pressure, and the residue was purified by column chromatography (NH, hexane/ethyl acetate) to give the title compound (240 mg).
MS (ESI+) : [M+H]⁺ 281.8

### B) (3S)-tert-butyl 6-((4-(3-cyano-3-cyclopropyl-2-oxopyrrolidin-1-yl)pyridin-2-yl)amino)-4-(trifluoromethyl)nicotinate

The title compound (260 mg) was obtained using tert-butyl 6-chloro-4-(trifluoromethyl)nicotinate obtained in Step A of Example 366 (240 mg) and (3S)-1-(2-aminopyridin-4-yl)-3-cyclopropyl-2-oxopyrrolidine-3-carbonitrile obtained in Example 2 (210 mg) in the same manner as in the method of Example 31.
MS (ESI+) : [M+H]⁺ 488.2

### C) (3S)-6-((4-(3-cyano-3-cyclopropyl-2-oxopyrrolidin-1-yl)pyridin-2-yl)amino)-4-(trifluoromethyl)nicotinic acid trifluoroacetate

To (3S)-tert-butyl 6-((4-(3-cyano-3-cyclopropyl-2-oxopyrrolidin-1-yl)pyridin-2-yl)amino)-4-(trifluoromethyl)nicotinate obtained in Step B of Example 366 (260 mg) was added 4 M hydrogen chloride ethyl acetate (5.0 mL) solution, and the mixture was stirred at 80°C for 2 days. To the reaction mixture was added trifluoroacetic acid (2.0 mL), and the mixture was stirred at 80°C for 5 hr, and the solvent was evaporated under reduced pressure. The residue was crystallized (ethyl acetate/diisopropyl ether) to give the title compound (200 mg).
MS (ESI+) : [M+H]⁺ 432.0

### D) 6-((4-((3S)-3-cyano-3-cyclopropyl-2-oxopyrrolidin-1-yl)pyridin-2-yl)amino)-4-(trifluoromethyl)nicotinamide

The title compound (5.0 mg) was obtained using (3S)-6-((4-(3-cyano-3-cyclopropyl-2-oxopyrrolidin-1-yl)pyridin-2-yl)amino)-4-(trifluoromethyl)nicotinic acid trifluoroacetate obtained in Step C of Example 366 (100 mg), in the same manner as in the method of Example 108.
MS (ESI+) : [M+H]⁺ 430.9

### Example 367

### 6-((4-((3S)-3-cyano-3-cyclopropyl-2-oxopyrrolidin-1-yl)pyridin-2-yl)amino)-N-methyl-4-(trifluoromethyl)nicotinamide

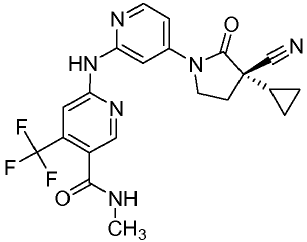

The title compound (37 mg) was obtained using (3S)-6-((4-(3-cyano-3-cyclopropyl-2-oxopyrrolidin-1-yl)pyridin-2-yl)amino)-4-(trifluoromethyl)nicotinic acid trifluoroacetate obtained in Step C of Example 366 (100 mg) and methylamine hydrochloride (27 mg), in the same manner as in the method of Step B of Example 5.
MS (ESI+) : [M+H]⁺ 445.0.

### Examples 368 to 369

The title compounds of Examples 368 to 369 shown in Table 46 were obtained using 6-((4-((3S)-3-cyano-3-cyclopropyl-2-oxopyrrolidin-1-yl)pyridin-2-yl)amino)-4-methylnicotinic acid dihydrochloride obtained in Step C of Example 320 and the reagents corresponding to the compounds of Examples 368 to 369 (these reagents can be produced according to a method known per se), in the same manner as in the method of Example 246 or a method analogous thereto. MS in the tables means actual measured value.

**Table 46**

| Ex. No. | IUPAC Name | Structure | Salt | MS | Corresponding Reagent |
|---|---|---|---|---|---|
| 368 | 6-((4-((3S)-3-cyano-3-cyclopropyl-2-oxopyrrolidin-1-yl)pyridin-2-yl)amino)-4-methyl-N-(2,2,2-trifluoroethyl)nicotinamide | 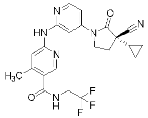 | free | 459.1 | 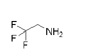 |
| 369 | 6-((4-((3S)-3-cyano-3-cyclopropyl-2-oxopyrrolidin-1-yl)pyridin-2-yl)amino)-N-(2,2-difluoroethyl)-4-methylnicotinamide | 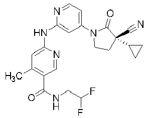 | free | 441.1 | 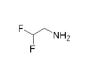 |

### Example 370

### 6-((4-(3-cyano-3-ethyl-2-oxopyrrolidin-1-yl)pyridin-2-yl)amino)-4-methylnicotinic acid hydrochloride (optically active compound)

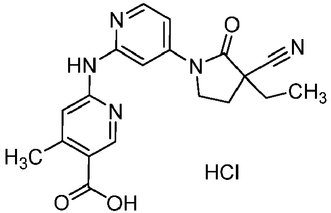

### A) (R or S)-3-ethyl-2-oxopyrrolidine-3-carbonitrile

3-ethyl-2-oxopyrrolidine-3-carbonitrile obtained in Step B of Example 125 (1.1 g) was resolved by HPLC (column: CHIRALPAK AS-H, 20 mmID×250 mmL, manufactured by Daicel Chemical Industries, mobile phase: carbon dioxide/2-propanol = 860/140) to give the title compound (510 mg: shorter retention time).
>99.9% ee (HPLC (column: CHIRALPAK AS-H, 4.6 mmID×150 mmL, manufactured by Daicel Chemical Industries, mobile phase: carbon dioxide/2-propanol= 820/180, flow rate: 4.0 mL/min, retention time: 1.49 min))
MS (ESI+) : [M+H]⁺ 139.3

### B) 1-(2-aminopyridin-4-yl)-3-ethyl-2-oxopyrrolidine-3-carbonitrile (optically active compound)

The title compound (730 mg) was obtained using 3-ethyl-2-oxopyrrolidine-3-carbonitrile obtained in Step A of Example 370 (optically active compound, 490 mg) and 4-iodopyridin-2-amine (780 mg), in the same manner as in the method of Example 2.
MS(ESI+): [M+H]⁺ 230.8

### C) tert-butyl 6-((4-(3-cyano-3-ethyl-2-oxopyrrolidin-1-yl)pyridin-2-yl)amino)-4-methylnicotinate (optically active compound)

The title compound (1.0 g) was obtained using 1-(2-aminopyridin-4-yl)-3-ethyl-2-oxopyrrolidine-3-carbonitrile obtained in Step B of Example 370 (optically active compound, 730 mg) and tert-butyl 6-chloro-4-methylnicotinate obtained in Step A of Example 320 (720 mg), in the same manner as in the method of Example 3.
MS (ESI+) : [M+H]⁺ 422.1

### D) 6-((4-(3-cyano-3-ethyl-2-oxopyrrolidin-1-yl)pyridin-2-yl)amino)-4-methylnicotinic acid hydrochloride (optically active compound)

To a solution of tert-butyl 6-((4-(3-cyano-3-ethyl-2-oxopyrrolidin-1-yl)pyridin-2-yl)amino)-4-methylnicotinate obtained in Step C of Example 370 (optically active compound, 1.0 g) in acetic acid (5 mL) was added 6 M hydrochloric acid (1.2 mL), and the mixture was stirred at 50°C for 1 hr, and then at 65°C for 4 hr. The reaction mixture was allowed to be cooled to room temperature, ethyl acetate (15 mL) was added thereto, and the mixture was stirred at 0°C for 30 min. The resulting solid was collected by filtration, and washed with ethyl acetate to give the title compound (760 mg).
MS(ESI+): [M+H]⁺ 366.1

### Example 371

### 6-((4-(3-cyano-3-ethyl-2-oxopyrrolidin-1-yl)pyridin-2-yl)amino)-4-methylnicotinic acid hydrochloride (optically active compound opposite to the compound obtained in Example 370)

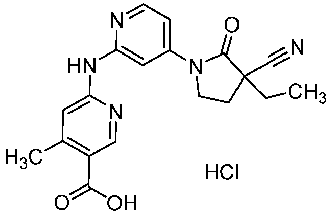

### A) 3-ethyl-2-oxopyrrolidine-3-carbonitrile (optically active compound opposite to the compound obtained in Step A of

### Example 370)

3-ethyl-2-oxopyrrolidine-3-carbonitrile obtained in Step B of Example 125 (1.1 g) was resolved by HPLC (column: CHIRALPAK AS-H, 20 mmID×250 mmL, manufactured by Daicel Chemical Industries, mobile phase: carbon dioxide/2-propanol = 860/140) to give the title compound (480 mg: longer retention time).
>99.9% ee (HPLC (column: CHIRALPAK AS-H, 4.6 mmID×150 mmL, manufactured by Daicel Chemical Industries, mobile phase: carbon dioxide/2-propanol= 820/180, flow rate: 4.0 mL/min, retention time: 2.23 min))
MS (ESI+) : [M+H]⁺ 139.3

### B) 1-(2-aminopyridin-4-yl)-3-ethyl-2-oxopyrrolidine-3-carbonitrile (optically active compound opposite to the compound obtained in Step B of Example 370)

The title compound (400 mg) was obtained using 3-ethyl-2-oxopyrrolidine-3-carbonitrile obtained in Step A of Example 371 (optically active compound opposite to the compound obtained in Step A of Example 370, 480 mg) and 4-iodopyridin-2-amine (760 mg), in the same manner as in the method of Example 2.
MS(ESI+): [M+H]⁺ 230.8

### C) tert-butyl 6-((4-(3-cyano-3-ethyl-2-oxopyrrolidin-1-yl)pyridin-2-yl)amino)-4-methylnicotinate (optically active compound opposite to the compound obtained in Step C of

### Example 370)

The title compound (460 mg) was obtained using 1-(2-aminopyridin-4-yl)-3-ethyl-2-oxopyrrolidine-3-carbonitrile obtained in Step B of Example 371 (optically active compound opposite to the compound obtained in Step B of Example 370, 400 mg) and tert-butyl 6-chloro-4-methylnicotinate obtained in Step A of Example 320 (400 mg), in the same manner as in the method of Example 3.
MS (ESI+) : [M+H]⁺ 422.1

### D) 6-((4-(3-cyano-3-ethyl-2-oxopyrrolidin-1-yl)pyridin-2-yl)amino)-4-methylnicotinic acid hydrochloride (optically active compound opposite to the compound obtained in Example 370)

The title compound (260 mg) was obtained using tert-butyl 6-((4-(3-cyano-3-ethyl-2-oxopyrrolidin-1-yl)pyridin-2-yl)amino)-4-methylnicotinate obtained in Step C of Example 371 (optically active compound opposite to the compound obtained in Step C of Example 370, 460 mg), in the same manner as in the method of Step C of Example 370.
MS (ESI+) : [M-H]⁻ 364.1

### Example 372

### (R or S)-6-((4-(3-cyano-3-ethyl-2-oxopyrrolidin-1-yl)pyridin-2-yl)amino)-N,N,4-trimethylnicotinamide (optically active compound)

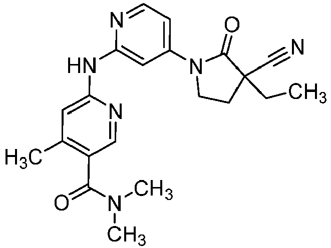

The title compound (75 mg) was obtained using 6-((4-(3-cyano-3-ethyl-2-oxopyrrolidin-1-yl)pyridin-2-yl)amino)-4-methylnicotinic acid hydrochloride obtained in Example 370 (optically active compound, 100 mg) and dimethylamine hydrochloride (61 mg), in the same manner as in the method of Example 246.
MS (ESI+) : [M+H]⁺ 393.2.

### Example 373

### (R or S)-6-((4-(3-cyano-3-ethyl-2-oxopyrrolidin-1-yl)pyridin-2-yl)amino)-N,N,4-trimethylnicotinamide (optically active compound opposite to the compound obtained in Example 372)

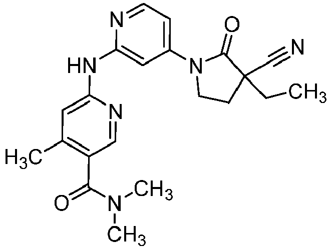

The title compound (65 mg) was obtained using 6-((4-(3-cyano-3-ethyl-2-oxopyrrolidin-1-yl)pyridin-2-yl)amino)-4-methylnicotinic acid hydrochloride obtained in Example 371 (optically active compound opposite to the compound obtained in Example 371, 80 mg) and dimethylamine hydrochloride (49 mg), in the same manner as in the method of Example 246.
MS (ESI+) : [M+H]⁺ 393.2.

### Examples 374 to 388

The title compounds of Examples 374 to 388 shown in Table 47-Table 48 were obtained using (3S)-1-(2-bromopyridin-4-yl)-3-cyclopropyl-2-oxopyrrolidine-3-carbonitrile obtained in Step E of Example 1 and the reagents corresponding to the compounds of Examples 374 to 388 (these reagents can be produced according to a method known per se), in the same manner as in the method of Example 179 or a method analogous thereto. MS in the tables means actual measured value.

**Table 47**

| Ex. No. | IUPAC Name | Structure | Salt | MS | Corresponding Reagent |
|---|---|---|---|---|---|
| 374 | (3S)-3-cyclopropyl-2-oxo-1-(2-((5-(1-(2,2,2-trifluoroet.hyl)piperidin-4-yl)pyridin-2-yl)amino)pyridin-4-yl)pyrrolidine-3-carbonitrile | 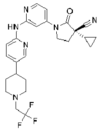 | free | 485.1 | 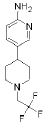 |
| 375 | (3S)-3-cyclopropyl-1-(2-((5-(1-methylpiperidin-4-yl)pyridin-2-yl)amino)pyridin-4-yl)-2-oxopyrrolidine-3-carbonitrile | 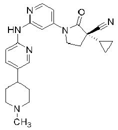 | free | 417.1 | 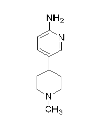 |
| 376 | (3S)-3-cyclopropyl-2-oxo-1-(2-((5-(1-(pyrimidin-2-yl)piperidin-4-yl)pyridin-2-yl)amino)pyridin-4-yl)pyrrolidine-3-carbonitrile | 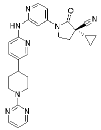 | free | 481.1 | 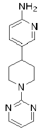 |
| 377 | (3S)-3-cyclopropyl-1-(2-((5-(1-methyl-1H-pyrazol-5-yl)pyridin-2-yl)amino)pyridin-4-yl)-2-oxopyrrolidine-3-carbonitrile | 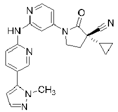 | free | 400.2 | 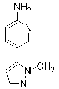 |
| 378 | (3S)-3-cyclopropyl-1-(2-(((5- (3,5-dimethyl-1,2-oxazol-4-yl)pyridin-2-yl)amino)pyridin-4-yl)-2-oxopyrrolidine-3-carbonitrile | 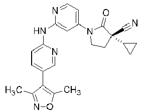 | free | 415.0 | 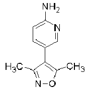 |
| 379 | (3S)-3-cyclopropyl-1-(2-(5-(3S)-3-methoxypiperidin-1-yl)pyridin-2-yl)amino)pyridin-4-yl)-2-oxopyrrolidine-3-carbonitrile | 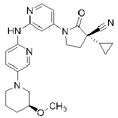 | free | 433.0 | 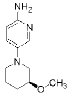 |
| 380 | (3S)-3-cyclopropyl-1-(2-(5-(methyl(tetrahydro-2H-pyran-4-yl)amino)pyridin-2-yl)amino)pyridin-4-yl)-2-oxopyrrolidine-3-carbonitrile | 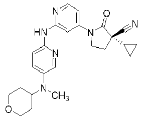 | free | 433.0 | 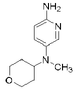 |
| 381 | (3S)-3-cyclopropyl-1-(2-(5-(2-oxa-6-azaspiro[3.5]non-6-yl)pyridin-2-yl)amino)pyridin-4-yl)-2-oxopyrrolidine-3-carbonitrile | 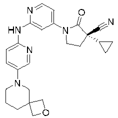 | free | 445.1 | 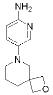 |

**Table 48**

| Ex. No. | IUPAC Name | Structure | Salt | MS | Corresponding Reagent |
|---|---|---|---|---|---|
| 382 | (3S)-3-cyclopropyl-1-(2-((5-(2-oxa-7-azaspiro[4.5]dec-7-yl)pyridin-2-yl)amino)pyridin-4-yl)-2-oxopyrrolidine-3-carbonitrile | 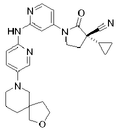 | free | 459.2 | 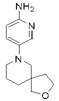 |
| 383 | (3S)-3-cyclopropyl-1-(2-((5-(3-methoxyazetidin-1-yl)ppyridin-2-yl)amino)pyridin-4-yl)-2-oxopyrrolidine-3-carbonitrile | 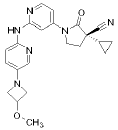 | free | 405.1 | 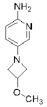 |
| 384 | (3S)-3-cyclopropyl-1-(2-((5-(4-methylpiperazin-1-yl)pyridin-2-yl)amino)pyridin-4-yl)-2-oxopyrrolidine-3-carbonitrile | 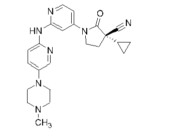 | free | 418.0 | 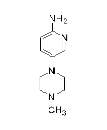 |
| 385 | (3S)-3-cyclopropyl-1-(2-(5-(4-methyl-1,4-diazepan-1-yl)pyridin-2-yl)amino)pyridin-4-yl)-2-oxopyrrolidine-3-carbonitrile | 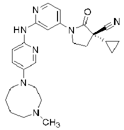 | free | 432.2 | 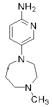 |
| 386 | (3S)-3-cyclopropyl-1-(2-((5-(3-(dimethylamino)pyrrolidin-1-yl)pyridin-2-yl)amino)pyridin-4-yl)-2-oxopyrrolidine-3-carbonitrile | 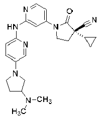 | free | 432.2 | 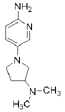 |
| 387 | 2-((4-((3S)-3-cyano-3-cyclopropyl-2-oxopyrrolidin-1-yl)pyridin-2-yl)amino)-5-(morpholin-4-yl)isonicotinonitrile | 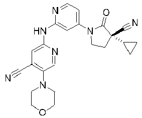 | free | 430.1 | 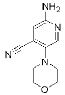 |
| 388 | (3S)-3-cyclopropyl-1-(2-((4-methyl-5-(4-methyl-1,4-diazepan-1-yl)pyridin-2-yl)amino)pyridin-4-yl)-2-oxopyrrolidine-3-carbonitrile | 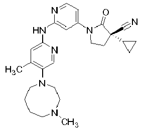 | free | 446.2 | 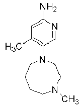 |

### Examples 390 to 417

The title compounds of Examples 390 to 417 shown in Table 49-Table 51 were obtained using (3S)-1-(2-aminopyridin-4-yl)-3-cyclopropyl-2-oxopyrrolidine-3-carbonitrile obtained in Example 2 and the reagents corresponding to the compounds of Examples 390 to 417 (these reagents can be produced according to a method known per se), in the same manner as in the method of Example 187 or a method analogous thereto. MS in the tables means actual measured value.

**Table 49**

| Ex. No. | IUPAC Name | Structure | Salt | MS | Corresponding Reagent |
|---|---|---|---|---|---|
| 390 | (3S)-3-cyclopropyl-1-(2-((6-(morpholin-4-yl)pyridazin-3-yl)amino)pyridin-4-yl)-2-oxopyrrolidine-3-carbonitrile | 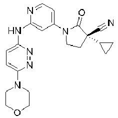 | free | 406.0 | 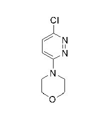 |
| 391 | (3S)-3-cyclopropyl-1-(2-((6-(hydroxymethyl)pyridin-2-yl)amino)pyridin-4-yl)-2-oxopyrrolidine-3-carbonitrile | 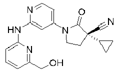 | free | 350.0 | 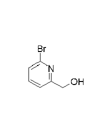 |
| 392 | (3S)-1-(2-((6-(cyanomethyl)pyridin-2-yl)amino)pyridin-4-yl)-3-cyclopropyl-2-oxopyrrolidine-3-carbonitrile | 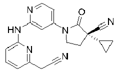 | free | 359.1 | 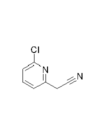 |
| 393 | N-((6-((4-((3S)-3-cyano-3-cyclopropyl-2-oxopyrrolidin-1-yl)pyridin-2-yl)amino)pyridin-2-yl)methyl)acetamide | 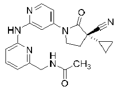 | free | 391.1 | 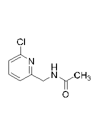 |
| 394 | N-((6-((4-((3S)-3-cyano-3-cyclopropyl-2-oxopyrrolidin-1-yl)pyridin-2-yl)amino)pyridin-2-yl)methyl)methanesulfonamide | 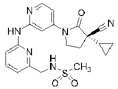 | free | 427.2 | 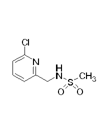 |
| 395 | (3S)-3-cyclopropyl-1-(2-((6-(2-hydroxypropan-2-yl)pyridin-2-yl)amino)pyridin-4-yl)-2-oxopyrrolidine-3-carbonitrile | 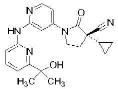 | free | 378.2 | 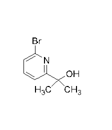 |
| 396 | N-(6-((4-((3S)-3-cyano-3-cyclopropyl-2-oxopyrrolidin-1-yl)pyridin-2-yl)amino)pyridin-2-yl)-N-methylacetamide | 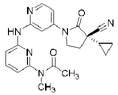 | free | 391.1 | 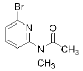 |
| 397 | N-((6-((4-((3S)-3-cyano-3-cyclopropyl-2-oxopyrrolidin-1-yl)pyridin-2-yl)amino)pyridin-2-yl)methyl)-N-methylacetamide | 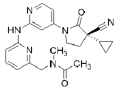 | free | 405.2 | 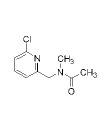 |
| 398 | N-((5-((4-((3S)-3-cyano-3-cyclopropyl-2-oxopyrrolidin-1-yl)pyridin-2-yl)amino)pyridin-2-yl)methyl)-N-methylmethanesulfonamide | 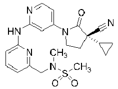 | free | 441.1 | 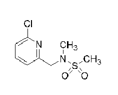 |
| 399 | (3S)-3-cyclopropyl-1-(2-((6-(methoxymethyl)pyridin-2-yl)amino)pyridin-4-yl)-2-oxopyrrolidine-3-carbonitrile | 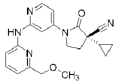 | free | 364.2 | 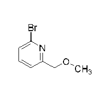 |

**Table 50**

| Ex. No. | IUPAC Name | Structure | Salt | MS | Corresponding Reagent |
|---|---|---|---|---|---|
| 400 | (3S)-3-cyclopropyl-1-(2-((6-(2-methoxypropan-2-yl)pyridin-2-yl)amino)pyridin-4-yl)-2-oxopyrrolidine-3-carbonitrile | 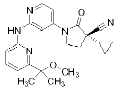 | free | 392.2 | 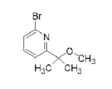 |
| 401 | N-(2-(6-((4-((3S)-3-cyano-3-cyclopropyl-2-oxopyrrolidin-1-yl)pyridin-2-yl)amino)pyridin-2-yl)propan-2-yl)acetamide | 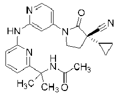 | free | 419.2 | 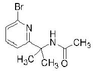 |
| 402 | N-(2-(6-((4-((3S)-3-cyano-3-cyclopropyl-2-oxopyrrolidin-1-yl)pyridin-2-yl)amino)pyridin-2-yl)propan-2-yl)-N-methylacetamide | 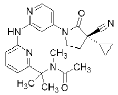 | free | 433.2 | 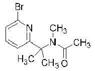 |
| 403 | (3S)-1-(2-((6-tert-butoxypyridin-2-yl)amino)pyridin-4-yl)-3-cyclopropyl-2-oxopyrrolidine-3-carbonitrile | 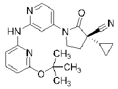 | free | 392.2 | 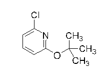 |
| 404 | 6-((4-((3S)-3-cyano-3-cyclopropyl-2-oxopyrrolidin-1-yl)pyridin-2-yl)amino)pyridine-2-carbonitrile | 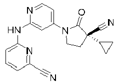 | free | 343.1 [M-H]⁻ | 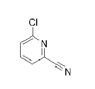 |
| 405 | (3S)-3-cyclopropyl-1-(2-((6-fluoropyridin-2-yl)amino)pyridin-4-yl)-2-oxopyrrolidine-3-carbonitrile | 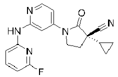 | free | 338.1 | 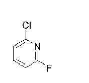 |
| 406 | (3S)-3-cyclopropyl-1-(2-((5-(morpholin-4-yl)pyridin-2-yl)amino)pyridin-4-yl)-2-oxopyrrolidine-3-carbonitrile | 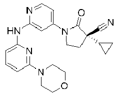 | free | 405.2 | 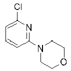 |
| 407 | (3S)-1-(2-((6-(benzyloxy)pyridin-2-yl)amino)pyridin-4-yl)-3-cyclopropyl-2-oxopyrrolidine-3-carbonitrile | 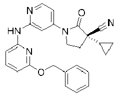 | free | 426.2 | 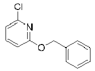 |
| 408 | (3S)-3-cyclopropyl-1-(2-((6-cyclopropylpyridin-2-yl)amino)pyridin-4-yl)-2-oxopyrrolidine-3-carbonitrile | 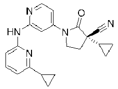 | free | 360.1 | 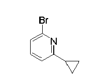 |
| 409 | (3S)-1-(2-((6-acetylpyridin-2-yl)amino)pyridin-4-yl)-3-cyclopropyl-2-oxopyrrolidine-3-carbonitrile | 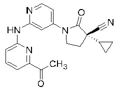 | free | 362.1 | 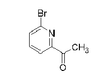 |

**Table 51**

| Ex. No. | IUPAC Name | Structure | Salt | MS | Corresponding Reagent |
|---|---|---|---|---|---|
| 410 | (3S)-3-cyclopropyl-1-(2-((6-ethoxypyridin-2-yl)amino)pyridin-4-yl)-2-oxopyrrolidine-3-carbonitrile | 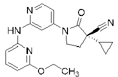 | free | 364.2 | 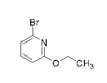 |
| 411 | (3S)-3-cyclopropyl-1-(2-((6-isopropoxypyridin-2-yl)amino)pyridin-4-yl)-2-oxopyrrolidine-3-carbonitrile | 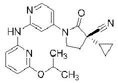 | free | 378.1 | 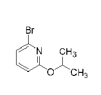 |
| 412 | (3S)-3-cyclopropyl-1(2-((6-(cyclopropylamino)pyridin-2-yl)amino)pyridin-4-yl)-2-oxopyrrolidine-3-carbonitrile | 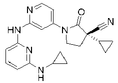 | free | 375.1 | 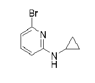 |
| 413 | (3S)-1(2-((6-tert-butylpyridin-2-yl)amino)pyridin-4-yl)-3-cyclopropyl-2-oxopyrrolidine-3-carbonitrile | 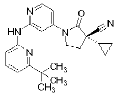 | free | 376.3 | 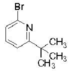 |
| 414 | (3S)-3-cyclopropyl-2-oxo-1-(2-((6-(pyrrolidin-1-yl)pyridin-2-yl)amino)pyridin-4-yl)pyrrolidine-3-carbonitrile | 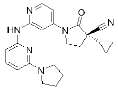 | free | 389.2 | 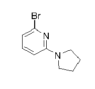 |
| 415 | (3S)-3-cyclopropyl-2-oxo-1-(2-((6-(1H-pyrazol-1-yl)pyridin-2-yl)amino)pyridin-4-yl)pyrrolidine-3-carbonitrile | 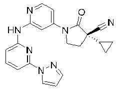 | free | 386.1 | 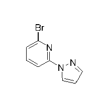 |
| 416 | (3S)-3-cyclopropyl-1-(2-((5-(difluoromethyl)pyridin-2-yl)amino)pyridin-4-yl)-2-oxopyrrolidine-3-carbonitrile | 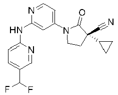 | free | 370.1 | 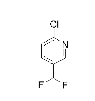 |
| 417 | (3S)-1-(2-((6-chloro-5-(morpholin-4-yl)pyridin-2-yl)amino)pyridin-4-yl)-3-cyclopropyl-2-oxopyrrolidine-3-carbonitrile | 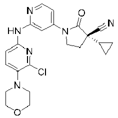 | free | 439.0 | 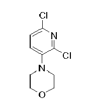 |

### Examples 418 to 421

The title compounds of Examples 418 to 421 shown in Table 52 were obtained using (3S)-1-(2-bromopyridin-4-yl)-3-cyclopropyl-2-oxopyrrolidine-3-carbonitrile obtained in Step E of Example 1 and the reagents corresponding to the compounds of Examples 418 to 421 (these reagents can be produced according to a method known per se), in the same manner as in the method of Example 179 or a method analogous thereto. MS in the tables means actual measured value.

**Table 52**

| Ex. No. | IUPAC Name | Structure | Salt | MS | Corresponding Reagent |
|---|---|---|---|---|---|
| 418 | (3S)-3-cyclopropyl-1-(2-((5-((3R)-3-(dimethylamino)pyrrolidin-1-yl)-4-methylpyridin-2-yl)amino)pyridin-4-yl)-2-oxopyrrolidine-3-carbonitrile | 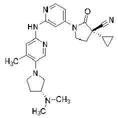 | HCl | 446.2 | 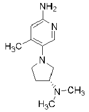 |
| 419 | (3S)-3-cyclopropyl-1-(2-((5-((3S)-3-(dimethylamino)pyrrolidin-1-yl)-4-methylpyridin-2-yl)amino)pyridin-4-yl)-2-oxopyrrolidine-3-carbonitrile | 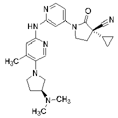 | free | 446.2 | 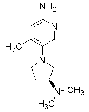 |
| 420 | (3S)-3-cyclopropyl-1-(2-((5-(-2,6-meso-dimethylmorpholin-4-yl)pyridin-2-yl)amino)pyridin-4-yl)-2-oxopyrrolidine-3-carbonitrile | 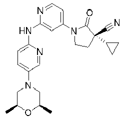 | free | 433.0 | 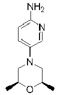 |
| 421 | (3S)-3-cyclopropyl-1-(2-((4-methyl-5-(morpholin-4-yl)pyridin-2-yl)amino)pyridin-4-yl)-2-oxopyrrolidine-3-carbonitrile | 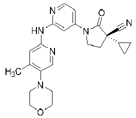 | free | 419.1 | 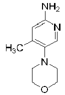 |

### Example 422

### 6-((6-amino-4-((3S)-3-cyano-3-cyclopropyl-2-oxopyrrolidin-1-yl)pyridin-2-yl)amino)-N,N-dimethylnicotinamide

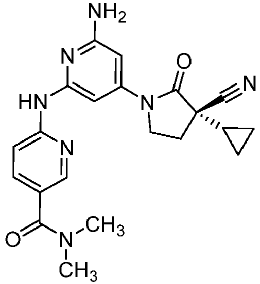

### A) (3S)-3-cyclopropyl-1-(2,6-diaminopyridin-4-yl)-2-oxopyrrolidine-3-carbonitrile

A mixture of (3S)-cyclopropyl-2-oxopyrrolidine-3-carbonitrile obtained in Step D of Example 1 (400 mg), 4-bromopyridine-2,6-diamine (460 mg), N,N'-dimethylethane-1,2-diamine (0.11 mL), potassium carbonate (670 mg) and copper(I) iodide (180 mg) in 1,2-dimethoxyethane (20 mL) was stirred at 130°C for 1 hr in a microwave reactor. The insoluble substance was removed through Celite, and the solvent was evaporated under reduced pressure. The residue was purified by silica gel column chromatography (NH, hexane/ethyl acetate) to give the title compound (370 mg).
MS (ESI+) : [M+H]⁺ 257.9

### B) 6-((6-amino-4-((3S)-3-cyano-3-cyclopropyl-2-oxopyrrolidin-1-yl)pyridin-2-yl)amino)-N,N-dimethylnicotinamide

The title compound (10 mg) was obtained using (3S)-3-cyclopropyl-1-(2,6-diaminopyridin-4-yl)-2-oxopyrrolidine-3-carbonitrile obtained in Step A of Example 422 (170 mg) and 6-bromo-N,N-dimethylnicotinamide (160 mg), in the same manner as in the method of Example 3.
MS (ESI+) : [M+H]⁺ 406.2

### Example 423

### 6-((4-((3S)-3-cyano-3-cyclopropyl-2-oxopyrrolidin-1-yl)-5-fluoropyridin-2-yl)amino)-N,N-dimethylnicotinamide

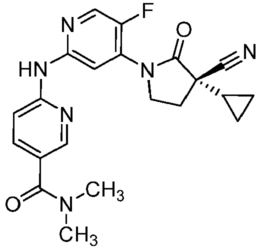

### A) (3S)-1-(2-amino-5-fluoropyridin-4-yl)-3-cyclopropyl-2-oxopyrrolidine-3-carbonitrile

The title compound (240 mg) was obtained using (3S)-cyclopropyl-2-oxopyrrolidine-3-carbonitrile obtained in Step D of Example 1 (400 mg) and 5-fluoro-4-iodopyridin-2-amine (580 mg), in the same manner as in the method of Step A of Example 422.
MS (ESI+) : [M+H]⁺ 260.8

### B) 6-((4-((3S)-3-cyano-3-cyclopropyl-2-oxopyrrolidin-1-yl)-5-fluoropyridin-2-yl)amino)-N,N-dimethylnicotinamide

The title compound (180 mg) was obtained using (3S)-1-(2-amino-5-fluoropyridin-4-yl)-3-cyclopropyl-2-oxopyrrolidine-3-carbonitrile obtained in Step A of Example 423 (200 mg) and 6-bromo-N,N-dimethylnicotinamide (180 mg), in the same manner as in the method of Example 3.
MS (ESI+) : [M+H]⁺ 409.1

### Example 424

### 6-((4-((3S)-3-cyano-3-cyclopropyl-2-oxopyrrolidin-1-yl)-5-methylpyridin-2-yl)amino)-N,N-dimethylnicotinamide

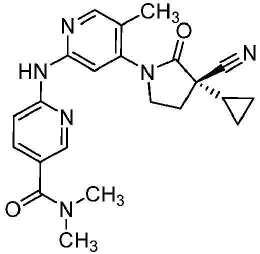

### A) (3S)-1-(2-amino-5-methylpyridin-4-yl)-3-cyclopropyl-2-oxopyrrolidine-3-carbonitrile

The title compound (200 mg) was obtained using (3S)-cyclopropyl-2-oxopyrrolidine-3-carbonitrile obtained in Step D of Example 1 (250 mg) and 4-iodo-5-methylpyridin-2-amine (300 mg), in the same manner as in the method of Step A of Example 422.
MS(ESI+): [M+H]⁺ 256.9

### B) 6-((4-((3S)-3-cyano-3-cyclopropyl-2-oxopyrrolidin-1-yl)-5-methylpyridin-2-yl)amino)-N,N-dimethylnicotinamide

The title compound (180 mg) was obtained using (3S)-1-(2-amino-5-methylpyridin-4-yl)-3-cyclopropyl-2-oxopyrrolidine-3-carbonitrile obtained in Step A of Example 424 (200 mg) and 6-bromo-N,N-dimethylnicotinamide (180 mg), in the same manner as in the method of Example 3.
MS (ESI+) : [M+H]⁺ 405.1

### Example 425

### 6-((5-chloro-4-((3S)-3-cyano-3-cyclopropyl-2-oxopyrrolidin-1-yl)pyridin-2-yl)amino)-N,N-dimethylnicotinamide

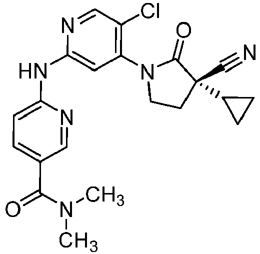

### A) (3S)-1-(2-amino-5-chloropyridin-4-yl)-3-cyclopropyl-2-oxopyrrolidine-3-carbonitrile

The title compound (100 mg) was obtained using (3S)-cyclopropyl-2-oxopyrrolidine-3-carbonitrile obtained in Step D of Example 1 (260 mg) and 5-chloro-4-iodopyridin-2-amine (400 mg), in the same manner as in the method of Step A of Example 422.
MS (ESI+) : [M+H]⁺ 276.8

### B) 6-((5-chloro-4-((3S)-3-cyano-3-cyclopropyl-2-oxopyrrolidin-1-yl)pyridin-2-yl)amino)-N,N-dimethylnicotinamide

The title compound (39 mg) was obtained using (3S)-1-(2-amino-5-chloropyridin-4-yl)-3-cyclopropyl-2-oxopyrrolidine-3-carbonitrile obtained in Step A of Example 425 (100 mg) and 6-bromo-N,N-dimethylnicotinamide (90 mg), in the same manner as in the method of Example 3.
MS (ESI+) : [M+H]⁺ + 425.

### Example 426

### tert-butyl ((1S,2S)-2-((6-((4-((3S)-3-cyano-3-cyclopropyl-2-oxopyrrolidin-1-yl)pyridin-2-yl)amino)pyridin-3-yl)amino)cyclohexyl)carbamate

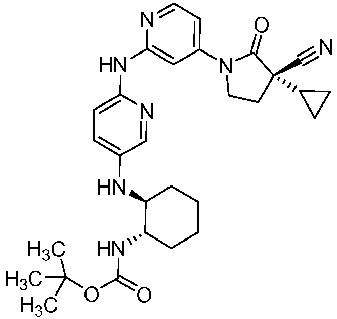

The title compound (75 mg) was obtained using (3S)-1-(2-bromopyridin-4-yl)-3-cyclopropyl-2-oxopyrrolidine-3-carbonitrile obtained in Step E of Example 1 (130 mg) and tert-butyl ((1S,2S)-2-((6-aminopyridin-3-yl))amino)cyclohexyl)carbamate (140 mg), in the same manner as in the method of Example 179.
MS (ESI+) : [M+H]⁺ 532.3

### Example 427

### (3S)-1-(2-((5-(((1S,2S)-2-aminocyclohexyl)amino)pyridin-2-yl)amino)pyridin-4-yl)-3-cyclopropyl-2-oxopyrrolidine-3-carbonitrile hydrochloride

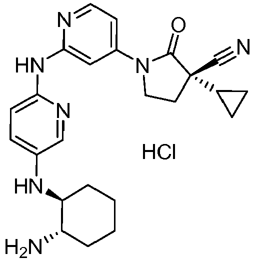

To a solution of tert-butyl ((1S,2S)-2-((6-((4-((3S)-3-cyano-3-cyclopropyl-2-oxopyrrolidin-1-yl)pyridin-2-yl)amino)pyridin-3-yl)amino)cyclohexyl)carbamate obtained in Example 426 (70 mg) in methanol (3.0 mL) was added 4 M hydrogen chloride ethyl acetate (3.0 mL) solution, and the mixture was stirred at room temperature for 3 hr. The solvent was evaporated under reduced pressure, and the residue was recrystallized (hexane/ethyl acetate) to give the title compound (48 mg).
MS (ESI+) : [M+H]⁺ 432.2

### Example 428

### (3S)-3-cyclopropyl-1-(2-((5-(methyl((1S,2S)-2-(methylamino)cyclohexyl)amino)pyridin-2-yl)amino)pyridin-4-yl)-2-oxopyrrolidine-3-carbonitrile

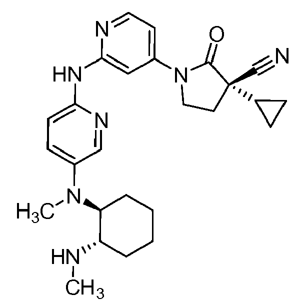

The title compound (30 mg) was obtained using (3S)-1-(2-bromopyridin-4-yl)-3-cyclopropyl-2-oxopyrrolidine-3-carbonitrile obtained in Step E of Example 1 (150 mg) and N5-methyl-N5-((1S,2S)-2-(methylamino)cyclohexyl)pyridine-2,5-diamine (140 mg), in the same manner as in the method of Example 179.
MS (ESI+) : [M+H]⁺ 460.3

### Reference Example 107

### 5-(1-(2,2,2-trifluoroethyl)piperidin-4-yl)pyridin-2-amine

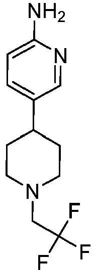

### A) 6-nitro-1',2',3',6'-tetrahydro-3,4'-bipyridine hydrochloride

To a mixture of 5-bromo-2-nitropyridine (800 mg), tert-butyl 4-(4,4,5,5-tetramethyl-1,3,2-dioxaborolan-2-yl)-5,6-dihydropyridine-1(2H)-carboxylate (1.2 g) and sodium carbonate (840 mg) in 1,2-dimethoxyethane/water (v/v = 5/1, 12 mL) was added 1,1'-bis(diphenylphosphino)ferrocene-palladium(II) dichloride-dichloromethane complex (160 mg), and the mixture was stirred in a microwave reactor at 130°C for 1 hr. To the reaction mixture was added water, and the mixture was extracted with ethyl acetate. The obtained organic layer was washed with saturated brine, and dried over magnesium sulfate, and the solvent was evaporated under reduced pressure. The residue was purified by silica gel column chromatography (hexane/ethyl acetate) to give tert-butyl 6-nitro-3',6'-dihydro-3,4'-bipyridine-1'(2'H)-carboxylate.

To a solution of the obtained tert-butyl 6-nitro-3',6'-dihydro-3,4'-bipyridine-1'(2'H)-carboxylate in methanol (10 mL) was added 4M hydrogen chloride ethyl acetate (10 mL) solution, and the mixture was stirred overnight at room temperature. The solvent was evaporated under reduced pressure to give the title compound (620 mg).
MS (ESI+) : [M+H]⁺ 206.6

### B) 6-nitro-1'-(2,2,2-trifluoroethyl)-1',2',3',6'-tetrahydro-3,4'-bipyridine

To a solution of 6-nitro-1',2',3',6'-tetrahydro-3,4'-bipyridine hydrochloride obtained in Step A of Reference Example 107 (300 mg) in N,N-dimethylformamide (10 mL) were added cesium carbonate (1.2 g) and 2,2,2-trifluoroethyl trifluoromethanesulfonate (0.22 mL), and the mixture was stirred overnight at room temperature. To the reaction mixture was added water, and the mixture was extracted with ethyl acetate. The obtained organic layer was washed successively with water and saturated brine, and dried over magnesium sulfate, and the solvent was evaporated under reduced pressure. The residue was purified by silica gel column chromatography (hexane/ethyl acetate) to give the title compound (120 mg). MS(ESI+): [M+H]⁺ 287.8

### C) 5-(1-(2,2,2-trifluoroethyl)piperidin-4-yl)pyridin-2-amine

A mixture of 6-nitro-1'-(2,2,2-trifluoroethyl)-1',2',3',6'-tetrahydro-3,4'-bipyridine obtained in Step B of Reference Example 107 (120 mg) and palladium-carbon (21 mg) in methanol/tetrahydrofuran (v/v = 1/1, 10 mL) was stirred at room temperature for 3 hr under hydrogen atmosphere. The palladium-carbon was removed through Celite, and the solvent was evaporated under reduced pressure to give the title compound (94 mg).
MS(ESI+): [M+H]⁺ 259.8

### Reference Example 108

### 5-(1-methylpiperidin-4-yl)pyridin-2-amine

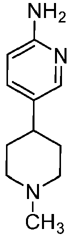

### A) 1'-methyl-6-nitro-1',2',3',6'-tetrahydro-3,4'-bipyridine

To a solution of 6-nitro-1',2',3',6'-tetrahydro-3,4'-bipyridine hydrochloride obtained in Step A of Reference Example 107 (200 mg) and iodomethane (0.10 mL) in N,N-dimethylformamide (10 mL) was added sodium hydride (60% in mineral oil, 73 mg), and the mixture was stirred overnight at room temperature. To the reaction mixture was added water, and the mixture was extracted with ethyl acetate. The obtained organic layer was washed successively with water and saturated brine, and dried over magnesium sulfate, and the solvent was evaporated under reduced pressure to give the crude title compound (150 mg).
MS (ESI+) : [M+H] ⁺ 219.

### B) 5-(1-methylpiperidin-4-yl)pyridin-2-amine

The crude title compound (140 mg) was obtained using the crude 1'-methyl-6-nitro-1',2',3',6'-tetrahydro-3,4'-bipyridine obtained in Step A of Reference Example 108 (150 mg), in the same manner as in the method of Step C of Reference Example 107.
MS (ESI+) : [M+H]⁺ 191.8

### Reference Example 109

### 5-(1-(pyrimidin-2-yl)piperidin-4-yl)pyridin-2-amine

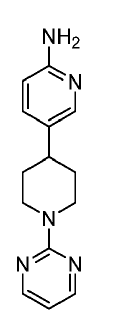

### A) 6-nitro-1'-(pyrimidin-2-yl)-1',2',3',6'-tetrahydro-3,4'-bipyridine

To a solution of 6-nitro-1',2',3',6'-tetrahydro-3,4'-bipyridine hydrochloride obtained in Step A of Reference Example 107 (200 mg) and 2-chloropyrimidine (95 mg) in N,N-dimethylformamide (2.0 mL) was added potassium carbonate (230 mg), and the mixture was stirred in a microwave reactor at 130°C for 30 min, and then overnight at 60°C. To the reaction mixture was added water, and the mixture was extracted with ethyl acetate. The obtained organic layer was washed successively with water and saturated brine, and dried over magnesium sulfate, and the solvent was evaporated under reduced pressure. The residue was purified by silica gel column chromatography (hexane/ethyl acetate) to give the title compound (60 mg).
MS (ESI+) : [M+H]⁺ 283.8

### B) 5-(1-(pyrimidin-2-yl)piperidin-4-yl)pyridin-2-amine

The crude title compound (56 mg) was obtained using 6-nitro-1'-(pyrimidin-2-yl)-1',2',3',6'-tetrahydro-3,4'-bipyridine obtained in Step A of Reference Example 109 (60 mg), in the same manner as in the method of Step C of Reference Example 107.
MS (ESI+) : [M+H]⁺ 255.9

### Reference Example 110

### 5-(1-methyl-1H-pyrazol-5-yl)pyridin-2-amine

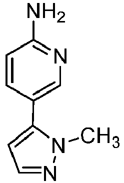

### A) 5-(1-methyl-1H-pyrazol-5-yl)-2-nitropyridine

The title compound (140 mg) was obtained using 5-bromo-2-nitropyridine (300 mg) and 1-methyl-5-(4,4,5,5-tetramethyl-1,3,2-dioxaborolan-2-yl)-1H-pyrazole, in the same manner as in the method of Step A of Reference Example 107.
MS(ESI+): [M+H]⁺ 204.8

### B) 5-(1-methyl-1H-pyrazol-5-yl)pyridin-2-amine

The crude title compound (120 mg) was obtained using 5-(1-methyl-1H-pyrazol-5-yl)-2-nitropyridine obtained in Step A of Reference Example 110 (140 mg), in the same manner as in the method of Step C of Reference Example 107.
MS (ESI+) : [M+H]⁺ 174.8

### Reference Example 111

### 5-(3,5-dimethyl-1,2-oxazol-4-yl)pyridin-2-amine

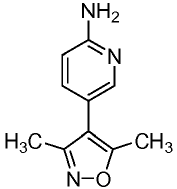

### A) 3,5-dimethyl-4-(6-nitropyridin-3-yl)isoxazole

The title compound (150 mg) was obtained using 5-bromo-2-nitropyridine (200 mg) and 3,5-dimethyl-4-(4,4,5,5-tetramethyl-1,3,2-dioxaborolan-2-yl)isoxazole (220 mg), in the same manner as in the method of Step A of Reference Example 107.
MS (ESI+) : [M+H]⁺ 220.2

### B) 5-(3,5-dimethyl-1,2-oxazol-4-yl)pyridin-2-amine

The crude title compound (110 mg) was obtained using 3,5-dimethyl-4-(6-nitropyridin-3-yl)isoxazole obtained in Step A of Reference Example 111 (150 mg), in the same manner as in the method of Step C of Reference Example 107.
MS (ESI+) : [M+H]⁺ 190.2

### Reference Example 112

### N5-methyl-N5-(tetrahydro-2H-pyran-4-yl)pyridine-2,5-diamine

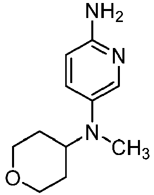

### A) N-methyl-6-nitro-N-(tetrahydro-2H-pyran-4-yl)pyridin-3-amine

To a solution of 5-bromo-2-nitropyridine (500 mg) in dimethyl sulfoxide (10 mL) were successively added potassium carbonate (850 mg), tetrabutylammonium iodide (91 mg) and tetrahydro-2H-pyran-4-amine (370 mg), and the mixture was stirred overnight at 90°C. To the reaction mixture was added water, and the mixture was extracted with ethyl acetate. The obtained organic layer was washed successively with water and saturated brine, and dried over magnesium sulfate, and the solvent was evaporated under reduced pressure.

To a solution of the residue and iodomethane (0.15 mL) in N,N-dimethylformamide (10 mL) was added sodium hydride (60% in mineral oil, 49 mg), and the mixture was stirred overnight at room temperature. To the reaction mixture was added water, and the mixture was extracted with ethyl acetate. The obtained organic layer was washed successively with water and saturated brine, and dried over magnesium sulfate, and the solvent was evaporated under reduced pressure. The residue was purified by silica gel column chromatography (NH, hexane/ethyl acetate) to give the title compound (60 mg).
MS (ESI+) : [M+H]⁺ 237.8

### B) N5-methyl-N5-(tetrahydro-2H-pyran-4-yl)pyridine-2,5-diamine

The crude title compound (56 mg) was obtained using N-methyl-6-nitro-N-(tetrahydro-2H-pyran-4-yl)pyridin-3-amine obtained in Step A of Reference Example 112 (60 mg), in the same manner as in the method of Step C of Reference Example 107.

### MS (ESI+) : [M+H]⁺ 208.2

### Reference Examples 113 to 116

The title compounds of Reference Examples 113 to 116 shown in Table 53 were obtained using 5-bromo-2-nitropyridine and the reagents corresponding to the compounds of Reference Examples 113 to 116 (these reagents can be produced according to a method known per se), in the same manner as in the method of Reference Example 50 or a method analogous thereto. MS in the tables means actual measured value.

**Table 53**

| Ref. No. | IUPAC Name | Structure | MS | Corresponding Reagent |
|---|---|---|---|---|
| 113 | 5-((3S)-3-methoxypiperidin-1-yl)pyridin-2-amine | 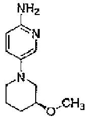 | 207.8 | 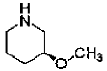 |
| 114 | 5-(2-oxa-6-azaspiro[3,5]non-6-yl)pyridin-2-amine | 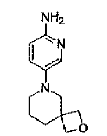 | 219.8 | 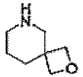 |
| 115 | 5-(2-oxa-7-azaspiro[4,5]dec-7-yl)pyridin-2-amine | 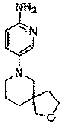 | 233.9 | 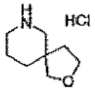 |
| 116 | 5-(2-oxa-7-azaspiro[4,5]dec-7-yl)pyridin-2-amine | 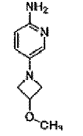 | 179.8 | 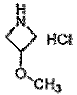 |

### Reference Examples 117 to 120

The title compounds of Reference Examples 117 to 120 shown in Table 54 were obtained using 5-bromo-4-methyl-2-nitropyridine and the reagents corresponding to the compounds of Reference Examples 117 to 120 (these reagents can be produced according to a method known per se), in the same manner as in the method of Reference Example 50 or a method analogous thereto. MS in the tables means actual measured value.

**Table 54**

| Ref. No. | IUPAC Name | Structure | MS | Corresponding Reagent |
|---|---|---|---|---|
| 117 | 4-methyl-5-(4-methyl-1,4-diazocan-l-yl)pyridin-2-amine | 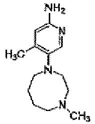 | 250.9 | 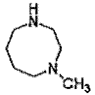 |
| 118 | (R)-5-(3-(dimethylamino)pyrrolidin-1-yl)-4-methylpyridin-2-amine | 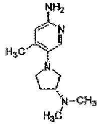 | 221.2 | 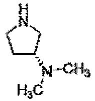 |
| 119 | (S)-5-(3-(dimethylamino)pyrrolidin-1-yl)-4-methylpyridin-2-amine | 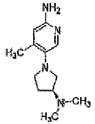 | 221.2 | 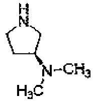 |
| 120 | 4-methyl-5-morpholino pyridin-2-amine | 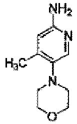 | 193.8 | 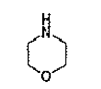 |

### Experimental Example (Tyk2 enzyme inhibition test)

Tyk2 enzyme inhibitory activity of test compounds was measured by LANCE method (PerkinElmer). First, a test compound diluted with assay buffer (50 mM HEPES (pH = 7.5), 10 mM MgCl₂, 1 mM EGTA, 2 mM DTT, 0.01% Tween20, 0.01% BSA) was added to 384-well plate at 2µL each. Then, a Tyk2 (Invitrogen) solution and a fluorescence-labeled peptide substrate (ULight-JAK1, PerkinElmer) solution diluted with assay buffer at 375 ng/mL and 300 nM, respectively were added at 2 µL each. Then, enzyme reaction was started by adding 2 µL each of ATP solution prepared with assay buffer at 30 µM. After the reaction at room temperature for 1 hr, Detection Buffer (PerkinElmer) prepared to be 20 mM EDTA, 4 nM europium-labeled anti-phosphotyrosine antibody (PerkinElmer) was added at 6 µL each. After standing at room temperature for 1 hr, fluorescence intensity (excitation wavelength 340 nm, fluorescence wavelength 665 nm, delay time 100 microsecond) was measured by a plate reader, Envision (PerkinElmer). The inhibitory activity of each compound was calculated as relative value where fluorescence intensity of a well without enzyme is considered as 100% inhibition.

**Table 55**

| Example No. | Tyk2 enzyme inhibitory activity (%, 1 µM) | Example No. | Tyk2 enzyme inhibitory activity (%, 1 µM) |
|---|---|---|---|
| 1 | 99 | 126 | 72 |
| 3 | 99 | 127 | 83 |
| 5 | 101 | 129 | 90 |
| 29 | 100 | 136 | 106 |
| 31 | 100 | 137 | 107 |
| 43 | 95 | 138 | 102 |
| 92 | 96 | | |

**Formulation Example 1 (production of capsule)**

| | |
|---|---|
| 1) compound of Example 1 | 30 mg |
| 2) fine powder cellulose | 10 mg |
| 3) lactose | 19 mg |
| 4) magnesium stearate | 1 mg |
| Total | 60 mg |

1), 2), 3) and 4) are mixed and filled in a gelatin capsule.

**Formulation Example 2 (production of tablet)**

| | |
|---|---|
| 1) compound of Example 1 | 30 g |
| 2) lactose | 50 g |
| 3) cornstarch | 15 g |
| 4) calcium carboxymethylcellulose | 44 g |
| 5) magnesium stearate | 1 g |
| 1000 tablets total | 140 g |

The total amount of 1), 2) and 3) and 4) (30 g) is kneaded with water, and the mixture is vacuum-dried, and sieved. The sieved powder is mixed with 4) (14 g) and 5) (1 g), and the mixture is punched by a tableting machine, whereby 1000 tablets containing 30 mg of the compound of Example 1 per tablet are obtained.

**Formulation Example 3 (production of ointment)**

| | |
|---|---|
| 1) compound of Example 1 | 0.5 g |
| 2) liquid paraffin | 1 g |
| 3) white vaseline | 98.5 g |
| Total | 100 g |

1) and 2) are mixed well in a mortar, and 3) is added gradually thereto with kneading to make the total weight 100 g. The obtained kneaded product is filled into tubes in parts to give an ointment.

### Industrial Applicability

The compound of the present invention has a superior Tyk2 inhibitory action, which is useful as an agent for the prophylaxis or treatment of autoimmune diseases (e.g., psoriasis, rheumatoid arthritis, inflammatory bowel disease, Sjogren's syndrome, Behcet's disease, multiple sclerosis, systemic lupus erythematosus etc.) and the like.

This application is based on patent application Nos. 2013-158306 and 2013-202738 filed in Japan, the contents of which are encompassed in full herein.

## Claims

1. A compound represented by the formula (I): 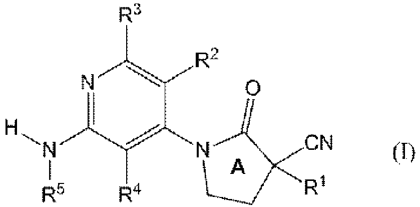 wherein
Ring A is an optionally further substituted pyrrolidine ring; R¹, R² and R⁴ are independently a hydrogen atom or a substituent;
R³ is a hydrogen atom, a halogen atom, an optionally halogenated C₁₋₆ alkyl group, a mono- or di-(optionally halogenated C₁₋₆ alkyl)amino group or an amino group; and
R⁵ is a hydrogen atom, an optionally substituted C₁₋₆ alkyl group, an optionally substituted C₆₋₁₄ aryl group, an optionally substituted aromatic heterocyclic group or an acyl group,
or a salt thereof.

2. The compound or salt of claim 1, wherein R³ is a hydrogen atom or an amino group.

3. The compound or salt of claim 1, wherein
R¹ is an optionally substituted C₁₋₆ alkyl group or an optionally substituted C₃₋₁₀ cycloalkyl group;
R² and R⁴ are independently a hydrogen atom, a halogen atom or an optionally substituted C₁₋₆ alkyl group;
R³ is a hydrogen atom or an amino group; and
R⁵ is
(1) a hydrogen atom,
(2) an optionally substituted C₃₋₁₀ cycloalkyl-carbonyl group,
(3) an optionally substituted C₆₋₁₄ aryl group,
(4) an optionally substituted 5- to 14-membered aromatic heterocyclic group,
(5) an optionally substituted C₁₋₆ alkyl-carbonyl group,
(6) an optionally substituted C₆₋₁₄ aryl-carbonyl group,
(7) an optionally substituted 5- or 6-membered aromatic heterocyclylcarbonyl group,
(8) an optionally substituted C₁₋₆ alkyl-carbamoyl group,
(9) an optionally substituted C₆₋₁₄ aryl-carbamoyl group,
(10) an optionally substituted C₃₋₁₀ cycloalkyl-carbamoyl group, or
(11) an optionally substituted 3- to 8-membered monocyclic non-aromatic heterocyclylcarbamoyl group.

4. (3S)-3-Cyclopropyl-l-(2-((5-(morpholin-4-yl)pyridin-2-yl)amino)pyridin-4-yl)-2-oxopyrrolidine-3-carbonitrile or a salt thereof.

5. (3S)-3-Cyclopropyl-1-(2-((5-(2-hydroxypropan-2-yl)pyridin-2-yl)amino)pyridin-4-yl)-2-oxopyrrolidine-3-carbonitrile or a salt thereof.

6. 3-((4-((3S)-3-Cyano-3-cyclopropyl-2-oxopyrrolidin-1-yl)pyridin-2-yl)amino)-N,1-dimethyl-1H-pyrazole-5-carboxamide or a salt thereof.

7. A medicament comprising the compound or salt of claim 1.

8. The medicament of claim 7, which is a tyrosine kinase 2 inhibitor.

9. The medicament of claim 7, which is an agent for the prophylaxis or treatment of autoimmune diseases.

10. The medicament of claim 9, wherein the autoimmune disease is psoriasis, rheumatoid arthritis, inflammatory bowel disease, Sjogren's syndrome, Behcet's disease, multiple sclerosis or systemic lupus erythematosus.

11. The compound or salt of claim 1 for use in the prophylaxis or treatment of autoimmune diseases.

12. The compound or salt of claim 11, wherein the autoimmune disease is psoriasis, rheumatoid arthritis, inflammatory bowel disease, Sjogren's syndrome, Behcet's disease, multiple sclerosis or systemic lupus erythematosus.

13. A method of inhibiting tyrosine kinase 2 in a mammal, which comprises administering an effective amount of the compound or salt of claim 1 to the mammal.

14. A method for the prophylaxis or treatment of autoimmune diseases in a mammal, which comprises administering an effective amount of the compound or salt of claim 1 to the mammal.

15. The method of claim 14, wherein the autoimmune disease is psoriasis, rheumatoid arthritis, inflammatory bowel disease, Sjogren's syndrome, Behcet's disease, multiple sclerosis or systemic lupus erythematosus.

16. Use of the compound or salt of claim 1 for the production of an agent for the prophylaxis or treatment of autoimmune diseases.

17. The use of claim 16, wherein the autoimmune disease is psoriasis, rheumatoid arthritis, inflammatory bowel disease, Sjogren's syndrome, Behcet's disease, multiple sclerosis or systemic lupus erythematosus.
